# EUROPÄISCHE PATENTSCHRIFT

(11) **EP 3 375 774 B1**
(45) Veröffentlichungstag und Bekanntmachung des Hinweises auf die Patenterteilung: **13.11.2024**
(21) Anmeldenummer: 17161718.6
(22) Anmeldetag: 17.03.2017
(51) Int. Cl.: C07D 213/30, C07C 49/755, C09D 5/14, C08K 5/08, A61L 2/08, C07C 69/54, C07C 69/76, C07C 217/12, C07C 225/18, C07C 271/16, C07C 271/20, C07C 233/31, C07C 233/38, C07D 303/22, C07F 7/04, C07F 7/18, C09D 133/14, C09D 175/04, C09D 183/10

(54) **PHENALEN-1-ON-HALTIGE PHOTOSENSIBILISATORZUSAMMENSETZUNG, PHENALEN-1-ON-VERBINDUNGEN SOWIE DEREN VERWENDUNG**
PHENALEN-1-ONE-CONTAINING PHOTOSENSITIZER COMPOSITION, PHENALEN-1-ONE COMPOUNDS AND THEIR USE
COMPOSITION PHOTOSENSIBILISANTE CONTENANT DU PHÉNALÉN-1-ONE, COMPOSÉS DE PHÉNALÉN-1-ONE ET LEUR UTILISATION

(43) Veröffentlichungstag der Anmeldung: 19.09.2018
(73) Patentinhaber: Jufico GmbH, 82152 Krailling (DE)
(72) Erfinder: SPÄTH, Andreas, 93055 Regensburg (DE); EICHNER, Anja, 93053 Regensburg (DE)
(74) Vertreter: Louis Pöhlau Lohrentz

(56) Entgegenhaltungen:
- EP-A1- 0 069 292
- EP-A2- 0 104 388
- WO-A1-00/78854
- WO-A2-2012/113860
- DATABASE CA [online] CHEMICAL ABSTRACTS SERVICE, COLUMBUS, OHIO, US; 1966, MEIROVICS, I. ET AL: "Enol ethers of 2-arylperinaphthindan-l,3-diones", XP002770280, retrieved from STN Database accession no. 1966:447525
- MEIROVICS, I. ET AL: "Enol ethers of 2-arylperinaphthindan-l,3-diones", LATVIJAS PSR ZINATNU AKADEMIJAS VESTIS, KIMIJAS SERIJA, CODEN: LZAKAM; ISSN: 0002-3248, no. 5, 1965, pages 579 - 586
- DAVID A FROST ET AL: "Naturally Occurring Compounds related t o Phenalenone. Part V.l Syn- thetic Approaches t o Structures Based on 8,9-Dihydro-8,8,9-trimethyl- phenaleno[l,2-b]f uran-7-one", JOURNAL OF THE CHEMICAL SOCIETY, PERKIN TRANSACTIONS 1, 1 January 1973 (1973-01-01), pages 2159 - 2169, XP055371432, DOI: 10.1039/P19730002159

## Beschreibung

Die vorliegende Erfindung betrifft eine Photosensibilisatorzusammensetzung, eine Phenalen-1-on-Verbindung, einen Gegenstand sowie deren nicht-medizinische Verwendung.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen.

Daher können gesunde, körpereigene Zellen eine Schädigung durch reaktive Sauerstoffspezies (ROS), beispielsweise Radikale oder Singulett-Sauerstoff, entgegen wirken.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen.

Die WO 00/78854 A1 betrifft ein Verfahren zum Bereitstellen einer antimikrobiellen Oberfläche, wobei das Verfahren das Kombinieren von einem oder mehreren Polymeren mit einem oder mehreren Photosensibilisatoren umfasst, um eine Oberfläche zu bilden, die eine gehärtete Polymerzusammensetzung aufweist, die ein oder mehrere Polymere und einen oder mehrere nicht-kovalent und nicht-ionisch gebundene Photosensibilisatoren aufweist, wobei mindestens einer davon ein Xanthen-Photosensibilisator ist.

Nachteilig dabei ist, dass der Photosensibilisator aus der Polymermatrix ausläuft und somit die antimikrobielle Wirksamkeit bei längerer Lagerung abnimmt.

Die US 5,830,526 A offenbart ein Substrat, an das ein lichtaktivierbarer Farbstoff allein oder in Kombination mit zusätzlichen herkömmlichen antimikrobiellen und/oder antiviralen Mitteln gebunden ist. Das Substrat wird mit einem lichtaktivierbaren Farbstoff mit antimikrobiellen und/oder antiviralen Eigenschaften imprägniert, wobei ein kationisches oder anionisches wasserlösliches Polymer den Farbstoff an das Substrat bindet.

Nachteilig dabei ist, dass der Photosensibilisator beispielsweise durch mechanische Beanspruchung von dem Substrat gelöst werden kann und somit die antimikrobielle Wirksamkeit reduziert wird.

Die WO 2012/113860 A2 ist auf die Bereitstellung von Phenalen-1-on-Derivaten, Verfahren zur Herstellung und Verwendung derselben gerichtet.

Aufgabe der vorliegenden Erfindung ist es, eine Photosensibilisator-haltige Beschichtung bereitzustellen, die eine einfache Auftragbarkeit auf vorzugsweise verschiedene Arten von Oberflächen gewährleistet und insbesondere zugleich eine gute antimikrobielle Wirksamkeit nach Belichtung mit elektromagnetischer Strahlung geeigneter Wellenlänge und Intensität zeigt.

Weiterhin soll die Photosensibilisator-haltige Beschichtung nach dem Auftragen auf eine Oberfläche, vorzugsweise eine verbesserte Adhäsion des Photosensibilisators gewährleisten, so dass ein Ausbluten des Photosensibilisators vorzugsweise vermieden wird.

Weiterhin soll vorzugsweise die Wirksamkeit des Photosensibilisators, insbesondere bei längerer Bestrahlung mit elektromagnetischer Strahlung geeigneter Wellenlänge und Intensität, verbessert werden.

Dabei soll die Photosensibilisator-haltige Beschichtung im Wesentlichen nicht die Anregung der in der Beschichtung enthaltenen Photosensibilisator-Moleküle durch Licht einer bestimmten Wellenlänge behindern.

Die Aufgabe der vorliegenden Erfindung wird gelöst durch die Bereitstellung Photosensibilisatorzusammensetzung nach Anspruch 1.

Bevorzugte Ausführungsformen der erfindungsgemäßen Photosensibilisator-zusammensetzung sind in den Ansprüchen 2 bis 3 und 6 bis 11 angegeben.

Die Aufgabe der vorliegenden Erfindung wird ebenfalls gelöst durch Bereitstellung einer Phenalen-1-on-Verbindung nach Anspruch 4.

Bevorzugte Ausführungsformen der erfindungsgemäßen Phenalen-1-on-Verbindung sind in Anspruch 5 angegeben.

Die Aufgabe der vorliegenden Erfindung wird weiterhin gelöst durch Bereitstellung eines Gegenstands nach Anspruch 12.

Bevorzugte Ausführungsformen des erfindungsgemäßen Gegenstands sind in den Ansprüchen 13 bis 14 angegeben.

Die Aufgabe der vorliegenden Erfindung wird ebenfalls gelöst durch Bereitstellung einer nicht-medizinischen Verwendung gemäß Anspruch 15, wobei eine Photosensibilisatorzusammensetzung nach einem der Ansprüche 1 bis 3 oder 6 bis 11 und/oder eine Phenalen-1-on-Verbindung nach einem der Ansprüche 4 bis 5 und/oder ein Gegenstand nach einem der Ansprüche 12 bis 14 zur, vorzugsweise photodynamischen, Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden, und/oder einem Biofilm davon verwendet wird.

Bevorzugte Ausführungsformen der erfindungsgemäßen nicht-medizinischen Verwendung sind in den Ansprüchen 16 bis 17 angegeben.

Unter dem Begriff "Biofilm" wird vorzugsweise eine Matrix aus extrazellulärer polymerer Substanzen (EPS), die vorzugsweise von Mikroorganismen gebildet und von ihnen in ihre unmittelbare, der Zelle angrenzenden, Umgebung abgegeben werden, verstanden, in der Mikroorganismen, vorzugsweise Bakterien, Algen, Pilze, Protozoen oder Kombinationen davon, zumindest teilweise angeordnet oder eingebettet sind.

Die Aufgabe der vorliegenden Erfindung wird ebenfalls gelöst durch Bereitstellung eines nicht-medizinischen Verfahrens zur, vorzugsweise photodynamischen, Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen und/oder einem Biofilm davon, nach Anspruch 18, wobei das Verfahren folgende Schritte umfasst:
(A) in Kontakt bringen der Mikroorganismen und/oder einem Biofilm davon mit wenigstens einer Beschichtung, die durch Aushärten einer Photosensibilisatorzusammensetzung nach einem der Ansprüche 1 bis 3 oder 6 bis 11 hergestellt wurde und/oder wenigstens eine Phenalen-1-on-Verbindung nach einem der Ansprüche 4 bis 5 enthält, und/oder wenigstens eines Gegenstandes nach einem der Ansprüche 12 bis 14, und
(B) Bestrahlen der Mikroorganismen und/oder einem Biofilm davon und der, in der Beschichtung und/oder dem Gegenstand enthaltenen wenigstens einen Phenalen-1-on-Verbindung mit elektromagnetischer Strahlung geeigneter Wellenlänge und Energiedichte.

Unter dem Begriff "Aushärten" bzw. "Härten" wird vorzugsweise der Übergang von einem flüssigen oder plastisch verformbaren Zustand in einen bei Standardbedingungen (Temperatur: 25°C, Druck: 1013 mbar) festen Zustand eines Stoffes oder Stoffgemisches verstanden.

Der Prozess des Aushärtens bzw. Härtens kann vorzugsweise durch Abkühlen, d.h. durch Reduzieren der Temperatur unter den Gefrierpunkt und/oder Glasübergangstemperatur eines Stoffes oder Stoffgemisches, physikalische Trocknung, d.h. durch Entfernung wenigstens eines flüssigen Bestandteils, beispielsweise Lösungsmittels, und/oder durch chemische Reaktion, beispielsweise durch Kettenpolymerisation, Polyaddition und/oder Polykondensation, erfolgen.

Unter dem Begriff "polymere Komponente und/oder Vorläufer davon" wird vorzugsweise ein Stoff oder Stoffgemisch verstanden, welches wenigstens ein, vorzugsweise organisches, Polymer und/oder wenigstens ein Vorläufer davon aufweist.

Unter dem Begriff "Polymer" wird vorzugsweise ein Stoff verstanden, der aus vorzugsweise wenigstens 10 Struktureinheiten, sogenannten konstitutionellen Repetiereinheiten, aufgebaut ist, die gleich oder voneinander verschieden sein können und durch chemische Reaktion, vorzugsweise Kettenpolymerisation, Polyaddition und/oder Polykondensation, das wenigstens eine organische Polymer bilden. Vorzugsweise ist eine konstitutionelle Repetiereinheit (KRE) die kleinste sich wiederholende Gruppe von Atomen innerhalb eines Polymers.

Ein Polymer im Sinne der Erfindung kann unverzweigt oder verzweigt sein.

Unter dem Begriff "Vorläufer einer polymeren Komponente" werden vorzugsweise Monomere oder Monomergemische sowie Oligomere und Mischungen davon verstanden, die sich jeweils zu dem entsprechenden unverzweigten oder verzweigten Polymer zusammenschließen können, vorzugsweise durch chemische Reaktion, weiter bevorzugt Kettenpolymerisation, Polyaddition und/oder Polykondensation.

Unter dem Begriff "reaktive funktionelle Gruppe" wird vorzugsweise eine funktionelle Gruppe verstanden, die sich durch chemische Reaktion, weiter bevorzugt Kettenpolymerisation, Polyaddition und/oder Polykondensation, an der Bildung des entsprechenden unverzweigten oder verzweigten Polymers beteiligen kann.

Monomere oder Monomergemische im Sinne der Erfindung sind vorzugsweise niedermolekulare, reaktionsfähige Moleküle oder Gemische von reaktionsfähigen Molekülen, die sich jeweils zu dem entsprechenden unverzweigten oder verzweigten Polymer durch chemische Reaktion, weiter bevorzugt Kettenpolymerisation, Polyaddition und/oder Polykondensation, zusammenschließen können und dadurch konstitutionelle Repetiereinheit des Polymers bilden. Vorzugsweise weist ein reaktionsfähiges Molekül, beispielsweise Monomer, Oligomer und optional Polymer wenigstens eine reaktive funktionelle Gruppe auf.

Unter dem Begriff "Oligomer" wird vorzugsweise ein Stoff verstanden, der aus vorzugsweise 2 bis 9 konstitutionellen Repetiereinheit aufgebaut ist, die gleich oder voneinander verschieden sein können und sich vorzugsweise durch chemische Reaktion, weiter bevorzugt Kettenpolymerisation, Polyaddition und/oder Polykondensation, zu einem unverzweigten oder verzweigten Polymer zusammenschließen können.

Unter dem Begriff "ausgehärtete polymere Komponente" wird vorzugsweise ein polymerer Stoff oder ein Gemisch polymerer Stoffe verstanden, der bzw. das bei Standardbedingungen (Temperatur: 25°C, Druck: 1013 mbar) einen festen Zustand aufweist und vorzugsweise nicht plastisch verformbar ist.

Hinsichtlich der Definition der vorgenannten Begriffe wird weiterhin auf Jenkins, A. D. et al.: "Glossary of basic terms in polymer science (IUPAC Recommendations 1996)" (Pure Appl. Chem. 68 (12), 1996, Seiten 2287 bis 2311, doi:10.1351/pac199668122287) verwiesen.

Vorzugsweise bildet eine erfindungsgemäße Photosensibilisatorzusammensetzung nach dem Aushärten eine ausgehärtete Polymerzusammensetzung, die wenigstens eine erfindungsgemäß verwendete Verbindung mit der Formel (1) und/oder wenigstens eine erfindungsgemäße Verbindung mit der Formel (1a) enthält.

Bei einer bevorzugten Ausführungsform wird beim Aushärten einer erfindungsgemäßen Photosensibilisatorzusammensetzung die wenigstens eine erfindungsgemäß verwendete Verbindung mit der Formel (1) und/oder die wenigstens eine erfindungsgemäße Verbindung mit der Formel (1a) kovalent und/oder elektrostatisch, vorzugsweise kovalent, in der ausgehärteten Polymerzusammensetzung gebunden. Weiter bevorzugt wird beim Aushärten einer erfindungsgemäßen Photosensibilisatorzusammensetzung die wenigstens eine erfindungsgemäß verwendete Verbindung mit der Formel (1) und/oder die wenigstens eine erfindungsgemäße Verbindung mit der Formel (1a) kovalent und/oder elektrostatisch, vorzugsweise kovalent, an die wenigstens eine ausgehärtete polymere Komponente gebunden.

Dadurch wird vorzugsweise ein Austreten der wenigstens einen erfindungsgemäß verwendeten Verbindung mit der Formel (1) und/oder der wenigstens einen erfindungsgemäßen Verbindung mit der Formel (1a) aus der ausgehärteten Photosensibilisatorzusammensetzung zumindest teilweise, vorzugsweise vollständig, verhindert.

Bei der erfindungsgemäß verwendeten Verbindung mit der Formel (1) und der erfindungsgemäßen Verbindung mit der Formel (1a) handelt es sich jeweils um ein 1H-Phenalen-1-on-Derivat, das im Folgenden auch so bezeichnet wird.

Vorzugsweise weist eine erfindungsgemäß verwendete Verbindung mit der Formel (1) und eine erfindungsgemäße Verbindung mit der Formel (1a) jeweils kein neutrales, protonierbares Stickstoffatom, beispielsweise als Aminorest, Methylaminorest oder Dimethylaminorest, und kein positiv geladenes, vorzugsweise quartäres, Stickstoffatom, beispielsweise als Pyridin-1-ium-1-yl-Rest oder Trimethylammonio-Rest, sowie kein positiv geladenes, vorzugsweise quartäres, Phosphoratom, das direkt an den Phenalen-Ring gebunden ist, auf.

Unter "direkt" wird dabei verstanden, dass das Stickstoffatom und/oder das Phosphoratom unmittelbar an den Phenalen-Ring gebunden ist.

Die Erfinder haben festgestellt, dass bei einer direkten Anordnung des Stickstoffatoms und/oder Phosphoratoms an den Phenalen-Ring es zu einer signifikanten Absenkung der Singulett-Sauerstoff-Quantenausbeute kommt.

Eine möglichst hohe Singulett-Sauerstoff-Quantenausbeute ist für eine antimikrobielle Wirksamkeit bei der photodynamischen Therapie oder bei der photodynamischen Reinigung bzw. photodynamische Entkeimung von Oberflächen erforderlich. Bei einer direkten Anordnung des Stickstoffatoms und/oder Phosphoratoms am Phenalen-Ring wird die aufgenommene Energie überwiegend durch Fluoreszenzeffekte abgegeben, was zu einer signifikanten Verringerung der Singulett-Sauerstoff-Quantenausbeute führt.

Vorzugsweise weist eine erfindungsgemäß verwendete Verbindung mit der Formel (1) und eine erfindungsgemäße Verbindung mit der Formel (1a) jeweils keine OH-Gruppe und/oder keine deprotonierte OH-Gruppe, die jeweils direkt an den Phenalen-Ring gebunden ist, auf.

Unter "direkt" wird dabei verstanden, dass die OH-Gruppe und/oder deprotonierte OH-Gruppe unmittelbar an den Phenalen-Ring gebunden ist.

Die Erfinder haben festgestellt, dass bei einer direkten Anordnung einer OH-Gruppe und/oder deprotonierte OH-Gruppe an den Phenalen-Ring zu einer signifikanten Verschlechterung der Photostabilität des Photosensibilisators kommt. Eine Verschlechterung der Photostabilität führt zu einem schnelleren Ausbleichen und somit zu einer schnelleren Inaktivierung des Photosensibilisators bei Bestrahlung mit elektromagnetischer Strahlung geeigneter Wellenlänge.

Als "Photosensibilisator" werden vorzugsweise Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Ein Photosensibilisator im Sinne der Erfindung weist vorzugsweise die allgemeine Formel (1) und/oder die allgemeine Formel (1a) auf.

Unter dem Begriff "photodynamische Entkeimung" wird vorzugsweise die lichtinduzierte Inaktivierung von Zellen oder Mikroorganismen und/oder einem Biofilm davon auf Oberflächen von Gegenständen verstanden.

Unter dem Begriff "photodynamische Reinigung" wird vorzugsweise die lichtinduzierte Reduzierung der Anzahl von Zellen oder Mikroorganismen und/oder einem Biofilm davon auf Oberflächen von Gegenständen verstanden.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäßen Verbindung mit der Formel (1a) und/oder wenigstens einer erfindungsgemäß verwendeten Verbindung mit der Formel (1) bestimmt werden.

Vorzugsweise kann eine Reduzierung der Lebensfähigkeit oder die Zerstörung von Mikroorganismen eines Biofilms, vorzugsweise dessen Zerstörung, eine Abgabe von extrazellulären polymeren Substanzen, die die Matrix eines Biofilms bilden, verringern bzw. verhindern. Dadurch wird vorzugsweise die Bildung eines Biofilms verlangsamt oder unterdrückt.

Erfindungsgemäß wird unter einer Reduzierung der Lebensfähigkeit verstanden, dass die Anzahl der Mikroorganismen, beispielsweise in einem Biofilm, um wenigstens 90,0 %, vorzugsweise wenigstens 95,0 %, vorzugsweise wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 % verringert wird.

Vorzugsweise wird die Reduzierung der Anzahl der Mikroorganismen gemäß Boyce, J. M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 - 46) als log10-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäßen Verbindung mit der Formel (1a) und/oder wenigstens einer erfindungsgemäß verwendeten Verbindung mit der Formel (1) verstanden. Beispielsweise können die Mikroorganismen in einem Biofilm enthalten sein.

Geeignete Methoden zur Bestimmung des log₁₀-Reduktionsfaktors sind beispielsweise in der DIN EN 14885:2007-01 "Chemische Desinfektionsmittel und Antiseptika -Anwendung Europäischer Normen für chemische Desinfektionsmittel und Antiseptika" oder in Rabenau, H. F. und Schwebke, I. ("Leitlinie der Deutschen Vereinigung zur Bekämpfung der Viruskrankheiten (DW) e. V. und der Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen und/oder eines Biofilms davon mit elektromagnetischer Strahlung in Gegenwart wenigstens einer erfindungsgemäßen Verbindung mit der Formel (1a) und/oder wenigstens einer erfindungsgemäß verwendeten Verbindung mit der Formel (1) mindestens 1 log₁₀, vorzugsweise mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀. Beispielsweise bedeutet eine Reduktion der Anzahl der Mikroorganismen nach einer Bestrahlung dieser Mikroorganismen und/oder eines Biofilms davon mit elektromagnetischer Strahlung in Gegenwart wenigstens einer erfindungsgemäßen Verbindung mit der Formel (1a) und/oder wenigstens einer erfindungsgemäß verwendeten Verbindung mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen und/oder eines Biofilms davon mit elektromagnetischer Strahlung in Gegenwart wenigstens einer erfindungsgemäßen Verbindung mit der Formel (1a) und/oder wenigstens einer erfindungsgemäß verwendeten Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Erfindungsgemäß wird unter einem Halogen jeweils unabhängig voneinander Fluor, Chlor, Brom oder lod verstanden. Erfindungsgemäß wird unter einem Halogenid jeweils unabhängig voneinander Fluorid, Chlorid, Bromid oder lodid verstanden.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1) und/oder der Formel (1a), sowohl die reinen Verbindungen als auch die Isomerengemische in jedem Verhältnis.

Falls nichts anderes angegeben, bezeichnet ein * in einer Formel einen Verknüpfungspunkt zwischen zwei Resten.

Eine erfindungsgemäße, vorzugsweise härtbare, Photosensibilisatorzusammensetzung umfasst:
(a) wenigstens eine erfindungsgemäß zu verwendende Phenalen-1-on-Verbindung mit der allgemeinen Formel (1): 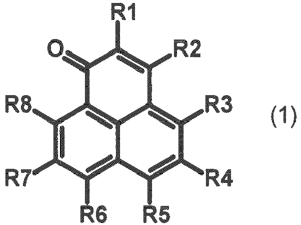 und
(b) wenigstens eine polymere Komponente und/oder Vorläufer davon.

Eine erfindungsgemäß zu verwendende Phenalen-1-on-Verbindung weist die allgemeine Formel (1) auf, wobei die Reste R1 bis R8, die jeweils unabhängig voneinander gleich oder voneinander verschieden sein können, jeweils Wasserstoff, Halogen, Alkyl mit 1 bis 12 C-Atomen, vorzugsweise 2 bis 9 C-Atomen, Alkylaryl mit 5 bis 20 C-Atomen, vorzugsweise 6 bis 9 C-Atomen, Aryl mit 5 bis 20 C-Atomen, vorzugsweise 6 bis 9 C-Atomen, *-O-Alkyl mit 1 bis 12 C-Atomen, vorzugsweise 2 bis 9 C-Atomen, *-O-Alkylaryl mit 5 bis 20 C-Atomen, vorzugsweise 6 bis 9 C-Atomen, *-O-Aryl mit 5 bis 20 C-Atomen, vorzugsweise 6 bis 9 C-Atomen, Ether mit 2 bis 12 C-Atomen, ein Rest der Formel *-O-C(=O)-R^{(Ia)}, ein Rest der Formel *-C(=O)-R^{(Ib)}, oder ein organischer Rest W1, der wenigstens eine reaktive funktionelle Gruppe enthält, bedeuten, mit der Maßgabe, dass wenigstens einer der Reste Rest R1 bis R7, vorzugsweise wenigstens einer der Reste R1, R2, R5 oder R6, weiter bevorzugt wenigstens einer der Reste R1 oder R2, vorzugsweise einer der Reste R1 oder R2, ein organischer Rest W1 ist, wobei der organische Rest W1 jeweils unabhängig voneinander ein Rest mit der allgemeinen Formel (2) bis (6):

*-[(C(D)(E))_{d}-B)ₐ-(C(D)(E))ₘ-X (2)

*-A-[(C(D)(E))_{d}-B]_{c}-(C(D)(E))ₘ-X (3)

*-(C(D)(E))_{d}-Ar-(C(D)(E))ₙ-X (4)

*-[(C(D)(E))_{d}-B]_{b}-(C(D)(E))_{g}-Ar-(C(D)(E))ₙ-X (5)

*-A-[(C(D)(E))_{d}-B]_{f}-(C(D)(E))_{g}-Ar-(C(D)(E))ₙ-X (6)

bedeutet,
wobei der Rest A jeweils unabhängig voneinander Sauerstoff, Schwefel oder ein Rest mit der allgemeinen Formel (10a) bis (11a), vorzugsweise Sauerstoff oder ein Rest mit der allgemeinen Formel (10a) bis (11a), weiter bevorzugt Sauerstoff oder ein Rest mit der allgemeinen Formel (10a), weiter bevorzugt Sauerstoff, bedeutet: 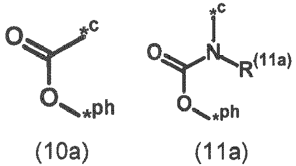
wobei *^{ph} jeweils einen Anknüpfungspunkt des Restes mit der allgemeinen Formel (10a) bis (11a) an ein C-Atom des Phenalen-Rings und *^{c} jeweils einen Anknüpfungspunkt des Restes mit der allgemeinen Formel (10a) bis (11a) an ein C-Atom des Restes (C(D)(E)) bezeichnet,
wobei der Rest B jeweils unabhängig voneinander Sauerstoff, Schwefel oder ein Rest mit der allgemeinen Formel (10) bis (14), vorzugsweise Sauerstoff oder ein Rest mit der allgemeinen Formel (10) bis (14), weiter bevorzugt Sauerstoff oder ein Rest mit der allgemeinen Formel (10), bedeutet: 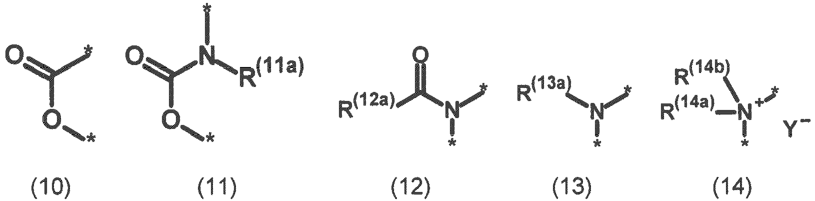
wobei die Reste R^{(Ia)}, R^{(Ib)}, R^{(11a)}, R^{(12a)}, R^{(13a)}, R^{(14a)} und R^{(14b)}, jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Halogen, Amino, Hydroxyl, Alkyl, das gradkettig oder verzweigt sein kann, mit 1 bis 3 Kohlenstoffatomen, Hydroxyalkyl, das gradkettig oder verzweigt sein kann, mit 1 bis 3 Kohlenstoffatomen und 1 bis 3 OH-Gruppen, Halogenalkyl, das gradkettig oder verzweigt sein kann, mit 1 bis 3 Kohlenstoffatomen und 1 bis 3 Halogen-Gruppen, und Kombinationen davon, vorzugsweise Chlor, Brom, Fluor, Methyl, Ethyl, n-Propyl, iso-Propyl, Methoxy, Ethoxy, und Kombinationen davon, besteht, ausgewählt werden, substituiert sein können, wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sulfonat-Anion einer Sulfonsäure mit 1 bis 12 C-Atomen oder eine Kombination davon bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxyalkyl, das gradkettig oder verzweigt sein kann, mit 1 bis 5 Kohlenstoffatomen und 1 bis 5 OH-Gruppen, Phenyl, Benzyl, ein Rest der Formel *-L-R^{(II)}, ein Rest der Formel *-L-C(=L)-R^{(III)}, ein Rest der Formel *-(CH₂)_{q}-X, ein Rest der Formel *-L- (CH₂)_{q}-X, oder ein Rest der Formel *-(CH₂)ₛ-L-(CH₂)ₜ-X bedeuten, wobei der Rest L jeweils unabhängig voneinander Sauerstoff oder Schwefel, vorzugsweise Sauerstoff, bedeutet, wobei die Reste R^{(II)} und R^{(III)} jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, die Indices q, s und t jeweils unabhängig voneinander eine ganze Zahl von 1 bis 5 bedeuten,
wobei die Indices a, c, f, g und n jeweils unabhängig voneinander eine ganze Zahl von 0 bis 5, vorzugsweise von 1 bis 4, bedeuten,
und wobei die Indices b, d und m jeweils unabhängig voneinander eine ganze Zahl von 1 bis 5, vorzugsweise von 2 bis bis 4, bedeuten,
wobei der Rest Ar jeweils unabhängig voneinander ein Rest der allgemeinen Formel (25a) bis (31) bedeutet, und
wobei der Rest X jeweils unabhängig voneinander eine reaktive funktionelle Gruppe ist, die *-N(R^{(VI)})(R^{(VII)}), *-OH, *-SH, *-NCO, *-NCS, *-Si(R^{(VIII)})(R^{(IX)})-[O-Si(R^{(X)})(R^{(XI)})]ₚ-Z oder ein Rest mit der allgemeinen Formel (20) bis (24) bedeutet: 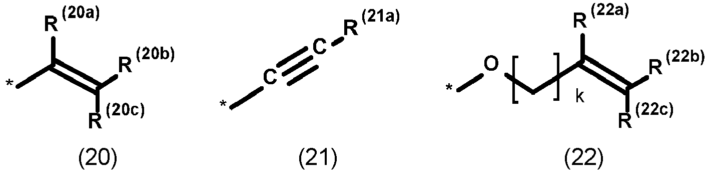 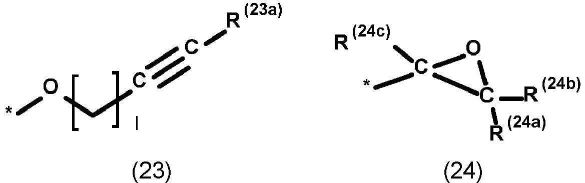
wobei die Reste R^{(20a)}, R^{(20b)}, R^{(20c)}, R^{(21a)}, R^{(22a)}, R^{(22b)}, R^{(22c)}, R^{(23a)}, R^{(24a)}, R^{(24b)}, und R^{(24c)} jeweils unabhängig voneinander Wasserstoff, Methyl, Ethyl, n-Propyl, iso-Propyl, n-Butyl, sec-Butyl, iso-Butyl, oder n-Pentyl, vorzugsweise Wasserstoff, Methyl oder Ethyl, bedeuten, und wobei die Indices I und k jeweils unabhängig voneinander eine ganze Zahl von 0 bis 4 bedeuten, wobei die Reste R^{(VI)}, R^{(VII)}, R^{(VIII)}, R^{(IX)}, R^{(X)} und R^{(XI)} jeweils unabhängig voneinander Wasserstoff, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, wobei der Rest Z jeweils unabhängig voneinander Halogen, Hydroxyl, Alkoxyl mit 1 bis 4 Kohlenstoffatomen oder Alkylcarboxyl mit 1 bis 4 Kohlenstoffatomen bedeutet, und wobei der Index p jeweils unabhängig voneinander eine ganze Zahl von 0 bis 4 bedeutet.

Erfindungsgemäß bedeutet wenigstens einer der Reste R1 bis R7, weiter bevorzugt wenigstens einer der Reste R1, R2, R5 oder R6, weiter bevorzugt wenigstens einer der Reste R1 oder R2, vorzugsweise einer der Reste R1 oder R2, der wenigstens einen erfindungsgemäß zu verwendenden Phenalen-1-on-Verbindung mit der allgemeinen Formel (1) jeweils unabhängig voneinander ein organischer Rest W1, wobei der wenigstens eine organische Rest W1 jeweils unabhängig voneinander ein Rest mit der allgemeinen Formel (2) bis (6) bedeutet und wobei der Rest Ar jeweils unabhängig voneinander ein Rest der allgemeinen Formel (25a) bis (31), weiter bevorzugt ein Rest der allgemeinen Formel (25a) bis (28b), bedeutet: 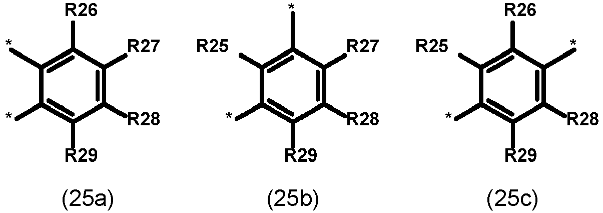 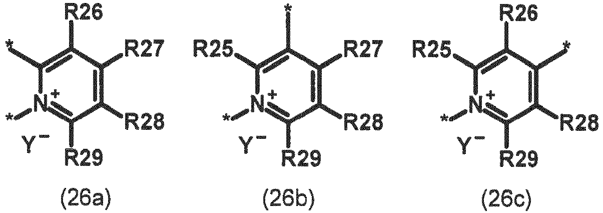 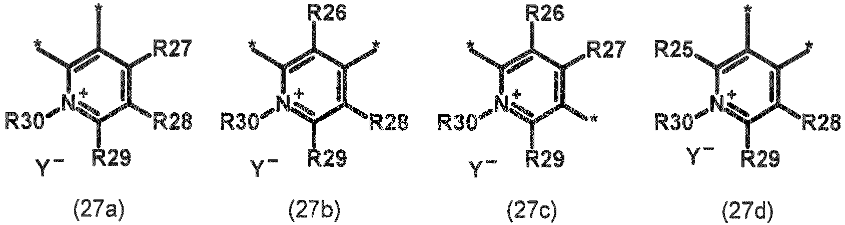 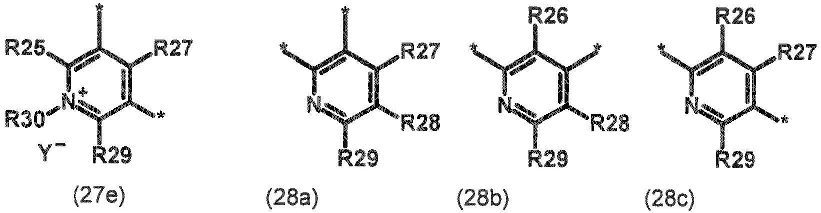 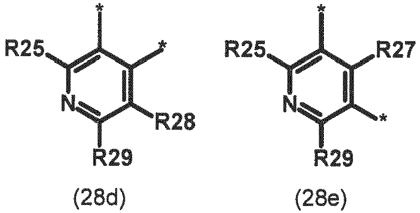 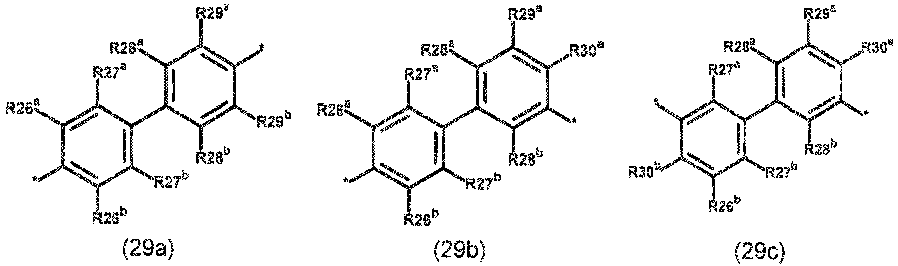 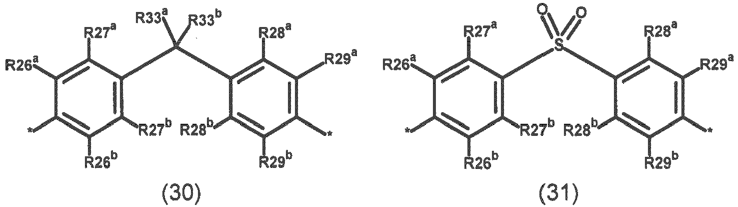
wobei die Reste R25, R26, R27, R28, R29, R26^{a}, R26^{b}, R27^{a}, R27^{b}, R28^{a}, R28^{b}, R29^{a}, R29^{b}, R30^{a} und R30^{b} jeweils unabhängig voneinander Wasserstoff, Hydroxy, Amino, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, und wobei der Rest R30 jeweils unabhängig voneinander Wasserstoff oder Alkyl, das gradkettig oder verzweigt sein kann, mit 1 bis 5 Kohlenstoffatomen bedeutet, und wobei die Reste R33^{a} und R33^{b} jeweils unabhängig voneinander Wasserstoff, Hydroxy, Amino, Alkyl, das gradkettig oder verzweigt sein kann, mit 1 bis 5 Kohlenstoffatomen, Perfluoralkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl, Benzyl oder wenn zusammen genommen ein Cycloalkyl, das gradkettig oder verzweigt sein kann, mit 4 bis 9 Kohlenstoffatomen, oder ein 9H-Fluoren-9-ylidenrest bedeuten, und
wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sufonat-Anion einer Sulfonsäure mit 1 bis 12 C-Atomen oder eine Kombination davon bedeutet.

Vorzugsweise ist wenigstens einer der Reste Rest R1 bis R7, weiter bevorzugt wenigstens einer der Reste R1, R2, R5 oder R6, weiter bevorzugt wenigstens einer der Reste R1 oder R2, weiter bevorzugt einer der Reste R1 oder R2, der wenigstens einen erfindungsgemäß zu verwendenden Phenalen-1-on-Verbindung mit der allgemeinen Formel (1) jeweils unabhängig ein organischer Rest W1, wobei der wenigstens eine organische Rest W1 jeweils unabhängig voneinander ein Rest der allgemeinen Formel (40) bis (67), oder (72) bis (98e), vorzugsweise ein Rest der allgemeinen Formel (40) bis (67) oder (72) oder (97), bedeutet: 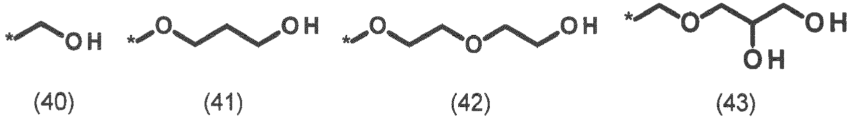 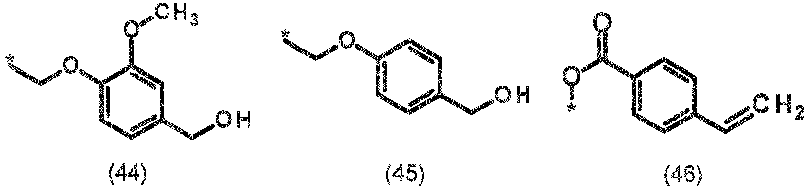 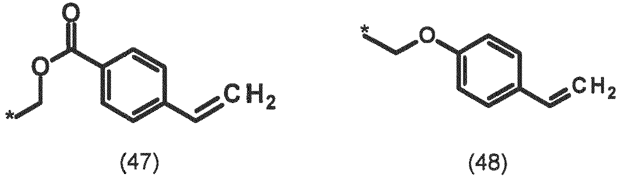 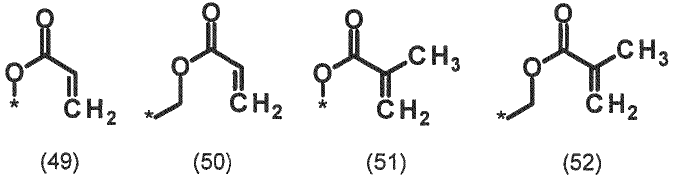 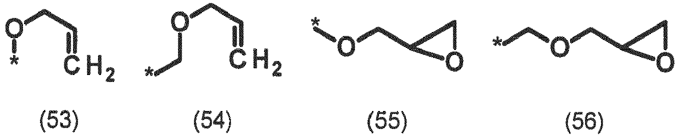 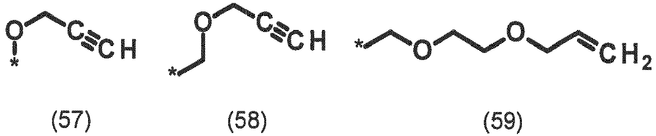 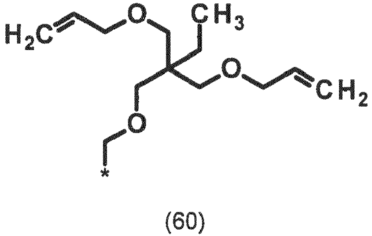 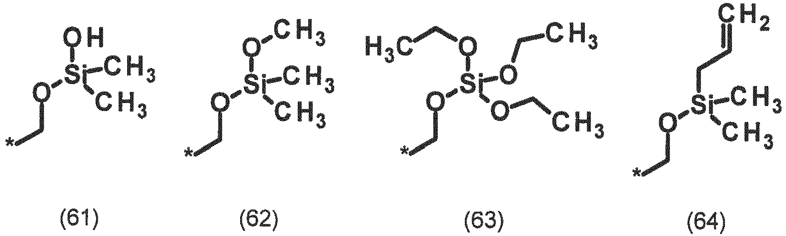 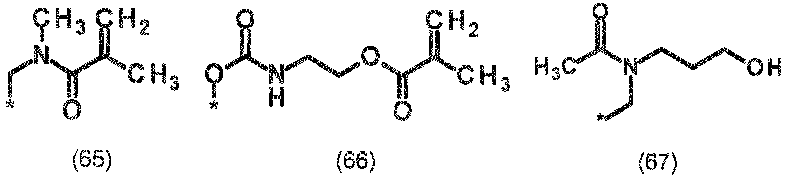 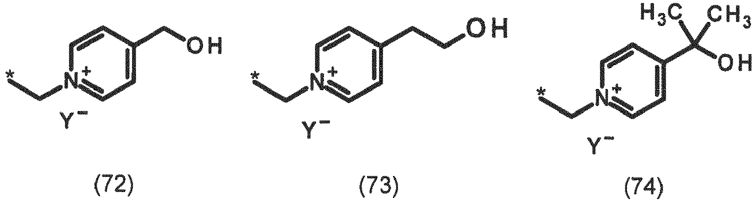 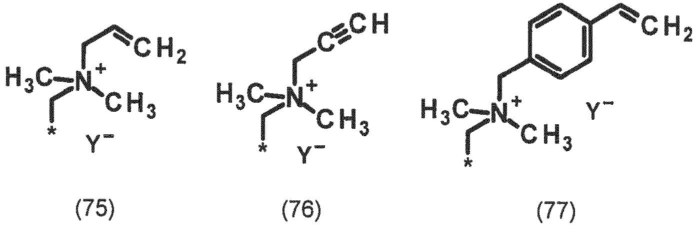 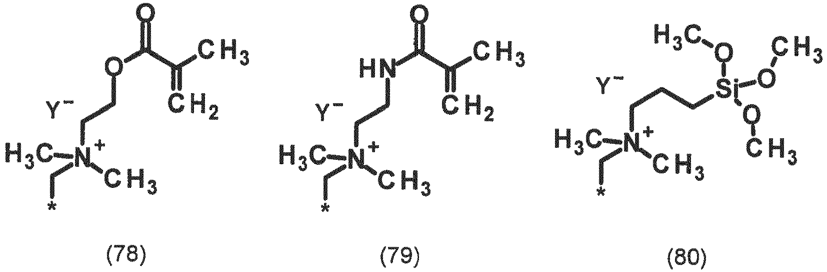 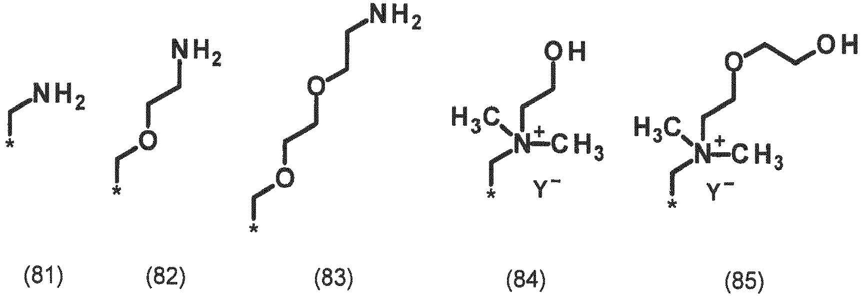 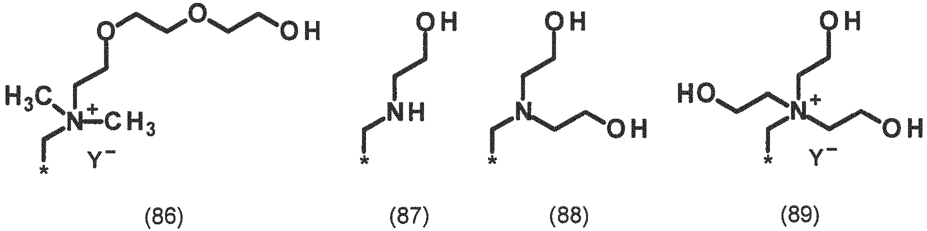 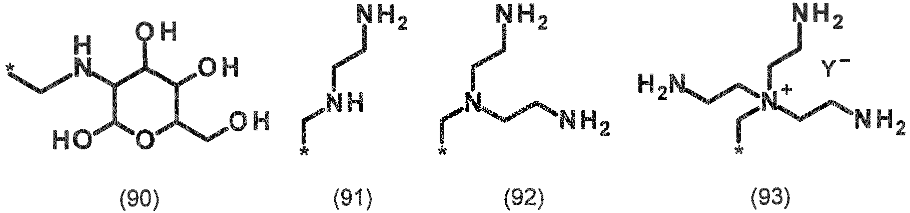 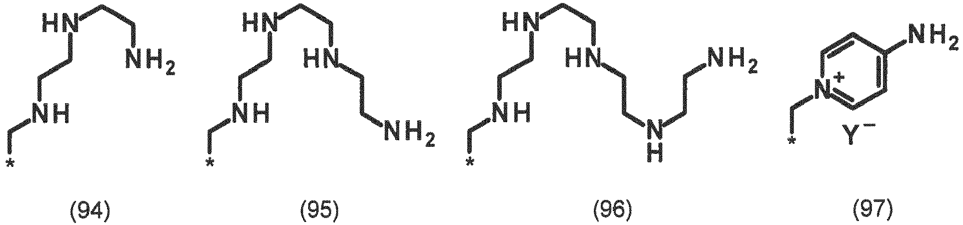 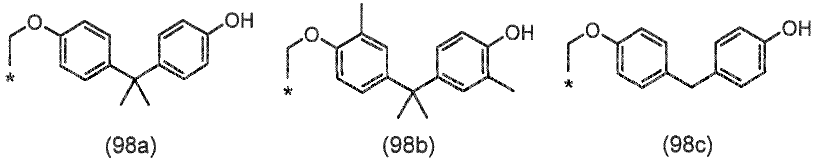 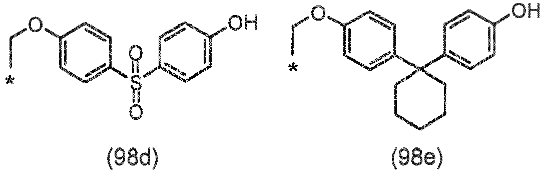 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sufonat-Anion einer Sulfonsäure mit 1 bis 12 C-Atomen oder eine Kombination davon bedeutet.

Weiter bevorzugt wird die wenigstens eine erfindungsgemäß zu verwendende Phenalen-1-on-Verbindung mit der allgemeinen Formel (1) jeweils unabhängig voneinander aus der Gruppe, die aus Verbindung der Formel (100) bis (127), (132) bis (166) und Kombinationen davon, vorzugsweise aus Verbindungen der Formel (100) bis (127), (132) bis (161) und Kombinationen davon, besteht: 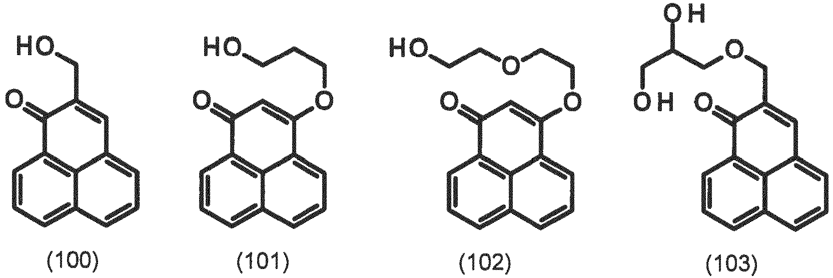 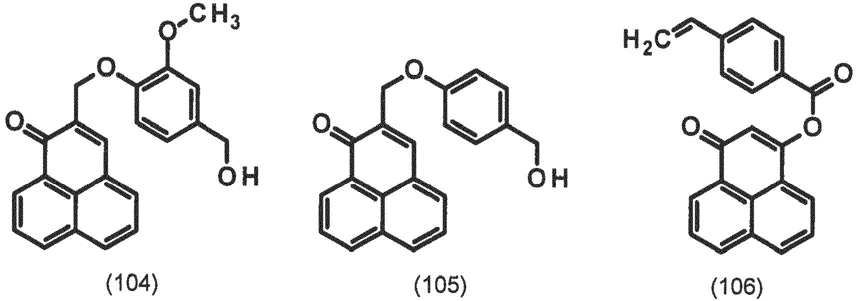 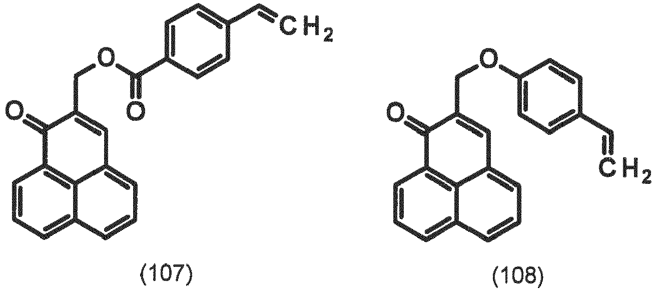 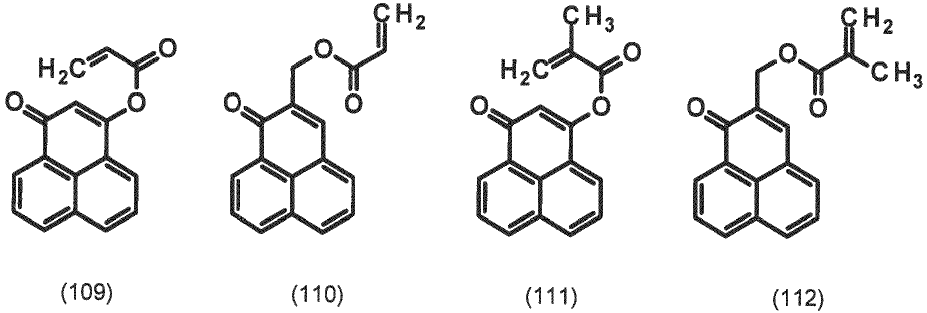 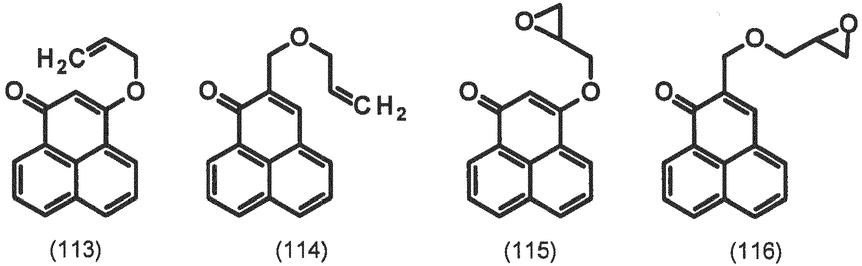 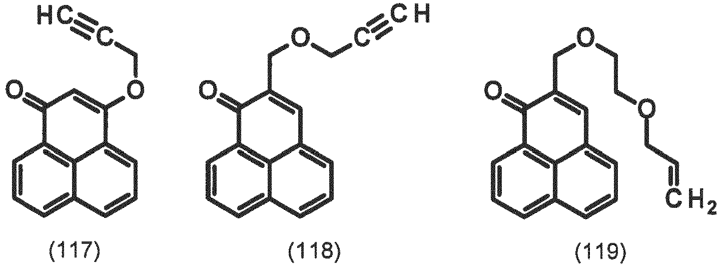 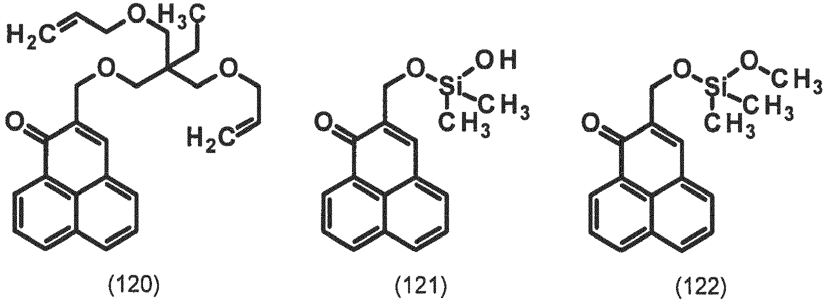 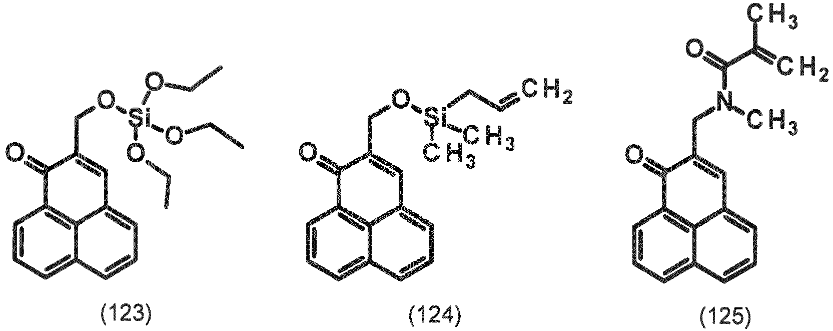 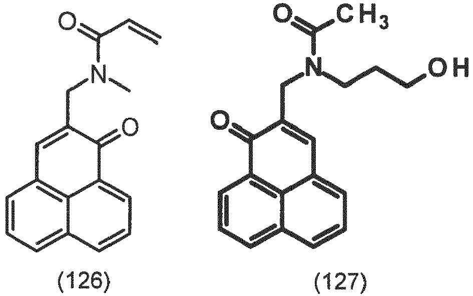 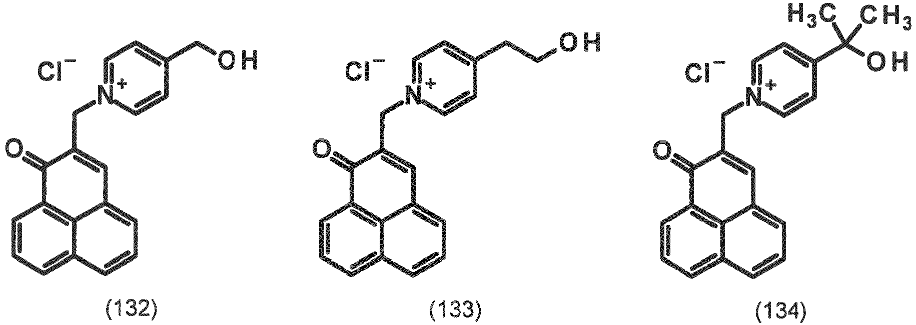 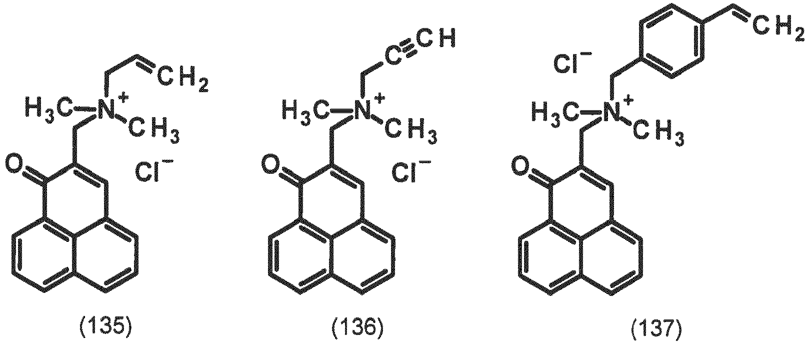 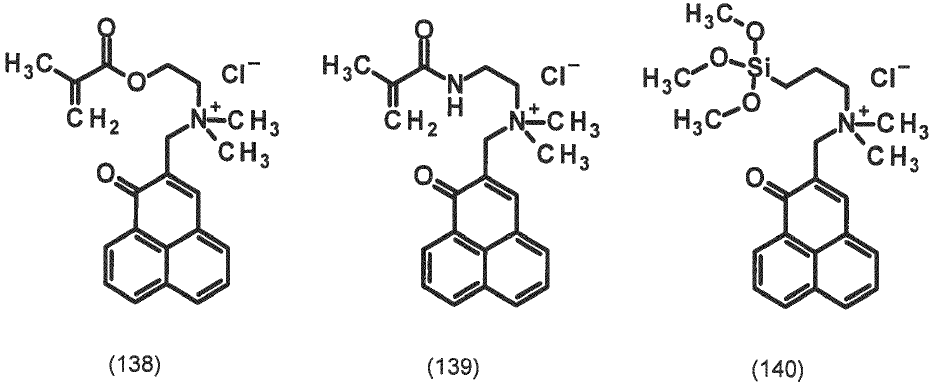 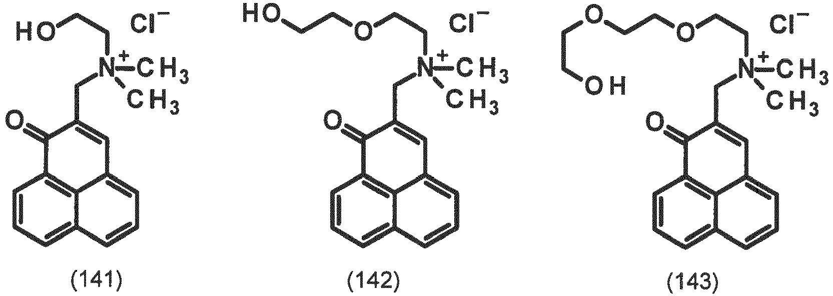 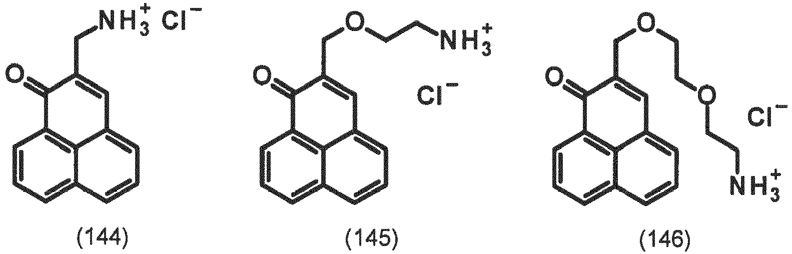 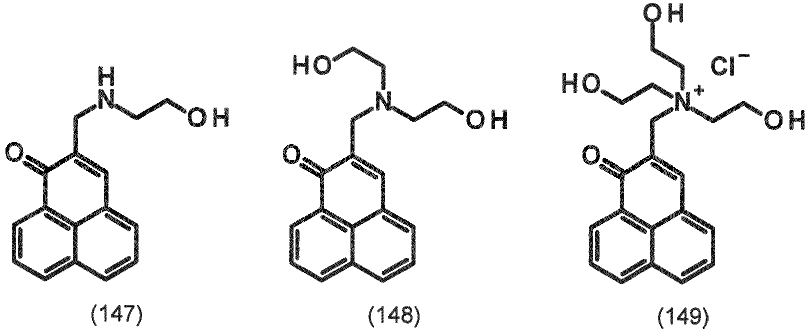 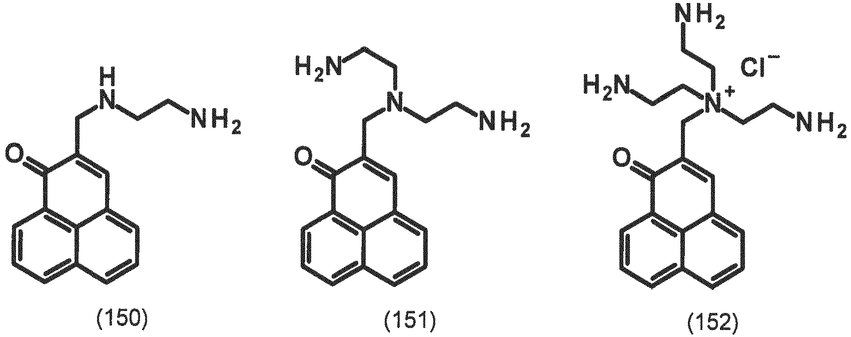 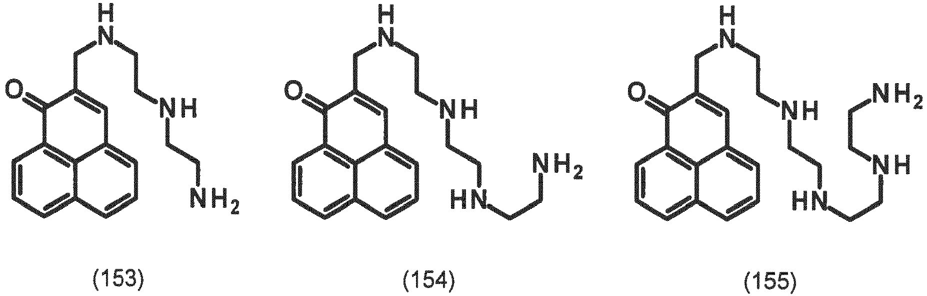 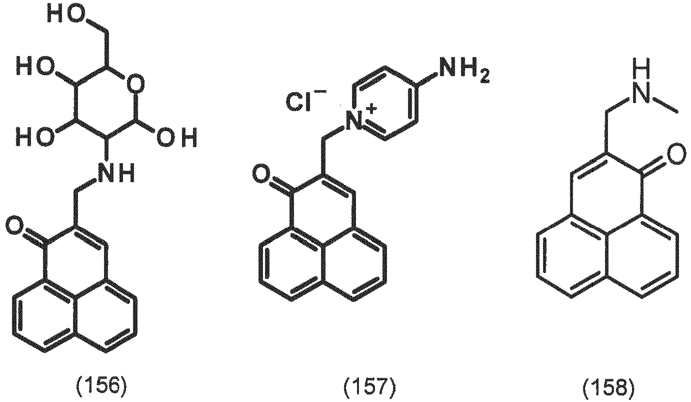 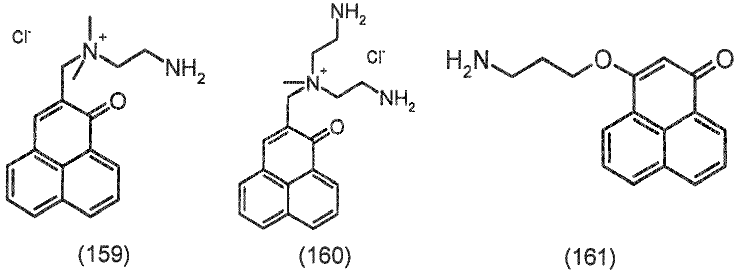 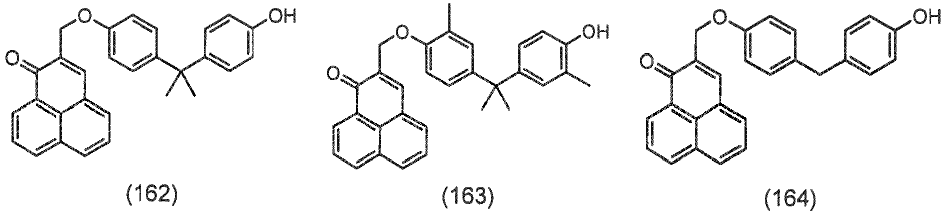 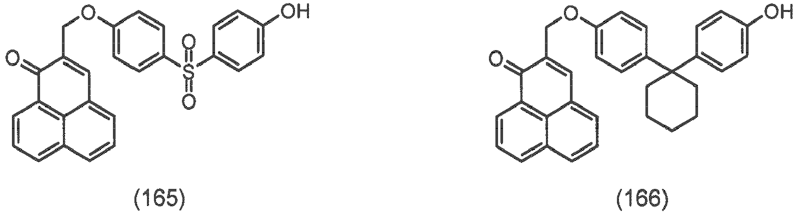 ausgewählt.

Weiter bevorzugt umfasst eine erfindungsgemäße Photosensibilisatorzusammensetzung
(a) wenigstens ein erfindungsgemäß Phenalen-1-on-Verbindung mit der allgemeinen Formel (1a) und
(b) wenigstens eine polymere Komponente und/oder Vorläufer davon.

Eine erfindungsgemäße Phenalen-1-on-Verbindung weist die allgemeine Formel (1a) auf: 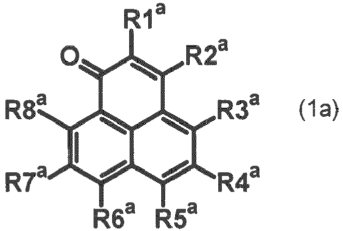 wobei die Reste R1^{a} bis R8^{a}, die jeweils unabhängig voneinander gleich oder voneinander verschieden sein können, jeweils Wasserstoff, Halogen, Alkyl mit 1 bis 12 C-Atomen, vorzugsweise 2 bis 9 C-Atomen, Alkylaryl mit 5 bis 20 C-Atomen, vorzugsweise 6 bis 9 C-Atomen, Aryl mit 5 bis 20 C-Atomen, vorzugsweise 6 bis 9 C-Atomen, *-O-Alkyl mit 1 bis 12 C-Atomen, vorzugsweise 2 bis 9 C-Atomen, *-O-Alkylaryl mit 5 bis 20 C-Atomen, vorzugsweise 6 bis 9 C-Atomen, *-O-Aryl mit 5 bis 20 C-Atomen, vorzugsweise 6 bis 9 C-Atomen, Ether mit 2 bis 12 C-Atomen, ein Rest der Formel *-O-C(=O)-R^{(Ia)}, ein Rest der Formel *-C(=O)-R^{(Ib)}, oder ein organischer Rest W1a, der wenigstens eine reaktive funktionelle Gruppe enthält, bedeutet, mit der Maßgabe, dass wenigstens einer der Reste R1^{a} oder R2^{a}, vorzugsweise einer der Reste R1^{a} oder R2^{a}, ein organischer Rest W1a ist, wobei der organische Rest W1a jeweils unabhängig voneinander ein Rest der allgemeinen Formel (41) bis (67), oder (98a) bis (98e), vorzugsweise ein Rest der allgemeinen Formel (41) bis (67) und Kombinationen davon, bedeutet:

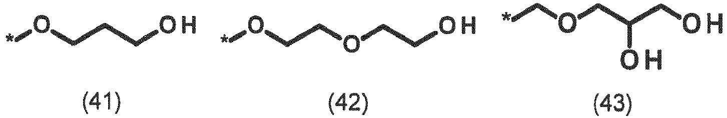

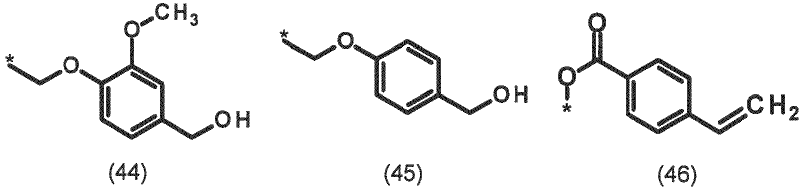

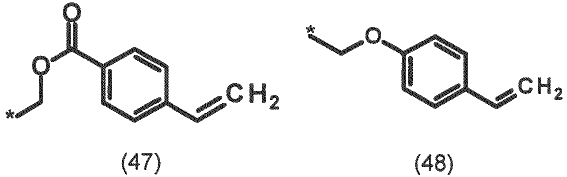

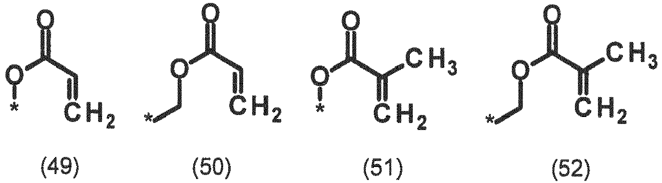

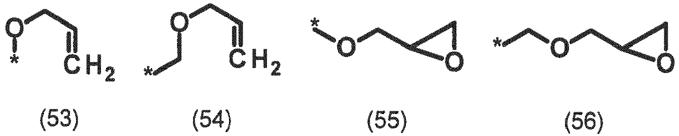

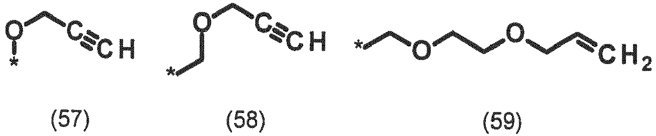

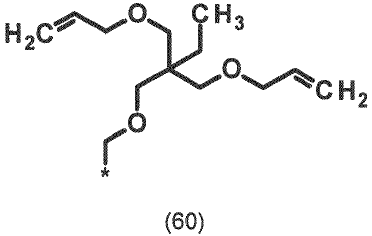

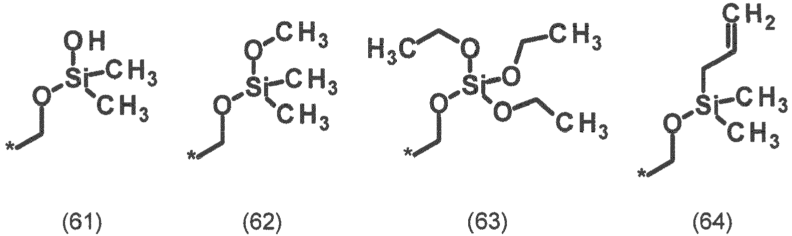

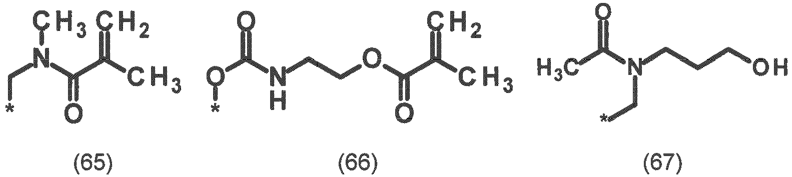

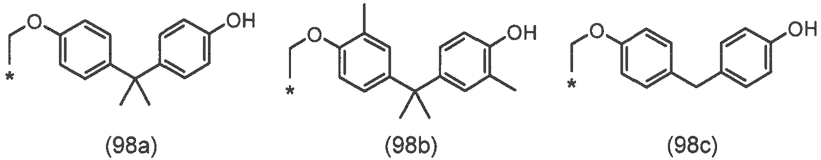

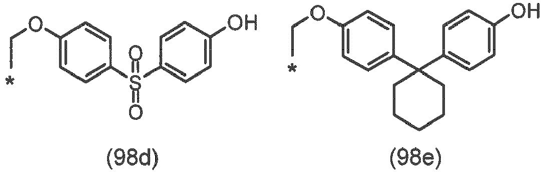

Weiter bevorzugt wird eine erfindungsgemäße Phenalen-1-on-Verbindung mit der allgemeinen Formel (1a) aus der Gruppe, die aus Verbindung der Formel (101) bis (127), (162) bis (166) und Kombinationen davon, vorzugsweise aus Verbindung der Formel (101) bis (127) und Kombinationen davon, besteht: 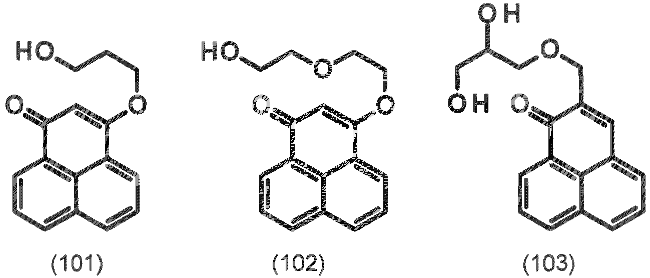 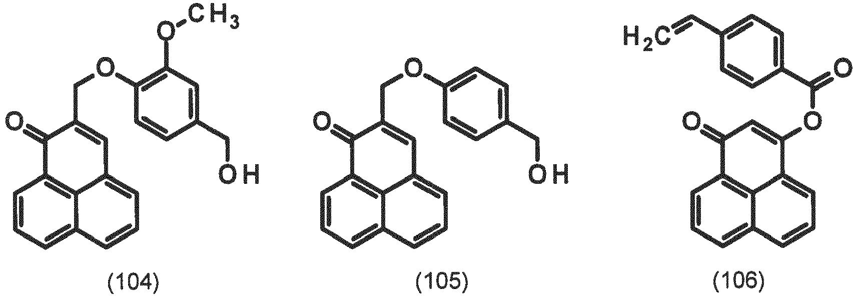 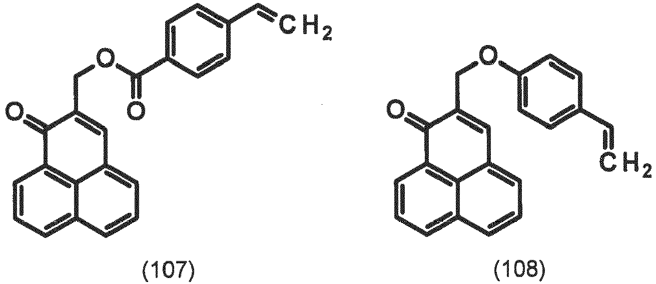 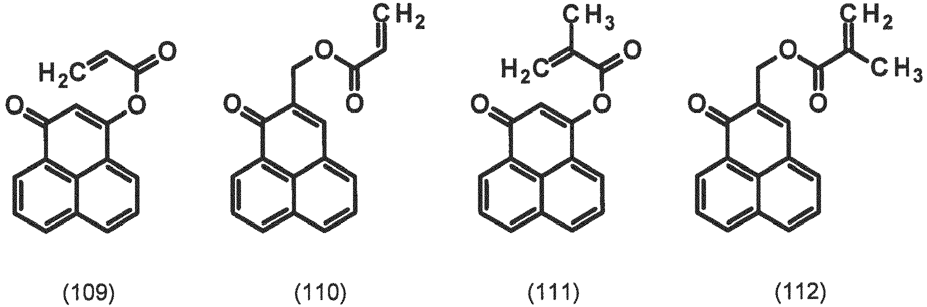 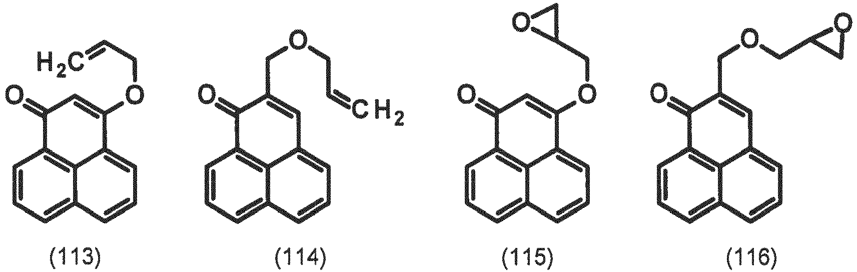 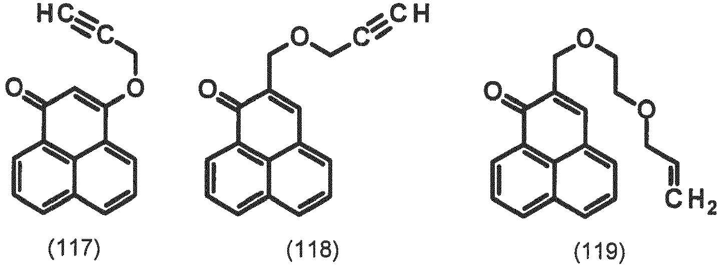 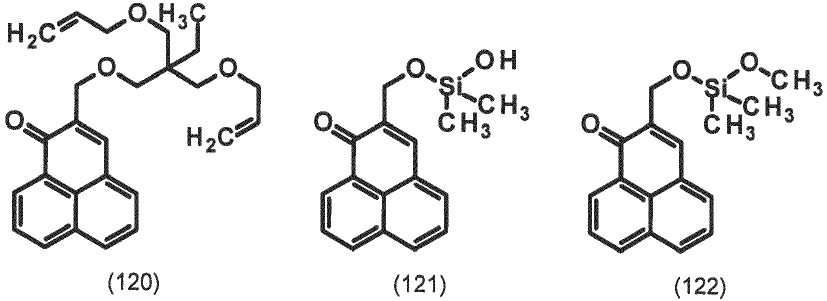 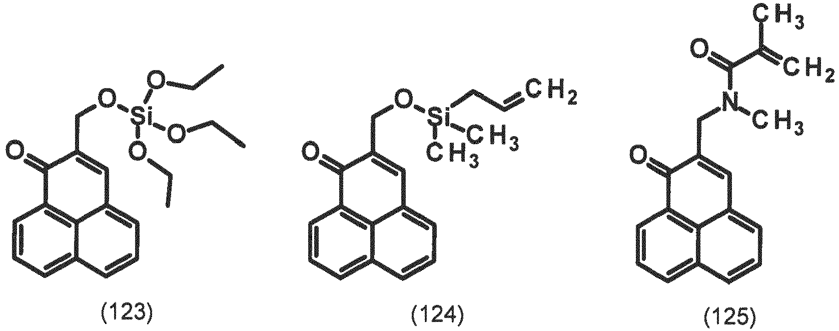 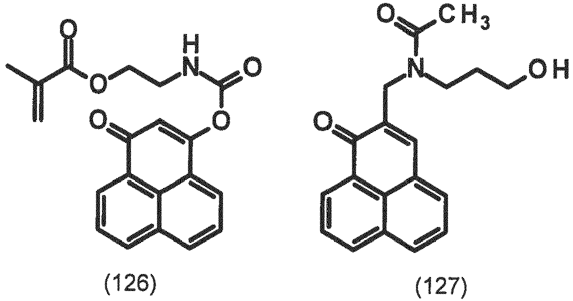 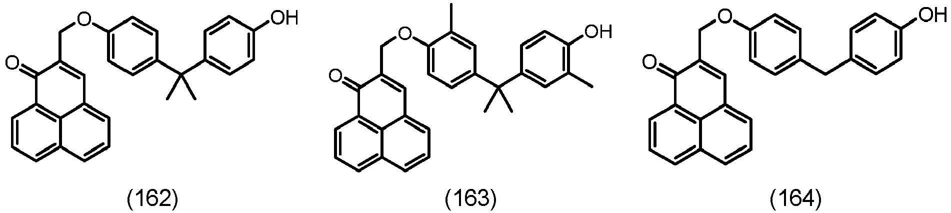 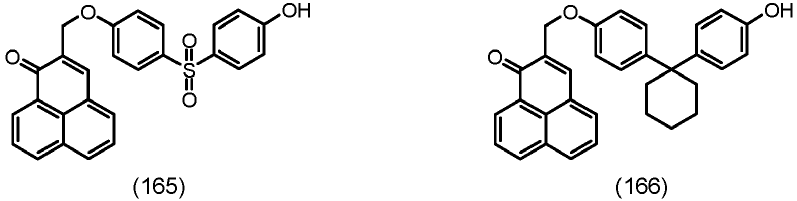 ausgewählt.

Bei einer bevorzugt Ausführunsform liegt eine Phenalen-1-on-Verbindung mit der allgemeinen Formel (1) als Salz vor, wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sulfonat-Anion einer Sulfonsäure mit 1 bis 12 C-Atomen oder eine Mischung davon bedeutet,

Ein geeignetes Carboxylat-Anion einer Carbonsäure mit 1 bis 15 Kohlenstoffatomen ist beispielsweise Acetat, Oxalat, Succinat, Tartrat oder eine Mischung davon.

Ein geeignetes Sulfonat-Anion einer Sulfonsäure mit 1 bis 12 C-Atomen ist beispielsweise Tosylat, Mesylat oder eine Mischung davon.

Bei einer bevorzugten Ausführungsform umfasst eine Photosensibilisatorzusammensetzung nach einem der Ansprüche 1 bis 3:
(a) wenigstens eine Phenalen-1-on-Verbindung mit der allgemeinen Formel (1), die eine Phenalen-1-on-Verbindung nach einem der Ansprüche 4 bis 5 ist, und
(b) wenigstens eine polymere Komponente und/oder Vorläufer davon.

Bei einer bevorzugten Ausführungsform ist eine erfindungsgemäße, vorzugsweise härtbare, Photosensibilisatorzusammensetzung für elektromagnetische Strahlung, die eine Wellenlänge aus einem Bereich von 280 bis 1000 nm, weiter bevorzugt von 320 bis 900 nm, noch weiter bevorzugt von 360 bis 800 nm, noch weiter bevorzugt von 380 bis 700 nm, aufweist, nach dem Aushärten der Zusammensetzung zumindest teilweise, vorzugsweise vollständig, transparent.

Vorzugsweise wird durch elektromagnetische Strahlung, die eine Wellenlänge aus einem Bereich von 280 bis 1000 nm, weiter bevorzugt von 320 bis 900 nm, noch weiter bevorzugt von 360 bis 800 nm, noch weiter bevorzugt von 380 bis 700 nm, aufweist, die in der Zusammensetzung enthaltene wenigstens eine erfindungsgemäß zu verwendende Phenalen-1-on-Verbindung mit der allgemeinen Formel (1) und/oder eine erfindungsgemäße Phenalen-1-on-Verbindung mit der allgemeinen Formel (1a) angeregt.

Die angeregte wenigstens eine erfindungsgemäß zu verwendende Phenalen-1-on-Verbindung mit der allgemeinen Formel (1), vorzugsweise der Formel (1a), kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Da eine erfindungsgemäße Photosensibilisatorzusammensetzung für elektromagnetische Strahlung, die für eine Anregung einer erfindungsgemäß zu verwendenden Phenalen-1-on-Verbindung mit der allgemeinen Formel (1) und/oder eine erfindungsgemäße Phenalen-1-on-Verbindung mit der allgemeinen Formel (1a) notwendige Wellenlänge aufweist, nach dem Aushärten zumindest teilweise, vorzugsweise vollständig, transparent ist, wird die eingestrahlte elektromagnetische Strahlung nur teilweise, vorzugsweise nicht, abgeschwächt, was zu einer signifikanten Verringerung der Singulett-Sauerstoff-Quantenausbeute führt.

Eine möglichst hohe Singulett-Sauerstoff-Quantenausbeute ist für eine antimikrobielle Wirksamkeit bei der photodynamischen Inaktivierung von Mikroorganismen auf Oberflächen erforderlich.

Weiter bevorzugt ist eine erfindungsgemäße Photosensibilisatorzusammensetzung nach dem Aushärten für Sauerstoff zumindest teilweise durchlässig.

Vorzugsweise ist erfindungsgemäße Photosensibilisatorzusammensetzung nach dem Aushärten sowohl für molekularen Sauerstoff (O₂), der in die ausgehärtete Photosensibilisatorzusammensetzung diffundiert, als auch für Singulett-Sauerstoff, der vorzugsweise nach Bestrahlen des in der Zusammensetzung enthaltenen wenigstens eine Phenalen-1-on-Verbindung mit der allgemeinen Formel (1), weiter bevorzug wenigstens eine Phenalen-1-on-Verbindung mit der allgemeinen Formel (1a), mit elektromagnetischer Strahlung geeigneter Wellenlänge und Strahlungsdichte gebildet wird, zumindest teilweise durchlässig.

Die Erfinder haben festgestellt, dass es durch Verwendung wenigstens einer Phenalen-1-on-Verbindung der allgemeinen Formel (1), weiter bevorzug wenigstens einer Phenalen-1-on-Verbindung mit der allgemeinen Formel (1a), in einer erfindungsgemäßen Photosensibilisator-Zusammensetzung möglich ist, nach Aushärten der Photosensibilisator-Zusammensetzung eine Oberfläche bereitzustellen, die nach Bestrahlung mit elektromagnetischer Strahlung geeigneter Wellenlänge und Energiedichte, vorzugsweise in Gegenwart von Sauerstoff und/oder einer sauerstoffabgebenden Verbindung, auf der Oberfläche anhaftende Mikroorganismen zuverlässig inaktiviert.

Die Erfinder haben weiterhin festgestellt, dass es bei Verwendung wenigstens einer Phenalen-1-on-Verbindung der allgemeinen Formel (1), vorzugsweise wenigstens einer Phenalen-1-on-Verbindung der allgemeinen Formel (1a), in einer erfindungsgemäßen Photosensibilisator-Zusammensetzung möglich ist, nach Aushärten der Photosensibilisator-Zusammensetzung eine Oberfläche bereitzustellen, die auch bei längerer Bestrahlung mit elektromagnetischer Strahlung geeigneter Wellenlänge und Energiedichte im wesentlichen keine, vorzugsweise keine, Abnahme der photodynamischen Aktivität der Oberfläche zeigt und auf der Oberfläche anhaftende Mikroorganismen zuverlässig inaktiviert werden.

Eine erfindungsgemäße Photosensibilisatorzusammensetzung weist wenigstens eine polymere Komponente und/oder Vorläufer davon auf, wobei die wenigstens eine polymere Komponente und/oder Vorläufer davon aus der Gruppe, die aus Polymerisaten und/oder Copolymerisaten von Acrylsäure und deren Ester, Methacrylsäure und deren Ester, Cyanacrylsäure und deren Ester, Acrylamid, Methacrylamid, Styrol, Siloxanen und deren Ester, Melamin, Acrylnitril, 1,3-Butadien, Epichlorhydrin, Polyolen, Polyisoprenen, Polyethern, Polyetherimiden, Polyvinylacetaten, Polycarbonaten, Polyethersulfonen, Carboxymethylcellulose, Alginat, und Kombinationen davon besteht, ausgewählt wird.

Vorzugsweise ist vorgenannte wenigstens eine polymere Komponente und/oder Vorläufer davon ein Präpolymer und/oder Monomer, weiter bevorzugt ein Harz. Vorzugsweise ist vorgenannte wenigstens eine polymere Komponente und/oder Vorläufer davon ein Polyurethanharz, ein Epoxidharz, ein Silikon oder Silikonharz, ein Polyacrylharz, Polymethacrylharz, ein Melamin-Formaldehyd-Harz, Phenol-Formaldehyd-Harz, ein ABS (Acrylnitril, 1,3-Butadien, Styrol) -Harz, ein Alkydharz, ein Polyesterharz, ein Alkydharz, ein Polyamidharz, ein Fluorkautschuk-Harz, ein Vinylesterharz oder eine Kombination davon. Vorzugsweise kann vorgenannte wenigstens eine polymere Komponente und/oder Vorläufer davon als 1 Komponenten (1K) Harz oder 2 Komponenten (2K) Harz vorliegen.

Weiter bevorzugt weist eine erfindungsgemäße Photosensibilisatorzusammensetzung wenigstens eine polymere Komponente und/oder Vorläufer davon und wenigstens eine Phenalen-1-on-Verbindung der allgemeinen Formel (1), vorzugsweise wenigstens eine Phenalen-1-on-Verbindung der allgemeinen Formel (1a), auf, wobei vorzugsweise der Rest X der wenigstens eine Phenalen-1-on-Verbindung der allgemeinen Formel (1), vorzugsweise wenigstens eine Phenalen-1-on-Verbindung der allgemeinen Formel (1a), auf die wenigstens eine polymere Komponente und/oder Vorläufer davon abgestimmt werden kann.

Bevorzugte Kombinationen aus wenigstens eine polymere Komponente und/oder Vorläufer davon und wenigstens einem Rest X der wenigstens eine Phenalen-1-on-Verbindung der allgemeinen Formel (1) sind beispielsweise:

| wenigstens eine polymere Komponente und/oder Vorläufer davon: | Rest X: |
|---|---|
| Polyurethanharz | -OH, -N(R^{(VI)})(R^{(VII)}), |
| Epoxidharz | -OH, -N(R^{(VI)})(R^{(VII)}), ein Rest mit der allgemeinen Formel (20), (22) oder (24), |
| Silikonharz, | -Si(R^{(VIII)})(R^{(IX)})-[O-Si(R^{(X)})(R^{(XI)})]ₚ-Z, ein Rest mit der allgemeinen Formel (20) oder (22), |
| Polyacrylharz | ein Rest mit der allgemeinen Formel (20) bis (23), |
| Polymethacrylharz, | ein Rest mit der allgemeinen Formel (20) bis (23), |
| Polyacrylamidharz | ein Rest mit der allgemeinen Formel (20) bis (23), |
| Melamin-Formaldehyd-Harz, | -OH, -N(R^{(VI)})(R^{(VII)}), ein Rest mit der allgemeinen Formel (20) oder (22), |
| Phenol-Formaldehyd-Harz, | -OH, -N(R^{(VI)})(R^{(VII)}), ein Rest mit der allgemeinen Formel (20) oder (22), |
| ABS (Acrylnitril, 1,3-Butadien, Styrol) -Harz, | -OH, -N(R^{(VI)})(R^{(VII)}), ein Rest mit der allgemeinen Formel (20) bis (23), |
| Alkydharz, | -OH, -N(R^{(VI)})(R^{(VII)}), ein Rest mit der allgemeinen Formel (20) bis (23), |
| Polyesterharz, | -OH, -N(R^{(VI)})(R^{(VII)}), ein Rest mit der allgemeinen Formel (20) bis (23), |
| Polyamidharz, | -OH, -N(R^{(VI)})(R^{(VII)}), ein Rest mit der allgemeinen Formel (20) bis (23), |
| Fluorkautschuk-Harz, | -OH, -N(R^{(VI)})(R^{(VII)}), ein Rest mit der allgemeinen Formel (20) oder (22), |
| Vinylesterharz | ein Rest mit der allgemeinen Formel (20) bis (23) |

Bevorzugte Kombinationen aus wenigstens eine polymere Komponente und/oder Vorläufer davon und wenigstens einem Rest X^{a} der wenigstens eine Phenalen-1-on-Verbindung der allgemeinen Formel (1a) sind beispielsweise:

| wenigstens eine polymere Komponente und/oder Vorläufer davon: | Rest X^{a}: |
|---|---|
| Polyurethanharz | -OH, |
| Epoxidharz | -OH, ein Rest mit der allgemeinen Formel (20), (22) oder (24), |
| Silikonharz, | -Si(R^{(VIII)})(R^{(IX)})-[O-Si(R^{(X)})(R^{(XI)})]ₚ-Z, ein Rest mit der allgemeinen Formel (20) oder (22), |
| Polyacrylharz | ein Rest mit der allgemeinen Formel (20) bis (23), |
| Polymethacrylharz, | ein Rest mit der allgemeinen Formel (20) bis (23), |
| Polyacrylamidharz | ein Rest mit der allgemeinen Formel (20) bis (23), |
| Melamin-Formaldehyd-Harz, | -OH, ein Rest mit der allgemeinen Formel (20) oder (22), |
| Phenol-Formaldehyd-Harz, | -OH, ein Rest mit der allgemeinen Formel (20) oder (22), |
| ABS (Acrylnitril, 1,3-Butadien, Styrol) -Harz, | -OH,ein Rest mit der allgemeinen Formel (20) bis (23), |
| Polyesterharz, | -OH, ein Rest mit der allgemeinen Formel (20) bis (23), |
| Alkydharz, | -OH, -N(R^{(VI)})(R^{(VII)}), ein Rest mit der allgemeinen Formel (20) bis (23), |
| Polyamidharz, | -OH, ein Rest mit der allgemeinen Formel (20) bis (23), |
| Fluorkautschuk-Harz, | -OH, ein Rest mit der allgemeinen Formel (20) oder (22), |
| Vinylesterharz ein | Rest mit der allgemeinen Formel (20) bis (23) |

Eine erfindungsgemäße Photosensibilisator-Zusammensetzung ermöglicht nach Aushärten der Photosensibilisator-Zusammensetzung eine Oberfläche bereitzustellen, die auch bei längerer Bestrahlung mit elektromagnetischer Strahlung geeigneter Wellenlänge und Energiedichte im wesentlichen keine, vorzugsweise keine, Abnahme der photodynamischen Aktivität der Oberfläche zeigt und auf der Oberfläche anhaftende Mikroorganismen zuverlässig inaktiviert werden.

Eine erfindungsgemäße Zusammensetzung weist wenigstens ein Vernetzungsmittel, beispielsweise wenigstens ein Polyisocyanat, ein blockiertes Isocyanat, wenigstens ein Alkyldiisocyanat oder Cycloalkyldiisocyanat oder Aryldiisocyanat, wenigstens eine Verbindung der allgemeinen Formel SiZ₄, (R^{XII})SiZ₃, (R^{XII})₂SiZ₂, eine Verbindung mit mindestens zwei Epoxidresten, eine Verbindung mit mindestens zwei Acrylamidresten, eine Verbindung mit mindestens zwei Acrylatresten, eine Verbindung mit mindestens zwei Aldehydresten, eine Verbindung mit mindestens zwei Alkoholgruppen, eine Verbindung mit mindestens zwei Aminresten, ein Divinylsulfon, und Kombinationen davon enthält, wobei der Rest R^{XII} jeweils unabhängig voneinander Alkyl, das gradkettig oder verzweigt sein kann, mit 1 bis 5 Kohlenstoffatomen, Phenyl oder Benzyl, vorzugsweise Methyl, Ethyl, n-Propyl, iso-Propyl, Phenyl oder Benzyl, bedeutet und wobei der Rest Z jeweils unabhängig voneinander Halogen, Hydroxyl, Alkoxyl mit 1 bis 4 Kohlenstoffatomen oder Alkylcarboxyl mit 1 bis 4 Kohlenstoffatomen, vorzugsweise Halogen oder Hydroxyl, bedeutet.

Geeignete Verbindung mit mindestens zwei Alkoholgruppen sind beipielsweise Bisphenole, beispielsweise die kommerziell erhältlichen Bisphenole Bisphenol A, Bisphenol AF, Bisphenol AP, Bisphenol B, Bisphenol BP, Bisphenol C, Bisphenol E, Bisphenol F, Bisphenol FL, Bisphenol G, Bisphenol M, Bisphenol P, Bisphenol PH, Bisphenol S, Bisphenol TMC, Bisphenol Z und Mischungen davon, vorzugsweise Bisphenol A (CAS 80-05-7), Bisphenol C (CAS 79-97-0), Bisphenol F (CAS 620-92-8), Bisphenol S (CAS 80-09-1), Bisphenol Z (CAS 843-55-0) und Mischungen davon.

Weiter bevorzugt weist eine erfindungsgemäße Photosensibilisatorzusammensetzung die wenigstens eine Phenalen-1-on-Verbindung mit der allgemeinen Formel (1), vorzugsweise mit der Formel 1 a), kovalent und/oder elektrostatisch, vorzugsweise kovalent, an die wenigstens eine polymere Komponente und/oder Vorläufer davon gebunden auf.

Vorzugsweise ist eine erfindungsgemäße Photosensibilisatorzusammensetzung ein Lack, eine Lasur, eine Dispersionsfarbe, eine Latexfarbe, eine Silikatfarbe, oder eine Kalkfarbe.

Weiter bevorzugt ist eine erfindungsgemäße Photosensibilisatorzusammensetzung ein Granulat.

Weiter bevorzugt liegt eine erfindungsgemäße Photosensibilisatorzusammensetzung in Form einer Beschichtung, eines selbsttragenden Films, eines Gewebes oder eines Formgegenstandes vor.

Ein erfindungsgemäßer Gegenstand, umfassend wenigstens eine ausgehärtete Polymerzusammensetzung, wobei die ausgehärtete Polymerzusammensetzung
(a) wenigstens eine der vorgenannten Phenalen-1-on-Verbindung mit der allgemeinen Formel (1), wenigstens eine Phenalen-1-on-Verbindung nach einem der Ansprüche 4 bis 5, oder eine Kombination davon, weiter bevorzugt wenigstens eine der vorgenannten Phenalen-1-on-Verbindung mit der allgemeinen Formel (1a), und
(b) wenigstens eine der vorgenannten ausgehärteten polymeren Komponente umfasst, wobei die wenigstens eine Phenalen-1-on-Verbindung mit der allgemeinen Formel (1), die wenigstens eine Phenalen-1-on-Verbindung nach einem der Ansprüche 4 bis 5, oder eine Kombination davon, weiter bevorzugt die wenigstens eine der vorgenannten Phenalen-1-on-Verbindung mit der allgemeinen Formel (1a), kovalent und/oder elektrostatisch, vorzugsweise kovalent, an die wenigstens eine ausgehärtete polymere Komponente gebunden ist.

Vorzugsweise kann ein erfindungsgemäßer Gegenstand durch Beschichten, beispielsweises durch Besprühen, Lackieren, Streichen und/oder Tauchen, des Gegenstandes mit einer erfindungsgemäßen Photosensibilisatorzusammensetzung, die vorzugsweise streichfähig, sprühfähig, oder fließfähig bei der Temperatur des Auftrags, vorzugsweise flüssig, ist und anschließendem Trocknen und/oder Aushärten erhalten werden.

Beispielsweise kann ein erfindungsgemäßer Gegenstand durch Laminieren, Kaschieren, Bekleben, mit einer erfindungsgemäßen Photosensibilisatorzusammensetzung, die in Form eines Gewebes oder eines selbsttragenden Films vorliegt, erhalten werden.

Beispielsweise kann ein erfindungsgemäßer Gegenstand durch Spritzen, Schmelzblasen, Extrudieren, oder anderen bekannten Verfahren zur Formgebung einer erfindungsgemäßen Photosensibilisatorzusammensetzung, die vorzugsweise streichfähig, sprühfähig, oder fließfähig bei der Temperatur der Herstellung, vorzugsweise flüssig, ist und anschließendem Trocknen und/oder Aushärten erhalten werden.

Weiter bevorzugt umfasst oder besteht ein erfindungsgemäßer Gegenstand aus einer gehärtete Polymerzusammensetzung, die
(a) wenigstens eine Phenalen-1-on-Verbindung nach einem der Ansprüche 4 bis 5, und
(b) wenigstens eine ausgehärtete polymere Komponente umfasst, die weiter bevorzugt in Form einer Beschichtung, eines selbsttragenden Films, eines Gewebes oder eines Formgegenstandes vorliegt, wobei die wenigstens eine Phenalen-1-on-Verbindung mit nach einem der Ansprüche 4 bis 5 kovalent und/oder elektrostatisch, vorzugsweise kovalent, an die wenigstens eine ausgehärtete polymere Komponente gebunden ist.

Die Aufgabe der vorliegenden Erfindung wird weiterhin gelöst durch die Bereitstellung einer nicht-medizinischen Verwendung einer Photosensibilisatorzusammensetzung nach einem der Ansprüche 1 bis 3 oder 6 bis 11 und/oder einer Phenalen-1-on-Verbindung nach einem der Ansprüche 4 bis 5 und/oder eines Gegenstandes nach einem der Ansprüche 12 bis 14 zur, vorzugsweise photodynamischen, Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, und/oder einem Biofilm davon ausgewählt werden.

Vorzugsweise wird eine erfindungsgemäße Photosensibilisatorzusammensetzung oder eine erfindungsgemäße Phenalen-1-on-Verbindung zur photodynamischen Oberflächenreinigung und/oder Oberflächenbeschichtung eines Gegenstandes oder Raumes verwendet.

Vorzugsweise wird eine erfindungsgemäße Photosensibilisatorzusammensetzung oder eine erfindungsgemäße Phenalen-1-on-Verbindung zur Oberflächenbeschichtung von Gegenständen, vorzugsweise Medizinprodukten, Lebensmittelverpackungen, Textilien, Baustoffen, elektronischen Geräten, Möbeln oder Hygieneartikeln verwendet.

Weitere Anwendungen sind beispielsweise Schnuller, Nuckel, Schläuche und Leitungen, Fensterscheiben, Glasflächen und/oder Fliesen von Nassräume und/oder Nasszellen, Touchscreenelemente, Personalcomputer (PC) und Peripheriegeräte, Lappen, Wischtücher, Arbeitsplatzauflagen, Behandlungs- und Vorbereitungsflächen in Kliniken und Altenheimen, Sanitäreinrichtung, Sanitägeräte, Wurstdärme, Getränkebehälter, Geschirr oder Abfallbehälter.

Ein erfindungsgemäßes nicht-medizinisches Verfahren zur, vorzugsweise photodynamischen, Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, und/oder einem Biofilm davon umfasst folgende Schritte:
(A) in Kontakt bringen der Mikroorganismen und/oder einem Biofilm davon mit wenigstens einer Beschichtung, die durch Aushärten einer Photosensibilisatorzusammensetzung nach einem der Ansprüche 1 bis 3 oder 6 bis 11 hergestellt wurde und/oder wenigstens eine Phenalen-1-on-Verbindung nach einem der Ansprüche 4 bis 5 enthält, und/oder wenigstens eines Gegenstandes nach einem der Ansprüche 12 bis 14, und
(B) Bestrahlen der Mikroorganismen und/oder einem Biofilm davon und der, in der Beschichtung und/oder dem Gegenstand enthaltenen wenigstens einen Phenalen-1-on-Verbindung mit elektromagnetischer Strahlung geeigneter Wellenlänge und Energiedichte.

Beispielsweise können transparente, durchsichtige oder transluzente Gegenstände, wie beispielsweise Nuckel, Schnuller, Schläuche, oder ähnliches, unter Verwendung einer erfindungsgemäße Photosensibilisatorzusammensetzung hergestellt werden und mit Hilfe des erfindungsgemäßen Verfahrens gereinigt und/oder entkeimt werden.

Weiter bevorzugt werden Gegenstände behandelt, die eine thermisch begrenzte Haltbarkeit aufweisen, beispielsweise Gegenstände aus thermoplastischen Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer bevorzugten Ausführungsform werden die vorgenannten Alkylreste mit 1 bis 12 C-Atomen, weiter bevorzugt 2 bis 9 C-Atomen, jeweils unabhängig voneinander aus der Gruppe, die aus Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl, n-Pentyl, n-Hexyl, n-Heptyl und n-Octyl und Kombinationen davon, weiter bevorzugt aus Methyl, Ethyl, n-Propyl, n-Butyl, n-Pentyl, n-Hexyl, n-Heptyl und n-Octyl und Kombinationen davon, besteht, ausgewählt.

Bei einer bevorzugten Ausführungsform werden die vorgenannten *-O-Alkylreste mit 1 bis 12 C-Atomen, vorzugsweise 2 bis 9 C-Atomen, jeweils unabhängig voneinander aus der Gruppe, die aus Methoxy, Ethoxy, n-Propoxy, iso-Propoxy, n-Butoxy, tert-Butoxy und Kombinationen davon besteht, ausgewählt.

Bei einer bevorzugten Ausführungsform werden die vorgenannten Arylreste mit 5 bis 20 C-Atomen, weiter bevorzugt 6 bis 9 C-Atomen, jeweils unabhängig voneinander aus der Gruppe, die aus Phenyl, Naphthyl, Anthracenyl, Phenanthrenyl und Pyrenyl besteht, ausgewählt.

Bei einer bevorzugten Ausführungsform werden die vorgenannten *-O-Arylreste mit 5 bis 20 C-Atomen, weiter bevorzugt 6 bis 9 C-Atomen, jeweils unabhängig voneinander aus der Gruppe, die aus *-O-Phenyl, *-O-Naphthyl, *-O-Anthracenyl, und Kombinationen davon besteht, ausgewählt.

Bei einer bevorzugten Ausführungsform werden die vorgenannten Alkylarylreste mit 5 bis 20 C-Atomen, weiter bevorzugt 6 bis 9 C-Atomen, jeweils unabhängig voneinander aus der Gruppe, die aus Benzyl, 2-Phenyl-eth-1-yl, 3-Phenyl-eth-1-yl, und Kombinationen davon, ausgewählt.

Bei einer bevorzugten Ausführungsform werden die vorgenannten *-O-Alkylarylreste mit 5 bis 20 C-Atomen, weiter bevorzugt 6 bis 9 C-Atomen, jeweils unabhängig voneinander aus der Gruppe, die aus 1-Methoxy-Phenyl, 1-Ethoxy-2-Phenyl, 1-Propoxy-3-Phenyl und Kombinationen davon, ausgewählt.

Bei einer weiteren bevorzugten Ausführungsform weisen die vorgenannten Etherreste jeweils unabhängig voneinander 2 bis 12 C-Atome, weiter bevorzugt 3 bis 9 C-Atome, weiter bevorzugt 4 bis 7 C-Atome, auf. Bei einer weiter bevorzugten Ausführungsform werden die vorgenannten Etherreste jeweils unabhängig voneinander aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl, i-Propoxymethyl, tert.-Butyloxymethyl, Dioxa-3,6-heptyl und Benzyloxymethyl besteht, ausgewählt. Bei einer weiter bevorzugten Ausführungsform können die vorgenannten Etherreste einfache Etherreste, Oligoetherreste, Polyetherreste oder Mischungen davon sein.

Die Erfindung wird nachfolgend durch Figuren und Beispiele erläutert, ohne hierauf beschränkt zu sein.

Fig. 1A zeigt die Ergebnisse der in Beispiel 3.1 erreichten Log-Reduktion der S. aureus Zahl.

Fig. 1B zeigt die Ergebnisse der in Beispiel 3.2 erreichten Log-Reduktion der S. aureus Zahl.

Fig. 2 zeigt die Ergebnisse der in Beispiel 3.3 erreichten Log-Reduktion der S. aureus Zahl.

Fig. 3 zeigt die Log-Reduktion von S. aureus auf der Silikonoberfläche (A - bedeutet 10 Shore ohne Farbstoff, A + bedeutet 10 Shore plus Farbstoff, B - bedeutet 45 Shore ohne Farbstoff, B + bedeutet 45 Shore plus Farbstoff).

Alle verwendeten Chemikalien wurden von gängigen Anbietern käuflich erworben (Thermo Fisher Scientific, Polysciences Europe GmbH, Sigma Aldrich, TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm x 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt.

NMR-Spektren wurden auf einem Bruker-Spektrometer Avance 300 (300 MHz [1H-NMR]) (Bruker Corporation, Billerica, US) gemessen.

Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für ¹H-NMR und 0.1 Hz für Kopplungskonstanten. Das verwendete Lösemittel ist jeweils für jedes Spektrum aufgeführt.

Die IR-Spektren wurden an einem Biorad Spektrometer Excalibur FTS 3000 (Bio-Rad Laboratories GmbH, München, DE) aufgenommen.

ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als lonisierungsgas für FAB.

Schmelzpunkte wurden mit Hilfe des Schmelzpunktmessgerätes Büchi SMP-20 (Büchi Labortechnik GmbH, Essen, DE) unter Verwendung einer Glaskapillare bestimmt.

Sämtliche UV/Vis-Spektren wurden mit einem Varian Cary 50 Bio UV/VIS Spektrometer, Fluoreszenzspektren mit einem Varian Cary Eclipse Spektrometer (jeweils Agilent Technologies, Santa Clara, US) aufgenommen.

Die Lösungsmittel für Absorptions- und Emissionsmessungen wurden in spezieller spektroskopischer Reinheit von Acros oder Baker bzw. Uvasol von Merck gekauft. Milliporewasser (18 MΩ, Milli QPlus) wurde für alle Messungen verwendet.

### Beispiel 1: Herstellung der verwendeten Phenale-1-on-Derivate

### Übersicht 1: Synthese der verwendeten Phenale-1-on-Derivate

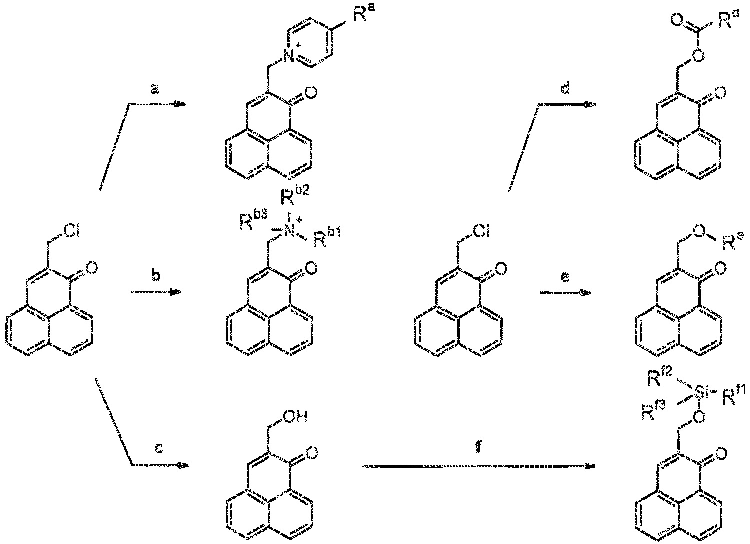

**Bedingungen: a**) Substituiertes Pyridin, MeCN, 50°C, 6h; **b**) Substituiertes Dimethylamin, MeCN, RT, 24h; **c**) 4N NaOH, Wasser, Toluen, Tetrabutylammoniumhydrogensulfat, RT, 6h; d) Variante 1: Carbonsäure als Natriumsalz, Adogen^{®} 464, Toluen, reflux, 4h; Variante 2: Carbonsäure, DMAP, DCC, THF, 0°C → RT, 4h; **e**) Substituierter Alkohol, Toluen, 4N NaOH, Tetrabutylammoniumhydrogensulfat, RT, 6h oder substituiertes Phenol, THF, PPh₃, DEAD, 0°C → RT, 6h; f) Substituiertes Silylchlorid, DCM oder THF, Triethylamin oder Imidazol, 0°C → RT;

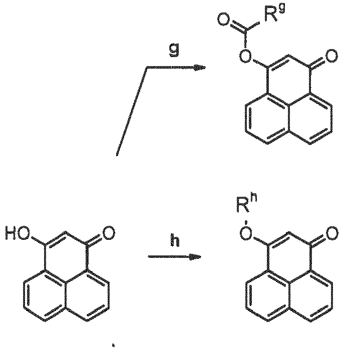

**Bedingungen: g**) Carbonsäure, THF, PPh3, DCC, 0°C → RT, 4h oder Carbonsäurechlorid, DCM, Triethylamin, 0°C → RT, 3h; h) substituierter Alkohol, DCM, PPh₃, DEAD, 0°C → RT, 6h;

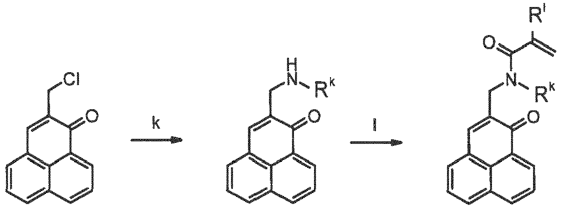

**Bedingungen: k**) Sekundäres Amin in Methanol, 0°C → RT, 6h; **l**) Carbonsäurechlorid, DCM oder THF, Triethylamin, 0°C → RT;

### 1. Herstellung von 2-Chloromethyl-1H-phenalen-1-on

2-Chloromethyl-1H-phenalen-1-on wurde gemäß der in US 2014/039184 A1, Beispiel 1 beschriebenen Methode hergestellt. Das NMR-Spektrum entsprach den in US 2014/039184 A1 offenbarten Werten.

### 2. Allgemeine Vorschrift a):

| | Substituiertes Pyridin in Übersicht 1, Schritt a) | | | | Produkt | |
|---|---|---|---|---|---|---|
| | | MW [g/mol] | Einwaage [mg] | | Formel | Bezeichnung |
| 2.1 | 4-Pyridinmethanol (Sigma-Aldrich) R^{e} = *-CH₂OH | 109,13 | 270 | → | 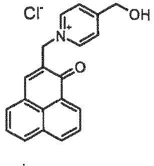 | SAPN-19a *[Verbindung (132)]* |
| 2.2 | 4-Pyridinethanol (Sigma-Aldrich) R^{a} = *-CH₂CH₂OH | 123,16 | 310 | → | 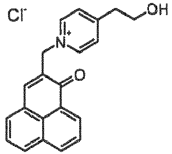 | SAPN-19b *[Verbindung (134)]* |
| 2.3 | 4-Pyridinpropan-2-ol (Sigma-Aldrich) R^{a} = *-C(CH₃)₂OH | 137,18 | 340 | → | 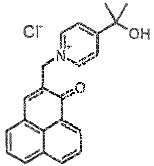 | SAPN-19c *[Verbindung (133)]* |

2-Chloromethyl-1H-phenalen-1-on (115 mg, 0,5 mmol) wurde in Acetonitril (6 mL) vorgelegt. Das oben angegebene substituierte Pyridin (2,5 mmol) wurde jeweils langsam in kleinen Portionen zugegeben. Die Suspension wurde 3 Tage bei Raumtemperatur im Dunklen gerührt.

Zur Reinigung wurde die Suspension auf zwei Polypropylen Röhrchen mit konischem Boden (Nennvolumen 15ml, Greiner Bio-One GmbH, Frickenhausen, DE) aufgeteilt und durch Zugabe von Diethylether auf 15 mL pro Röhrchen gefällt. Das Produkt wurde abzentrifugiert (60 mins, 4400 rpm, 0°C) und der Überstand verworfen. Das Präzipitat wurde in Diethylether suspendiert. Nach dem Absetzen des Produktes wurde jeweils der Überstand verworfen. Der Reinigungsschritt wurde noch einmal wiederholt und das Produkt anschließend getrocknet.

### 3. Allgemeine Vorschrift b):

| | Substituiertes Dimethylamin in Übersicht 1, Schritt b) | | | | Produkt | |
|---|---|---|---|---|---|---|
| | | MW [g/mol] | Einwaage [mg] | | Formel | Bezeichnung |
| 3.1 | N,N-Dimethylallylamin (Sigma-Aldrich) R^{b1} = 2-Propen-1-yl R^{b2}, R^{b3} = Methyl | 85,16 | 170 | → | 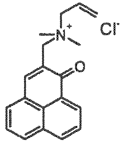 | SAPN-35a *[Verbindung (135)]* |
| 3.2 | 1-N,N-Dimethylamino-2-propin (Sigma-Aldrich) R^{b1} = 2-Propin-1-yl R^{b2}, R^{b3} = Methyl | 83,13 | 166 | → | 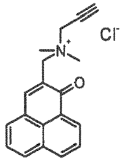 | SAPN-35a *[Verbindung (136)]* |
| 3.3 | N-(4-Vinylbenzyl)-N,N-dimethylamin (Thermo Fisher Scientific) R^{b1} = 4-Vinylphen-1-yl R^{b2}, R^{b3} = Methyl | 161,24 | 322 | → | 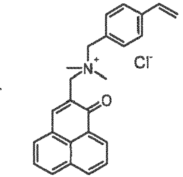 | SAPN-36 *[Verbindung (137)]* |
| 3.4 | 2-(N,N-Dimethylamino)-ethylmethacrylat (Sigma-Aldrich) R^{b1} = Eth-1-ylmethacrylat R^{b2}, R^{b3} = Methyl | 157,21 | 314 | → | 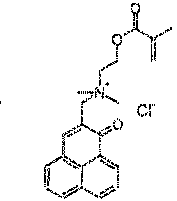 | SAPN-37a *[Verbindung (138)]* |
| 3.5 | N-[2-(N,N-Dimethylamino)-ethyl]-methacrylamid (Polysciences Europe GmbH) R^{b1} = Eth-1-ylmethacrylamid R^{b2}, R^{b3} = Methyl | 156,22 | 312 | → | 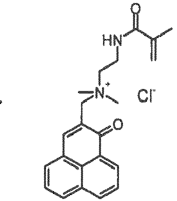 | SAPN-37b *[Verbindung (139)]* |
| 3.6 | (N,N-Dimethyl-3-aminopropyl)-trimethoxysilan (Sigma-Aldrich) R^{b1} = 3-(Trimethoxysilyl)-prop-1-yl R^{b2}, R^{b3} = Methyl | 207,34 | 414 | → | 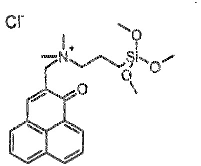 | SA-PN-38 *[Verbindung (140)]* |
| 3.7I | 1-N-Bocethylendiamin (Sigma-Aldrich) R^{b1} = 2-N-Boc-ethylenamin-1-yl R^{b2}, R^{b3} = H | 160,2 | 320 | → | 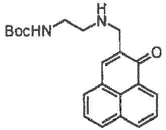 | SA-PN-25c-boc |
| 3.8 | 2-N-Boc-aminoethyl-1-N,N-dimethylamin R^{b1} = 2-N-Boc-ethylenamin-1-yl R^{b2}, R^{b3} = Methyl | 198,2 | 396 | → | 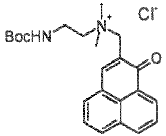 | SA-PN-25a-boc |
| 3.9 | 1,1-N,N-(2-N-Boc-aminoethyl)-N-methylamin R^{b1}, R^{b2} = 2-N-Boc-ethylenamin-1-yl R^{b3} = Methyl | 327,2 | 654 | → | 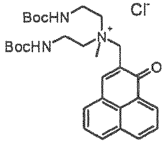 | SA-PN-34a-boc |
| 3.10 | R^{b1} = *-(CH₂)₂O(CH₂)₂OH R^{b2}, R^{b3} = CH3 | 133,19 | 400 | → | 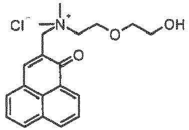 | SA-PN-11 *[Verbindung (142)]* |
| 3.11 | R^{b1} = *-(CH₂)₂O[(CH₂)₂O]₂H R^{b2}, R^{b3} = CH₃ | 177,25 | 530 | → | 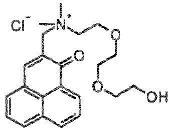 | SA-PN-12 *[Verbindung (143)]* |
| 3.12 | N,N-Dimethylaminoethanol R^{b1} = *-(CH₂)₂OH R^{b2}, R^{b3} = CH₃ | 89,14 | 270 | → | 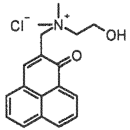 | SA-PN-09 *[Verbindung (141)]* |

2-Chloromethyl-1H-phenalen-1-on (115 mg, 0,5 mmol) wurde in Acetonitril (6 mL) vorgelegt. Das oben angegebene substituierte Dimethylamin (2 mmol) wurde jeweils langsam in kleinen Portionen zugegeben. Die Suspension wurde 48 h bei Raumtemperatur im Dunklen gerührt.

Zur Reinigung wurde die Suspension auf zwei Polypropylen Röhrchen mit konischem Boden (Nennvolumen 15ml, Greiner Bio-One GmbH) aufgeteilt und durch Zugabe von Diethylether auf 15 mL pro Röhrchen gefällt. Das Produkt wurde abzentrifugiert (60 mins, 4400 rpm, 0°C) und der Überstand verworfen. Das Präzipitat wurde in Diethylether suspendiert. Nach dem Absetzen des Produktes wurde jeweils der Überstand verworfen. Der Reinigungsschritt wurde noch einmal wiederholt und das Produkt anschließend getrocknet.

### 4. Vorschrift c): Herstellung von 2-Hydroxymethyl-1H-phenalen-1-on [Bezeichnung: PNOH, Verbindung (100)]

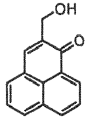

2-Chloromethyl-1 H-phenalen-1-on (230 mg, 1 mmol) wurde in 20 mL Toluen gelöst. Wässriges Natriumhydroxid (4N, 5 mL) und der Phasentransferkatalysator Tetrabutylammoniumhydrogensulfat (100 mg) wurden zugegeben. Das Reaktionsgemisch wurde 6 h bei Raumtemperatur lebhaft gerührt. Es bildete sich ein gelbbrauner Niederschlag. Der Niederschlag wurde abfiltriert, mit Wasser (4 mal, 20 ml), Toluen und Petrolether (jeweils 1 mal, 20 ml) gewaschen. Das Produkt wurde an der Luft bis zur Gewichtskonstanz getrocknet.

### 5. Allgemeine Vorschrift d):

| | Carbonsäure in Übersicht 1, Schritt d) | | | | Produkt | |
|---|---|---|---|---|---|---|
| | | MW [g/mol] | Einwaage [mg] | | Formel | Bezeichnung |
| 5.1 | Acrylsäure (Sigma-Aldrich) R^{d} = *-CH=CH₂ | 72,06 | 72 | → | 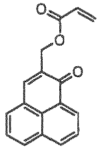 | PN-AMO-07a *[Verbindung (110)]* |
| 5.2 | Methacrylsäure (Sigma-Aldrich) R^{d} = *-C(CH₃)=CH₂ | 87,09 | 87 | → | 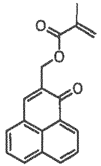 | PN-AMO-07b *[Verbindung (112)]* |
| 5.3 | 4-Vinylbenzoesäure (Sigma-Aldrich) R^{d} = 4-Vinylphen-1-yl | 148,16 | 148 | → | 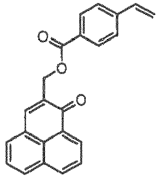 | PN-AMO-08 *[Verbindung (107)]* |

### Variante 1:

Die oben angegebenen Carbonsäuren (1 mmol) wurde jeweils mit einer äquimolaren Menge Natriumhydroxid neutralisiert und das erhaltene Natriumsalz der Carbonsäure durch Gefriertrocknung isoliert.

2-Chloromethyl-1H-phenalen-1-on (115 mg, 0,5 mmol) wurde in Toluen gelöst (3 mL). Das Natriumsalz der oben angegebenen Carbonsäure, Hydrochinon (11 mg, 0.1 mmol) und Adogen^{®} 464 (200 mg) wurden zugegeben und das Reaktionsgemisch unter kräftigem Rühren 4 h refluxiert. Nach Abkühlen auf Raumtemperatur wurde mit Toluen verdünnt (20 mL) und mit Wasser ausgeschüttelt (30 mL). Die wässrige Phase wurde zweimal mit Essigsäureethylester (20 mL) extrahiert. Die organischen Phasen wurden vereinigt, mit Wasser (50 mL) gewaschen, abgetrennt und über Magnesiumsulfat getrocknet.

Nach Filtration und Einengen unter vermindertem Druck wurde das Rohprodukt durch Flashchromatographie gereinigt (DCM/PE 2:1).

### Variante 2:

Verbindung (100) (65 mg, 0,3 mmol) wurde in trockenem THF gelöst (1 mL). Die oben angegebene Carbonsäure, Hydrochinon (11 mg, 0.1 mmol), DMAP (61 mg, 0,5 mmol) und DCC (103 mg, 0,5 mmol) wurden bei 2-5°C zugegeben. Das Reaktionsgemisch wurde kräftig für 4 h bei RT gerührt. Es wurde mit Essigsäureethylester verdünnt (20 mL) und mit Wasser ausgeschüttelt (30 mL). Die wässrige Phase wurde zweimal mit Essigsäureethylester (20 mL) extrahiert. Die organischen Phasen wurden vereinigt, mit Wasser (50 mL) gewaschen, abgetrennt und über Magnesiumsulfat getrocknet.

Nach Filtration und Einengen unter vermindertem Druck wurde das Rohprodukt durch Flashchromatographie gereinigt (DCM/PE 2:1).

### 6. Allgemeine Vorschrift e):

| | Substituierter Alkohol in Übersicht 1, Schritt e) | | | | Produkt | |
|---|---|---|---|---|---|---|
| | | MW [g/mol] | Einwaage [mg] | | Formel | Bezeichnung |
| 6.1 | Allylalkohol (Sigma-Aldrich) R^{e} = 2-Propen-1-yl | 58,08 | 58 | → | 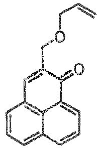 | PN-AMO-04a *[Verbindung (114)]* |
| 6.2 | Propargylalkohol (Sigma-Aldrich) R^{e} = 2-Propin-1-yl | 56,06 | 56 | → | 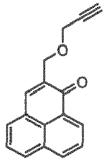 | PN-AMO-04b *[Verbindung (118)]* |
| 6.3 | 4-Vinylphenol (Sigma-Aldrich) R^{a} = 4-Vinylphen-1-yl | 120,15 | 120 | → | 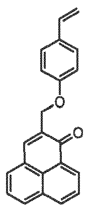 | PN-AMO-06 *[Verbindung (108)]* |
| 6.4 | 2-Allyloxyethanol (Sigma-Aldrich) R^{a} = 2-Allyloxyeth-1-yl | 102,13 | 102 | → | 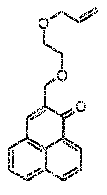 | PN-AMO-05 *[Verbindung (119)]* |
| 6.5 | Glycerin (Sigma-Aldrich) R^{e} = 2,3-Dihydroxyprop-1-yl | 92,09 | 92 | → | 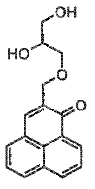 | PN-AMO-10 *[Verbindung (103)]* |
| 6.6 | 4-Hydroxybenzylalkohol (Sigma-Aldrich) R^{e} = 4-(Hydroxymethyl)phen-1-yl | 124,14 | 124 | → | 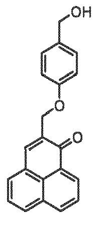 | PN-AMO-02b *[Verbindung (105)]* |
| 6.7 | 4-Hydroxy-3-methoxy-benzylalkohol (Sigma-Aldrich) R^{e} = 4-(Hydroxymethyl)-2-methoxy-phen-1-yl | 154,17 | 154 | → | 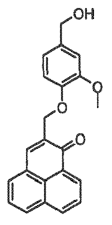 | PN-AMO-04a *[Verbindung (104)]* |

2-Chloromethyl-1H-phenalen-1-on (115 mg, 0,5 mmol) wurde in Toluen (4 mL) vorgelegt. Der oben angegebene substituierte Alkohol (1 mmol), Hydrochinon (11 mg, 0.1 mmol) und Tetrabutylammoniumhydrogensulfat (100 mg) wurden zugegeben. Zur lebhaft rührenden Lösung wurden bei 2-5°C 2 mL 4N wässrige Natronlauge zugegeben. Das Eisbad wurde entfernt und das Reaktionsgemisch weitere 6 h kräftig gerührt. Es wurde mit 30 mL DCM verdünnt und mehrmals mit Wasser (4 mal, je 20 mL) ausgeschüttelt.

Die organische Phase wurde abgetrennt und über Magnesiumsulfat getrocknet. Nach Filtration und Einengen unter vermindertem Druck wurde das Rohprodukt durch Flashchromatographie gereinigt (DCM/PE 2:1).

### 7. Allgemeine Vorschrift f):

| | Substituiertes Silylchlorid in Übersicht 1, Schritt f) | | | | Produkt | |
|---|---|---|---|---|---|---|
| | | MW [g/mol] | Einwaage [mg] | | Formel | Bezeichnung |
| 7.1 | Triethoxychlorosilan (Sigma-Aldrich) | 198,72 | 76 | → | 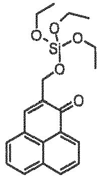 | PN-AMO-14a *[Verbindung (123)]* |
| | R^{f1}, R^{f2}, R^{f3} = Ethoxy | | | | | |
| 7.2 | Allyldimethylchlorosilan (Sigma-Aldrich) | 134,68 | 54 | → | 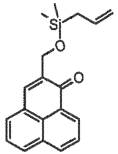 | PN-AMO-14b *[Verbindung (124)]* |
| | R^{f1} = 2-Propen-1-yl | | | | | |
| | R^{f2}, R^{f3} = Methyl | | | | | |

2-Hydroxymethyl-1H-phenalen-1-on (70 mg, 0,3 mmol) wurde zusammen mit Imidazol (30 mg, 0,4 mmol) in trockenem DCM (3 mL) in einem 10 mL Rundkolben mit Septum unter Stickstoff vorgelegt. Das oben angegebene substituierte Silylchlorid (0,4 mmol) in 2 mL trockenem DCM wurde langsam mittels einer Spritze über das Septum bei ca. 0°C zugetropft. Das Eisbad wurde entfernt und das Reaktionsgemisch über Nacht bei Raumtemperatur gerührt. Es wurde mit 30 mL DCM verdünnt und mehrmals mit Wasser (4 mal, je 20 mL) ausgeschüttelt. Die organische Phase wurde abgetrennt und über Magnesiumsulfat getrocknet. Nach Filtration und Einengen unter vermindertem Druck wurde das Rohprodukt durch Flashchromatographie gereinigt (DCM/PE 2:1).

### 8. Allgemeine Vorschrift g):

| | Carbonsäure in Übersicht 1, Schritt g) | | | | Produkt | |
|---|---|---|---|---|---|---|
| | | MW [g/mol] | Einwaage [mg] | | Formel | Bezeichnung |
| 8.1 | Acrylsäure | 72,06 | 53 | → | 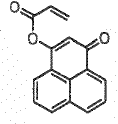 | PN-AMO-12a *[Verbindung (109))* |
| | R^{g} = *-CH=CH₂ | | | | | |
| 8.2 | Methacrylsäure | 87,09 | 62 | → | | PN-AMO-12b *[Verbindung (111)]* |
| | R^{g} = *-C(CH₃)=CH₂ | | | | 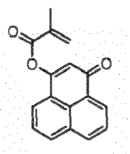 | |
| 8.3 | 4-Vinylbenzoesäure | 148,16 | 88 | → | 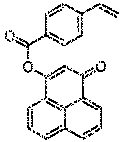 | PN-AMO-12c *[Verbindung (106)]* |
| | R^{g} = 4-Vinylphen-1-yl | | | | | |

3-Hydroxy-phenalen-1-on (Sigma-Aldrich) (100 mg, 0,5 mmol) wurde zusammen mit Triphenylphosphin (50 mg, 1 mmol) und der oben angegebenen Carbonsäure (0,6 mmol) in trockenem THF (5 mL) in einem 10 mL Rundkolben mit Septum unter Stickstoff vorgelegt. DCC (103 mg, 0,5 mmol) in trockenem THF (1 mL) wurde langsam mittels einer Spritze über das Septum bei ca.0°C zugetropft. Nach 2 h Rühren im Eisbad wurde eine weitere Portion DCC (103 mg, 0,5 mmol) in trockenem THF (1 mL) zugetropft. Das Reaktionsgemisch wurde im auftauenden Eisbad, dann bei Raumtemperatur für 6 h gerührt. Das THF wurde abgezogen, der Rückstand in 30 mL DCM aufgenommen und mehrmals mit Wasser (4 mal, 20 mL) ausgeschüttelt.

Die organische Phase wurde abgetrennt und über Magnesiumsulfat getrocknet. Nach Filtration und Einengen unter vermindertem Druck wurde das Rohprodukt durch Flashchromatographie gereinigt (DCM/PE 2:1).

### 9. Allgemeine Vorschrift h):

| | substituierter Alkohol in Übersicht 1, Schritt h) | | | | Produkt | |
|---|---|---|---|---|---|---|
| | | MW [g/mol] | Einwaage [mg] | | Formel | Bezeichnung |
| 9.1 | Allylalkohol | 58,08 | 23 | → | 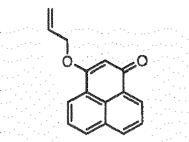 | PN-AMO-11a *[Verbindung (113)]* |
| | R^{h} = 2-Propen-1-yl | | | | | |
| 9.2 | Propargylalkohol | 56,06 | 22 | → | 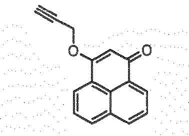 | PN-AMO-11b *[Verbindung (117)]* |
| | R^{h} = 2-Propin-1-yl | | | | | |
| 9.3 | Glycidol (Sigma-Aldrich) | 74,08 | 30 | → | 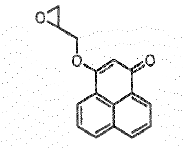 | PN-AMO-13 *[Verbindung (115)]* |
| | R^{h} = 2,3-Epoxy-prop-1-yl | | | | | |
| 9.4 | Bis(2-hydroxyethyl)ether (Thermo Fisher Scientific) | 106,12 | 42 | → | 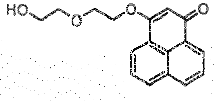 | PN-AMO-03a *[Verbindung (102)]* |
| | R^{h} = *-(CH₂)₂O(CH₂)₂OH | | | | | |
| 9.5 | 1,3-Propandiol (Sigma-Aldrich) | 76,10 | 31 | → | 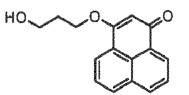 | PN-AMO-03b *[Verbindung (101)]* |
| | R^{h} = *-(CH₂)₂OH | | | | | |
| 9.6l | 3-Boc-aminopropan-1-ol (Sigma-Aldrich) | 175,2 | 70 | → | 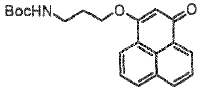 | SAPN-32-boc |
| | R^{h} = *-(CH₂)₂NHBoc | | | | | |

3-Hydroxy-phenalen-1-on (60 mg, 0,3 mmol) wurde zusammen mit Triphenylphosphin (30 mg, 0,6 mmol) in DCM (3 mL) und dem oben angegebenen substituierten Alkohol (0,4 mmol) in einem 10 mL Rundkolben mit Septum unter Stickstoff vorgelegt. DEAD in Toluen (40%, 0,2 mL, 0,4 mmol) wurde langsam mittels einer Spritze über das Septum bei ca.0°C zugetropft. Das Reaktionsgemisch wurde im auftauenden Eisbad, dann bei Raumtemperatur für 6 h gerührt. Es wurde mit 30 mL DCM verdünnt und mehrmals mit Wasser (4 mal, 20 mL) ausgeschüttelt. Die organische Phase wurde abgetrennt und über Magnesiumsulfat getrocknet. Nach Filtration und Einengen unter vermindertem Druck wurde das Rohprodukt durch Flashchromatographie gereinigt (DCM/PE 2:1).

### 10. Vorschrift k): Herstellung von 2-(N-Methylamino)methyl-1H-phenalen-1-on hydrochlorid [Bezeichnung: SAPN-02c, Verbindung 158]

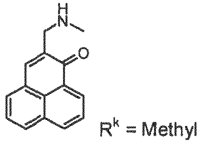

2-Chloromethyl-1H-phenalen-1-on (113 mg, 0.5 mmol) in Methanol (10 mL) wurde zu einer eiskalten Lösung des Amins in Methanol (10 mL, 5 M) über 2h zugetropft. Nach 1 h lebhaftem Rühren bei Raumtemperatur wurde das Lösungsmittel zusammen mit dem überschüssigen Amin im Stickstoffstrom abgetrieben. Der Rückstand wurde in möglichst wenig DCM/Ethanol 4:1 gelöst und durch Zugabe von Diethylether gefällt. Das Produkt wurde abzentrifugiert (60 mins, 4400 rpm, 0°C) und der Überstand verworfen. Das Präzipitat wurde in Diethylether suspendiert und erneut zentrifugiert. Der Reinigungsschritt wurde noch einmal wiederholt und das Produkt anschließend getrocknet.

### 11. Allgemeine Vorschrift I):

| | Carbonsäurechlorid in Übersicht 1, Schritt I) | | | | Produkt | |
|---|---|---|---|---|---|---|
| | | MW [g/mol] | Einwaage [mg] | | Formel | Bezeichnung |
| 11.1 | Acrylsäurechlorid (Merck-Millipore) | 90,5 | 36 | → | 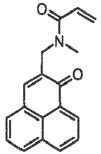 | PN-AMO-07a *[Verbindung (126)]* |
| | R^{k} = Methyl | | | | | |
| | R^{l} = 2-Ethen-1-yl | | | | | |
| 11.2 | Methacrylsäurechlorid | 104,53 | 41 | → | 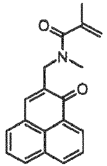 | PN-AMO-07b *[Verbindung (126)]* |
| | R^{k} = Methyl | | | | | |
| | R^{l} = 1-Methyl-2-ethen-1-yl | | | | | |

2-(N-Methylamino)methyl-1H-phenalen-1-on hydrochlorid (80 mg, 0,3 mmol) wurde mit Triethylamin (100 mg, 1 mmol) in DCM (3 mL) gelöst und im Eisbad unter Feuchtigkeitsausschluss gerührt. Das entsprechende Carbonsäuresäurechlorid (0,4 mmol) in DCM (0,5 mL) wurde zugetropft. Das Reaktionsgemisch wurde im auftauenden Eisbad, dann bei Raumtemperatur für 4 h gerührt. Es wurde mit 30 mL DCM verdünnt und mehrmals mit Wasser (4 mal, 20 mL) ausgeschüttelt. Die organische Phase wurde abgetrennt und über Magnesiumsulfat getrocknet. Nach Filtration und Einengen unter vermindertem Druck wurde das Rohprodukt durch Flashchromatographie gereinigt.

### 12. Synthese von Verbindung (127) [Bezeichnung: PN-AMO-01]

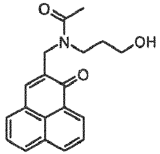

2-Chlormethyl-1H-phenalen-1-on (230 mg, 1,0 mmol) in Acetonitril (20 mL) wurde zu einer Lösung von 3-Aminopropanol (1,5 mL, 20 mmol) in Acetonitril (50 mL) über 30 mins. zugetropft. Nach Rühren bei Raumtemperatur über Nacht, wurde Triethylamin (2,02 g, 2,66 mL, 20 mmol) zugegeben und die Lösung im Eisbad gerührt. Essigsäureanhydrid (3,06 g, 2,83 mL, 30 mmol) wurde bei ca. 0°C zugetropft. Das Reaktionsgemisch wurde 2h bei Raumtemperatur gerührt, dann für 1 h auf 50°C erwärmt. Alle flüchtigen Bestandteile wurden unter vermindertem Druck abgezogen.

Das Produkt wurde durch Säulenchromatographie mit Dichloromethan / Ethanol 20:1 gereinigt. Man erhält 261 mg gelben Sirup. Dieses Material wurde in Methanol (2 mL) gelöst. Wässrige Natronlauge (1 M, 0,5 mL) wurde zugegeben und die Mischung über Nacht bei Raumtemperatur gerührt. Der Alkohol wurde abgezogen und die restliche Lösung mit Wasser (10 mL) verdünnt. Das Produkt wurde mit Dichloromethan (2x 10 mL) extrahiert. Die organischen Phasen wurden über Magnesiumsulfat getrocknet, gefiltert und bei vermindertem Druck eingeengt. Das Produkt war ein gelblicher, schmieriger Feststoff (215 mg, 69 %, 0,69 mmol).

### 13. Entschützung der Boc-Gruppe

| | | | Produkt | |
|---|---|---|---|---|
| | geschütztes Phenalenonderivat | | Formel | Bezeichnung |
| 13.1 | 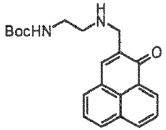 (aus Synthese 3.7) | → | 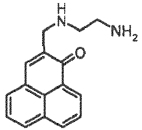 | SA-PN-25c *[Verbindung (150)]* |
| 13.2 | 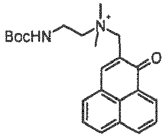 (aus Synthese 3.8) | → | 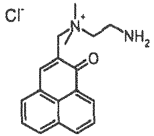 | SA-PN-25b *[Verbindung (159)]* |
| 13.3 | 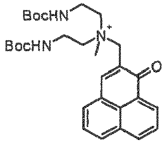 (aus Synthese 3.9) | → | 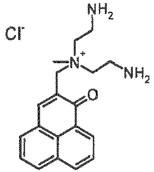 | SA-P N-34 b *[Verbindung (160)]* |
| 13.4 | 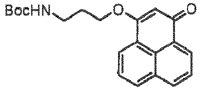 (aus Synthese 9.6) | → | 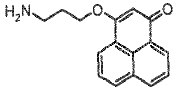 | SAPN-32 *[Verbindung (161)]* |

Das entsprechende tert-Butyloxycarbonyl (Boc -geschützte Phenalenonderivat wurde in Dichlormethan (3 mL pro 100 mg) vorgelegt. Eine gesättigte Lösung von Chlorwasserstoff in Diethylether (0,5 mL pro mmol Boc-Gruppe) wurde zugetropft. Der Ansatz wurde 3h unter Feuchtigkeits-ausschluss gerührt. Das Produkt wurde durch Zugabe von 30 mL Diethylether gefällt. Der Niederschlag wurde abzentrifugiert und mit Diethylether gründlich gewaschen. Das Produkt wurde bei vermindertem Druck getrocknet.

Nachfolgend sind für die hergestellten Verbindungen jeweils die berechnete Molekülmasse (MW) und Summenformel (MF) sowie die Daten der gemessenen Massenspektren (MS) und ₁H-NMR Spektren angegeben.

| | **Substanz Nr.** | **Struktur** | **Molekülmasse (MW)** / **Summenformel (MF)** | **Massenspektrum (MS)** | **NMR** |
|---|---|---|---|---|---|
| 2.1 | SAPN-19a | 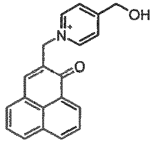 | **MW:** 302,3 + 35,45 = 337,75 g/mol | **MS** (ESI-MS, CH₂Cl₂/MeOH + 10 mmol NH₄OAc): m/z = 302.1 (100%, M+) | **¹H-NMR** (300 MHz, DMSO-d6): δ [ppm] = 9,18 (d, *J* = 6,8 Hz, 2H), 8,54 - 8,45 (m, 2H), 8,42 (s, 1H), 8,35 (d, *J* = 7,8 Hz, 1H), 8,15 (d, *J* = 6,4 Hz, 1H), 8,04 (d, *J* = 6,7 Hz, 2H), 7,91 (t, *J* = 7,7 Hz, 1H), 7,80 (dd, *J* = 8,2 & 7,2 Hz, 1H), 6,17 (t, *J* = 5,4 Hz, 1H), 5,79 (s, 2H), 4,80 (d, *J* = 4,5 Hz, 2H). |
| | | | **MF:** C₂₀H₁₆NO₂Cl | | |
| 2.2 | SAPN-19c | 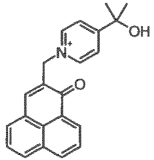 | **MW:** 330,4 + 35,45 = 365,85 g/mol | **MS** (ESI-MS, CH₂Cl₂/MeOH + 10 mmol NH₄OAc): m/z = 330.1 (100%, M+) | **¹H-NMR** (300 MHz, DMSO-d6): δ [ppm] = 9,18 (d, *J* = 6,8 Hz, 2H), 8,52 (dd, *J* = 8,9, 7,7 Hz, 2H), 8,45 (s, 1H), 8,36 (d, *J* = 8,1 Hz, 1H), 8,22 - 8,13 (m, 3H), 7,93 (t, *J* = 7,7 Hz, 1H), 7,82 (dd, *J* = 8,1 & 7,3 Hz, 1H), 5,91 (s, 1H), 5,78 (s, 2H), 1,49 (s, 6H). |
| | | | **MF:** C₂₂H₂₀NO₂Cl | | |
| 2.3 | SAPN-19b | 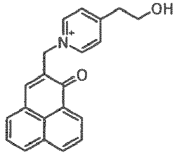 | **MW:** 316,4 + 35,45 = 351,85 g/mol | **MS** (ESI-MS, CH₂Cl₂/MeOH + 10 mmol NH₄OAc): m/z = 316.1 (100%, M+) | **¹H-NMR** (300 MHz, DMSO-d6): δ [ppm] = 9,11 (d, *J* = 6,6 Hz, 2H), 8,60 - 8,45 (m, 2H), 8,46 - 8,31 (m, 2H), 8,17 (d, *J* = 6,9 Hz, 1H), 8,02 (d, *J* = 6,6 Hz, 2H), 7,93 (t, *J* = 7,7 Hz, 1H), 7,87 - 7,72 (m, 1H), 5,75 (s, 2H), 4,95 (s, 1H), 3,75 (t, *J* = 5,9 Hz, 2H), 3,01 (t, *J* = 6,0 Hz, 2H). |
| | | | **MF:** C₂₁H₁₈NO₂Cl | | |
| 3.1 | SAPN-35a | 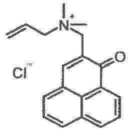 | **MW:** 278,38 + 35,45 = 313,83 g/mol | **MS** (ESI-MS, CH₂Cl₂/MeOH + 10 mmol NH₄OAc): m/z = 278.2 (100%, M+) | **¹H-NMR** (300 MHz, MeOD): δ [ppm] = 8,66 (dd, *J* = 7,4 & 1,1 Hz, 1H), 8,49 - 8,37 (m, 2H), 8,36 - 8,24 (m, 1H), 8,18 - 8,06 (m, 1H), 7,91 (t, *J* = 7,8 Hz, 1H), 7,77 (dd, J = 8,2 & 7,2 Hz, 1H), 5,87 - 5,69 (m, 2H), 4,56 (s, 2H), 4,10 (d, *J* = 7,3 Hz, 2H), 3,11 (s, 6H). |
| | | | **MF:** C₁₉H₂₀NOCl | | |
| 3.2 | SAPN-35b | 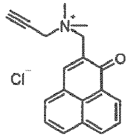 | **MW:** 276,36 + 35,45 = 311,81 g/mol | **MS** (ESI-MS, CH₂Cl₂/MeOH + 10 mmol NH₄OAc): m/z = 276.1 (100%, M+) | **¹H-NMR** (300 MHz, MeOD): δ [ppm] = 8,66 (dd, *J* = 7,4 & 1,1 Hz, 1H), 8,51 - 8,37 (m, 2H), 8,30 (d, *J* = 8,3 Hz, 1H), 8,12 (d, *J* = 6,5 Hz, 1H), 7,91 (t, *J* = 7,8 Hz, 1H), 7,77 (dd, *J* = 8,2 & 7,2 Hz, 1H), 4,67 (s, 2H), 4,47 (s, 2H), 3,64 (m, 1H), 3,25 (s, 6H). |
| | | | **MF:** C₁₉H₁₈NOCl | | |
| 3.3 | SAPN-36 | 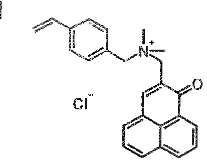 | **MW:** 354,48 + 35,45 = 389,93 g/mol | **MS** (ESI-MS, CH₂Cl₂/MeOH + 10 mmol NH₄OAc): m/z = 354.2 (100%, M+) | **¹H-NMR** (300 MHz, CDCl₃): δ [ppm] = 8,92 (s, 1H), 8,57 (dd, *J* = 7,4 & 0,9 Hz, 1H), 8,25 (d, *J* = 7,3 Hz, 1H), 8,12 (d, *J* = 7,7 Hz, 2H), 7,79 (t, *J* = 7,7 Hz, 1H), 7,66 (m, 3H), 7,57 (m, 2H), 7,43 (d, *J* = 8,1 Hz, 2H), 7,57 (m, 2H), 6,68 (m, 1H), 5,80 (d, *J* = 16,8 Hz, 1H), 5,33 (d, *J* = 10,9 Hz, 1H), 5,15 (s, 2H), 5,03 (s, 2H), 3,19 (s, 6H). |
| | | | **MF:** C₂₅H₂₄NOCl | | |
| 3.4 | SAPN-37a | 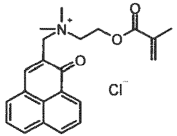 | **MW:** 350,44 + 35,45 = 385,89 g/mol | **MS** (ESI-MS, CH₂Cl₂/MeOH + 10 mmol NH₄OAc): m/z = 350.2 (100%, M+) | **¹H-NMR** (300 MHz, MeOD): δ [ppm] = 8,58 (dd, *J* = 7,4, 1,1 Hz, 1H), 8,52 - 8,32 (m, 2H), 8,25 (dd, *J* = 8,3 & 0,7 Hz, 1H), 8,18 - 8,03 (m, 1H), 7,93 - 7,78 (m, 1H), 7,76 - 7,63 (m, 1H), 6,19 (m, 1H), 5,72 (m, 1H), 4,89 (m, 2H), 4,67 (m, 2H), 3,98 - 3,76 (m, 2H), 3,26 (s, 6H), 1,97 (s, 3H). |
| | | | **MF:** C₂₂H₂₄NO₃Cl | | |
| 3.5 | SAPN-37b | 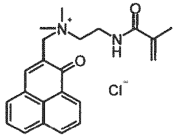 | **MW:** 349,46 + 35,45 = 384,91 g/mol | **MS** (ESI-MS, CH₂Cl₂/MeOH + 10 mmol NH₄OAc): m/z = 349.2 (100%, M+) | Nicht gemessen |
| | | | **MF:** C₂₂H₂₅N₂O₂Cl | | |
| 3.6 | SA-PN-38 | 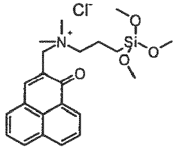 | **MW:** 400,57 + 35,45 = 436,02 g/mol | **MS** (ESI-MS, CH₂Cl₂/MeOH + 10 mmol NH₄OAc): m/z = 358.1 (100%, (M-3Me)+) | **¹H-NMR** (300 MHz, DMSO-d6): δ [ppm] = 8,81 - 7,29 (m, 7H), 5,67 (m, 1H), 4,55 (s, 2H), 3,64 (m, 2H), 3,45 - 2,98 (m, 15H), 2,03 (m, 2H), 0,77 (m, 2H). |
| | | | **MF:** C₂₂H₃₀NO₄SiCl | | |
| 3.7 | SA-PN-25c-boc | 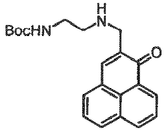 | **MW:** 352,44 g/mol | **MS** (ESI-MS, CH₂Cl₂/MeOH + 10 mmol NH₄OAc): m/z = 353.1 (100%, MH+) | **¹H-NMR** (300 MHz, CDCl₃): δ [ppm] = 8,64 (dd, *J* = 7,4 & 1,0 Hz, 1H), 8,21 (d, *J* = 8,2 Hz, 1H), 8,03 (d, *J* = 8,2 Hz, 1H), 7,92 (s, 1H), 7,79 (dd, *J* = 16,7 & 7,4 Hz, 2H), 7,65 - 7,57 (m, 1H), 5,40 (s, 1H), 3,98 (s, 2H), 3,41 (d, *J* = 5,3 Hz, 2H), 3,03 - 2,95 (m, 2H), 1,42 (s, 9H). |
| | | | **MF:** C₂₁H₂₄N₂O₃ | | |
| 3.8 | SA-PN-25a | 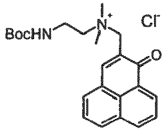 | **MW:** 381,50 + 35,45 = 416,95 g/mol | **MS** (ESI-MS, CH₂Cl₂/MeOH + 10 mmol NH₄OAc): m/z = 381.0 (100%, M+) | **¹H-NMR** (300 MHz, DMSO-d6): δ [ppm] = 8,66 - 8,47 (m, 2H), 8,39 (d, *J* = 8,1 Hz, 1H), 8,18 (d, *J* = 6,8 Hz, 1H), 7,96 (t, *J =* 7,7 Hz, 1H), 7,83 (dd, *J* = 8,1 & 7,3 Hz, 1H), 7,27 (t, *J* = 5,1 Hz, 1H), 4,57 (s, 2H), 3,55 - 3,40 (m, 4H), 3,13 (s, 6H), 1,39 (s, 9H). |
| | | | **MF:** C₂₃H₂₉N₂O₃Cl | | |
| 3.9 | SA-PN- 34a | 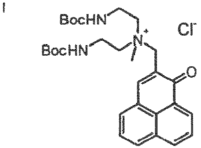 | **MW:** 510,66 + 35,45 = 546,12 g/mol | **MS** (ESI-MS, CH₂Cl₂/MeOH + 10 mmol NH₄OAc): m/z = 510.3 (100%, M+) | **¹H-NMR** (300 MHz, CDCl₃): δ [ppm] = 8,89 (s, 1H), 8,58 (d, *J* = 7,3 Hz, 1H), 8,31 - 8,02 (m, 3H), 7,78 (t, *J* = 7,7 Hz, 1H), 7,66 (t, *J* = 7,7 Hz, 1H), 6,53 (s, 2H), 4,82 (s, 2H), 3,98 - 3,58 (m, 8H), 3,22 (s, 3H), 1,39 (s, 18H). |
| | | | **MF:** C₂₉H₄₀N₃O₅Cl | | |
| 3.10 | SA-PN- 11 | 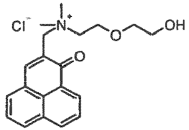 | **MW:** 326,4 + 35,45 = 361,85 g/mol | **MS** (ESI-MS, CH₂Cl₂/MeOH + 10 mmol NH₄OAc): e/z (%) = 326.2 (100, M⁺); | **¹H-NMR** (300 MHz, CDCl₃): δ [ppm] = 8,69 (s, 1H), 8,48 - 8,34 (m, 1H), 8,17 - 7,91 (m, 3H), 7,65 (t, *J* = 7,6 Hz, 1H), 7,56 (t, *J* = 7,7 Hz, 1H), 5,03 - 4,78 (bs, 1H), 4,81 (s, 2H), 4,06 (m, 2H), 3,84 (m, 2H), 3,69 (m, 2H), 3,63 (m, 2H), 3,30 (s, 6H). |
| | | | **MF:** C₂₀H₂₄NO₃Cl | | |
| 3.11 | SA-PN- 12 | 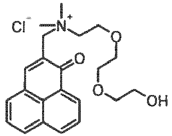 | **MW:** 370,5 + 35,45 = 405,95 g/mol | **MS** (ESI-MS, CH₂Cl₂/MeOH + 10 mmol NH₄OAc): e/z (%) = 370.2 (100, M⁺); | **¹H-NMR** (300 MHz, DMSO): δ [ppm] = 8,65 - 8,45 (m, 3H), 8,39 (d, *J* = 8,0 Hz, 1H), 8,17 (d, *J* = 6,5 Hz, 1H), 7,96 (t, *J* = 7,7 Hz, 1H), 7,83 (dd, *J* = 8,2 & 7,2 Hz, 1H), 4,60 (s, 2H), 3,97 (s, 2H), 3,75 - 3,41 (m, 13H), 3,14 (s, 6H). |
| | | | **MF:** C₂₂H₂₈NO₄Cl | | |
| 3.12 | SA-PN- 09 | 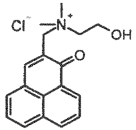 | **MW:** 282,4 + 35,45 = 317,85 g/mol | **MS** (ESI-MS, CH₂Cl₂/MeOH + 10 mmol NH₄OAc): e/z (%) = 282.1 (100, M⁺); | **¹H-NMR** (300 MHz, DMSO-d6): δ[ppm] = 8,58 - 8,54 (dd, *J* = 1,0 & 7,3 Hz, 1H), 8,55 - 8,50 (dd, *J* = 1,2 & 7,4 Hz, 1H), 8,52 (s, 1H), 8,37 (dd, *J* = 1,0 & 7,8 Hz, 1H), 8,18 (dd, *J* = 0,9 Hz, *J* = 7,1 Hz, 1H), 7,95 (t, *J* = 7,7 Hz, 1H), 7,84 - 7,78 (m, 1H), 5,62 (t, *J* = 5,1 Hz, 1H), 4,61 (s, 2H), 3,99 - 3,88 (m, 2H), 3,59 - 3,51 (m, 2H), 3,15 (s,6H). |
| | | | **MF:** C₁₈H₂₀NO₂Cl | | |
| 4 | PNOH | 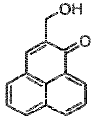 | **MW:** 210,33 g/mol | **MS** (ESI-MS, CH₂Cl₂/MeOH + 10 mmol NH₄OAc): m/z = 211.1 (100%, MH+), 193.1 (64%, MH+ - H₂O) | **¹H-NMR** (300 MHz, CDCl₃): δ [ppm] = 8,66 (dd, *J* = 7,4 & 1,2 Hz, 1H), 8,22 (dd, *J* = 8,1, 1,1 Hz, 1H), 8,01 (m, 2H), 7,89 - 7,74 (m, 2H), 7,62 (dd, *J* = 8,2 & 7,1 Hz, 1H), 4,82 (d, *J* = 1,4 Hz, 2H). |
| | | | **MF:** C₁₄H₁₀NO₂ | | |
| 5.1 | PN-AMO-07a | 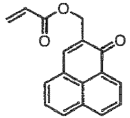 | **MW:** 264,28 g/mol | **MS** (ESI-MS, CH₂Cl₂/MeOH + 10 mmol NH₄OAc): m/z = 265.1 (100%, MH+) | **¹H-NMR** (300 MHz, CDCl₃): δ [ppm] = 8,67 (d, *J* = 7,4 Hz, 1H), 8,23 (d, *J* = 8,0 Hz, 1H), 8,03 (d, *J* = 8,4 Hz, 2H), 7,87 - 7,76 (m, 2H), 7,65 - 7,59 (m, 1H), 6,64 (d, *J* = 16,6 Hz, 1H), 6.35 (dd, *J* = 16,6, 10,1 Hz, 1H), 6,18 (d, *J* = 10,1 Hz, 1H), 4,83 (s, 2H). |
| | | | **MF:** C₁₇H₁₂O₃ | | |
| 5.2 | PN-AMO-07b | 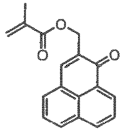 | **MW:** 278,31 g/mol | **MS** (ESI-MS, CH₂Cl₂/MeOH + 10 mmol NH₄OAc): m/z = 279.1 (100%, MH+) | Nicht gemessen |
| | | | **MF:** C₁₈H₁₄O₃ | | |
| 5.3 | PN-AMO-08 | 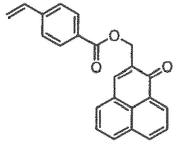 | **MW:** 340,38 g/mol | **MS** (ESI-MS, CH₂Cl₂/MeOH + 10 mmol NH₄OAc): m/z = 341.1 (100%, MH+) | **¹H-NMR** (300 MHz, CDCl₃): δ [ppm] = 8,69 (d, *J* = 7,4 Hz, 1H), 8,25 (d, *J* = 8,1 Hz, 1H), 8,22 (d, *J* = 8,4 Hz, 2H), 8,05 (d, *J* = 8,4 Hz, 2H), 7,89 - 7,74 (m, 2H), 7,67 - 7,62 (m, 1H), 7,58 (d, *J* = 8,3 Hz, 2H), 6,90 - 6,73 (m, 2H), 5,94 (d, *J* = 17,5 Hz, 1H), 5,48 (d, *J* = 10,8 Hz, 1H), 4,85 (s, 1H). |
| | | | **MF:** C₂₃H₁₆O₃ | | |
| 6.1 | PN-AMO-04a | 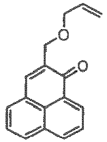 | **MW:** 250,30 g/mol | **MS** (ESI-MS, CH₂Cl₂/MeOH + 10 mmol NH₄OAc): m/z = 193.1 (52%, MH+ -C3H6O), 251.1 (100%, MH+), 273.1 (13%, MNa+) | **¹H-NMR** (300 MHz, CDCl₃): δ [ppm] = 8,63 (d, *J* = 7,4 Hz, 1H), 8,19 (d, *J* = 8,1 Hz, 1H), 7,99 (d, *J* = 8,3 Hz, 1H), 7,85 (m, 1H), 7,75 (m, 2H), 7,58 (t, *J* = 7,7 Hz, 1H), 6,06 (m, 1H), 5,40 (dd, *J* = 17,2 & 1,6 Hz, 1H), 5,26 (dd, *J* = 10,4 & 1,1 Hz, 1H), 4,61 (s, 2H), 4,20 (d, *J* = 5,6 Hz, 2H). |
| | | | **MF:** C₁₇H₁₄O₂ | | |
| 6.2 | PN-AMO-04b | 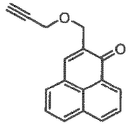 | **MW:** 248,28 g/mol | **MS** (ESI-MS, CH₂Cl₂/MeOH + 10 mmol NH₄OAc): m/z = 249.1 (100%, MH+) | **¹H-NMR** (300 MHz, CDCl₃): δ [ppm] = 8,66 (dd, *J* = 7,4, 1,1 Hz, 1H), 8,22 (dd, *J* = 8,0, 0,9 Hz, 1H), 8,02 (d, *J* = 8,1 Hz, 1H), 7,87 (t, *J* = 1,4 Hz, 1H), 7,79 (t, *J* = 7,7 Hz, 2H), 7,61 (dd, *J* = 8,2, 7,2 Hz, 1H), 4,71 (d, *J* = 1,5 Hz, 2H), 4,37 (d, *J* = 2,4 Hz, 2H), 2,51 (t, *J* = 2,4 Hz, 1H). |
| | | | **MF:** C₁₇H₁₂O₂ | | |
| 6.3 | PN-AMO-06 | 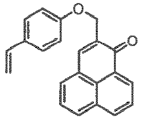 | **MW:** 312,37 g/mol | **MS** (ESI-MS, CH₂Cl₂/MeOH + 10 mmol NH₄OAc): m/z = 313.1 (100%, MH+) | **¹H-NMR** (300 MHz, CDCl₃): δ [ppm] = 8,70 (d, *J* = 6,4 Hz, 1H), 8,25 (d, *J* = 8,1 Hz, 1H), 8,04 (d, *J* = 8,1 Hz, 1H), 7,95 (s, 1H), 7,86 - 7,78 (m, 2H), 7,66 - 7,58 (m, 1H), 7,38 (d, *J* = 8,7 Hz, 2H), 7,03 (d, *J* = 8,7 Hz, 2H), 6,67 (dd, *J* = 17,6, 10,9 Hz, 1H), 5,63 (d, *J* = 17,6 Hz, 1H), 5,20 (d, *J* = 1,5 Hz, 2H), 5,14 (d, *J* = 11,0 Hz, 1H). |
| | | | **MF:** C₂₂H₁₆O₂ | | |
| 6.4 | PN-AMO-05 | 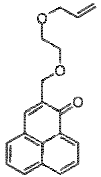 | **MW:** 294,35 g/mol | **MS** (ESI-MS, CH₂Cl₂/MeOH + 10 mmol NH₄OAc): m/z = 193.1 (27%, MH+ -C5H10O2), 295.1 (100%, MH+), 317.1 (10%, MNa+) | **¹H-NMR** (300 MHz, CDCl₃): δ [ppm] = 8,63 (dd, *J* = 7,4 & 1,1 Hz, 1H), 8,19 (dd, *J* = 8,1 & 0,9 Hz, 1H), 7,99 (d, *J* = 8,1 Hz, 1H), 7,89 (t, *J =* 1,5 Hz, 1H), 7,76 (t, *J* = 7,6 Hz, (ddd, *J* = 17,2 *J* = & 1H), 5,94 (m, 1H), 5,32 2H), 7,58 (dd, & 3,2 & 1,6 Hz, 1H), 5,26 - 5,16 (m, 1H), 4,66 (d, *J* = 1,5 Hz, 2H), 4,09 (dt, *J* = 5,6 & 1,3 Hz, 2H), 3,83 (m, 2H), 3,72 (m, 2H). |
| | | | **MF:** C₁₉H₁₈O₃ | | |
| 6.5 | PN-AMO-10 | 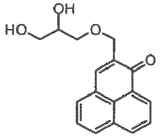 | **MW:** 284,31 g/mol | **MS** (ESI-MS, CH₂Cl₂/MeOH + 10 mmol NH₄OAc): m/z = 285.1 (100%, MH+), 263.1 (29%, MH+ - H₂O) | **¹H-NMR** (300 MHz, CDCl₃): δ [ppm] = 8,62 (d, *J* = 7,6 Hz, 1H), 8,20 (d, *J* = 8,1 Hz, 1H), 7,97 (d, *J* = 8,2 Hz, 1H), 7,87 (s, 1H), 7,76 (t, *J* = 7,5 Hz, 2H), 7,56 (t, *J* = 7,6 Hz, 1H), 4,61 (s, 2H), 4,12 - 3,56 (m, 7H). |
| | | | **MF:** C₁₇H₁₆O₄ | | |
| 6.6 | PN-AMO-02b | 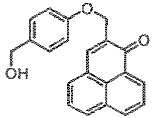 | **MW:** 316,36 g/mol | **MS** (ESI-MS, CH₂Cl₂/MeOH + 10 mmol NH₄OAc): m/z = 317.1 (100%, MH+) | **¹H-NMR** (300 MHz, CDCl₃): δ [ppm] = 8,69 (d, *J* = 7,4 Hz, 1H), 8,23 (d, *J* = 8,1 Hz, 1H), 7,98 (d, *J* = 8,1 Hz, 1H), 7,93 (s, 1H), 7,78 - 7,73 (m, 2H), 7,62 - 7,51 (m, 1H), 7,01 (d, *J* = 7,8 Hz, 2H), 6,78 (d, *J* = 7,8 Hz, 2H), 5,26 (m, 2H), 4,46 (s, 2H). |
| | | | **MF:** C₂₁H₁₆O₃ | | |
| 6.7 | PN-AMO-02a | 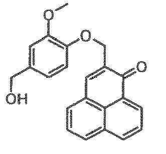 | **MW:** 346,39 g/mol | **MS** (ESI-MS, CH₂Cl₂/MeOH + 10 mmol NH₄OAc): m/z = 347.1 (100%, MH+), 369.1 (9%, MNa+) | **¹H-NMR** (300 MHz, CDCl₃): δ [ppm] = 8,67 (dd, *J* = 7,3 & 0,9 Hz, 1H), 8,21 (d, *J* = 8,0 Hz, 1H), 8,01 (d, *J* = 8,2 Hz, 1H), 7,94 (s, 1H), 7,78 (dd, *J* = 10,8 & 4,4 Hz, 2H), 7,65 - 7,52 (m, 1H), 6,97 - 6,89 (m, 2H), 6,84 (dd, *J* = 8,2 & 1,7 Hz, 1H), 5,24 (d, *J* = 1,3 Hz, 2H), 4,63 (s, 2H), 3,95 (s, 3H). |
| | | | **MF:** C₂₂H₁₈O₄ | | |
| 7.1 | PN-AMO-14a | 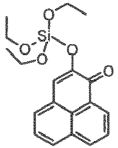 | **MW:** 372,50 g/mol | **MS** (ESI-MS, CH₂Cl₂/MeOH + 10 mmol NH₄OAc): m/z = 373.1 (13%, MH+), 286.1 (100%, (M -3 C₂H₅)+) | **¹H-NMR** (300 MHz, CDCl₃): δ [ppm] = 8,67 (d, *J* = 7,4 Hz, 1H), 8,22 (d, *J* = 7,5 Hz, 1H), 8,03 (d, *J* = 8,6 Hz, 2H), 7,90 - 7,74 (m, 2H), 7,67 - 7,57 (m, 1H), 4,82 (s, 2H), 3,76 - 3,69 (m, 6H), 1,26 (d, *J* = 7,0 Hz, 9H). |
| | | | **MF:** C₂₀H₂₄O₅Si | | |
| 7.2 | PN-AMO- 14b | 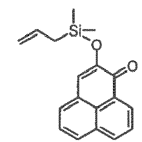 | **MW:** 308,46 g/mol | **MS** (ESI-MS, CH₂Cl₂/MeOH + 10 mmol NH₄OAc): m/z = 309.1 (26%, MH+), 193.1 (100%, (M -H₂O - C₅H₁₁Si)+) | **¹H-NMR** (300 MHz, CDCl₃): δ [ppm] = 8,67 (d, *J* = 7,4 Hz, 1H), 8,22 (d, *J* = 8,2 Hz, 1H), 8,03 (d, *J* = 8,4 Hz, 2H), 7,90 - 7,74 (m, 2H), 7,68 - 7,57 (m, 1H), 4,98 (dd, *J* = 28,1, 14,7 Hz, 1H), 4,83 (s, 2H), 1,72 (dt, *J* = 6,4, 1,6 Hz, 1H), 0,51 (d, *J* = 5,2 Hz, 1 H), 0,23 - 0,07 (m, 6H). |
| | | | **MF:** C₁₉H₂₀O₂Si | | |
| 8.1 | PN-AMO-12a | 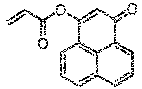 | **MW:** 250,26 g/mol | **MS** (ESI-MS, CH₂Cl₂/MeOH + 10 mmol NH₄OAc): m/z = 251.1 (100%, MH+) | **¹H-NMR** (300 MHz, CDCl₃): δ [ppm] = 8,70 (d, *J* = 7,3 Hz, 1H), 8,57 (d, *J* = 6,6 Hz, 1H), 8,27 (d, *J* = 8,0 Hz, 1H), 8,19 (d, *J* = 7,8 Hz, 1H), 8,11 (s, 1H), 7,79 (dt, *J* = 21,7, 7,8 Hz, 2H), 6,70 (dd, *J* = 17,3, 1,1 Hz, 1H), 6,41 (dd, *J* = 17,3 & 10,4 Hz, 1H), 6,11 (dd, *J* = 10,4 & 1,1 Hz, 1H). |
| | | | **MF:** C₁₆H₁₀O₃ | | |
| 8.2 | PN-AMO-12b | 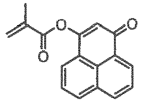 | **MW:** 264,28 g/mol | **MS** (ESI-MS, CH₂Cl₂/MeOH + 10 mmol NH₄OAc): m/z = 265.1 (100%, MH+) | Nicht gemessen |
| | | | **MF:** C₁₇H₁₂O₃ | | |
| 8.3 | PN-AMO-12c | 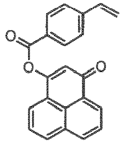 | **MW:** 326,36 g/mol | **MS** (ESI-MS, CH₂Cl₂/MeOH + 10 mmol NH₄OAc): m/z = 327.1 (100%, MH+) | **¹H-NMR** (300 MHz, CDCl₃): δ [ppm] = 8,55 (d, *J* = 7,3 Hz, 1H), 8,19 (d, *J* = 8,3 Hz, 2H), 8,12 (d, *J* = 8,0 Hz, 1H), 8,00 (d, *J* = 8,2 Hz, 1H), 7,91 (d, *J* = 7,2 Hz, 1H), 7,69 (t, *J* = 7,7 Hz, 1H), 7,61 - 7,48 (m, 3H), 6,91 - 6,60 (m, 2H), 5,92 (d, *J* = 17,6 Hz, 1H), 5,45 (d, *J* = 10,9 Hz, 1H). |
| | | | **MF:** C₂₂H₁₄O₃ | | |
| 9.1 | PN-AMO-11a | 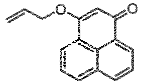 | **MW:** 236,27 g/mol | **MS** (ESI-MS, CH₂Cl₂/MeOH + 10 mmol NH₄OAc): m/z = 237.1 (100%, MH+) | **¹H-NMR** (300 MHz, CDCl₃): δ [ppm] = 8,59 (dd, *J* = 7,3, 1,1 Hz, 1H), 8,31 (dd, *J* = 7,3, 0,9 Hz, 1H), 8,22 - 8,14 (m, 1H), 8,08 (d, *J* = 7,5 Hz, 1H), 7,78 - 7,57 (m, 2H), 6,23 - 6,08 (m, 2H), 5,55 (dd, *J* = 17,3, 1,4 Hz, 1H), 5,42 (dd, *J* = 10,5, 1,2 Hz, 1H), 4,73 (d, *J* = 5,4 Hz, 2H). |
| | | | **MF:** C₁₆H₁₂O₂ | | |
| 9.2 | PN-AMO-11b | 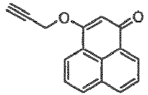 | **MW:** 234,26 g/mol | **MS** (ESI-MS, CH₂Cl₂/MeOH + 10 mmol NH₄OAc): m/z = 235.1 (100%, MH+) | **¹H-NMR** (300 MHz, CDCl₃): δ [ppm] = 8,56 (d, *J* = 7,2 Hz, 1H), 8,47 (d, *J* = 7,3 Hz, 1H), 8,20 (d, *J* = 8,0 Hz, 1H), 8,10 (d, *J* = 8,1 Hz, 1H), 7,81 - 7,63 (m, 2H), 7,57 (s, 1H), 4,35 - 4,10 (m, 2H), 2,63 (s, 1H). |
| | | | **MF:** C₁₆H₁₀O₂ | | |
| 9.3 | PN-AMO-13 | 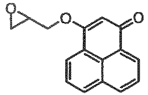 | **MW:** 252,27 g/mol | **MS** (ESI-MS, CH₂Cl₂/MeOH + 10 mmol NH₄OAc): m/z = 253.1 (100%, MH+) | **¹H-NMR** (300 MHz, CDCl₃): δ [ppm] = 8,59 (dd, *J* = 7,1 & 0,9 Hz, 1H), 8,29 (m, 2H), 8,17 (d, *J* = 7,6 Hz, 1H), 7,47 (t, *J* = 7,8 Hz, 1H), 7,31 (t, *J* = 7,8 Hz, 1H), 7,16 (m, 1H), 4,18 (dd, *J* = 11,4 & 4,6 Hz, 1H), 3,93 (dd, *J* = 11,2 & 5,8 Hz, 1H), 3,31 (m, 1H), 2,88 (m, 1H), 2,69 (m, 1H). |
| | | | **MF:** C₁₆H₁₂O₃ | | |
| 9.4 | PN-AMO-03a | 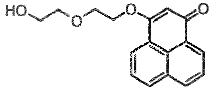 | **MW:** 284,31 g/mol | **MS** (ESI-MS, CH₂Cl₂/MeOH + 10 mmol NH₄OAc): m/z = 285.1 (100%, MH+) | **¹H-NMR** (300 MHz, CDCl₃): δ [ppm] = 8,26 (dd, *J* = 7,2 & 0,8 Hz, 1H), 7,94 - 7,74 (m, 2H), 7,66 (d, *J* = 7,9 Hz, 1H), 7,43 (t, *J* = 7,7 Hz, 1H), 7,23 (t, *J* = 7,8 Hz, 1H), 5,79 (s, 1H), 4,13 - 3,97 (m, 2H), 3,88 - 3,79 (m, 2H), 3,77 - 3,69 (m, 2H), 3,68 - 3,51 (m, 3H). |
| | | | **MF:** C₁₇H₁₆O₄ | | |
| 9.5 | PN-AMO-03b | 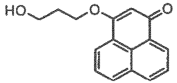 | **MW:** 254,29 g/mol | **MS** (ESI-MS, CH₂Cl₂/MeOH + 10 mmol NH₄OAc): m/z = 255.1 (100%, MH+) | **H-NMR** (300 MHz, MeOD): δ [ppm] = 8,66 - 8,58 (m, 1H), 8,45 (m, 2H), 8,27 (m, 1H), 7,96 - 7,72 (m, 3H), 3,22 (t, *J* = 7,1 Hz, 2H), 2,47 (m, 2H), 1,81 (m, 2H). |
| | | | **MF:** C₁₆H₁₄O₃ | | |
| 9.6 | SAPN-32boc | 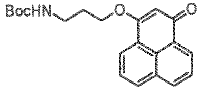 | **MW:** 353,42 g/mol | **MS** (ESI-MS, CH₂Cl₂/MeOH + 10 mmol NH₄OAc): m/z = 354.2 (100%, MH+) | **¹H-NMR** (300 MHz, CDCl₃): δ [ppm] = 8,56 (dd, *J* = 7,3 & 1,0 Hz, 1H), 8,22 (d, *J* = 7,2 Hz, 1H), 8,19 - 8,11 (m, 1H), 8,05 (d, *J* = 7,9 Hz, 1H), 7,72 (t, *J* = 7,7 Hz, 1H), 7,67 - 7,55 (m, 1H), 6,13 (s, 1H), 4,81 (s, 1H), 4,22 (t, *J* = 5,9 Hz, 2H), 3,43 (s, 2H), 2,14 (p, *J* = 6,3 Hz, 2H), 1,45 (s, 9H). |
| | | | **MF:** C₂₁H₂₃NO₄ | | |
| 10 | SAPN-02c | 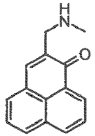 | **MW:** 223,28 g/mol | **MS** (ESI-MS, CH₂Cl₂/MeOH + 10 mmol NH₄OAc): m/z = 223.1 (100%, M+) | **H-NMR** (300 MHz, MeOD): δ [ppm] = 8,68 (dd, *J* = 7,4 & 1,0 Hz, 1H), 8,51 - 8,38 (m, 1H), 8,27 (d, *J* = 7,9 Hz, 1H), 8,20 (s, 1H), 8,07 (d, *J* = 7,0 Hz, 1H), 7,91 (t, *J* = 7,8 Hz, 1H), 7,76 (dd, *J* = 8,2 & 7,2 Hz, 1H), 4,21 (s, 2H), 2,79 (s, 3H). |
| | | | **MF:** C₁₅H₁₃NO | | |
| 11.1 | PN-AMO-09a | 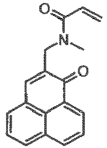 | **MW:** 291,35 g/mol | **MS** (ESI-MS, CH₂Cl₂/MeOH + 10 mmol NH₄OAc): m/z = 292.1 (100%, MH+) | **1H-NMR** (300 MHz, CDCI3): δ [ppm] = 8,72 - 8,59 (m, 1H), 8,30 - 8,16 (m, 1H), 8,02 (dd, *J* = 14,3 & 8,2 Hz, 1H), 7,85 - 7,72 (m, 2H), 7,60 (m, 1H), 7,47 (s, 1H), 6,62 (m, 1H), 6,41 (m, 1H), 5,70 (m, 1H), 4,72 - 4,60 (m, 2H), 3,20 (d, *J* = 27,8 Hz, 3H). |
| | | | **MF:** C₁₉H₁₇NO₂ | | |
| 11.2 | PN-AMO-09b | 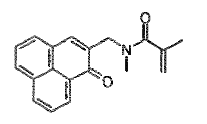 | **MW:** 277,33 g/mol | **MS** (ESI-MS, CH₂Cl₂/MeOH + 10 mmol NH₄OAc): m/z = 278.1 (100%, MH+) | Nicht gemessen |
| | | | **MF:** C₁₈H₁₅NO₃ | | |
| 12 | PN-AMO-01 | 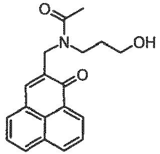 | **MW:** 309,37 g/mol | **MS** (ESI-MS, CH₂Cl₂/MeOH + 10 mmol NH₄OAc): m/z = 310.1 (100%, MH+) | **¹H-NMR** (300 MHz, CDCl₃): δ [ppm] = 8,69 (dd, *J* = 1,2 & 7,4 Hz, 1H), 8,26 (dd, *J* = 1,0 & 8,1 Hz, 1H), 8,08 (dd, *J* = 0,6 & 8,2 Hz, 1H), 7,78 - 7,90 (m, 2H), 7,62 (dd, *J* = 7,0 & 1,2 Hz, 1H), 7,51 (s, 1H), 4,65 (s, 2H), 3,83 (bs, 1H), 3,66 (t, *J* = 5,6 Hz, 2H), 3,59 (t, *J* = 5,6 Hz, 2H), 2,16 (s, 3H), 1,78 (m, 2H). |
| | | | **MF:** C₁₉H₁₉NO₃ | | |
| 13.1 | SA-PN-25c | 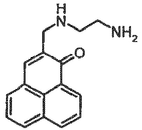 | **MW:** 252,32 g/mol | **MS** (ESI-MS, CH₂Cl₂/MeOH + 10 mmol NH₄OAc): m/z = 253.1 (100%, MH+) | **H-NMR** (300 MHz, MeOD): δ [ppm] = 8,71 (dd, *J* = 7,4 & 1,0 Hz, 1H), 8,46 (d, *J* = 8,1 Hz, 1H), 8,29 (t, *J* = 3,9 Hz, 2H), 8,10 (d, *J* = 7,1 Hz, 1H), 7,92 (t, *J* = 7,8 Hz, 1H), 7,77 (dd, *J* = 8,2 & 7,2 Hz, 1H), 4,33 (s, 2H), 3,46 (m, 4H). |
| | | | **MF:** C₁₆H₁₆N₂O | | |
| 13.2 | SA-PN-25b | 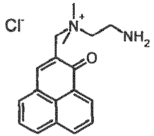 | **MW:** 281,38 + 35,45 = 316,83 g/mol | **MS** (ESI-MS, CH₂Cl₂/MeOH + 10 mmol NH₄OAc): m/z = 281.1 (100%, M+) | **¹H-NMR** (300 MHz, DMSO-d6): δ [ppm] = 8,68 (s, 3H), 8,57 (dd, *J* = 10,0 & 6,5 Hz, 3H), 8,40 (d, *J* = 8,3 Hz, 1H), 8,22 (d, *J* = 7,0 Hz, 1H), 7,97 (t, *J* = 7,7 Hz, 1H), 7,91 - 7,76 (m, 1H), 4,62 (s, 2H), 3,77 - 3,65 (m, 2H), 3,43 (m, 2H), 3,20 (s, 6H). |
| | | | **MF:** C₁₈H₂₁N₂OCl | | |
| 13.3 | SA-PN-34b | 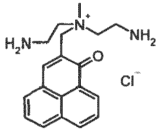 | **MW:** 310,42 + 35,45 = 345, 87 g/mol | **MS** (ESI-MS, CH₂Cl₂/MeOH + 10 mmol NH₄OAc): m/z = 310.1 (100%, M+) | **H-NMR** (300 MHz, MeOD): δ [ppm] = 8,74 (dd, *J* = 7,5 & 0,9 Hz, 1H), 8,47 (d, *J* = 8,0 Hz, 1H), 8,31 (t, *J* = 3,8 Hz, 2H), 8,12 (d, *J* = 7,2 Hz, 1H), 7,93 (t, *J* = 7,6 Hz, 1H), 7,79 (dd, *J* = 8,2 & 7,4 Hz, 1H), 4,35 (s, 2H), 3,58 - 3,35 (m, 8H). |
| | | | **MF:** C₁₉H₂₄N₃O | | |
| 13.4 | SAPN-32 | 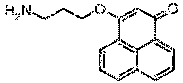 | **MW:** 253,30 g/mol | **MS** (ESI-MS, CH₂Cl₂/MeOH + 10 mmol NH₄OAc): m/z = 254.1 (100%, MH+) | **H-NMR** (300 MHz, MeOD): δ [ppm] = 8,62 - 8,50 (m, 1H), 8,42 (m, 2H), 8,26 (m, 1H), 7,98 - 7,70 (m, 3H), 3,05 (t, *J* = 7,0 Hz, 2H), 2,88 - 2,78 (m, 2H), 1,86 (dd, *J* = 9,5 & 3,6 Hz, 2H). |
| | | | **MF:** C₁₆H₁₅NO₂ | | |

### Beispiel 2: Herstellung von antimikrobiellen Oberflächenbeschichtungen

Die in Beispiel 1 hergestellten Photosensibilisatoren wurden nachfolgend in verschiedenen Lacksystemen getestet.

### 1) 2 Komponenten (2K) Polyurethanlack, Lösungsmittel-haltig

Der jeweilige Photosensibilisator (0,06 mmol) wurde in 100 mL klarem Grundlack (polyisocyanatvernetzbares, hydroxylgruppenhaltiges Acrylharz, 20% in Xylol/n-Butylacetat) gelöst. Der Vernetzer (Hexamethylendiisocyanat (HDI) - haltiges Polymer, 20% in Xylol/n-Butylacetat, "Desmodur^{®} N75" von Covestro AG, Leverkusen, DE) wurde im Verhältnis 5:1 (V/V) mit dem Grundlack gemischt. Die viskose, blassgelbe Lösung wurde mittels Spritzpistole auf verschiedene entfettete Oberfläche aufgetragen. Getestet Oberflächen waren PMMA, PVC, Glas, Aluminium, Edelstahl und Holz. Nach Trocknen für 4h bei Raumtemperatur wurde die vollständige Aushärtung durch Erwärmen auf 60°C für 30 min erreicht.

Folgende Photosensibilisatoren wurden durch diese Methode verarbeitet:

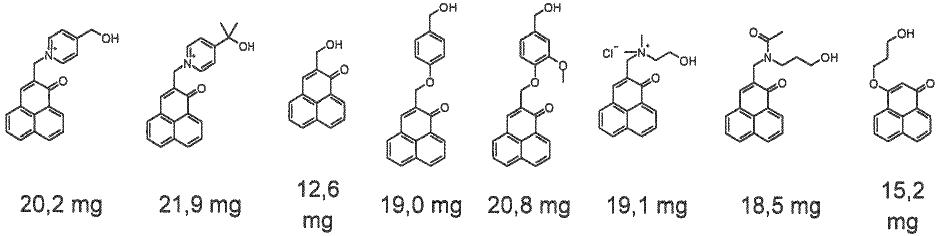

**Ergebnis:** Transparente, blassgelbe, homogene Beschichtung; Schichtstärke zwischen 20 und 40 µm

### 2) 1 Komponenten (1K) Polyurethanlack, Wasserbasiert

Der jeweilige Photosensibilisator (0,05 mmol) wurde dem wasserbasierten Lack (100 mL, Acryl-Polyurethan-Dispersion, fettsäuremodifiziert, 20% in Wasser) zugelöst. Die Mischung wurde 30 min bei Raumtemperatur lebhaft gerührt. Der Lack wurde auf i) ein unbehandeltes Holzbrett, ii) auf PMMA-Platten oder iii) PVC-Platten mit einem Pinsel gleichmäßig aufgebracht. Alternativ wurde der Lack mit einer Sprühpistole aus ca. 30cm Abstand gleichmäßig appliziert. Man ließ die Beschichtung an der Luft antrocknen und über Nacht bei Raumtemperatur aushärten.

Folgende Photosensibilisatoren wurden durch diese Methode verarbeitet:

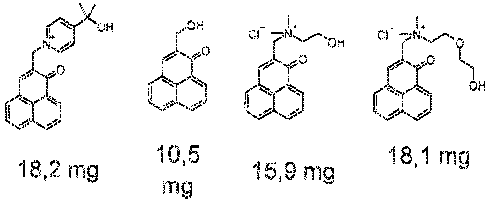

**Ergebnis:** Transparente, matte, blassgelbe, homogene Beschichtung; Schichtstärke ca. 50 µm.

### 3) 2K Epoxidharze

### Zusammensetzung des Grundharzes (typische technische Produkte):

| | |
|---|---|
| *Rezeptur I (Harz mit Reaktivverdünner):* | Bisphenol-A-Epichlorhydrin-Harz mit durchschnittlichem Molekulargewicht < 700 g/mol (25-50%) |
| | C12-C14 Aliphatischer Glycidylether (25-50%) |
| | Alkylglycidylether (5-25%) |
| *Rezeptur II (Harz mit Reaktivverdünner):* | Bisphenol-A-Epichlorhydrin-Harz mit durchschnittlichem Molekulargewicht < 700 g/mol (60-80%) |
| | 1,4-Bis(2,3-epoxypropox)butan (20-40%) |
| *Rezeptur III (Harz mit Reaktivverdünner):* | Bisphenol-A-Epichlorhydrin-Harz mit durchschnittlichem Molekulargewicht < 700 g/mol (75-90%) |
| | Hexandioldiglycidylether (10-25%) |
| *Rezeptur IV (Harz mit Reaktivverdünner):* | Bisphenol-A-Epichlorhydrin-Harz mit durchschnittlichem Molekulargewicht < 700 g/mol (50-90%) |
| | Bisphenol F Epoxidharz (25 - 50%) |
| | C12-C14 Aliphatischer Glycidylether (10-20%) |
| | 1,4-Bis(2,3-epoxypropoxy)butan (10-20%) |

### Zusammensetzung des Härters (typische technische Produkte):

| | |
|---|---|
| *Rezeptur I (Normalhärter):* | Isophorondiamin (Aminomethyl-3,5,5-trimethyl-cyclohexylamin) (10-25%) |
| | Benzylalkohol (25-50%) |
| | 2,2,4-Trimethylhexan-1,6-diamin (3-10%) |
| *Rezeptur II (Normalhärter):* | Isophorondiamin (Aminomethyl-3,5,5-trimethyl-cyclohexylamin) (44%) |
| | Xylidendiamin (10%) |
| | Trimethylhexamethylendiamin (5%) |
| | Salicylsäure (1%) |
| | Polyethylenamin (10%) |
| | Benzylalkohol (30%) |
| *Rezeptur III (Schnellhärter):* | Diaminocyclohexan (18%) |
| | Pentamethylendiamin (25%) |
| | Salicylsäure (2%) |
| | Polyethylenamin (20%) |
| | Benzylalkohol (35%) |

| | |
|---|---|
| Optional mit Beschleuniger/Katalysator: | N-Benzyldimethylamin (BDMA) und/oder |
| N,N,N,N-Tetramethyl-1.3 Butandiamin (TMBDA) | |
| und/oder | |
| 2-Methyl Imidazol (2MI) | |

### Variante a)

Der jeweilige Photosensibilisator (0,05 mmol) wurde in 25 mL des klaren Härters (Rezeptur I oder II) gelöst. Vom Grundharz (Rezeptur II, III oder IV) wurden 75 mL mit dem Härter gemischt. Die viskose, blassgelbe Lösung wurde mittels Spritzpistole auf entfettete Oberfläche aus i) PMMA oder ii) PVC aufgetragen.

Alternativ wurde die Mischung mit einem Pinsel auf unbehandeltes Holz gleichmäßig in mehreren Wiederholungen aufgetragen, um eine antimikrobielle Versiegelung zu erhalten. Nach Aushärten für 12h bei Raumtemperatur wurde durch Erwärmen auf 40°C für 6h eine Nachhärtung durchgeführt.

Folgende Photosensibilisatoren wurden durch diese Methode verarbeitet:

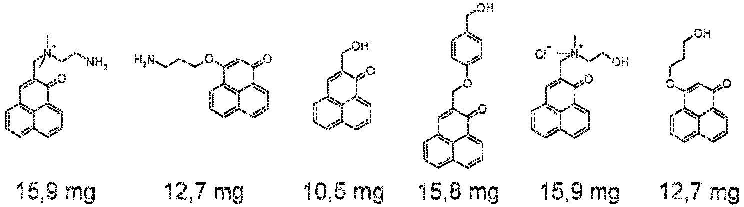

**Ergebnis:** Transparente, blassgelbe, homogene Beschichtung; Schichtstärke zwischen 20 und 40 µm

### Variante b)

Der jeweilige Photosensibilisator (0,05 mmol) wurde in 30 mL des klaren Härters (Rezeptur II oder III) gelöst. Vom Grundharz (Rezeptur I) wurden 70mL mit dem Härter gemischt. Eine flache Edelstahlwanne wurde mit Trennwachs gleichmäßig bestrichen. Darin wurde i) eine Glasfasermatte, ii) eine Stück CFK-Gewebe oder iii) ein Stück Aramidgewebe (je 20x20 cm²) mit einem Pinsel gleichmäßig mit der Harzmischung durchtränkt, so dass das Gewebe die Mischung vollständig aufnehmen konnte. Nach Aushärten für 24h bei Raumtemperatur wurde durch langsames Erwärmen auf 60°C und halten für 30 min eine Nachhärtung durchgeführt.

Folgende Photosensibilisatoren wurden durch diese Methode verarbeitet:

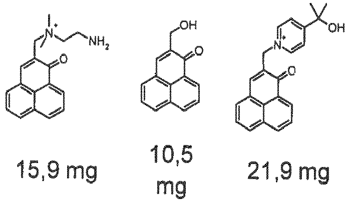

**Ergebnis:** Zähe, begrenzt flexible Fasermatte; Eigenfarbe des Farbstoffes ist nicht zu erkennen

### Variante c)

Der jeweilige Photosensibilisator (0,05 mmol) wurde in 70 mL des Grundharzes (Rezeptur II, III oder IV) gelöst. Vom Härter (Rezeptur III) wurden 30mL zugemischt. Die viskose, blassgelbe Lösung wurde mittels Spritzpistole auf entfettete Oberfläche aus i) PMMA oder ii) aufgerautem Glas aufgetragen.

Alternativ wurde die Mischung mit einem Pinsel auf unbehandeltes Holz gleichmäßig in mehreren Wiederholungen aufgetragen, um eine antimikrobielle Versiegelung zu erhalten. Nach Aushärten für 6h bei Raumtemperatur wurde durch Erwärmen auf 40°C für 3h eine Nachhärtung durchgeführt. Folgende Vertreter wurden durch diese Methode verarbeitet:

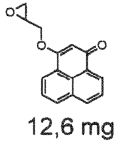

**Ergebnis:** Transparente, blassgelbe, homogene Beschichtung; Schichtstärke zwischen 20 und 40 µm, bei Verwendung eines Pinsels > 50 µm

### 4) Raumtemperatur vulkanisierendes 1 Komponenten (RTV-1) Silikon,

Typische Zusammensetzung des Silikons (technisches Produkt von Wacker) Polydimethylsiloxandiol und Polydimethylsiloxan im Gemisch mit Siliciumdioxid (Füllstoff) und Triacetoxymethylsilan (Vernetzer, <5 %)

### Variante a)

Klares Sanitärsilikon (1K, Raumtemperatur-vernetzend, essigvernetzend, 5,0 g) wurde in Hexan (10 mL) gelöst. Eine Lösung des jeweiligen Photosensibilisators (0,012 mmol) in getrocknetem Dichlormethan (20 mL) wurde zugemischt. Die leicht trübe, blassgelbe, viskose Lösung wurde gleichmäßig auf i) eine entfettete Glasplatte, ii) eine entfettete PMMA-Platte, iii) eine Keramikplatte oder iv) eine Polyesterfolie aufgetragen. Nach ca. 15 min bei Raumtemperatur ist die Beschichtung angetrocknet, weitere ca. 10 min später fand eine erste Hautbildung statt. Nach 6h bei Raumtemperatur ist die ca. 0,1 mm starke Schicht vollständig durchgehärtet.

**Ergebnis:** Transparente, matte, blassgelbe, homogene, gummiartige Beschichtung

### Variante b)

Der jeweilige Photosensibilisator (0,02 mmol) wurde in getrocknetem Ethylacetat (100 mL) gelöst. Klares Sanitärsilikon (1K, Raumtemperatur-vernetzend, essigvernetzend, 8,0 g) wurde zugegeben und die Lösung wurde gerührt bis diese homogen war. Die leicht trübe, blassgelbe Lösung wurde gleichmäßig auf i) eine Tonplatte, ii) eine Keramikfliese, iii) eine entfettete PE-Platte oder iii) eine Aluminiumplatte mit einer Spritzpistole aufgetragen. Nach ca. 20 min bei Raumtemperatur war die Beschichtung angetrocknet, weitere ca. 10 min später fand eine erste Hautbildung statt. Nach 6 h bei Raumtemperatur war die ca. 0,1 mm starke Schicht vollständig durchgehärtet.

**Ergebnis:** Transparente, matte, blassgelbe, homogene, gummiartige Beschichtung

### Variante c)

Der feingepulverte Photosensibilisator (0,012 mmol) wurde in klares Sanitärsilikon (1K, Raumtemperatur-vernetzend, essigvernetzend, 5,0 g) in einem Mischgerät SpeedMixer^{™} DAC 600 (Hauschild Engineering, Deutschland) bei Raumtemperatur eingerührt, bis eine makroskopisch homogene Paste erhalten wurde. Die blassgelbe, viskose Masse wurde mit einer Einwegspritze gleichmäßig in i) eine Fließenfuge eingetragen oder ii) zum Verkleben zweier aufeinander senkrecht stehenden Glasplatten benutzt. Nach ca. 30 min bei Raumtemperatur war das Silikon angetrocknet, weitere ca. 20 min später fand eine erste Hautbildung statt. Nach 6h bei Raumtemperatur war die Schicht vollständig durchgehärtet.

Folgende Photosensibilisatoren wurden nach den drei Varianten zu blassgelben, gummiartigen Silikonfugen verarbeitet:

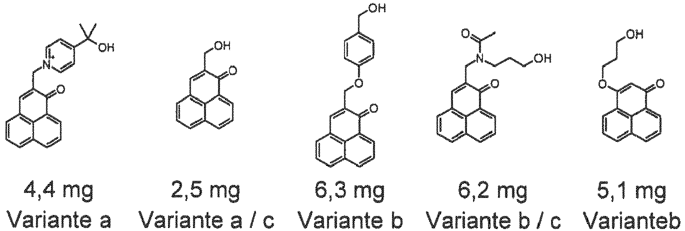

**Ergebnis:** Transparente, matte, blassgelbe, homogene, gummiartige Fugenfüllung

### 5) Raumtemperatur vulkanisierendes 2 Komponenten (RTV-2) Silikon

Typische Zusammensetzung des Silikons (technisches Produkt von Wacker): Komponente A) Vinyl-gruppenhaltiges oligomeres Silan / Vinylsilan und Komponente B) Polydimethylsiloxan im Gemisch mit Siliciumdioxid (Füllstoff) und Hilfsstoffen und Platinkatalysator

Das Basispolymer (A) wurde mit dem Vernetzter (B) 9:1 gemischt.

Der feingepulverte Photosensibilisator (0,05 mmol) wurde in 90 g des Basispolymers (A) bei Raumtemperatur eingerührt, bis eine makroskopisch homogene Paste erhalten wurde. Die blassgelbe, viskose Masse wurde mit dem Vernetzter (B) im Verhältnis 9:1 gemischt und mit Hilfe einer flachen Spachtel gleichmäßig auf i) eine Polyesterfolie, ii) eine Keramikfliese, eine entfettete Glasplatte, iv) eine entfettete PE-Platte oder v) eine Aluminiumplatte aufgetragen (50 mL pro m²).

Nach ca. 30 min bei Raumtemperatur fand eine erste Hautbildung statt. Nach 8 h bei Raumtemperatur war die Schicht vollständig durchgehärtet.

Folgende Photosensibilisatoren wurden zu Silikonbeschichtungen verarbeitet:

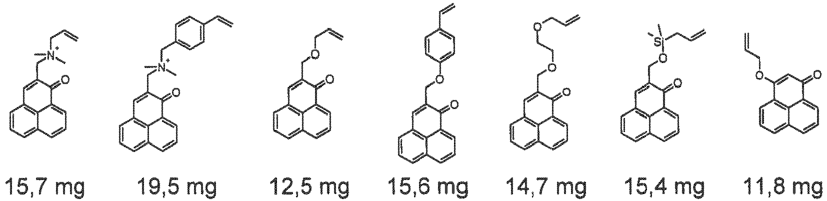

**Ergebnis:** Transparente, matte, blassgelbe, homogene, gummiartige Beschichtung

### 6) Polyacrylat 1K

Der feingepulverte Photosensibilisator (0,05 mmol) wurde in 100 mL Methacrylsäureethylester (trockene Bedingungen) gelöst. Campherchinon (1,7 mg, 0,01 mmol) und 4-Dimethylaminobenzoesäuremethylester (0,9mg, 0,005 mmol, Aminbeschleuniger) wurden zugegeben und die Mischung 15 min im Dunklen gerührt. Die Lösung war gekühlt im Dunklen über Wochen haltbar.

Die Lösung wurde auf i) Holz mit einem Pinsel aufgetragen oder ii) ein Filterpapier getränkt oder iii) auf eine entfettete PMMA-Platte gleichmäßig aufgesprüht.

Im 2.Schritt wurde mit einer Polymerisationslampe (LED Bluephase; Fa. ivociarVivadent AG bei 650 mW/cm²) für 30 Sekunden belichtet. Die Prüfkörper wurden nach der Herstellung in einem Trockenschrank bei 37 °C für 24 h gelagert um die Polymerisation zu vervollständigen. Folgende Photosensibilisatoren wurden in den Versuchen verwendet:

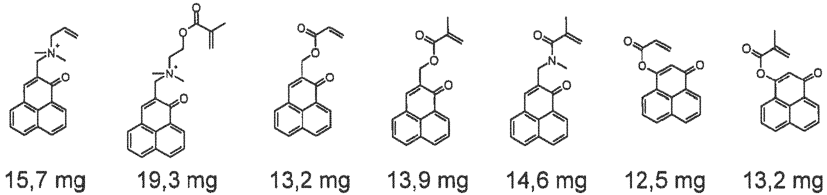

**Ergebnis:** Man erhält eine i) transparente, polymere Versiegelung, ii) ein teilflexibles, blassgelbes Plättchen oder iii) transparente, blassgelbe, lackartige Beschichtung.

### 7) Polyacrylat 2K

### Variante a)

Der jeweilige Photosensibilisator (0,05 mmol) wurde in 100 mL Methacrylsäuremethylester (Methylmethacrylat, MMA, Sigma Aldrich) gelöst (Lösung A). tert-Butylperoxybenzoat (Peroxan PB, 0,5 g) wurde in THF (2 mL) gelöst (Lösung B). Lösung B wurde zu Lösung A gegeben und das Gemisch wurde für 15 min bei 90°C im Wasserbad erwärmt. Die leicht viskose Lösung wurde auf i) eine entfettete PMMA-Platte oder ii) eine entfettete Glasplatte aufgesprüht oder iii) auf Kiefernholz aufgetragen und mit einem Pinsel gleichmäßig verteilt (50 mL pro m²). Zum vollständigen Aushärten der Acrylglasbeschichtung wurde der Träger 24 Stunden bei 60 °C im Trockenschrank gelagert. Man erhält eine blassgelbe, klare Kunststoffbeschichtung bzw. polymere Versiegelung des Holzes

Ergebnis: Transparente, homogene, lackartige Beschichtung; bei i) und ii) war die gelbe Eigenfarbe der Schicht schwach zu erkennen, bei iii) war die Eigenfarbe nicht vom Untergrund zu unterscheiden

### Variante b)

Der jeweilige Photosensibilisator (0,05 mmol) wurde in 100 mL Methacrylsäuremethylester (Methylmethacrylat, MMA, Sigma Aldrich) gelöst (Lösung A). Benzoylperoxid (0,5 g) wurde in THF (2 mL) gelöst (Lösung B). Lösung B wurde zu Lösung A gegeben und das Gemisch für 20 min bei 80°C im Wasserbad erwärmt. Mit der Mischung wurde i) ca. 6 mm starkes Baumwollflies oder ii) eine ca. 6 mm starke Filzmatte oder iii) ein Stück Aramidgewebe (je 20x20 cm²) mit einem Pinsel gleichmäßig mit der Mischung durchtränkt, so dass das Gewebe die Mischung vollständig aufnehmen konnte.

Zum vollständigen Aushärten des Acrylglases wurden die Fasermatten 12 Stunden bei 60°C getrocknet. Man erhielt eine gelbliche feste Platte. Folgende Photosensibilisatoren wurden verarbeitet:

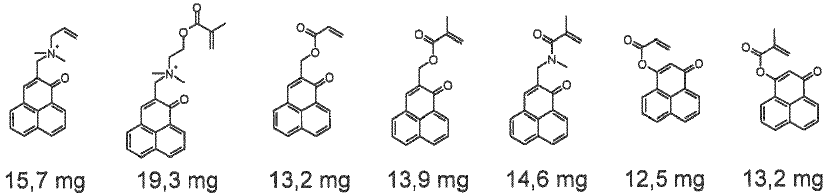

**Ergebnis:** Zähe, begrenzt flexible Fasermatte; Eigenfarbe des Farbstoffes ist nur bei i) schwach zu erkennen

### 8) Polyacrylamid

Eine 40 %ige Lösung von Acrylamide/bis-acrylamide (37,5 : 1) in Wasser (2,5 mL) wurde mit Tris-Puffer (3,75 mL, 1M, pH 8,8) und Propylenglykol (0,6 mL) gemischt. Der jeweilige Photosensibilisator (0,005 mmol) in destilliertem Wasser (3,0 mL) wurde zugegeben. Die Lösung wurde mit einer Vakuumpumpe 5 Minuten entgast (5-10 mbar) und anschließend mit TEMED versetzt (10 µl). Zuletzt wurde der Lösung 0,05 ml Ammoniumpersulfat (10 % in Wasser) unter Rühren zugesetzt.

Es wurde i) eine 3 mm dünne Kassette (Platten vorher mit 70 %igem Alkohol gesäubert) mit der Mischung gefüllt und vorsichtig mit Isopropanol überschichtet. Nach 30 min Polymerisation bei Raumtemperatur war das Gel fest und der Überstand konnte abgegossen wurden. Nach Abnehmen der Kammerplatten erhielt man eine flexible, dicke blassgelbe Folie. Alternativ wurde ii) die Mischung auf ein Flies gleichmäßig aufgebracht..

Während der Polymerisation wurde das Material mit Frischhaltefolie abgedeckt. Nach 30 min Polymerisation bei Raumtemperatur war das Gel fest und die Folie wurde vorsichtig abgezogen. Man erhielt eine gelartige gelbliche Beschichtung.

Folgende Photosensibilisatoren wurden auf diese Art und Weise einpolymerisiert:

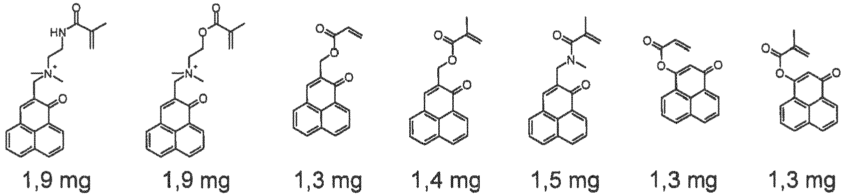

Ergebnis: i) Gummiartige, leicht trübe, blassgelbe Matte; ii) transparente, matte, blassgelbe, homogene, gummiartige Beschichtung

### 9) Cyanacrylat

### Variante a)

Der jeweilige Photosensibilisator (0,01 mmol) wurde in 100 mL Dichlormethan (getrocknet über Calciumchlorid) gelöst. Zu 1 g der Lösung wurden 0,2 g käufliches Cyanacrylat (Loctite, Henkel AG & Co. KGaA, Düsseldorf, DE) zugemischt (trockene Bedingungen). Die Mischung wurde auf a) eine entfettete Glasplatte oder b) auf trockenes Fichtenholz gleichmäßig aufgesprüht. Bei Raumtemperatur und eine Umgebungsfeuchtigkeit zwischen 20 und 70 % härtete die Schicht binnen 20 min vollständig aus.

### Variante b)

Der jeweilige Photosensibilisator (0,01 mmol) wurde in 100 mL getrocknetem Methylethylketon gelöst. Käufliches Cyanacrylat (Loctite.) wurde mit 10 g der Lösung im Verhältnis 1:5 (w/w) gemischt. Die Lösung wurde gleichmäßig auf einen mit Isopropanol gereinigten Untergrund, z.B. i) Glas oder ii) PE oder iii) Melaminplatte aufgetragen. Nach Verdampfen des Lösungsmittels härtete die Beschichtung durch Luftfeuchtigkeit aus (ca. 15 min, Luftfeuchtigkeit zwischen 20 und 70 %).

Folgende Photosensibilisatoren wurden nach der jeweiligen Variante verarbeitet:

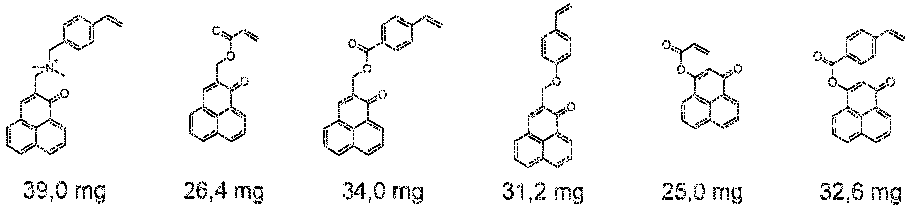

### Variante a Variante a Variante b Variante b Variante b Variante b

**Ergebnis:** Transparente, leicht trübe, blassgelbe, homogene Beschichtung; Schichtstärke zwischen 20 und 40 µm

### 10) Polystyrol

Der jeweilige Photosensibilisator (0,01 mmol) wurde in 20 mL frisch destilliertem Polystyrol (Sigma Aldrich) gelöst (Lösung A). Benzoylperoxid (0,5 g) wurde in THF (2 mL) gelöst (Lösung B). Lösung B wurde zu Lösung A gegeben und das Gemisch für 20 min bei 80°C im Wasserbad erwärmt. Mit 5 bis 6 mL der Mischung wurde i) eine saubere, unbehandelte Spanholzplatte oder ii) eine entfette PMMA-Platte oder iii) eine entfettete Glasplatte (je 20x20 cm²) mit einem Pinsel gleichmäßig bestrichen. Zum vollständigen Aushärten ließ man den Träger 12 Stunden bei 60°C trocknen. Man erhält eine gelbliche feste Platte. Folgende Photosensibilisatoren wurden verarbeitet:

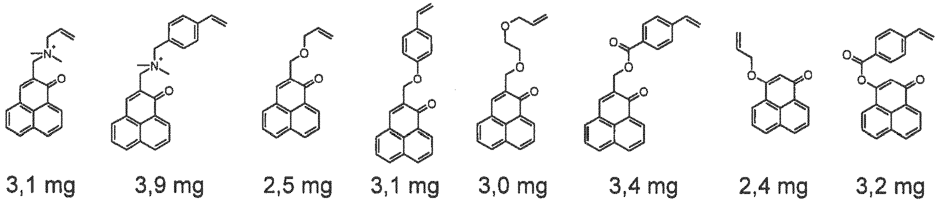

### 11) Carboxymethylcellulose-Beschichtungen

Der jeweilige Photosensibilisator (0,05 mmol) wurde zusammen mit fein gemörserter Carboxymethylcellulose (4,0 g, Akzo Nobel) in 100 ml Wasser gelöst. Mit 5 bis 6 mL der klaren, etwas zähflüssigen Lösung wurde i) eine saubere, unbehandelte Spanholzplatte oder ii) eine entfette PMMA-Platte (je 20x20 cm²) mit einem Pinsel gleichmäßig bestrichen.

Alternativ wurde iii) ein Baumwollflies (20x20 cm²) mit einem Pinsel gleichmäßig durchtränkt, so dass das Gewebe die Mischung vollständig aufnehmen konnte. Zum Aushärten ließ man den Träger 3 Stunden bei Raumtemperatur trocknen. Folgende Photosensibilisatoren wurden verarbeitet:

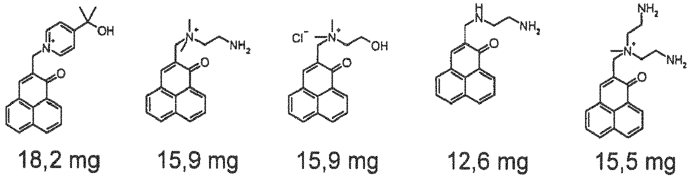

**Ergebnis:** i) transparente, polymere Versiegelung ohne erkennbare Eigenfarbe; ii) transparente, leicht trübe, blassgelbe, homogene Beschichtung; iii) zähe, begrenzt flexible Fasermatte; Eigenfarbe des Photosensibilisators war kaum zu erkennen

### 12) Alginat-Beschichtungen

Natriumhydroxid (850 mg, 21,25 mmol) wurden in 95 mL destilliertem Wasser gelöst. Zu der warmen Lösung (ca. 35°C) wurden 5g Alginsäure (Sigma Aldrich) zugegeben und für 3 h gerührt. Es entstand eine leicht viskose, klare Lösung. Eine Stammlösung des jeweiligen Photosensibilisators (5 mL, 12 mmol/L) wurde unter Rühren zugetropft. Mit der gelben, leicht viskosen Lösung wurde i) ein Stück Papier oder ii) ein Stück Baumwollflies oder iii) ein Zellstofflappen/kompresse (je 20x20 cm²) getränkt. Man ließ die überschüssige Lösung abtropfen. Der feuchte Träger wurde zügig in eine 1%ige Calciumchloridlösung getaucht. Man ließ das Material gut abtropfen, tupft es anschließend vorsichtig ab und trocknet es bei Raumtemperatur an der Luft für 3 Stunden.

Folgende Photosensibilisatoren wurden verarbeitet:

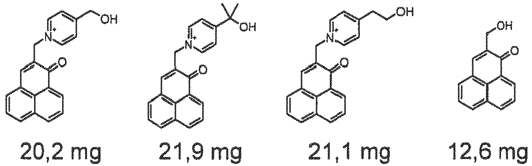

**Ergebnis:** zähe, begrenzt flexible Fasermatte bzw. Papierstück; die Eigenfarbe des Photosensibilisators war kaum zu erkennen

### Beispiel 3: Testung der Wirksamkeit der antimikrobiellen Oberflächenbeschichtungen

### 1.) Herstellung der Probenträger

Für die nachfolgenden Versuche wurde folgender Photosensibilisator verwendet 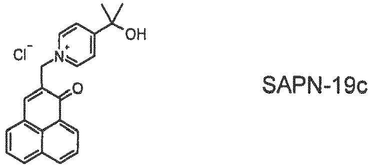
**Absorptionsmaximum** 360 - 420 nm
**Molarer Extinktionskoeffizient** 9800 L mol⁻¹ cm⁻¹

Der Photosensibilisator wurde in dem jeweiligen Lacksystem gelöst und auf verschiedene rechteckige Probenträger (Breite: 4cm, Länge: 4cm, Dicke: 3 mm) aus PVC bzw. PMMA aufgebracht und getrocknet. Nach dem Trocknen der Beschichtung wurde eine definierte Menge an Bakterien der Spezies *Staphylococcus aureus* ATCC 25923 auf die Oberfläche aufgebracht.

Dazu wurde zunächst wurde eine über Nacht (üN) Kultur von S. aureus in 5 mL Mueller Hinton Boullion (Carl Roth GmbH + Co. KG, Karlsruhe, DE) mit einer Einzelkolonie von einer Agarplatte angeimpft und bei 37 °C und 180 rpm auf einem Schüttler für 18-20 Std. inkubiert. Am Versuchstag wurde jeweils 1 mL üN Kultur zentrifugiert (Hettich Zentrifuge Universal 320 R (Andreas Hettich GmbH & Co.KG, Tuttlingen, DE); Ausschwingrotor 1494; 10 Min, 3000 rpm, RT). Der Überstand wurde verworfen und das Pellet in 1 mL Milli-Q-Wasser, das von der Firma Merck (KGaA, Darmstadt, DE) kommerziell bezogen wurde (Bezeichnung "Milli-Q Integral Reinstwasser (Typ 1)"), resuspendiert.

Nach Bestimmung der optischen Dichte bei 600 nm (Spektrometer "Specord 50 plus", Analytik Jena) erfolgte eine Verdünnung der resuspendierten Bakterien mit Milli-Q-Wasser auf eine Zellzahl von 10⁵ Bakterien/mL. Von der Verdünnung wurden jeweils 100 µL auf den Probenträger mit einer Pipette aufgetropft (Größe des Tropfens etwa 1 cm²).

Somit befanden sich etwa 10.000 Bakterien pro cm² auf der Oberfläche des jeweiligen Probenträgers. Der Probenträger wurde anschließend an Luft unter einer Sterilwerkbank im Dunklen ca. 2 Stunden getrocknet, bis kein Wasser mehr auf der Oberfläche zu sehen war.

### 2.) Bestrahlung und quantitative Bestimmung der Kolonie-bildenden Einheiten (KBE) / mL

Die jeweiligen Probenträger wurden mit monochromen Licht einer Wellenlänge von 405 nm mit Hilfe eines LED Moduls (Versuchsfeld 5 × 5 cm homogen ausgeleuchtet; high power LEDs) beziehungsweise mit Raumlicht (Osram Lumilux Cool White, Farbtemperatur 4000 K) in den unten angegeben Intensitäten und Bestrahlungszeiten bestrahlt. Direkt nach der Bestrahlung wurden die Bakterien mittels eines sterilen Tupfers von der Oberfläche des jeweiligen Probenträger gewischt und in 1 mL Mueller-Hinton-Boullion resuspendiert.

Danach wurde die Probe seriell verdünnt (1:10 Verdünnungsstufen: 180 + 20 µL; bis Verdünnungsstufe 10⁻⁵).

Aus jeder Verdünnungsstufe (100 bis 10⁻⁵) wurde 3 × jeweils 20 µL Boullion auf eine Mueller-Hinton-Agarplatte (Carl Roth GmbH + Co. KG) mit einer Pipette aufgetropft und verteilt. Die Agarplatten wurden anschließend bei 37°C üN bebrütet.

Am nächsten Tag wurden die zählbaren Kolonien aller Verdünnungsstufen eines Experimentes ausgezählt und die KBE/mL errechnet.

Verdünnungsstufen, die einen Bakterienrasen oder gar keine Kolonien aufwiesen, wurden mit "∞" bzw. "0" bezeichnet und gingen in die Berechnung der KBE/mL nicht mit ein.

Aus dem Wert der KBE/mL wurde im Anschluss die log₁₀ Abtötungsrate berechnet. Als Bezugspunkt diente in jedem Experiment die Referenzkontrolle (= 100% Anzahl der eingesetzten Bakterien).

Ein Versuch war wie folgt zusammengesetzt:
- beschichteter Probenträger: ohne Photosensibilisator, nicht bestrahlt ("Referenzkontrolle")
- beschichteter Probenträger: mit Photosensibilisator, nicht bestrahlt ("Dunkelkontrolle")
- beschichteter Probenträger: ohne Photosensibilisator und bestrahlt ("Lichtkontrolle")
- beschichteter Probenträger: mit Photosensibilisator und bestrahlt ("Probe")

### 3.) Verwendete Lacksysteme und Bestrahlungsparamete

### 3.1.) 2K Polyurethanlack, Lösungsmittel Butylacetat/Xylen aus Beispiel 2.1

Beschichtetes Material: PMMA, 3 mm Dicke, 4 x 4 cm
Trocknungszeit ca. 1 Std.
Trocknungstemperatur 15 - 30 °C
Applikation mit einer Spritzpistole
Verwendet Konzentration des Phosensibilisators: 200 µm (61,55 mg/l)

### Bestrahlungsbedingungen:

| Versuchs-Nr. | Bestrahlungsquelle | Bestrahlungsintensität | Bestrahlungsdauer |
|---|---|---|---|
| 3.1.1 | LED Modul (405 nm) | 10 mW/cm² | 2 min |
| 3.1.2 | LED Modul (405 nm) | 20 mW/cm² | 2 min |
| 3.1.3 | LED-Modul (405 nm) | 0,7 mW/cm² | 0 bis 150 min |

### 3.2.) 1K Polyurethanlack, wasserbasiert aus Beispiel 2.2

Beschichtetes Material: PVC, 3 mm Dicke, 4 x 4 cm
Trocknungszeit ca. 2 Std.
Trocknungstemperatur 15 - 30 °C
Relative Luftfeuchte für die Trocknungszeit 30 - 70 %
Applikation mit einer Spritzpistole
Verwendet Konzentration des Phosensibilisators: 200 µm (61,55 mg/l)

### Bestrahlungsbedingungen:

| Versuchs-Nr. | Bestrahlungsquelle | Bestrahlungsintensität | Bestrahlungsdauer |
|---|---|---|---|
| 3.2.1 | LED Modul (405 nm) | 10 mW/cm² | 10 min |

### 3.3) Raumtemperatur vulkanisierendes 2 Komponenten (RTV-2) Silikon aus Beispiel 2.5

Beschichtetes Material: PVC, 3 mm Dicke, 4 x 4 cm
Trocknungszeit ca. 2 Std.
Trocknungstemperatur 15 - 30 °C
Relative Luftfeuchte für die Trocknungszeit 30 - 70 %
Applikation mit einer Spritzpistole
Verwendet Konzentration des Phosensibilisators: 200 µm (61,55 mg/l)
Zwei Silikonhärtegrade (sog. "Shores"): A = 10 Shore; B = 45 Shore
Bestrahlungsbedingungen:

| Versuchs-Nr. | Bestrahlungsquelle | Bestrahlungsintensität | Bestrahlungsdauer |
|---|---|---|---|
| 3.3.1 | LED Modul (405 nm) | 10 mW/cm² | 5 min |
| 3.3.2 | LED Modul (405 nm) | 10 mW/cm² | 10 min |

### 4.) Ergebnisse und Auswertung

### 4.1.) 2K Polyurethanlack (Lösungsmittel: Butylacetat/Xylen) aus Beispiel 2.1

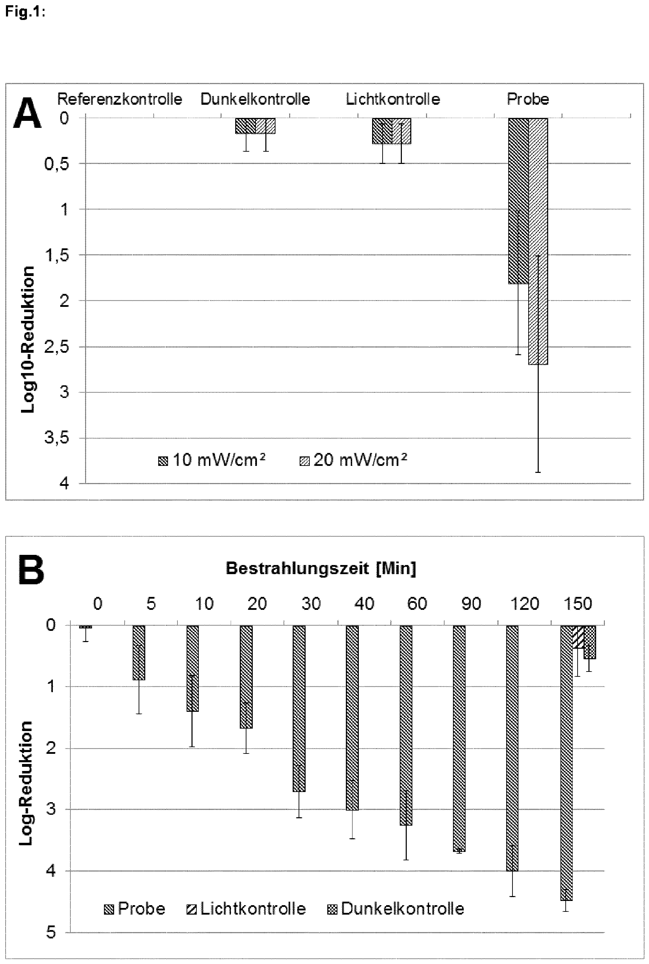

In einer ersten Studie mit dem 2K Lacksystem wurden zunächst hohe Intensitäten von 10 und 20 mW/cm² eingesetzt, um die Wirksamkeit des verwendeten Photosensibilisators SAPN-19c gegen S. aureus zu testen. Die Ergebnisse sind in Fig. 1A gezeigt.

Fig. 1A zeigt jeweils die Mittelwerte der Reduktion von S. aureus auf der 2K-Lackoberfläche aus drei unabhängigen Versuchen. Die Bestrahlung erfolgte mit 10 bzw. 20 mW/cm² und 2 Minuten (entspricht einer Energiedichte von 1,2 bzw. 2,4 J/cm²).

Fig. 1A zeigt, dass das 2K Lacksystem bereits nach 2 Minuten Bestrahlungszeit bei einer Lichtintensität von 10 mW/cm² eine Log₁₀-Reduktion von 1,8 (Mittelwert aus 3 unabhängigen Experimenten). Eine Erhöhung der Lichtintensität auf 20 mW/cm² führt zu einer effektiveren Abtötung von 2,7 log₁₀ Stufen.

Anschließend wurde die Effektivität bei niedrigen Intensitäten von 0,7 mW/cm² bestimmt. Die Ergebnisse sind in Fig. 1B gezeigt.

Fig. 1B zeigt jeweils die Mittelwerte der Reduktion von S. aureus auf der 2K-Lackoberfläche (farbloser lösungsmittelbasierter 2K Lack auf durchsichtigen PMMA Probenträger) aus zwei unabhängigen Versuchen.

Die Bestrahlung erfolgte mit 0,7 mW/cm² bis zu 150 Minuten (entspricht einer Energiedichte von bis zu 6,3 J/cm²). Der Mittelwert der Referenzkontrolle (kein Licht, kein Katalysator) beträgt 3,2 x 10⁴ Bakterien pro mL. Die Licht- und Dunkelkontrolle erfolgte nur bei 150 Minuten.

Die Abnahme der Koloniebildenden Einheiten bezogen auf eine nicht-bestrahlte Probe (0 min) wurde in Intervallen gemessen, wobei nach den in Fig. 1b angegeben Zeitpunkten (5 min., 10 min.,20 min., 30 min., 40 min., 60 min.,90 min., 120 min. und 150 min.) jeweils 2 beschichtete Probenträger aus der Bestrahlung entfernt wurden. Die Ergebnisse aus zwei unabhängigen Versuchen sind in Fig. 1B dargestellt.

Der Mittelwert der Referenzkontrolle (kein Licht, kein Katalysator) betrug 3,2 × 10⁴ Bakterien pro mL. Die Licht- und Dunkelkontrolle erfolgte nur bei 150 Minuten.

Abbildung 1B zeigt, das bereits nach 20 Minuten das 2K Lacksystem eine Abtötung von S. aureus um 1,9 log10 Stufen erreicht.

Das 2K Lacksystem zeigte bei Bestrahlung mit einer künstlichen Lichtquelle eine biologische Wirksamkeit gegen S. aureus, sowohl bei hohen Intensitäten und niedrigen Bestrahlungszeiten (Fig. 1A), als auch bei niedrigen Intensitäten und längeren Bestrahlungszeiten (Fig. 1B).

### 4.2.) 1K Polyurethanlack, wasserbasiert aus Beispiel 2.2

Ein erster Versuch mit einer hohen Intensität (10 mW/cm²) und einer langen Bestrahlungszeit (10 Minuten) zeigte die Wirksamkeit des 1K Lacksystems.

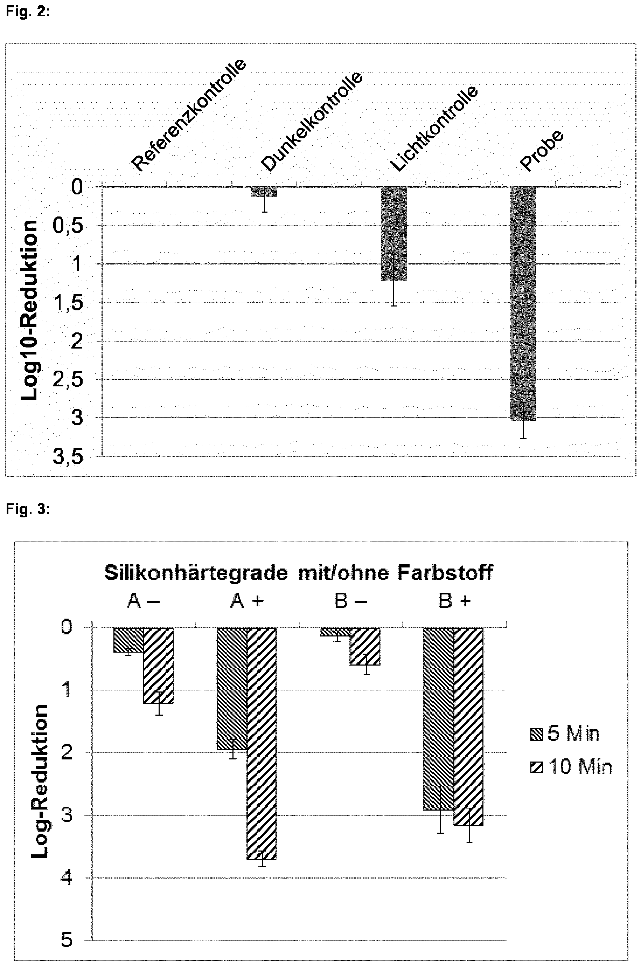

Die Ergebnisse sind in Fig. 2 gezeigt.

Fig. 2 zeigt die Mittelwerte der Reduktion von S. aureus auf der 1K-Lackoberfläche aus drei unabhängigen Versuchen. Die Bestrahlung erfolgte mit 10 mW/cm² und 10 Minuten (entspricht einer Energiedichte von 6 J/cm²).

Die Experimente mit dem 1K Lacksystem zeigen eindeutig, dass erst beim Zusammenspiel von Licht und dem photodynamischen Katalysator eine effektive Abtötung von S. aureus auf der beschichteten Oberfläche stattfindet. Drei unabhängige Versuche zeigen im Mittel eine Abtötung von 3.0 Log₁₀ Stufen bei einer Energiedichte von 6 J/cm².

Die Lichtkontrolle zeigte in den Experimenten im Mittel eine Reduktion um 1.2 Log₁₀ Stufen, was vermutlich auf den dunklen Probenträger zurückzuführen ist.

### 3.3) Raumtemperatur vulkanisierendes 2 Komponenten (RTV-2) Silikon aus Beispiel 2.5

Ein erster Versuch mit einer hohen Intensität (10 mW/cm²) und einer Bestrahlungszeit von 5 Minuten bzw. 10 Minuten zeigte die Wirksamkeit des RTV-2 Silikonsystems.

Die Ergebnisse sind in Fig. 3 dargestellt, wobei jeweils der Mittelwert aus 3 Versuchen gezeigt ist
Fig. 3 zeigt die Log-Reduktion von S. aureus auf der Silikonoberfläche (A - bedeutet 10 Shore ohne Farbstoff, A + bedeutet 10 Shore plus Farbstoff, B - bedeutet 45 Shore ohne Farbstoff, B + bedeutet 45 Shore plus Farbstoff).

### 4.3) Zusammenfassung

In den getesteten Lacksystemen (2K PUR Lacksystem; 1K PUR Lacksystem, RTV-2 Silikon) zeigte der verwendete Photosensibilisator SAPN-19c gegen S. aureus eine biologische Wirksamkeit auf der lackierten Oberflächenbeschichtung.

Höhere Lichtintensitäten und/oder längere Bestrahlungszeiten erzeugen mehr Singulett Sauerstoff, was zu einer schnelleren und effektiveren Abtötung von Mikroorganismen auf der Oberfläche führt.

Die in beiden Lacksysteme verwendete Konzentration von 200 µM (61,55 mg/L) war auf gefärbten Oberflächen nicht sichtbar. Darüber hinaus erreichte die Beschichtung bei einer Lichtintensität von 6 J/cm² eine Reduktion um 3 log10 Stufen (= 99,9%) der eingesetzten Bakterien.

### Beispiel 4. Testung der phototoxischen Wirkung einer beschichteten Oberflächen bei Bürobeleuchtung (Neonröhre)

In einem anschließenden Test wurde das 2K Lacksystem mit Raumlicht bestrahlt. Dazu wurden die in Beispiel 2.1 mit dem Photosensibilisator SAPN-19c und SAPN-19 hergestellten photoaktiven Oberflächen zusätzlich offen in Räumen mit normaler Bürobeleuchtung (Standard Leuchtstofflampen, Osram Cool White, 840) gelagert und die phototoxische Wirkung auf S. aureus mit den gleichen Keimzahlen wie in Beispiel 3 nach definierten Zeiten analysiert. Dabei waren die photoaktiven Schichten ca. 40 cm von der Lichtquelle entfernt.

Es gezeigte sich, dass für den Photosensibilisator SAPN-19c nach einer Lagerung von 24 h unter Standard-Bürobeleuchtungszeiten mit eine Beleuchtungsdauer von 12 h eine vollständige Abtötung aller Keime auf der Oberfläche stattgefunden hatte (ca. 5,5 log₁₀ - Stufen, entspricht > 99,999 % Reduktion).

### Beispiel 5: Langzeitversuch zur photoaktiven Stabilität

Um die Stabilität der hergestellten Lacke zu testen wurde ein Langzeitversuch durchgeführt.

Die Test-Oberflächen wurden für verschiedene Zeiten (5, 25, 50, 75 h) bei Leistung von 5 mW cm⁻² vorbestrahlt, um festzustellen, ob der jeweilige Photosensibilisator in PUR dahingehend beeinflusst wird, dass die phototoxische Aktivität der Oberfläche abnimmt:

| **Applizierte Energie [J cm**⁻² | **5 mW cm**⁻² | **0,7 mW cm⁻²** | **"Bestrahlungstage"** |
|---|---|---|---|
| 90 | 5 h | 35,70 h | 7 Tage (d) |
| 450 | 25 h | 178,5 h | 36 d |
| 900 | 50 h | 357,0 h | 71 d |
| 1350 | 75 h | 535,5 h | 107 d |

Im Anschluss an die Vorbestrahlung wurden die Oberflächen wie oben beschrieben mit S. *aureus* verkeimt, danach 2 h bestrahlt (entsprechend bei 5 mW cm⁻²) und die phototoxische Effektivität gemessen.

| | |
|---|---|
| 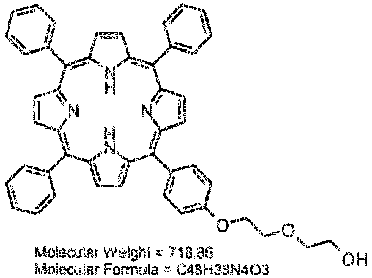 | 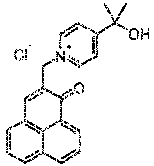 |
| TPP-Sensitizer WBII/2 | SAPN-19c |
| Eingesetzte Menge: 5 10⁻⁴ mol/L | Eingesetzte Menge: 5 10⁻⁴ mol/L |

Der TPP-Sensitizer WBII/2 wurde gemäß dem in Felgenträger A., Maisch T., Späth A., Schröder J.A., Bäumler W., "Singlet oxygen generation in porphyrin-doped polymeric surface coating enables antimicrobial effects on Staphylococcus aureus.", (Phys Chem Chem Phys. 2014 Oct 14;16(38):20598-607. doi: 10.1039/c4cp02439g.) beschriebenen Verfahren hergestellt.

Für die untersuchten Porphyrin-dotierten Oberflächen (TPP-Sensitizer WBII/2) ergab sich ein auffallende Verminderung der phototoxischen Effektivität. Dabei zeigten sich nach einem Monat bereits deutlich Verfärbungen der Testoberflächen und diese verloren an phototoxischer Wirksamkeit.

Dies kann damit begründet werden, dass der Ring des Farbstoffs unter Einfluss des aktiven Sauerstoffs aufbricht, wobei Billirubin-analoga gebildet werden:

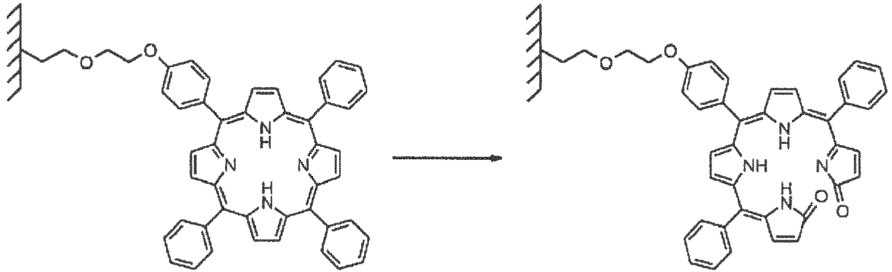

Für den SAPN-19c wurde kein Unterschied hinsichtlich phototoxischer Effektivität bis hin zu einer Vorbestrahlung von 75 h mit 5 mW cm ⁻².

Dies entspricht extrapolierten 312 Bestrahlungstagen (d) mit der Annahme, dass Büro-Leuchtstoffröhren bei einer Leistung von 100 µW cm ⁻² (auf Büro-Arbeitsplatz) für 12 Stunden Belichtung pro Tag durchgehend eingesetzt werden.

## Patentansprüche

1. Photosensibilisatorzusammensetzung umfassend
(a) wenigstens eine Phenalen-1-on-Verbindung mit der allgemeinen Formel (1): 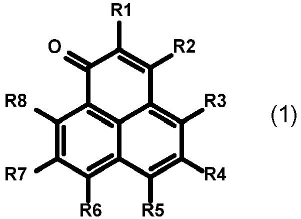
wobei die Reste R1 bis R8, die jeweils unabhängig voneinander gleich oder voneinander verschieden sein können, jeweils Wasserstoff, Halogen, Alkyl mit 1 bis 12 C-Atomen, Alkylaryl mit 5 bis 20 C-Atomen, Aryl mit 5 bis 20 C-Atomen, *-O-Alkyl mit 1 bis 12 C-Atomen, *-O-Alkylaryl mit 5 bis 20 C-Atomen, *-O-Aryl mit 5 bis 20 C-Atomen, Ether mit 2 bis 12 C-Atomen, ein Rest der Formel *-O-C(=O)-R^{(Ia)}, ein Rest der Formel *-C(=O)-R^{(Ib)}, oder ein organischer Rest W1, der wenigstens eine reaktive funktionelle Gruppe enthält, bedeuten, mit der Maßgabe, dass wenigstens einer der Reste R1 bis R7 ein organischer Rest W1 ist, wobei der organische Rest W1 jeweils unabhängig voneinander ein Rest mit der allgemeinen Formel (2) bis (6) bedeutet:
* - [(C(D)(E))_{d} - B]ₐ - (C(D)(E))ₘ - X (2)
* - A - [(C(D)(E))_{d} - B]_{c} - (C(D)(E))ₘ - X (3)
* - (C(D)(E))_{d} - Ar - (C(D)(E))ₙ - X (4)
* - [(C(D)(E))_{d} - B]_{b} - (C(D)(E))_{g} - Ar - (C(D)(E))ₙ - X (5)
* - A - [(C(D)(E))_{d} - B]_{f} - (C(D)(E))_{g} - Ar - (C(D)(E))ₙ - X (6)
wobei A jeweils unabhängig voneinander Sauerstoff, Schwefel, oder ein Rest mit der allgemeinen Formel (10a) bis (11a) bedeutet: 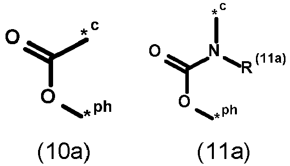
wobei *^{ph} jeweils einen Anknüpfungspunkt des Restes mit der allgemeinen Formel (10a) bis (11a) an ein C-Atom des Phenalen-Rings und *^{c} jeweils einen Anknüpfungspunkt des Restes mit der allgemeinen Formel (10a) bis (11a) an ein C-Atom des Restes (C(D)(E)) bezeichnet, und
wobei der Rest B jeweils unabhängig voneinander Sauerstoff, Schwefel oder ein Rest mit der allgemeinen Formel (10) bis (14) bedeutet: 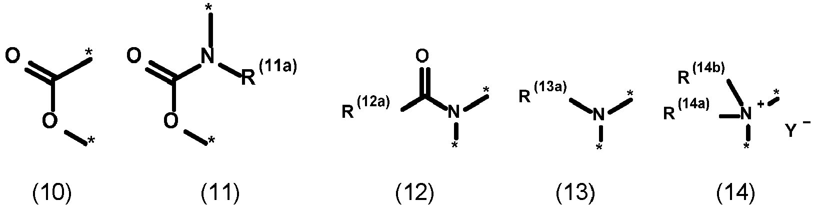
wobei die Reste R^{(Ia)}, R^{(Ib)}, R^{(11a)}, R^{(12a)}, R^{(13a)}, R^{(14a)} und R^{(14b)}, jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Halogen, Amino, Hydroxyl, *- O-Alkyl, das gradkettig oder verzweigt sein kann, mit 1 bis 3 Kohlenstoffatomen, Alkyl, das gradkettig oder verzweigt sein kann, mit 1 bis 3 Kohlenstoffatomen, Hydroxyalkyl, das gradkettig oder verzweigt sein kann, mit 1 bis 3 Kohlenstoffatomen und 1 bis 3 OH-Gruppen, Halogenalkyl, das gradkettig oder verzweigt sein kann, mit 1 bis 3 Kohlenstoffatomen und 1 bis 3 Halogen-Gruppen, und Kombinationen davon, vorzugsweise Chlor, Brom, Fluor, Methyl, Ethyl, n-Propyl, iso-Propyl, Methoxy, Ethoxy und Kombinationen davon, besteht, ausgewählt werden, substituiert sein können, wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sulfonat-Anion einer Sulfonsäure mit 1 bis 12 C-Atomen oder eine Kombination davon bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxyalkyl, das gradkettig oder verzweigt sein kann, mit 1 bis 5 Kohlenstoffatomen und 1 bis 5 OH-Gruppen, Phenyl, Benzyl, ein Rest der Formel *-L-R^{(II)}, ein Rest der Formel *-L-C(=L)-R^{(III)}, ein Rest der Formel *-(CH₂)_{q}-X, ein Rest der Formel *-L- (CH₂)_{q}-X, oder ein Rest der Formel *-(CH₂)ₛ-L-(CH₂)ₜ-X bedeuten, wobei der Rest L jeweils unabhängig voneinander Sauerstoff oder Schwefel, vorzugsweise Sauerstoff, bedeutet, wobei die Reste R^{(II)} und R^{(III)} jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, die Indices q, s und t jeweils unabhängig voneinander eine ganze Zahl von 1 bis 5 bedeuten,
wobei die Indices a, c, f, g und n jeweils unabhängig voneinander eine ganze Zahl von 0 bis 5 bedeuten,
und wobei die Indices b, d und m jeweils unabhängig voneinander eine ganze Zahl von 1 bis 5 bedeuten,
wobei der Rest Ar jeweils unabhängig voneinander einen Rest der allgemeinen Formel (25a) bis (31) bedeutet: 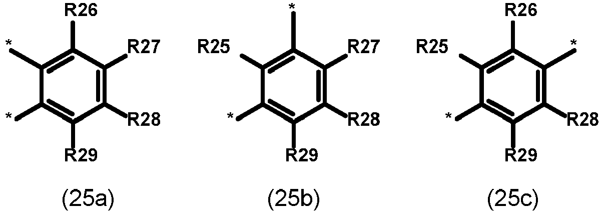 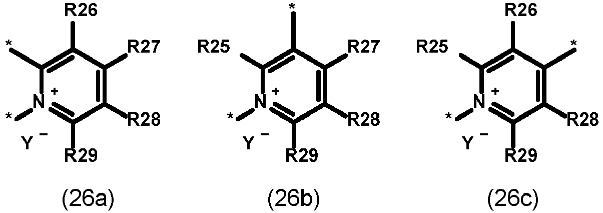 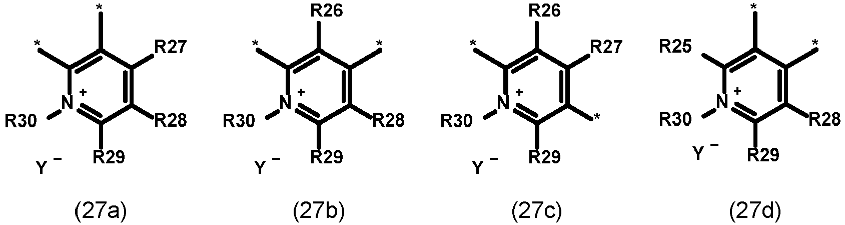 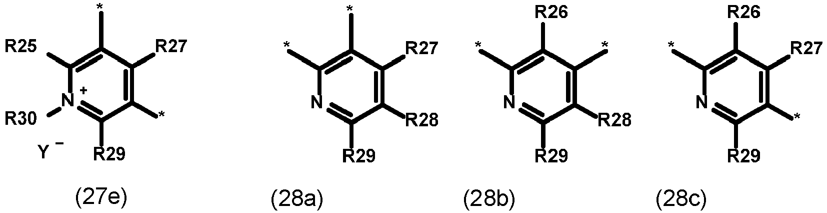 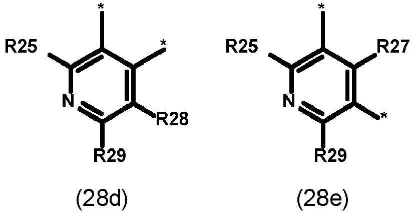 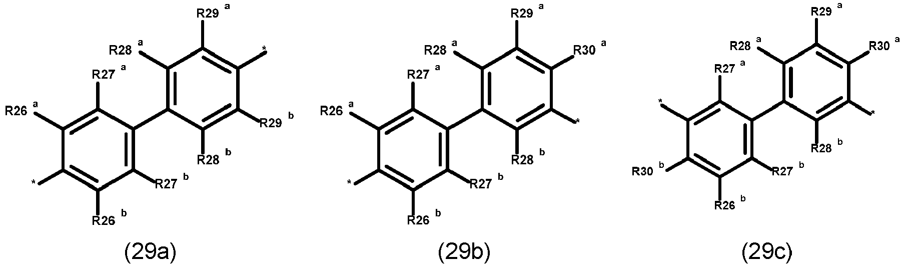 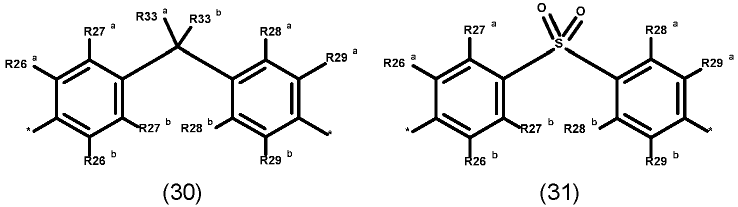
wobei die Reste R25, R26, R27, R28, R29, R26^{a}, R26^{b}, R27^{a}, R27^{b}, R28^{a}, R28^{b}, R29^{a}, R29^{b}, R30^{a} und R30^{b} jeweils unabhängig voneinander Wasserstoff, Hydroxy, Amino, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, und wobei der Rest R30 jeweils unabhängig voneinander Wasserstoff oder Alkyl, das gradkettig oder verzweigt sein kann, mit 1 bis 5 Kohlenstoffatomen bedeutet, und wobei die Reste R33^{a} und R33^{b} jeweils unabhängig voneinander Wasserstoff, Hydroxy, Amino, Alkyl, das gradkettig oder verzweigt sein kann, mit 1 bis 5 Kohlenstoffatomen, Perfluoralkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl, Benzyl oder wenn zusammen genommen ein Cycloalkyl, das gradkettig oder verzweigt sein kann, mit 4 bis 9 Kohlenstoffatomen, oder ein 9H-Fluoren-9-ylidenrest bedeuten, und
wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sufonat-Anion einer Sulfonsäure mit 1 bis 12 C-Atomen oder eine Kombination davon bedeutet, und
wobei der Rest X jeweils unabhängig voneinander eine reaktive funktionelle Gruppe ist, die *-N(R^{(VI)})(R^{(VII)}), *-OH, *-SH, *-NCO, *-NCS, *-Si(R^{(VIII)})(R^{(IX)})-[O-Si(R^{(X)})(R^{(XI)})]ₚ-Z oder ein Rest mit der allgemeinen Formel (20) bis (24) bedeutet: 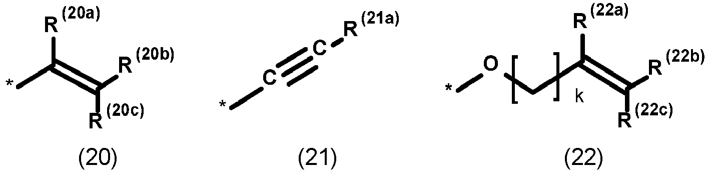 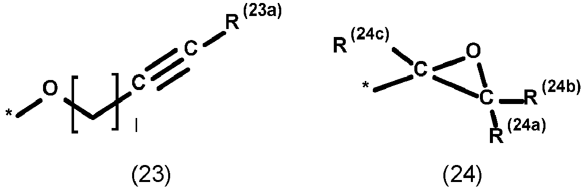
wobei die Reste R^{(20a)}, R^{(20b)}, R^{(20c)}, R^{(21a)}, R^{(22a)}, R^{(22b)}, R^{(22c)}, R^{(23a)}, R^{(24a)}, R^{(24b)}, und R^{(24c)} jeweils unabhängig voneinander Wasserstoff, Methyl, Ethyl, n-Propyl, iso-Propyl, n-Butyl, sec-Butyl, iso-Butyl, oder n-Pentyl bedeuten, und wobei die Indices l und k jeweils unabhängig voneinander eine ganze Zahl von 0 bis 4 bedeuten,
wobei die Reste R^{(VI)} und R^{(VII)} jeweils unabhängig voneinander Wasserstoff, Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei die Reste R^{(VIII)}, R^{(IX)}, R^{(X)} und R^{(XI)} jeweils unabhängig voneinander Wasserstoff, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, wobei der Rest Z jeweils unabhängig voneinander Halogen, Hydroxyl, Alkoxyl mit 1 bis 4 Kohlenstoffatomen oder Alkylcarboxyl mit 1 bis 4 Kohlenstoffatomen, vorzugsweise Halogen oder Hydroxyl, bedeutet, und wobei der Index p jeweils unabhängig voneinander eine ganze Zahl von 0 bis 4 bedeutet, und
(b) wenigstens eine polymere Komponente und/oder Vorläufer davon.

2. Photosensibilisatorzusammensetzung nach Anspruch 1,
wobei der wenigstens eine organische Rest W1 jeweils unabhängig voneinander ein Rest der allgemeinen Formel (40) bis (67) oder (72) bis (98e) bedeutet: 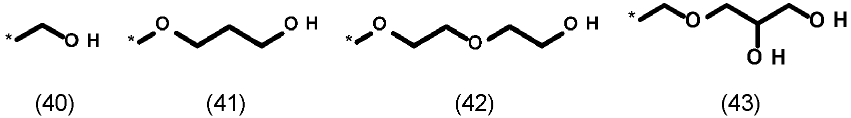 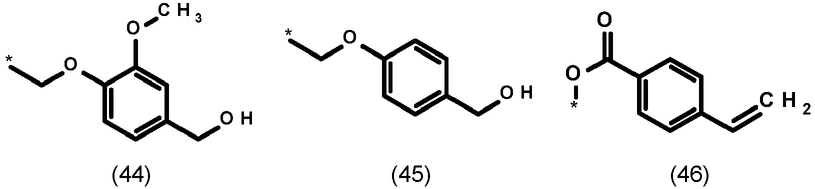 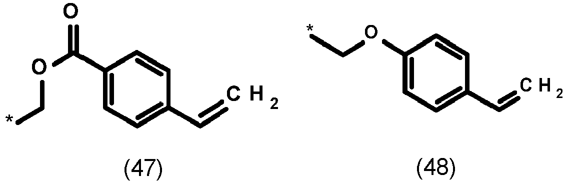 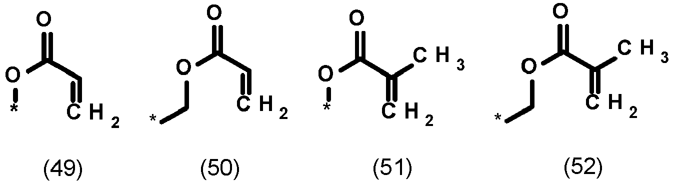 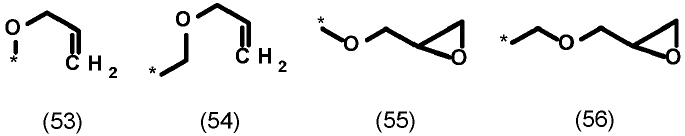 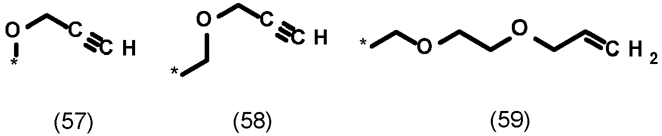 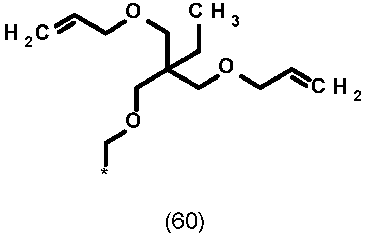 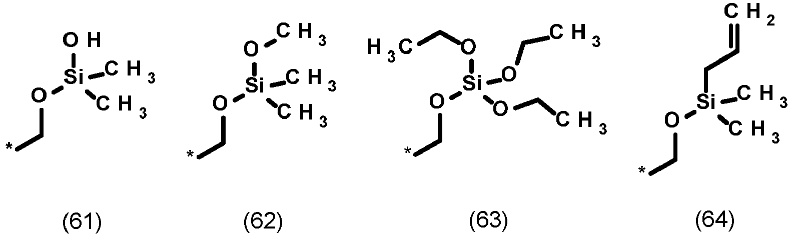 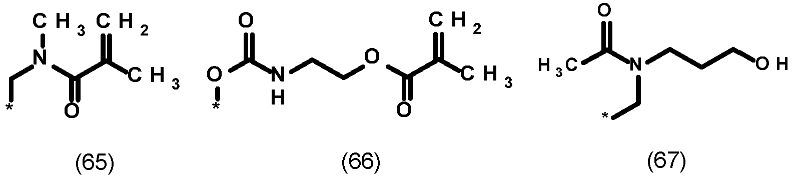 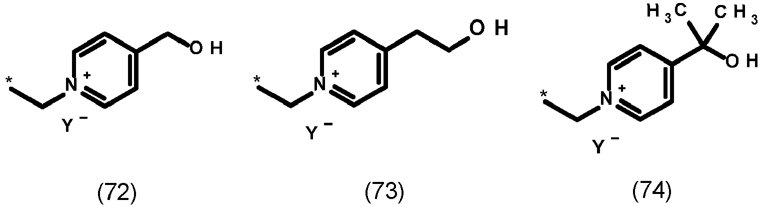 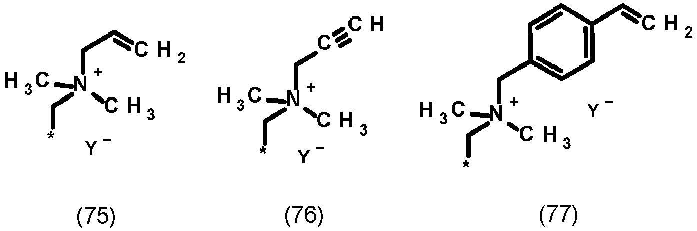 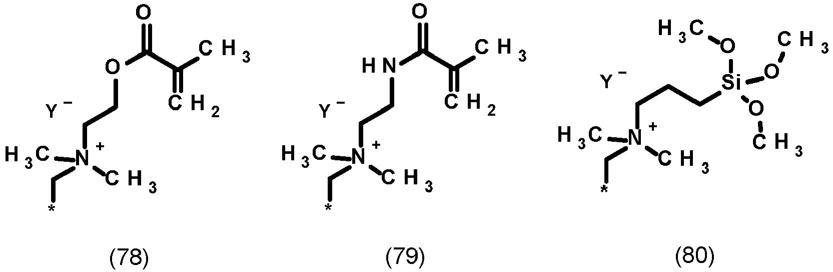 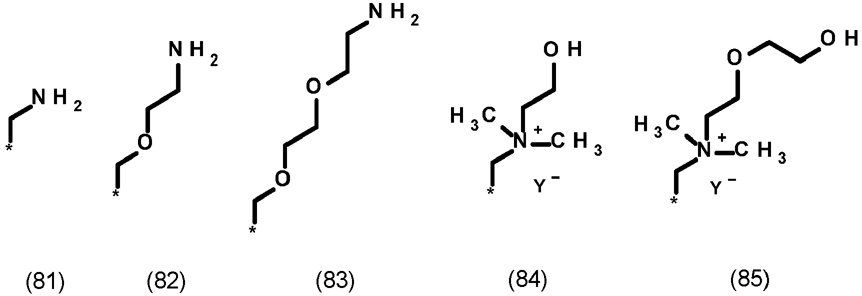 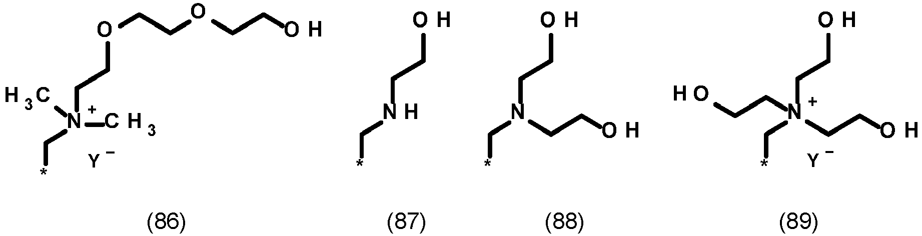 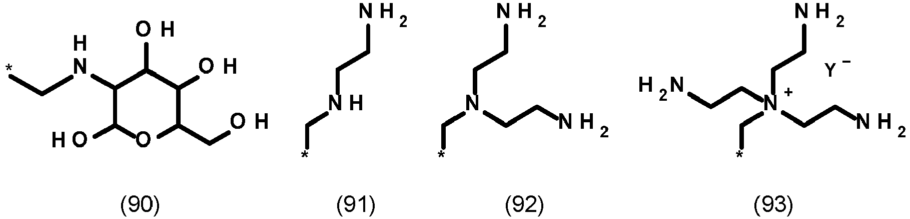 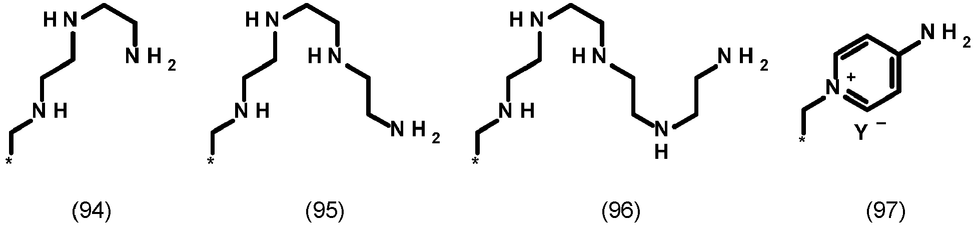 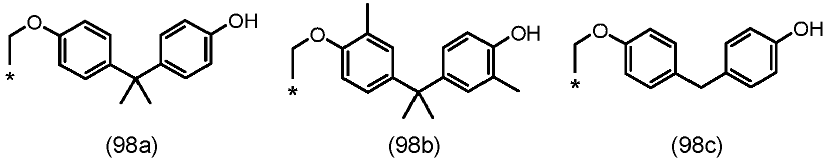 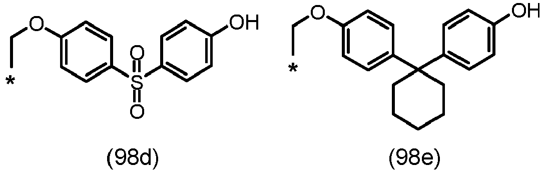 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sulfonat-Anion einer Sulfonsäure mit 1 bis 12 C-Atomen oder eine Kombination davon bedeutet.

3. Photosensibilisatorzusammensetzung nach einem der Ansprüche 1 oder 2, umfassend:
(a) wenigstens eine Phenalen-1-on-Verbindung mit der allgemeinen Formel (1), die aus der Gruppe, die aus Verbindung der Formel (100) bis (127), (132) bis (166): 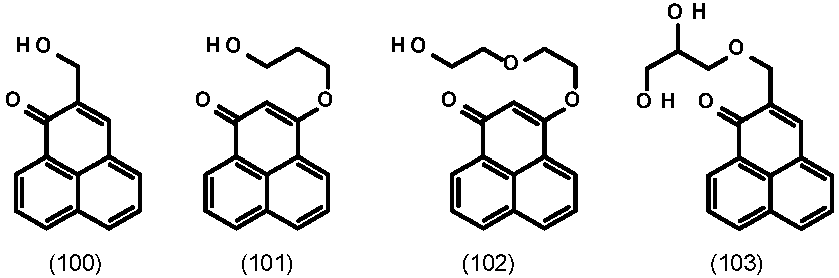 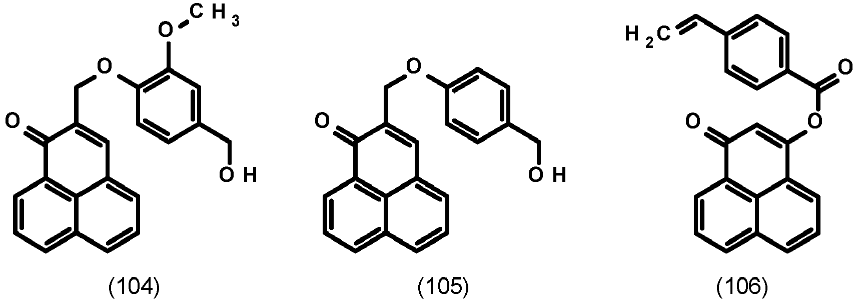 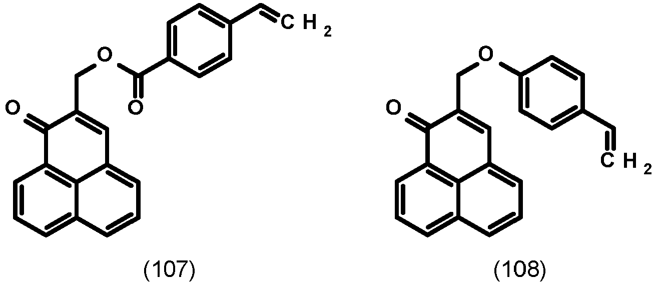 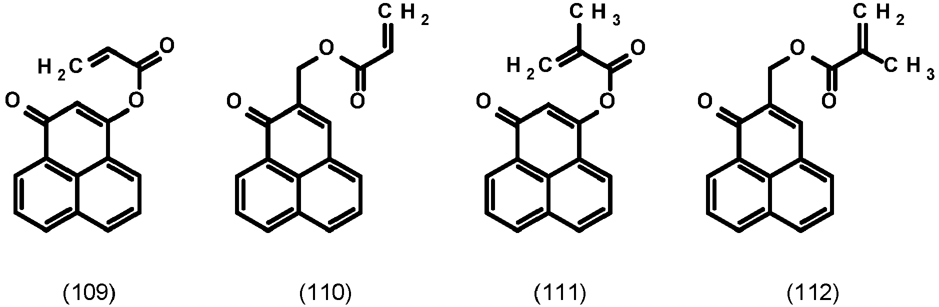 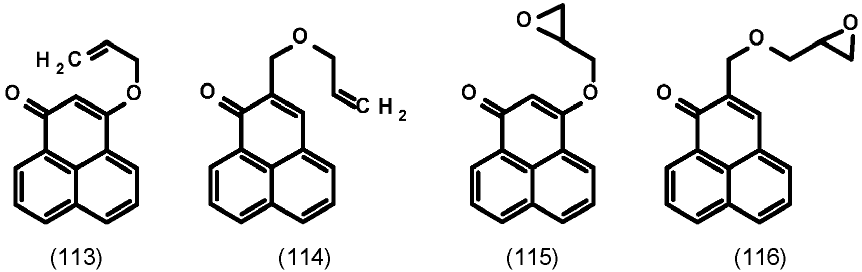 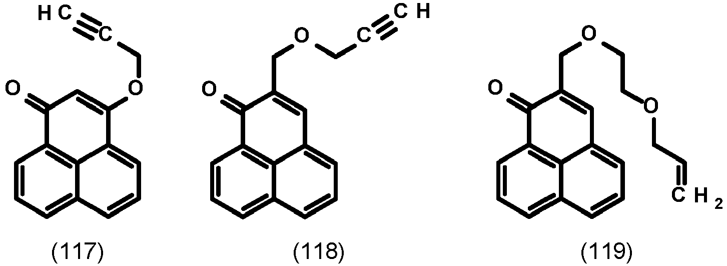 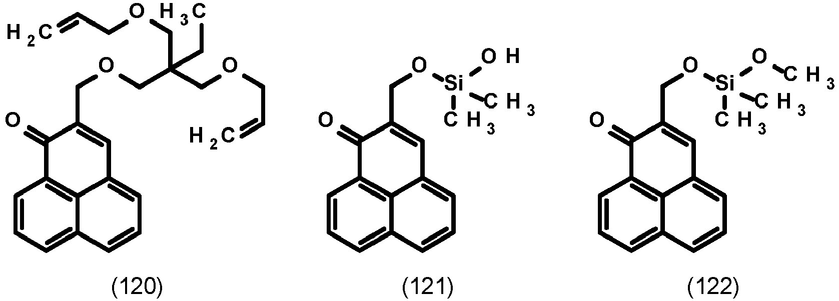 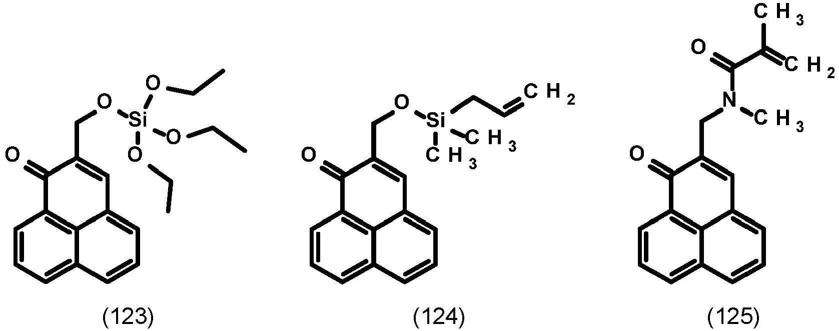 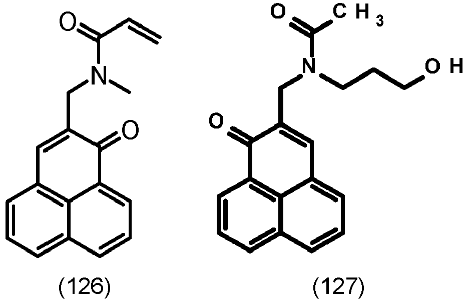 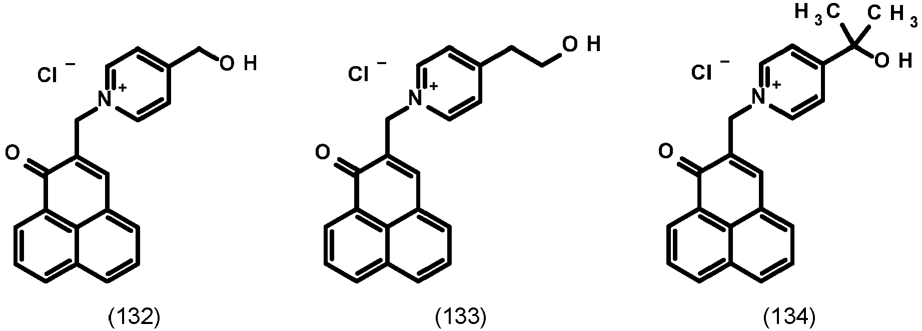 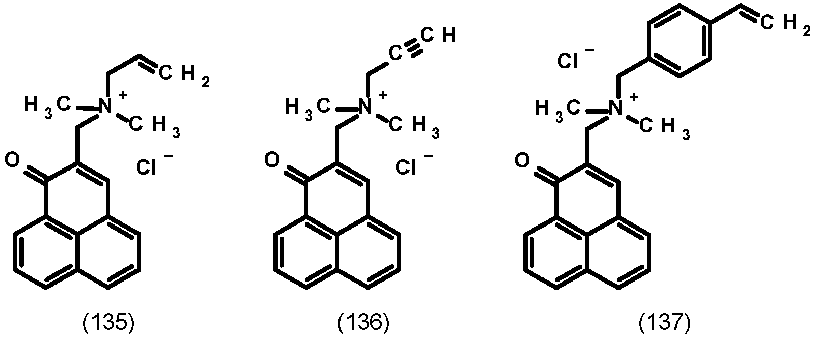 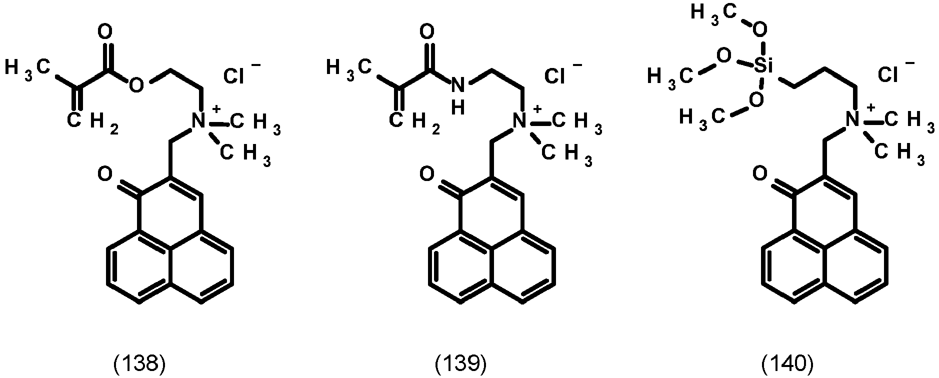 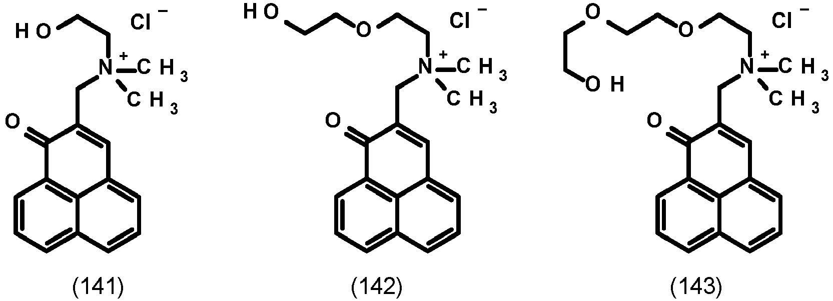 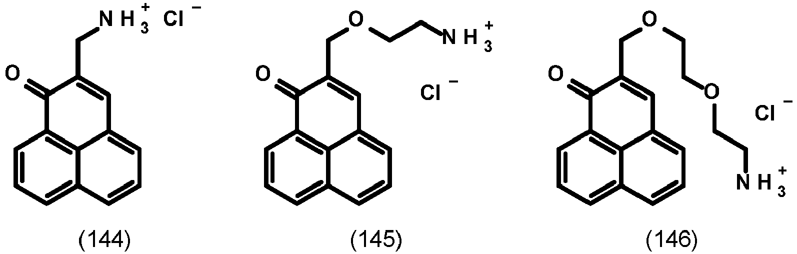 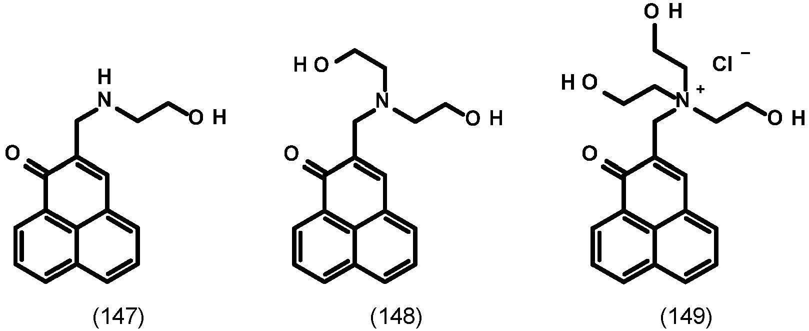 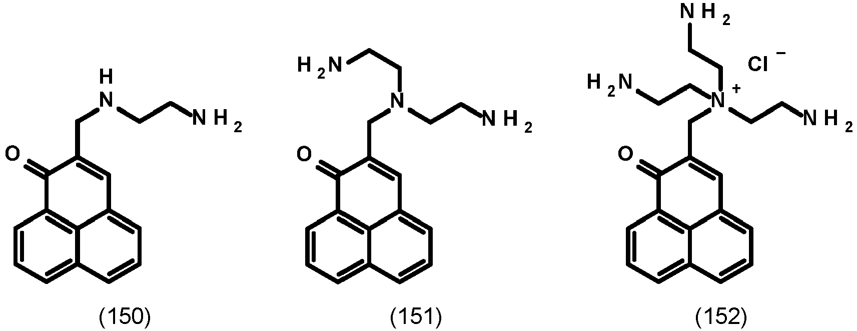 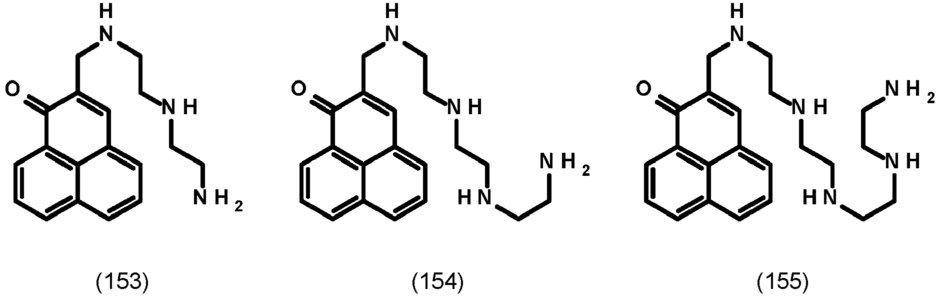 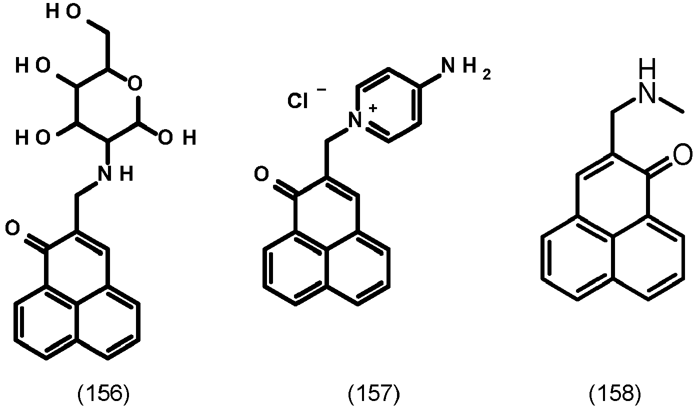 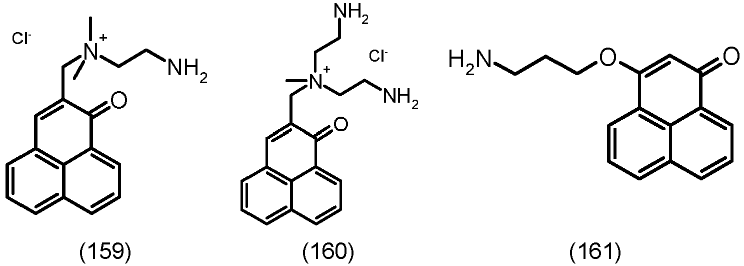 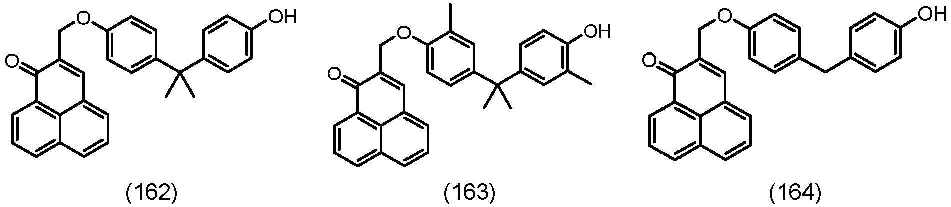 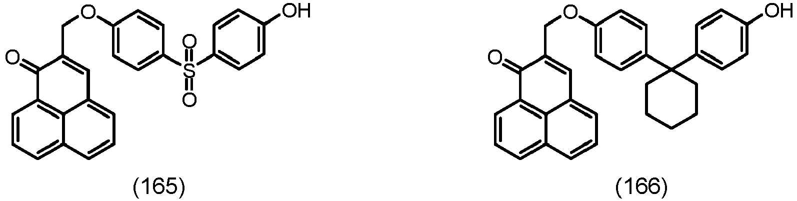 und Kombinationen davon besteht, ausgewählt wird, und
(b) wenigstens eine polymere Komponente und/oder Vorläufer davon.

4. Phenalen-1-on-Verbindung mit der allgemeinen Formel (1a): 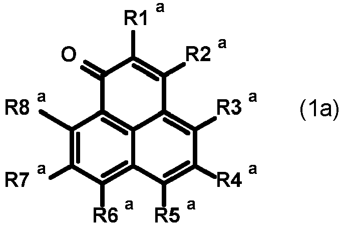 wobei die Reste R1^{a} bis R8^{a}, die jeweils unabhängig voneinander gleich oder voneinander verschieden sein können, jeweils Wasserstoff, Halogen, Alkyl mit 1 bis 12 C-Atomen, Alkylaryl mit 5 bis 20 C-Atomen, Aryl mit 5 bis 20 C-Atomen, *-O-Alkyl mit 1 bis 12 C-Atomen, *-O-Alkylaryl mit 5 bis 20 C-Atomen, *-O-Aryl mit 5 bis 20 C-Atomen, Ether mit 2 bis 12 C-Atomen, ein Rest der Formel *-O-C(=O)-R^{(Ia)}, ein Rest der Formel *-C(=O)-R^{(Ib)}, oder ein organischer Rest W1a, der wenigstens eine reaktive funktionelle Gruppe enthält, bedeutet, mit der Maßgabe, dass wenigstens einer der Reste R1^{a} oder R2^{a} ein organischer Rest W1a ist, wobei der wenigstens eine organische Rest W1a jeweils unabhängig voneinander ein Rest der allgemeinen Formel (41) bis (67) oder (98a) bis (98e) bedeutet: 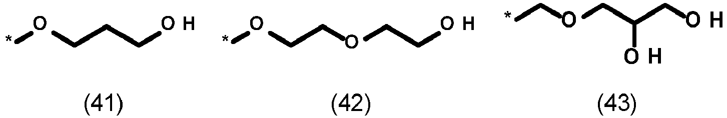 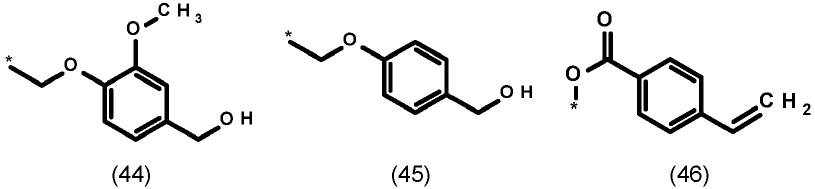 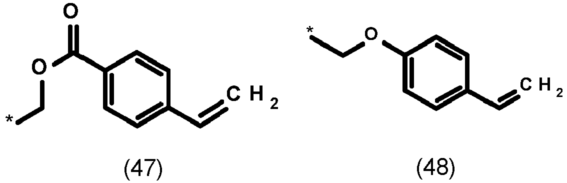 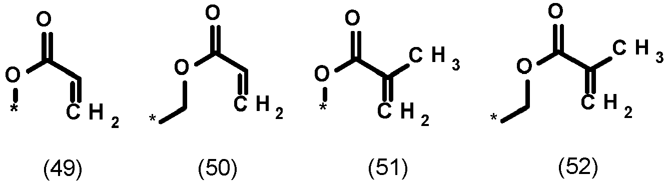 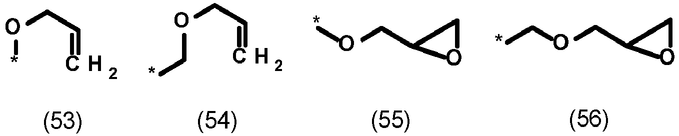 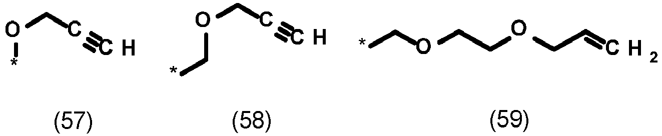 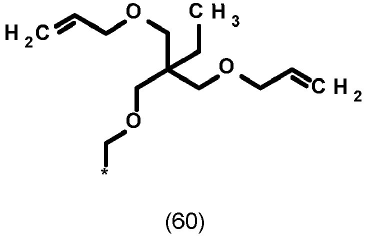 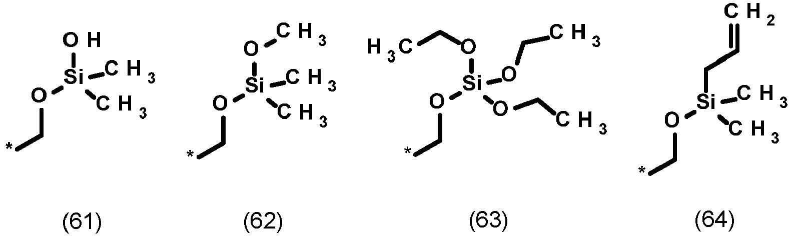 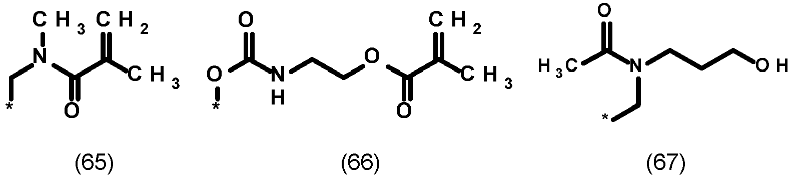 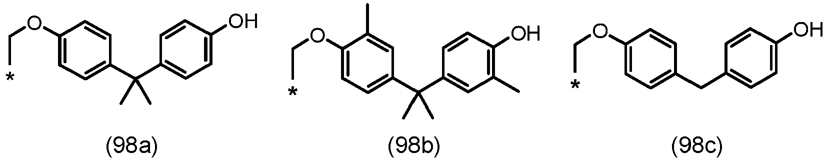 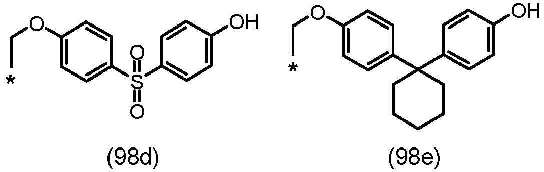 und wobei die Reste R^{(Ia)} und R^{(Ib)}jeweils unabhängig voneinander Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können.

5. Phenalen-1-on-Verbindung nach Anspruch 4,
wobei die wenigstens eine Phenalen-1-on-Verbindung mit der allgemeinen Formel (1a) aus der Gruppe, die aus Verbindung der Formel (101) bis (127), (162) bis (166) und Kombinationen davon besteht: 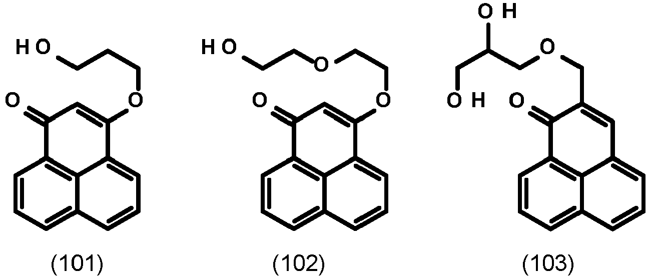 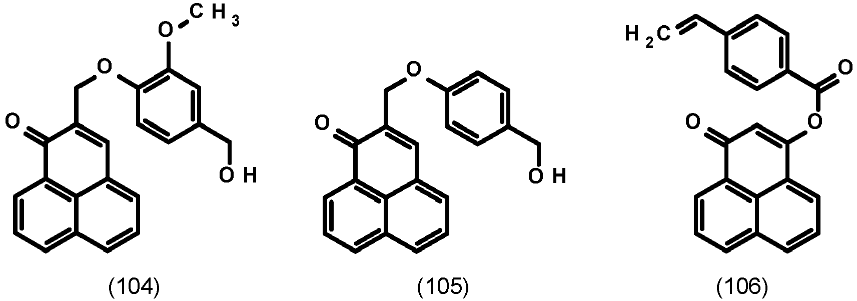 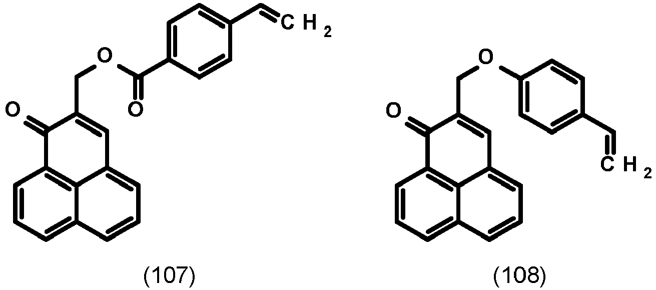 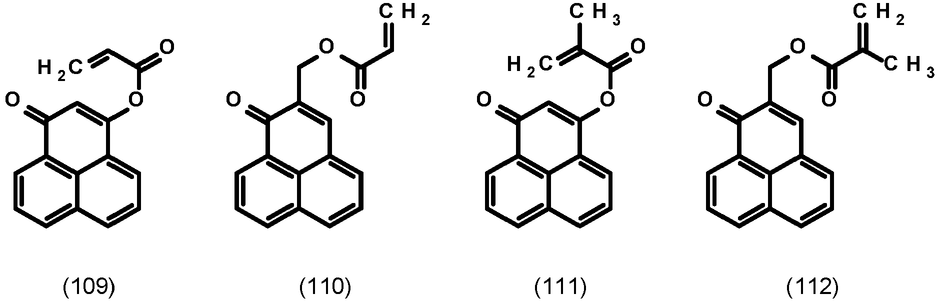 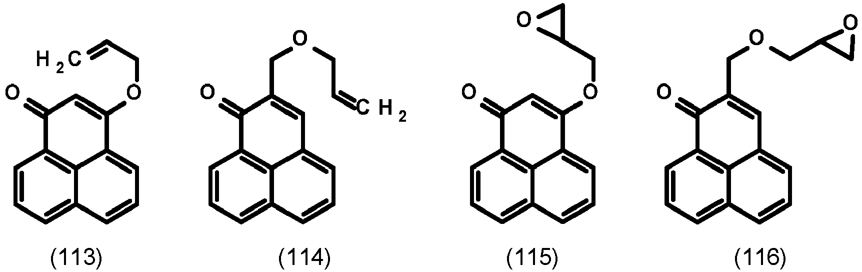 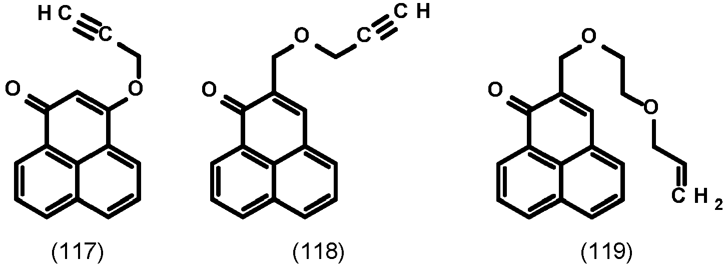 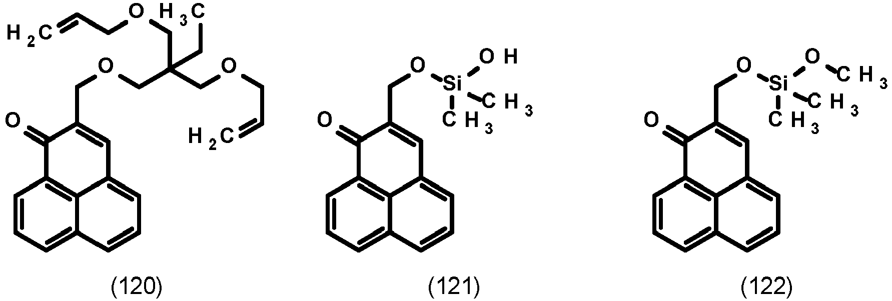 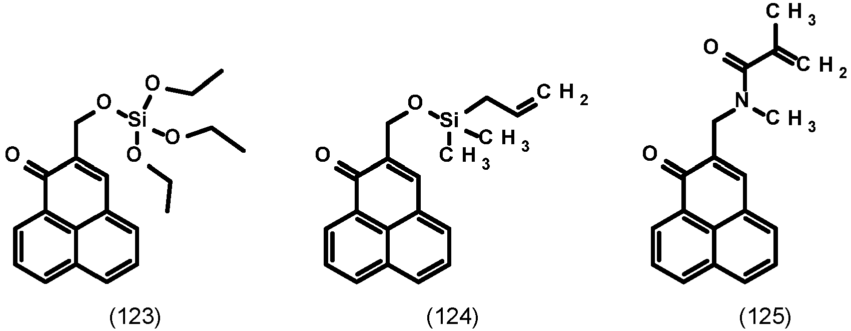 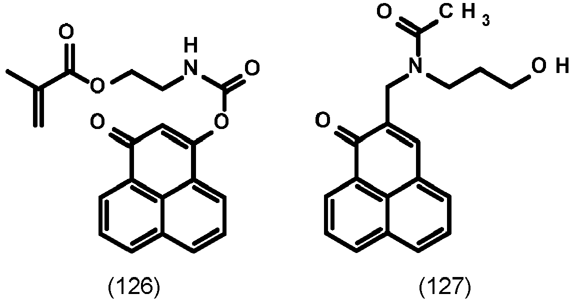 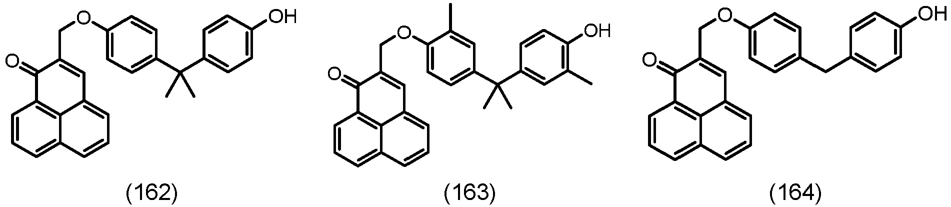 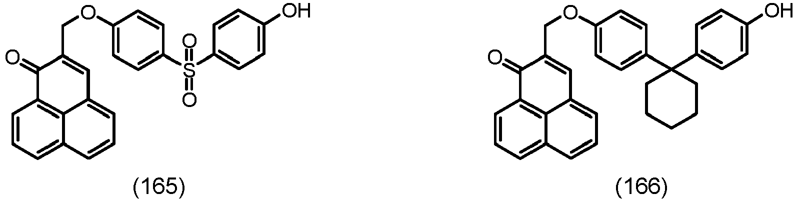 ausgewählt wird.

6. Photosensibilisatorzusammensetzung nach einem der Ansprüche 1 bis 3 umfassend:
(a) wenigstens eine Phenalen-1-on-Verbindung mit der allgemeinen Formel (1), die eine Phenalen-1-on-Verbindung nach einem der Ansprüche 4 bis 5 ist, und
(b) wenigstens eine polymere Komponente und/oder Vorläufer davon.

7. Photosensibilisatorzusammensetzung nach einem der Ansprüche 1 bis 3 oder 6, wobei die wenigstens eine polymere Komponente und/oder Vorläufer davon aus der Gruppe, die aus Polymerisaten und/oder Copolymerisaten von Acrylsäure und deren Ester, Methacrylsäure und deren Ester, Cyanacrylsäure und deren Ester, Acrylamid, Methacrylamid, Styrol, Siloxanen und deren Ester, Melamin, Acrylnitril, 1,3-Butadien, Epichlorhydrin, Polyolen, Polyisoprenen, Polyethern, Polyetherimiden, Polyvinylacetaten, Polycarbonaten, Polyethersulfonen, Carboxymethylcellulose, Alginat, und Kombinationen davon besteht, ausgewählt wird.

8. Photosensibilisatorzusammensetzung nach einem der Ansprüche 1 bis 3 oder 6 bis 7, wobei die Zusammensetzung weiterhin wenigstens ein Vernetzungsmittel, das ein Polyisocyanat, ein blockiertes Isocyanat, ein Alkyldiisocyanat oder Cycloalkyldiisocyanat oder Aryldiisocyanat, eine Verbindung der allgemeinen Formel SiZ₄, (R^{XII})SiZ₃, (R^{XII})₂SiZ₂, eine Verbindung mit mindestens zwei Epoxidresten, eine Verbindung mit mindestens zwei Acrylamidresten, eine Verbindung mit mindestens zwei Acrylatresten, eine Verbindung mit mindestens zwei Aldehydresten, eine Verbindung mit mindestens zwei Alkoholgruppen, eine Verbindung mit mindestens zwei Aminresten, ein Divinylsulfon, oder ein Kombinationen davon enthält, wobei der Rest R^{XII} jeweils unabhängig voneinander Alkyl, das gradkettig oder verzweigt sein kann, mit 1 bis 5 Kohlenstoffatomen, Phenyl oder Benzyl, vorzugsweise Methyl, Ethyl, n-Propyl, iso-Propyl, Phenyl oder Benzyl, bedeutet und wobei der Rest Z jeweils unabhängig voneinander Halogen, Hydroxyl, Alkoxyl mit 1 bis 4 Kohlenstoffatomen oder Alkylcarboxyl mit 1 bis 4 Kohlenstoffatomen, vorzugsweise Halogen oder Hydroxyl, bedeutet.

9. Photosensibilisatorzusammensetzung nach einem der Ansprüche 1 bis 3 oder 6 bis 8, wobei die wenigstens eine Phenalen-1-on-Verbindung mit der allgemeinen Formel (1) kovalent und/oder elektrostatisch, vorzugsweise kovalent, an die wenigstens eine polymere Komponente und/oder Vorläufer davon gebunden ist.

10. Photosensibilisatorzusammensetzung nach einem der Ansprüche 1 bis 3 oder 6 bis 9, wobei es sich bei der Photosensibilisatorzusammensetzung um einen Lack, eine Lasur, eine Dispersionsfarbe, eine Latexfarbe, eine Silikatfarbe, oder eine Kalkfarbe handelt.

11. Photosensibilisatorzusammensetzung nach einem der Ansprüche 1 bis 3 oder 6 bis 10, wobei es sich bei der Photosensibilisatorzusammensetzung um ein Granulat handelt.

12. Gegenstand, umfassend wenigstens eine ausgehärtete Polymerzusammensetzung, wobei die ausgehärtete Polymerzusammensetzung
(a) wenigstens eine Phenalen-1-on-Verbindung mit der allgemeinen Formel (1): 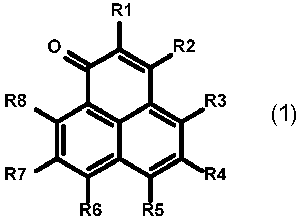
wobei die Reste R1 bis R8, die jeweils unabhängig voneinander gleich oder voneinander verschieden sein können, jeweils Wasserstoff, Halogen, Alkyl mit 1 bis 12 C-Atomen, Alkylaryl mit 1 bis 18 C-Atomen, Aryl mit 5 bis 20 C-Atomen, *-O-Alkyl mit 1 bis 12 C-Atomen, *-O-Alkylaryl mit 1 bis 18 C-Atomen, *-O-Aryl mit 5 bis 20 C-Atomen, Ether mit 2 bis 12 C-Atomen, ein Rest der Formel *-O-C(=O)-R^{(Ia)}, ein Rest der Formel *-C(=O)-R^{(Ib)}, oder ein organischer Rest W1, der wenigstens eine reaktive funktionelle Gruppe enthält, bedeuten, mit der Maßgabe, dass wenigstens einer der Reste R1 bis R7 ein organischer Rest W1 ist, wobei der organische Rest W1 jeweils unabhängig voneinander ein Rest mit der allgemeinen Formel (2) bis (6) bedeutet:
* - [(C(D)(E))_{d} - B]ₐ - (C(D)(E))ₘ - X (2)
* - A - [(C(D)(E))_{d} - B]_{c} - (C(D)(E))ₘ - X (3)
* - (C(D)(E))_{d} - Ar - (C(D)(E))ₙ - X (4)
* - [(C(D)(E))_{d} - B]_{b} - (C(D)(E))_{g} - Ar - (C(D)(E))ₙ - X (5)
* - A - [(C(D)(E))_{d} - B]_{f} - (C(D)(E))_{g} - Ar - (C(D)(E))ₙ - X (6)
wobei der Rest A jeweils unabhängig voneinander Sauerstoff, Schwefel, oder ein Rest mit der allgemeinen Formel (10a) bis (11a) bedeutet: 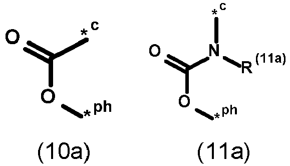
wobei *^{ph} jeweils einen Anknüpfungspunkt des Restes mit der allgemeinen Formel (10a) bis (11a) an ein C-Atom des Phenalen-Rings und *^{c} jeweils einen Anknüpfungspunkt des Restes mit der allgemeinen Formel (10a) bis (11a) an ein C-Atom des Restes (C(D)(E)) bezeichnet, und
wobei der Rest B jeweils unabhängig voneinander Sauerstoff, Schwefel oder ein Rest mit der allgemeinen Formel (10) bis (14) bedeutet: 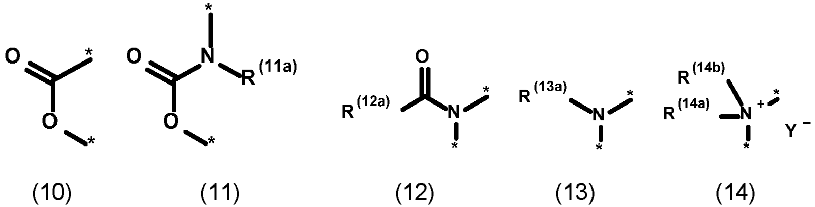
wobei die Reste R^{(Ia)}, R^{(Ib)}, R^{(11a)}, R^{(12a)}, R^{(13a)}, R^{(14a)} und R^{(14b)}, jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Halogen, Amino, Hydroxyl, *- O-Alkyl, das gradkettig oder verzweigt sein kann, mit 1 bis 3 Kohlenstoffatomen, Alkyl, das gradkettig oder verzweigt sein kann, mit 1 bis 3 Kohlenstoffatomen, Hydroxyalkyl, das gradkettig oder verzweigt sein kann, mit 1 bis 3 Kohlenstoffatomen und 1 bis 3 OH-Gruppen, Halogenalkyl, das gradkettig oder verzweigt sein kann, mit 1 bis 3 Kohlenstoffatomen und 1 bis 3 Halogen-Gruppen, und Kombinationen davon, vorzugsweise Chlor, Brom, Fluor, Methyl, Ethyl, n-Propyl, iso-Propyl, Methoxy, Ethoxy und Kombinationen davon, besteht, ausgewählt werden, substituiert sein können, wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sulfonat-Anion einer Sulfonsäure mit 1 bis 12 C-Atomen oder eine Kombination davon bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxyalkyl, das gradkettig oder verzweigt sein kann, mit 1 bis 5 Kohlenstoffatomen und 1 bis 5 OH-Gruppen, Phenyl, Benzyl, ein Rest der Formel *-L-R^{(II)}, ein Rest der Formel *-L-C(=L)-R^{(III)}, ein Rest der Formel *-(CH₂)_{q}-X, ein Rest der Formel *-L- (CH₂)_{q}-X, oder ein Rest der Formel *-(CH₂)ₛ-L-(CH₂)ₜ-X bedeuten, wobei der Rest L jeweils unabhängig voneinander Sauerstoff oder Schwefel, vorzugsweise Sauerstoff, bedeutet, wobei die Reste R^{(II)} und R^{(III)} jeweils unabhängig voneinander Wasserstoff, Methyl, Ethyl, n-Propyl, iso-Propyl, n-Butyl, sec-Butyl, iso-Butyl, n-Pentyl, Phenyl oder Benzyl bedeuten, und wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, die Indices q, s und t jeweils unabhängig voneinander eine ganze Zahl von 1 bis 5 bedeuten,
wobei die Indices a, c, f, g und n jeweils unabhängig voneinander eine ganze Zahl von 0 bis 5 bedeuten,
und wobei die Indices b, d und m jeweils unabhängig voneinander eine ganze Zahl von 1 bis 5 bedeuten,
wobei der Rest Ar jeweils unabhängig voneinander einen Rest der allgemeinen Formel (25a) bis (31) bedeutet: 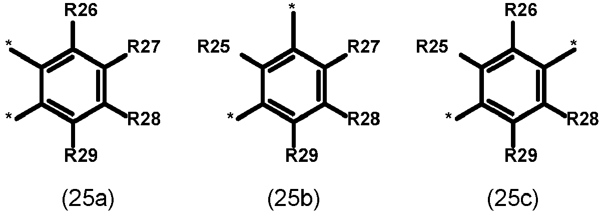 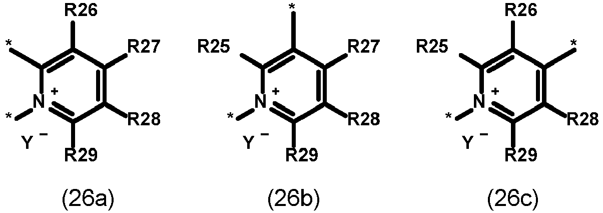 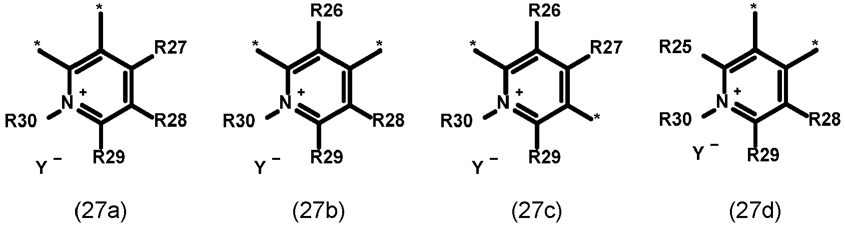 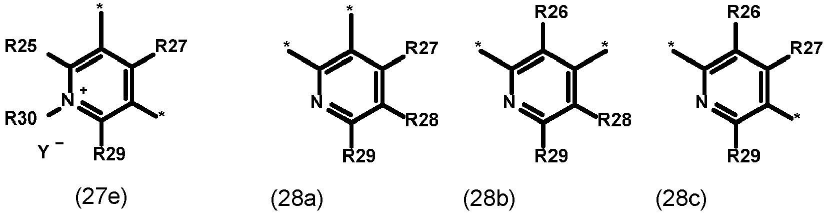 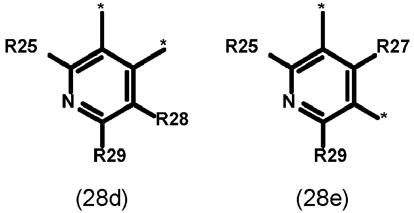 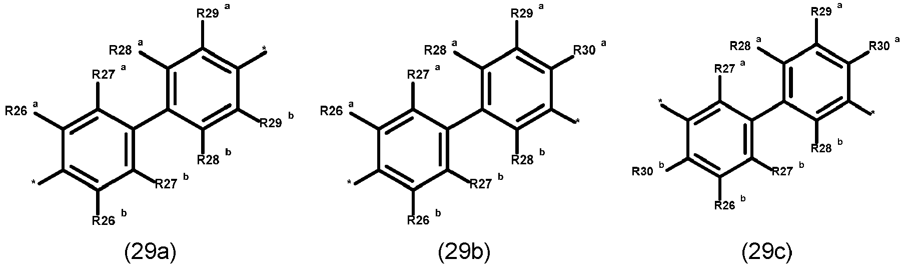 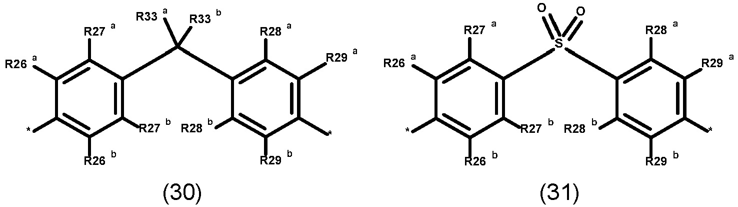
wobei die Reste R25, R26, R27, R28, R29, R26^{a}, R26^{b}, R27^{a}, R27^{b}, R28^{a}, R28^{b}, R29^{a}, R29^{b}, R30^{a} und R30^{b} jeweils unabhängig voneinander Wasserstoff, Hydroxy, Amino, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, und wobei der Rest R30 jeweils unabhängig voneinander Wasserstoff oder Alkyl, das gradkettig oder verzweigt sein kann, mit 1 bis 5 Kohlenstoffatomen bedeutet, und wobei die Reste R33^{a} und R33^{b} jeweils unabhängig voneinander Wasserstoff, Hydroxy, Amino, Alkyl, das gradkettig oder verzweigt sein kann, mit 1 bis 5 Kohlenstoffatomen, Perfluoralkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl, Benzyl oder wenn zusammen genommen ein Cycloalkyl, das gradkettig oder verzweigt sein kann, mit 4 bis 9 Kohlenstoffatomen, oder ein 9H-Fluoren-9-ylidenrest bedeuten, und
wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, wenigstens ein Carboxylat-Anion einer Carbonsäure mit 1 bis 15 Kohlenstoffatomen, wenigstens ein Sufonat-Anion einer Sulfonsäure mit 1 bis 12 C-Atomen oder eine Kombination davon bedeutet, und
wobei der Rest X jeweils unabhängig voneinander eine reaktive funktionelle Gruppe ist, die *-N(R^{(VI)})(R^{(VII)}), *-OH, *-SH, *-NCO, *-NCS, *-Si(R^{(VIII)})(R^{(IX)})-[O-Si(R^{(X)})(R^{(XI)})]ₚ-Z oder ein Rest mit der allgemeinen Formel (20) bis (24) bedeutet: 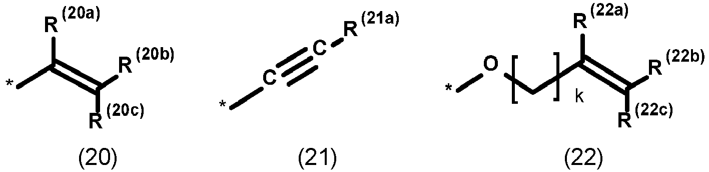 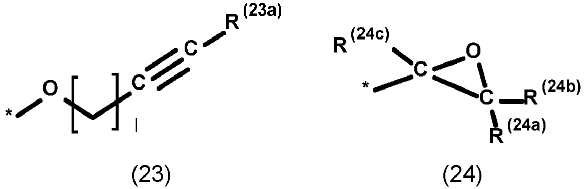
wobei die Reste R^{(20a)}, R^{(20b)}, R^{(20c)}, R^{(21a)}, R^{(22a)}, R^{(22b)}, R^{(22c)}, R^{(23a)}, R^{(24a)}, R^{(24b)}, und R^{(24c)} jeweils unabhängig voneinander Wasserstoff, Methyl, Ethyl, n-Propyl, iso-Propyl, n-Butyl, sec-Butyl, iso-Butyl, oder n-Pentyl bedeuten, und wobei die Indices l und k jeweils unabhängig voneinander eine ganze Zahl von 0 bis 4 bedeuten,
wobei die Reste R^{(VI)} und R^{(VII)} jeweils unabhängig voneinander Wasserstoff, Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei die Reste R^{(VIII)}, R^{(IX)}, R^{(X)} und R^{(XI)} jeweils unabhängig voneinander Wasserstoff, Alkyl, das gradkettig oder verzweigt sein kann, mit 1 bis 5 Kohlenstoffatomen, *-O-Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, wobei der Rest Z jeweils unabhängig voneinander Halogen, Hydroxyl, Alkoxyl mit 1 bis 4 Kohlenstoffatomen oder Alkylcarboxyl mit 1 bis 4 Kohlenstoffatomen, vorzugsweise Halogen oder Hydroxyl, bedeutet, und wobei der Index p jeweils unabhängig voneinander eine ganze Zahl von 0 bis 4 bedeutet, und
(b) wenigstens eine ausgehärtete polymere Komponente umfasst,
wobei die wenigstens eine Phenalen-1-on-Verbindung mit der allgemeinen Formel (1) kovalent und/oder elektrostatisch, vorzugsweise kovalent, an die wenigstens eine ausgehärtete polymere Komponente gebunden ist.

13. Gegenstand nach Anspruch 12, wobei die gehärtete Polymerzusammensetzung
(a) wenigstens eine Phenalen-1-on-Verbindung mit der Formel (1), wenigstens eine Phenalen-1-on-Verbindung nach einem der Ansprüche 4 bis 5 oder eine Kombination davon, und
(b) wenigstens eine ausgehärtete polymere Komponente umfasst,
wobei die wenigstens eine Phenalen-1-on-Verbindung mit der Formel (1), die wenigstens eine Phenalen-1-on-Verbindung nach einem der Ansprüche 5 bis 6 oder eine Kombination davon kovalent und/oder elektrostatisch, vorzugsweise kovalent, an die wenigstens eine ausgehärtete polymere Komponente gebunden ist.

14. Gegenstand nach einem der Ansprüche 12 oder 13, wobei die ausgehärtete Polymerzusammensetzung in Form einer Beschichtung, eines selbsttragenden Films, eines Gewebes oder eines Formgegenstandes vorliegt.

15. Nicht-medizinische Verwendung einer Photosensibilisatorzusammensetzung nach einem der Ansprüche 1 bis 3 oder 6 bis 11 und/oder einer Phenalen-1-on-Verbindung nach einem der Ansprüche 4 bis 5 und/oder eines Gegenstandes nach einem der Ansprüche 12 bis 14 zur, vorzugsweise photodynamischen, Inaktivierung von Mikroorganismen, die vorzugsweise aus der Gruppe, die aus Viren, Archäeen, Bakterien, Bakteriensporen, Biofilme von Bakterien, Pilzen, Pilzsporen, Protozoen, Algen und blutübertragbaren Parasiten besteht, und/oder einem Biofilm davon ausgewählt werden.

16. Nicht-medizinische Verwendung einer Photosensibilisatorzusammensetzung nach einem der Ansprüche 1 bis 3 oder 6 bis 11 und/oder einer Phenalen-1-on-Verbindung nach einem der Ansprüche 4 bis 5 zur photodynamischen Oberflächenreinigung und/oder Oberflächenbeschichtung eines Gegenstandes oder Raumes.

17. Nicht-medizinische Verwendung nach einem der Ansprüche 15 oder 16 zur Oberflächenbeschichtung von Gegenständen, vorzugsweise Medizinprodukten, Lebensmittelverpackungen, Verpackungsfolien, Textilien, Baustoffen, Spielzeugen, elektronischen Geräten, Möbeln oder Hygieneartikeln.

18. Nicht-medizinisches Verfahren zur, vorzugsweise photodynamischen, Inaktivierung von Mikroorganismen, die vorzugsweise Viren, Archäeen, Bakterien, Bakteriensporen, Biofilme von Bakterien, Pilzen, Pilzsporen, Protozoen, Algen, blutübertragbaren Parasiten oder Kombinationen davon umfassen, und/oder einem Biofilm davon, wobei das Verfahren folgende Schritte umfasst:
(A) in Kontakt bringen der Mikroorganismen und/oder einem Biofilm davon mit wenigstens einer Beschichtung, die durch Aushärten einer Photosensibilisatorzusammensetzung nach einem der Ansprüche 1 bis 3 oder 6 bis 11 hergestellt wurde und/oder wenigstens eine Phenalen-1-on-Verbindung nach einem der Ansprüche 4 bis 5 enthält, und/oder wenigstens eines Gegenstandes nach einem der Ansprüche 12 bis 14, und
(B) Bestrahlen der Mikroorganismen und/oder einem Biofilm davon und der, in der Beschichtung und/oder dem Gegenstand enthaltenen wenigstens einen Phenalen-1-on-Verbindung mit elektromagnetischer Strahlung geeigneter Wellenlänge und Energiedichte.

## Claims

1. Photosensitizer composition comprising
(a) at least one phenalen-1-one compound having the general formula (1): 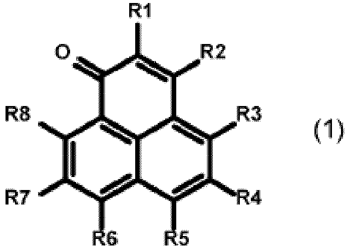
where the R1 to R8 radicals, which may each independently be the same or different, are each hydrogen, halogen, alkyl having 1 to 12 carbon atoms, alkylaryl having 5 to 20 carbon atoms, aryl having 5 to 20 carbon atoms, *-O-alkyl having 1 to 12 carbon atoms, *-O-alkylaryl having 5 to 20 carbon atoms, *-O-aryl having 5 to 20 carbon atoms, ethers having 2 to 12 carbon atoms, a radical of the formula *-O-C(=O)-R^{(Ia)}, a radical of the formula *-C (=O)-R^{(Ib)}, or an organic radical W1 containing at least one reactive functional group, with the proviso that at least one of the R1 to R7 radicals is an organic radical W1, where the organic radical W1 is in each case independently a radical having the general formula (2) to (6):
*-[(C(D) (E))_{d}-B]ₐ-(C(D) (E)ₘ-X (2)
*-A-[(C(D)(E))_{d}-B]_{c}-(C(D)(E))ₘ-X (3)
*-(C(D)(E))_{d}-Ar-(C(D)(E))ₙ-X (4)
*-[(C(D)(E))_{d}-B]_{b}-(C(D)(E))_{g}-Ar-(C(D)(E))ₙ-X (5)
*-A-[(C(D) (E))_{d}-B]_{f}-(C(D) (E))_{g}-Ar-(C(D)(E))ₙ-X (6)
where A is in each case independently oxygen, sulfur, or a radical having the general formula (10a) to (11a) 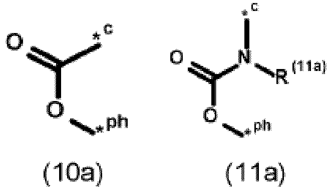
where *^{ph} in each case denotes a point of attachment of the radical having the general formula (10a) to (11a) to a carbon atom of the phenalene ring and *^{c} in each case denotes a point of attachment of the radical having the general formula (10a) to (11a) to a carbon atom of the (C(D)(E)) radical, and
where the B radical is in each case independently oxygen, sulfur or a radical having the general formula (10) to (14): 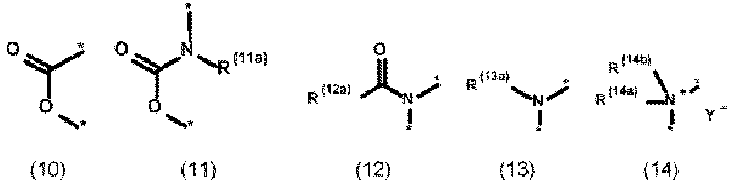
where the R^{(Ia)}, R^{(Ib)}, R^{(IIa)}, R^{(12a)}, R^{(13a)}, R^{(14a)} and R^{(14b)} radicals are each independently hydrogen, methyl, ethyl, n-propyl, isopropyl, n-butyl, sec-butyl, isobutyl, n-pentyl, phenyl or benzyl, and where phenyl or benzyl may each independently be unsubstituted or substituted by one or more radicals selected from the group consisting of halogen, amino, hydroxyl, *-O-alkyl which may be straight-chain or branched and has 1 to 3 carbon atoms, alkyl which may be straight-chain or branched and has 1 to 3 carbon atoms, hydroxyalkyl which may be straight-chain or branched and has 1 to 3 carbon atoms and 1 to 3 OH groups, haloalkyl which may be straight-chain or branched and has 1 to 3 carbon atoms and 1 to 3 halogen groups, and combinations thereof, preferably chlorine, bromine, fluorine, methyl, ethyl, n-propyl, isopropyl, methoxy, ethoxy and combinations thereof, where Y⁻ is an anion which is in each case independently fluoride, chloride, bromide, iodide, sulfate, hydrogensulfate, phosphate, hydrogenphosphate, dihydrogenphosphate, at least one carboxylate anion of a carboxylic acid having 1 to 15 carbon atoms, at least one sulfonate anion of a sulfonic acid having 1 to 12 carbon atoms or a combination thereof, and
where the D and E radicals are each independently hydrogen, halogen, hydroxyl, alkyl which may be straight-chain or branched and has 1 to 5 carbon atoms, hydroxyalkyl which may be straight-chain or branched and has 1 to 5 carbon atoms and 1 to 5 OH groups, phenyl, benzyl, a radical of the formula *-L-R^{(II)}, a radical of the formula *-L-C(=L)-R^{(III)}, a radical of the formula *-(CH₂)_{q}-X, a radical of the formula *-L-(CH₂)_{q}-X, or a radical of the formula *-(CH₂)ₛ-L-(CH₂)ₜ-X, where the L radical is in each case independently oxygen or sulfur, preferably oxygen, where the R^{(II)} and R^{(III)} radicals are each independently hydrogen, methyl, ethyl, n-propyl, isopropyl, n-butyl, sec-butyl, isobutyl, n-pentyl, phenyl or benzyl, and where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, the indices q, s and t are each independently an integer from 1 to 5,
where the indices a, c, f, g and n are each independently an integer from 0 to 5,
and where the indices b, d and m are each independently an integer from 1 to 5,
where the Ar radical is in each case independently a radical of the general formula (25a) to (31): 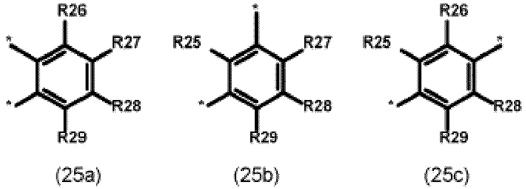 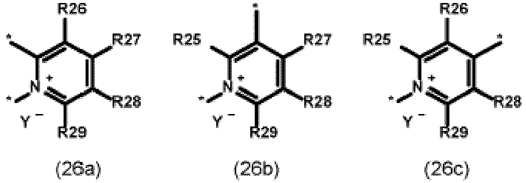 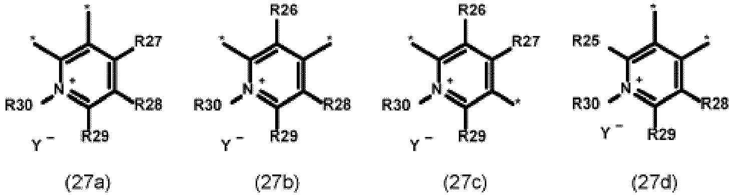 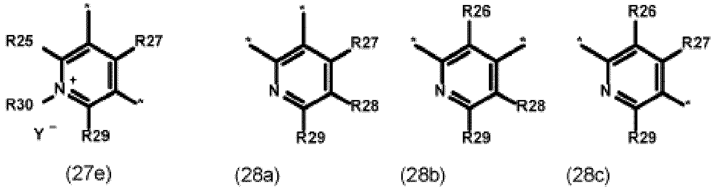 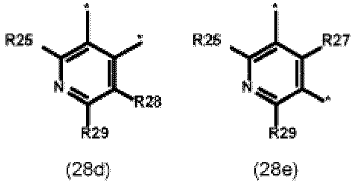 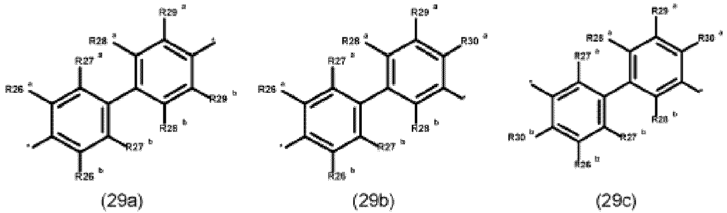 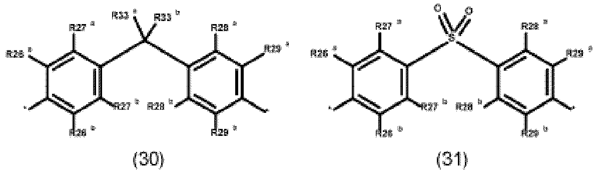
where the R25, R26, R27, R28, R29, R26^{a}, R26^{b}, R27^{a}, R27^{b}, R28^{a}, R28^{b}, R29^{a}, R29^{b}, R30^{a} and R30^{b} radicals are each independently hydrogen, hydroxy, amino, alkyl which may be straight-chain or branched and has 1 to 5 carbon atoms, *-O-alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, and where the R30 radical is in each case independently hydrogen or alkyl which may be straight-chain or branched and has 1 to 5 carbon atoms, and where the R33^{a} and R33^{b} radicals are each independently hydrogen, hydroxy, amino, alkyl which may be straight-chain or branched and has 1 to 5 carbon atoms, perfluoroalkyl which may be straight-chain or branched and has 1 to 5 carbon atoms, *-O-alkyl which may be straight-chain or branched and has 1 to 5 carbon atoms, phenyl, benzyl or, taken together, a cycloalkyl which may be straight-chain or branched and has 4 to 9 carbon atoms, or a 9H-fluoren-9-ylidene radical, and
where Y⁻ is an anion which is in each case independently fluoride, chloride, bromide, iodide, sulfate, hydrogensulfate, phosphate, hydrogenphosphate, dihydrogenphosphate, at least one carboxylate anion of a carboxylic acid having 1 to 15 carbon atoms, at least one sulfonate anion of a sulfonic acid having 1 to 12 carbon atoms or a combination thereof, and
where the X radical is in each case independently a reactive functional group which is *-N(R^{(VI)})(R^{(VII)}), *-OH, *-SH, *-NCO, *-NCS, *-Si(R^{(VIII)})(R^{(IX)})-[O-Si(R^{(x)})(R^{(XI)})]ₚ-Z or a radical having the general formula (20) to (24) : 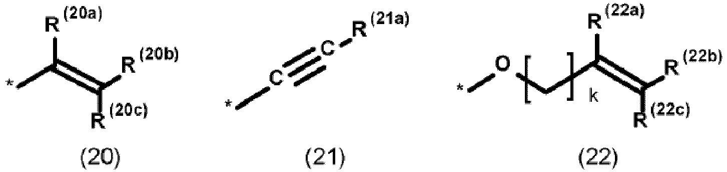 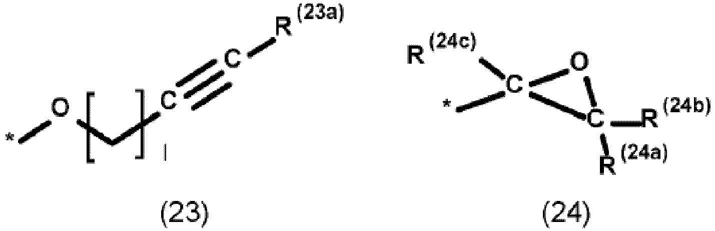
where the R^{(20a)}, R^{(20b)}, R^{(20c)}, R^{(21a)}, R^{(22a)}, R^{(22b)}, R^{(22c)}, R^{(23a)}, R^{(24a)}, R^{(24b)} and R^{(24c)} radicals are each independently hydrogen, methyl, ethyl, n-propyl, isopropyl, n-butyl, sec-butyl, isobutyl or n-pentyl, and where the indices 1 and k are each independently an integer from 0 to 4,
where the R^{(VI)} and R^{(VII)} radicals are each independently hydrogen, alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof,
where the R^{(VIII)}, R^{(IX)}, R^{(x)} and R^{(XI)} radicals are each independently hydrogen, alkyl which may be straight-chain or branched and has 1 to 5 carbon atoms, *-O-alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, where the Z radical is in each case independently halogen, hydroxy, alkoxy having 1 to 4 carbon atoms or alkylcarboxy having 1 to 4 carbon atoms, preferably halogen or hydroxy, and where the index p is in each case independently an integer from 0 to 4, and
(b) at least one polymeric component and/or precursor thereof.

2. Photosensitizer composition according to Claim 1,
wherein the at least one organic radical W1 is in each case independently a radical of the general formula (40) to (67) or (72) to (98e): 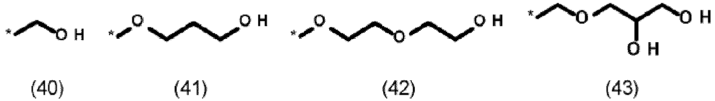 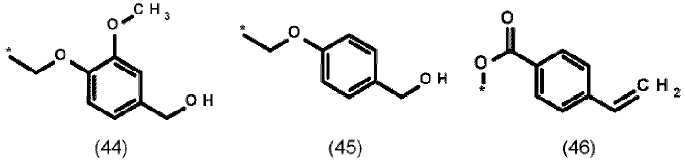 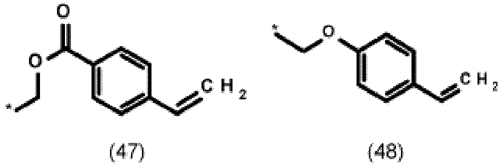 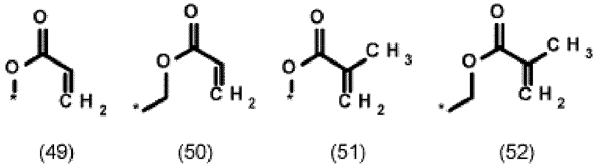 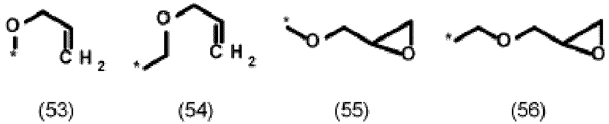 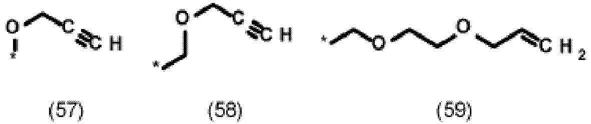 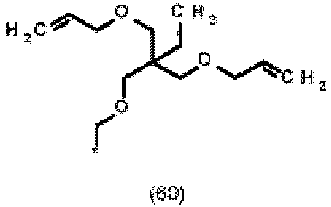 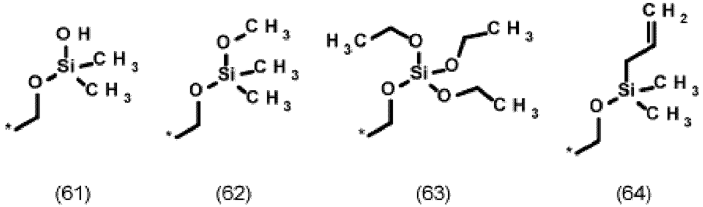 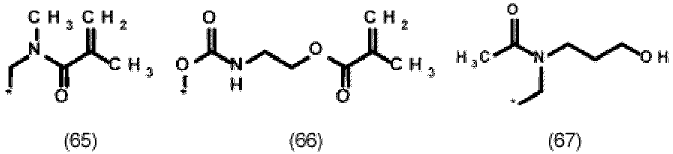 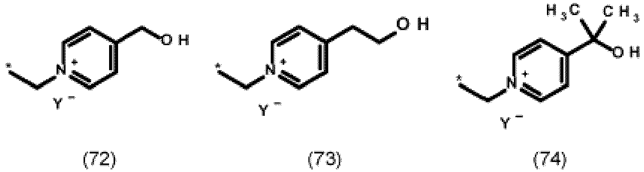 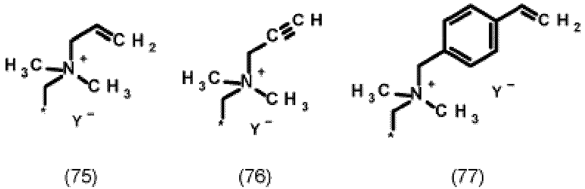 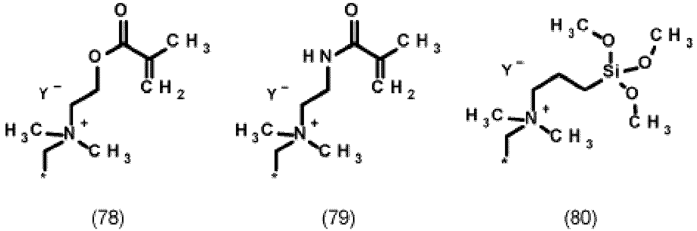 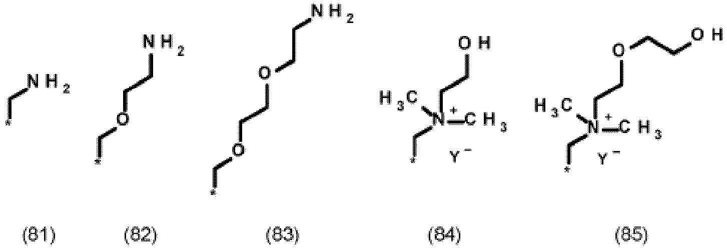 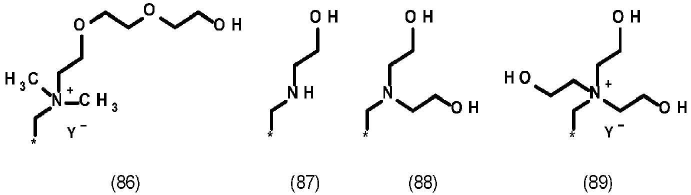 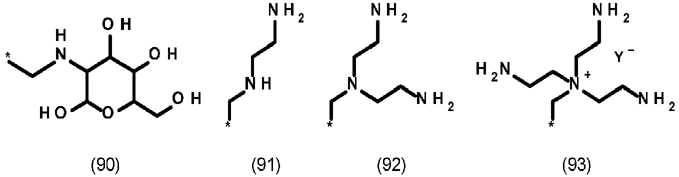 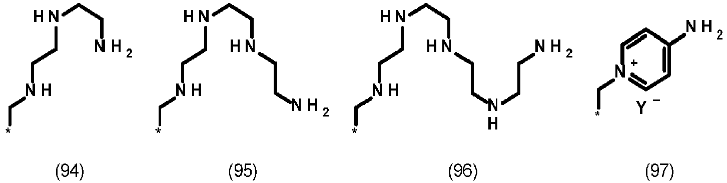 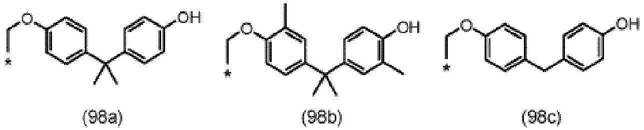 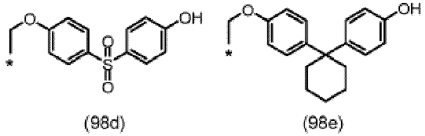 where Y⁻ is an anion which is in each case independently fluoride, chloride, bromide, iodide, sulfate, hydrogensulfate, phosphate, hydrogenphosphate, dihydrogenphosphate, at least one carboxylate anion of a carboxylic acid having 1 to 15 carbon atoms, at least one sulfonate anion of a sulfonic acid having 1 to 12 carbon atoms or a combination thereof.

3. Photosensitizer composition according to either of Claims 1 and 2, comprising:
(a) at least one phenalen-1-one compound having the general formula (1) which is selected from the group consisting of compound of the formula (100) to (127), (132) to (166): 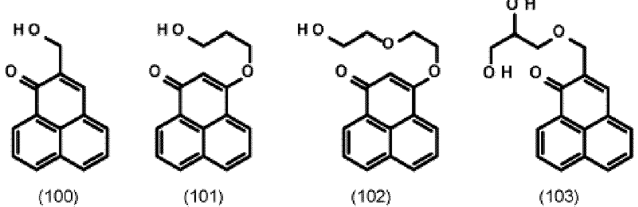 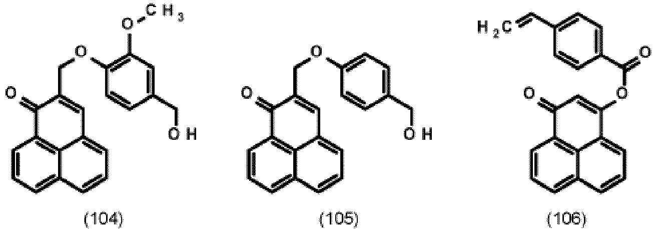 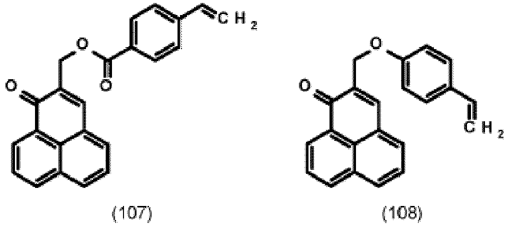 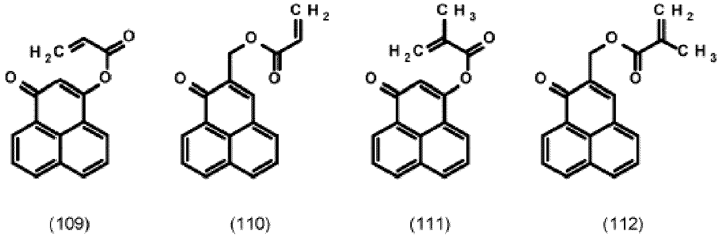 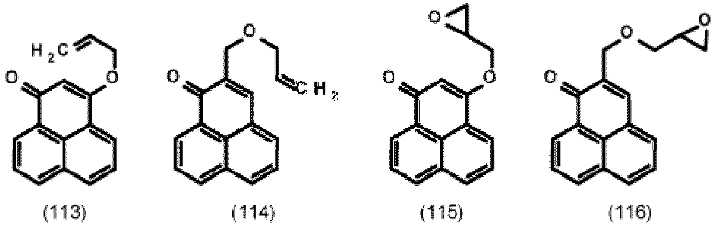 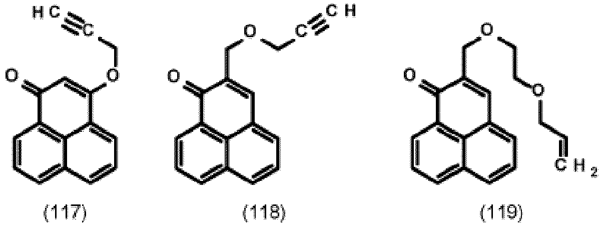 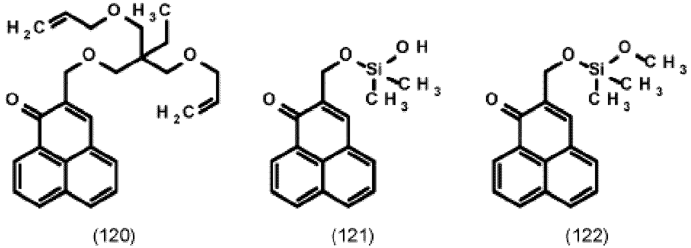 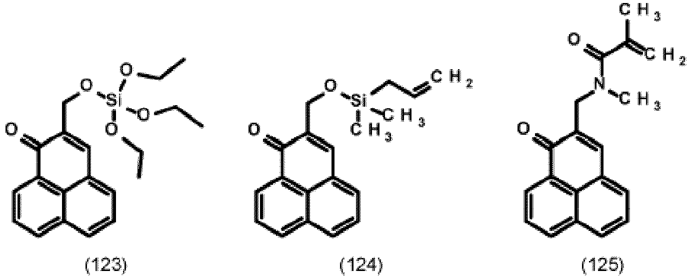 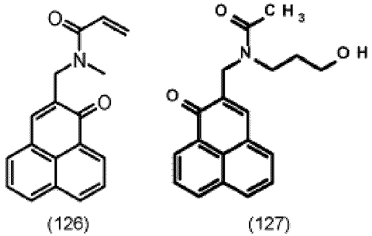 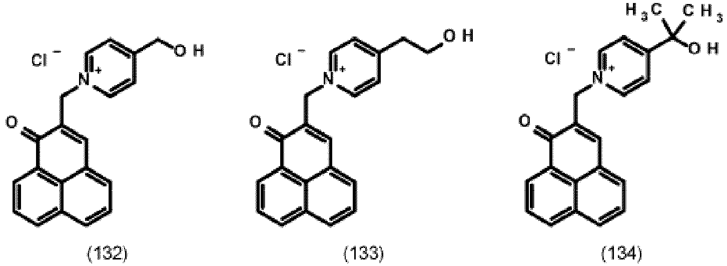 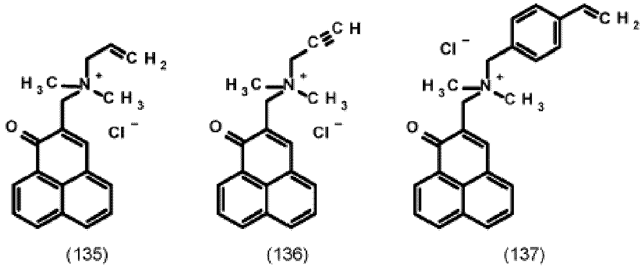 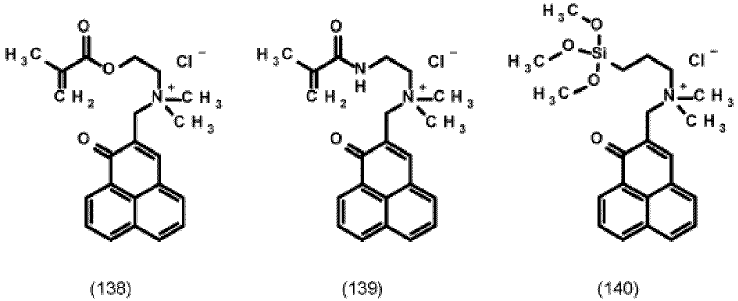 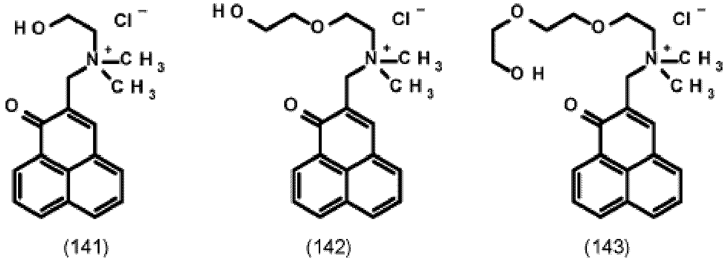 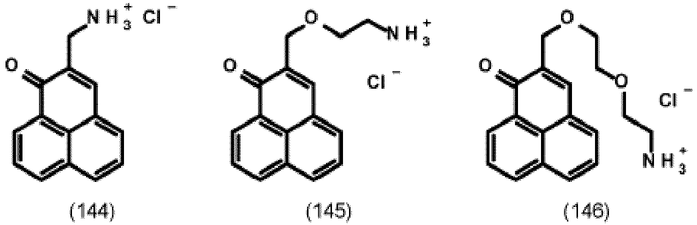 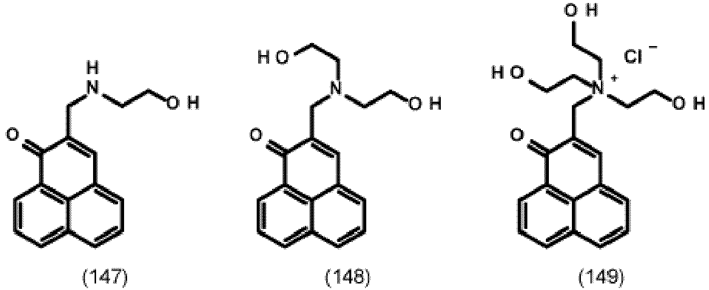 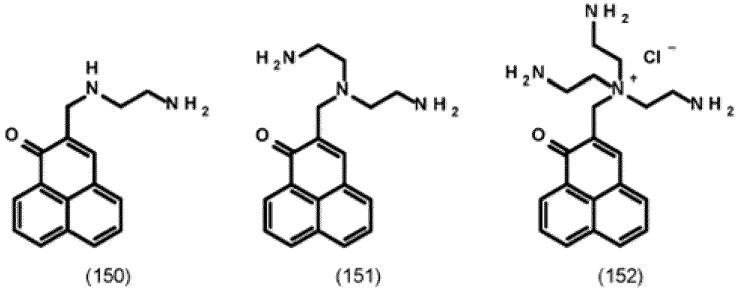 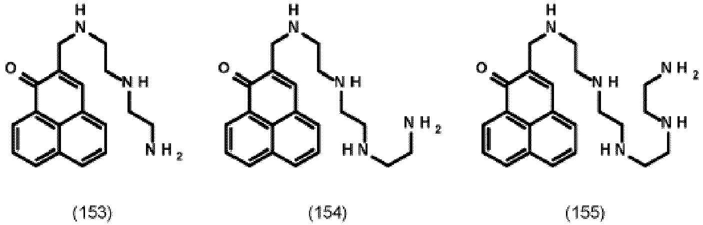 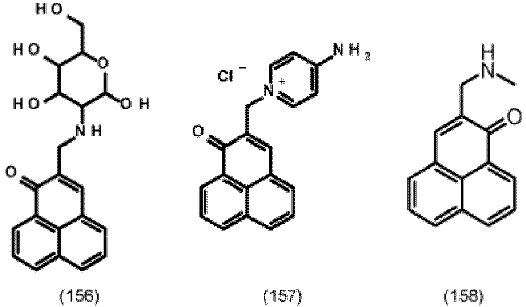 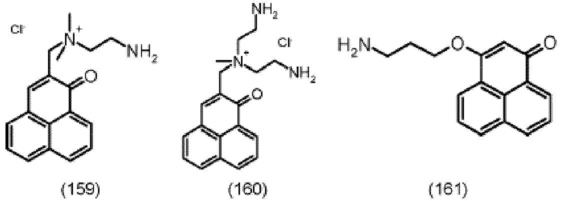 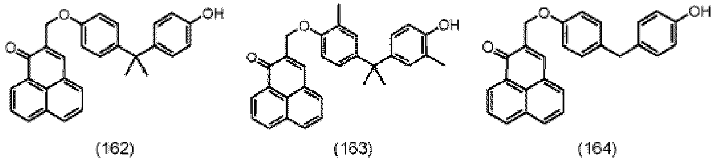 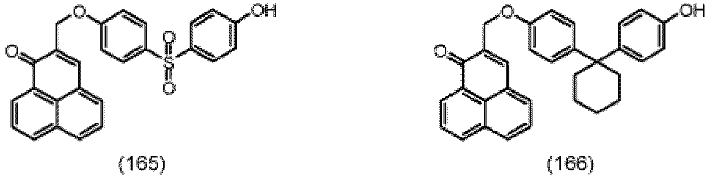 and combinations thereof, and
(b) at least one polymeric component and/or precursor thereof.

4. Phenalen-1-one compound having the general formula (1a) : 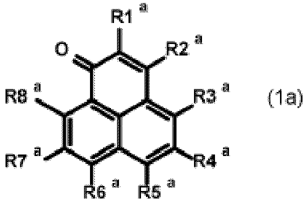 where the R1^{a} to R8^{a} radicals, which may each independently be the same or different, are each hydrogen, halogen, alkyl having 1 to 12 carbon atoms, alkylaryl having 5 to 20 carbon atoms, aryl having 5 to 20 carbon atoms, *-O-alkyl having 1 to 12 carbon atoms, *-O-alkylaryl having 5 to 20 carbon atoms, *-O-aryl having 5 to 20 carbon atoms, ethers having 2 to 12 carbon atoms, a radical of the formula *-O-C(=O)-R^{(Ia)}, a radical of the formula *-C (=O)-R^{(Ib)}, or an organic radical W1a containing at least one reactive functional group, with the proviso that at least one of the R1^{a} and R2^{a} radicals is an organic radical W1a, where the at least one organic radical W1a is in each case independently a radical having the general formula (41) to (67) or (98a) to (98e) : 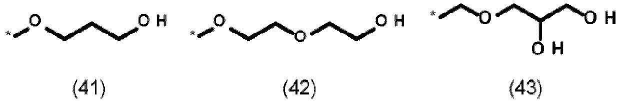 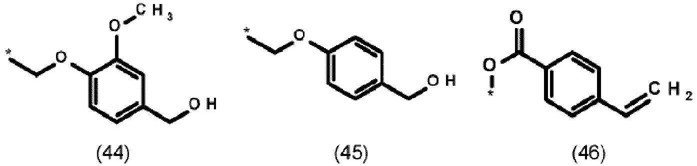 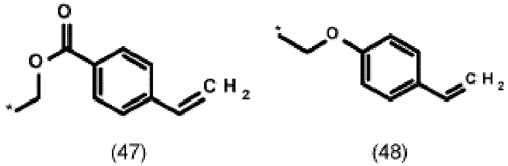 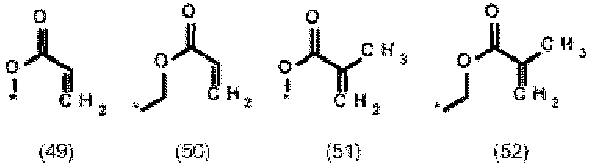 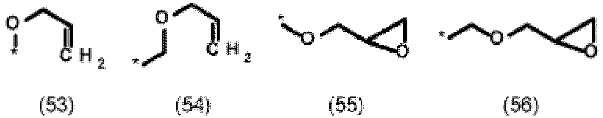 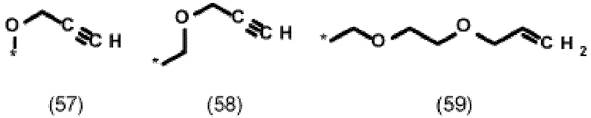 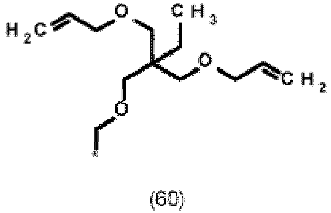 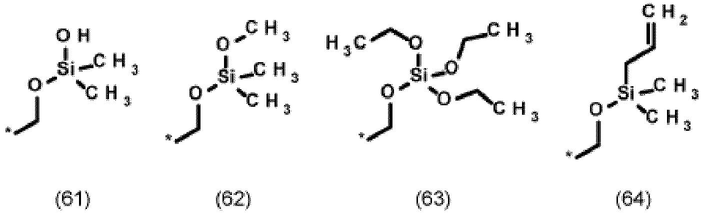 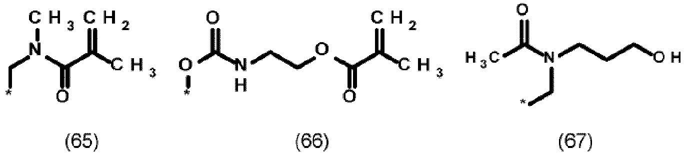 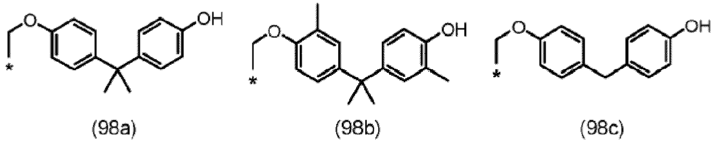 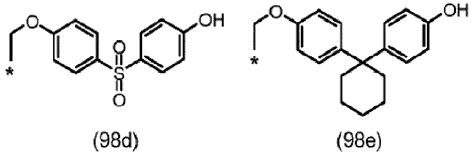 and where the R^{(Ia)} and R^{(Ib)} radicals are each independently methyl, ethyl, n-propyl, isopropyl, n-butyl, sec-butyl, isobutyl, n-pentyl, phenyl or benzyl, and where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof.

5. Phenalen-1-one compound according to Claim 4,
where the at least one phenalen-1-one compound having the general formula (1a) is selected from the group consisting of compound of the formula (101) to (127), (162) to (166) and combinations thereof:

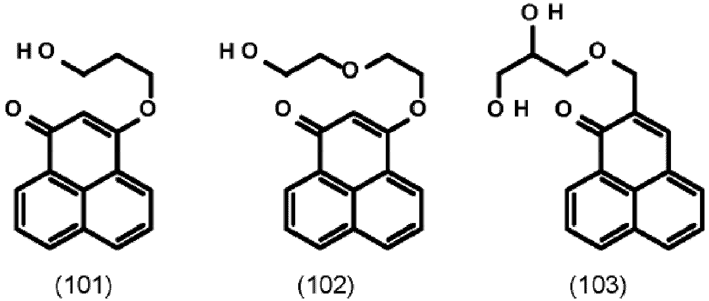

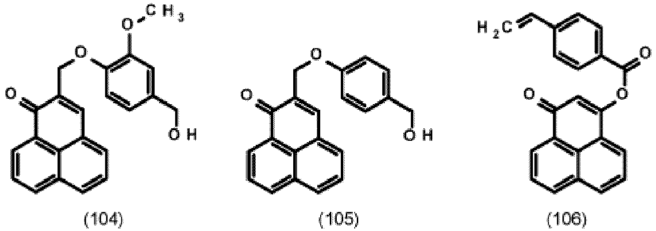

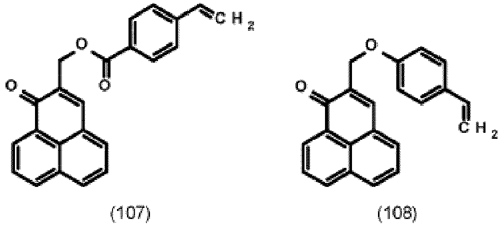

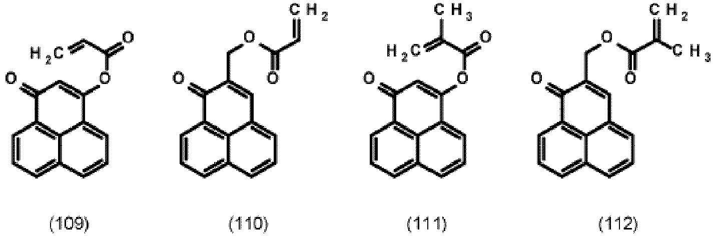

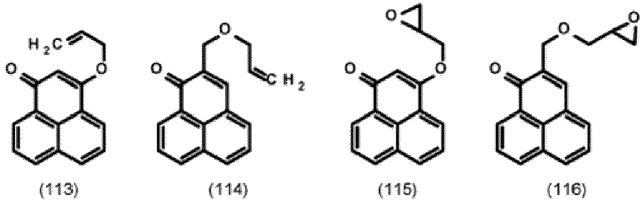

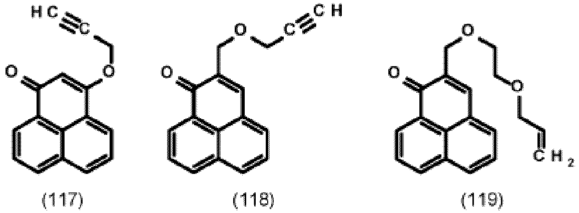

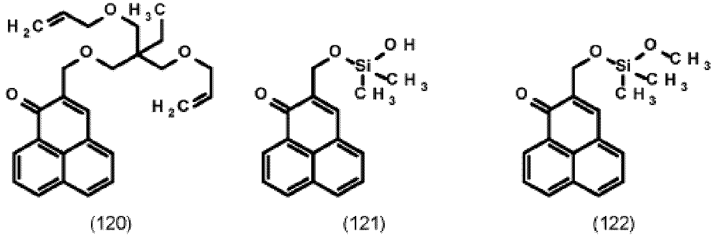

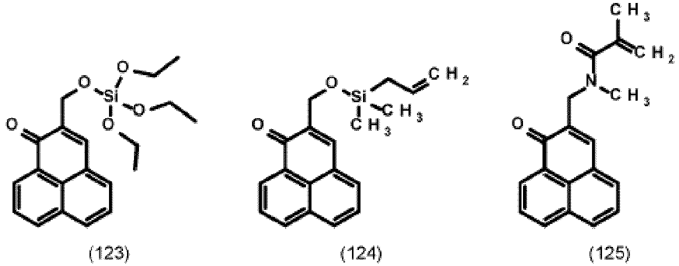

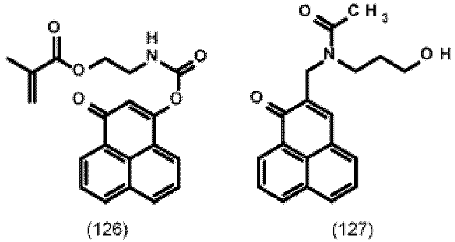

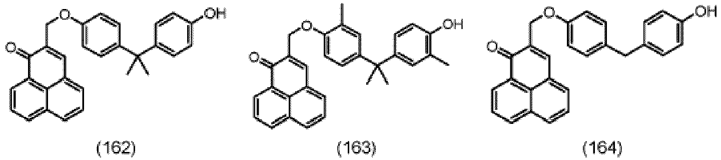

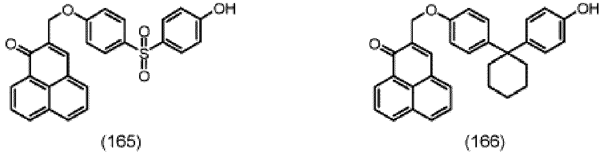

6. Photosensitizer composition according to any of Claims 1 to 3, comprising:
(a) at least one phenalen-1-one compound having the general formula (1), which is a phenalen-1-one compound according to either of Claims 4 and 5, and
(b) at least one polymeric component and/or precursor thereof.

7. Photosensitizer composition according to any of Claims 1 to 3 or 6, wherein the at least one polymeric component and/or precursor thereof is selected from the group consisting of polymers and/or copolymers of acrylic acid and esters thereof, methacrylic acid and esters thereof, cyanoacrylic acid and esters thereof, acrylamide, methacrylamide, styrene, siloxanes and esters thereof, melamine, acrylonitrile, 1,3-butadiene, epichlorohydrin, polyols, polyisoprenes, polyethers, polyetherimides, polyvinylacetates, polycarbonates, polyether sulfones, carboxymethylcellulose, alginate, and combinations thereof.

8. Photosensitizer composition according to any of Claims 1 to 3 or 6 and 7, wherein the composition further comprises a crosslinking agent which is a polyisocyanate, a blocked isocyanate, an alkyl diisocyanate or cycloalkyl diisocyanate or aryl diisocyanate, a compound of the general formula SiZ₄, (R^{XII})SiZ₃, (R^{XII})₂SiZ₂, a compound having at least two epoxy radicals, a compound having at least two acrylamide radicals, a compound having at least two acrylate radicals, a compound having at least two aldehyde radicals, a compound having at least two alcohol groups, a compound having at least two amine radicals, a divinyl sulfone, or a combination thereof, where the R^{XII} radical is in each case independently alkyl which may be straight-chain or branched and has 1 to 5 carbon atoms, phenyl or benzyl, preferably methyl, ethyl, n-propyl, isopropyl, phenyl or benzyl, and where the Z radical is in each case independently halogen, hydroxy, alkoxy having 1 to 4 carbon atoms or alkylcarboxy having 1 to 4 carbon atoms, preferably halogen or hydroxy.

9. Photosensitizer composition according to any of Claims 1 to 3 and 6 to 8, wherein the at least one phenalen-1-one compound having the general formula (1) is bonded covalently and/or electrostatically, preferably covalently, to the at least one polymeric component and/or precursor thereof.

10. Photosensitizer composition according to any of Claims 1 to 3 and 6 to 9, wherein the photosensitizer composition is a lacquer, a varnish, an emulsion paint, a latex paint, a silicate paint, or a limewash.

11. Photosensitizer composition according to any of Claims 1 to 3 and 6 to 10, wherein the photosensitizer composition is a pelletized material.

12. Article comprising at least one cured polymer composition, wherein the cured polymer composition comprises
(a) at least one phenalen-1-one compound having the general formula (1): 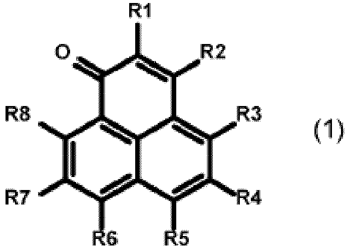
where the R1 to R8 radicals, which may each independently be the same or different, are each hydrogen, halogen, alkyl having 1 to 12 carbon atoms, alkylaryl having 1 to 18 carbon atoms, aryl having 5 to 20 carbon atoms, *-O-alkyl having 1 to 12 carbon atoms, *-O-alkylaryl having 1 to 18 carbon atoms, *-O-aryl having 5 to 20 carbon atoms, ethers having 2 to 12 carbon atoms, a radical of the formula *-O-C(=O)-R^{(Ia)}, a radical of the formula *-C (=O)-R^{(Ib)}, or an organic radical W1 containing at least one reactive functional group, with the proviso that at least one of the R1 to R7 radicals is an organic radical W1, where the organic radical W1 is in each case independently a radical having the general formula (2) to (6):
*-[(C(D)(E))_{d}-B]ₐ-(C(D)(E)ₘ-X (2)
*-A-[(C(D)(E))_{d}-B]_{c}-(C(D) (E))ₘ-X (3)
*-(C(D) (E))_{d}-Ar-(C(D)(E))ₙ-X (4)
*-[(C(D)(E))_{d}-B]_{b}-(C(D)(E))_{g}-Ar-(C(D)(E))ₙ-X (5)
*-A-[(C(D)(E))_{d}-B]_{f}-(C(D)(E))_{g}-Ar-(C(D)(E))ₙ-X (6)
where the A radical is in each case independently oxygen, sulfur, or a radical having the general formula (10a) to (11a) 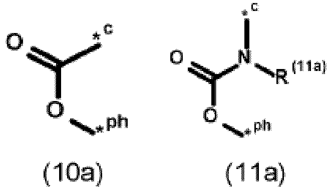
where *^{ph} in each case denotes a point of attachment of the radical having the general formula (10a) to (11a) to a carbon atom of the phenalene ring and *^{c} in each case denotes a point of attachment of the radical having the general formula (10a) to (11a) to a carbon atom of the (C(D)(E)) radical, and
where the B radical is in each case independently oxygen, sulfur or a radical having the general formula (10) to (14): 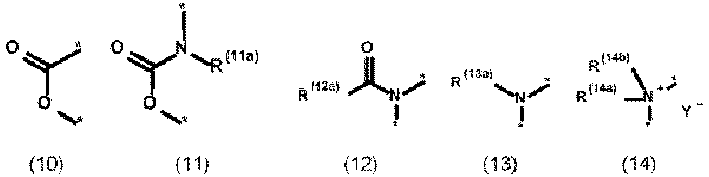
where the R^{(Ia)}, R^{(Ib)}, R^{(11a)}, R^{(12a)}, R^{(13a)}, R^{(14a)} and R^{(14b)} radicals are each independently hydrogen, methyl, ethyl, n-propyl, isopropyl, n-butyl, sec-butyl, isobutyl, n-pentyl, phenyl or benzyl, and where phenyl or benzyl may each independently be unsubstituted or substituted by one or more radicals selected from the group consisting of halogen, amino, hydroxyl, *-O-alkyl which may be straight-chain or branched and has 1 to 3 carbon atoms, alkyl which may be straight-chain or branched and has 1 to 3 carbon atoms, hydroxyalkyl which may be straight-chain or branched and has 1 to 3 carbon atoms and 1 to 3 OH groups, haloalkyl which may be straight-chain or branched and has 1 to 3 carbon atoms and 1 to 3 halogen groups, and combinations thereof, preferably chlorine, bromine, fluorine, methyl, ethyl, n-propyl, isopropyl, methoxy, ethoxy and combinations thereof, where Y- is an anion which is in each case independently fluoride, chloride, bromide, iodide, sulfate, hydrogensulfate, phosphate, hydrogenphosphate, dihydrogenphosphate, at least one carboxylate anion of a carboxylic acid having 1 to 15 carbon atoms, at least one sulfonate anion of a sulfonic acid having 1 to 12 carbon atoms or a combination thereof, and
where the D and E radicals are each independently hydrogen, halogen, hydroxyl, alkyl which may be straight-chain or branched and has 1 to 5 carbon atoms, hydroxyalkyl which may be straight-chain or branched and has 1 to 5 carbon atoms and 1 to 5 OH groups, phenyl, benzyl, a radical of the formula *-L-R^{(II)}, a radical of the formula *-L-C(=L)-R^{(III)}, a radical of the formula *-(CH₂)_{q}-X, a radical of the formula *-L-(CH₂)_{q}-X, or a radical of the formula *-(CH₂)ₛ-L-(CH₂)ₜ-X, where the L radical is in each case independently oxygen or sulfur, preferably oxygen, where the R^{(II)} and R^{(III)} radicals are each independently hydrogen, methyl, ethyl, n-propyl, isopropyl, n-butyl, sec-butyl, isobutyl, n-pentyl, phenyl or benzyl, and where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, the indices q, s and t are each independently an integer from 1 to 5,
where the indices a, c, f, g and n are each independently an integer from 0 to 5,
and where the indices b, d and m are each independently an integer from 1 to 5,
where the Ar radical is in each case independently a radical of the general formula (25a) to (31): 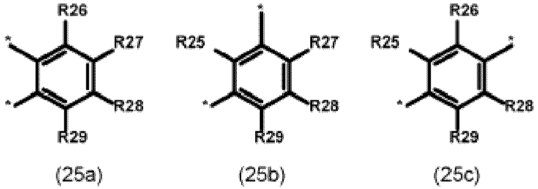 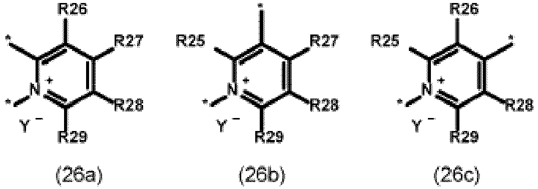 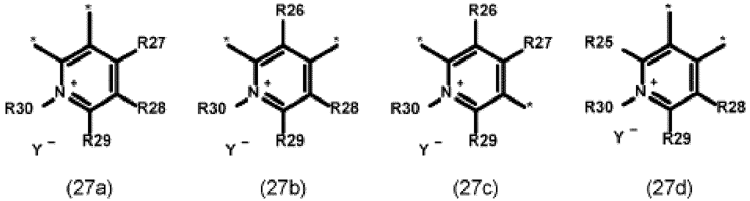 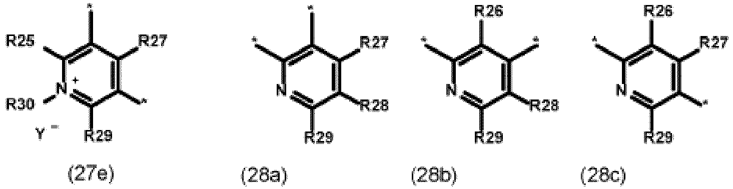 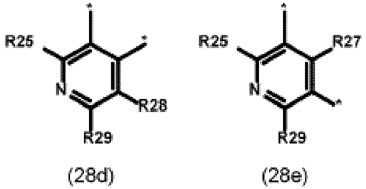 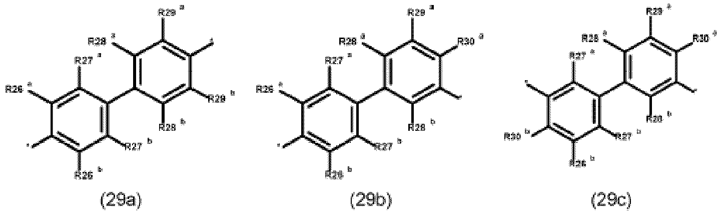 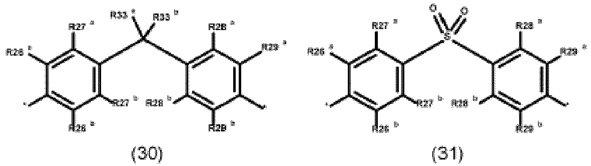
where the R25, R26, R27, R28, R29, R26^{a}, R26^{b}, R27^{a}, R27^{b}, R28^{a}, R28^{b}, R29^{a}, R29^{b}, R30^{a} and R30^{b} radicals are each independently hydrogen, hydroxy, amino, alkyl which may be straight-chain or branched and has 1 to 5 carbon atoms, *-O-alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, and where the R30 radical is in each case independently hydrogen or alkyl which may be straight-chain or branched and has 1 to 5 carbon atoms, and where the R33^{a} and R33^{b} radicals are each independently hydrogen, hydroxy, amino, alkyl which may be straight-chain or branched and has 1 to 5 carbon atoms, perfluoroalkyl which may be straight-chain or branched and has 1 to 5 carbon atoms, *-O-alkyl which may be straight-chain or branched and has 1 to 5 carbon atoms, phenyl, benzyl or, taken together, a cycloalkyl which may be straight-chain or branched and has 4 to 9 carbon atoms, or a 9H-fluoren-9-ylidene radical, and
where Y⁻ is an anion which is in each case independently fluoride, chloride, bromide, iodide, sulfate, hydrogensulfate, phosphate, hydrogenphosphate, dihydrogenphosphate, at least one carboxylate anion of a carboxylic acid having 1 to 15 carbon atoms, at least one sulfonate anion of a sulfonic acid having 1 to 12 carbon atoms or a combination thereof, and
where the X radical is in each case independently a reactive functional group which is *-N(R^{(VI)})(R^{(VII)}), *-OH, *-SH, *-NCO, *-NCS, *-Si(R^{(VIII)})(R^{(IX)})-[O-Si(R^{(X)})(R^{(XI)})]ₚ-Z or a radical having the general formula (20) to (24) : 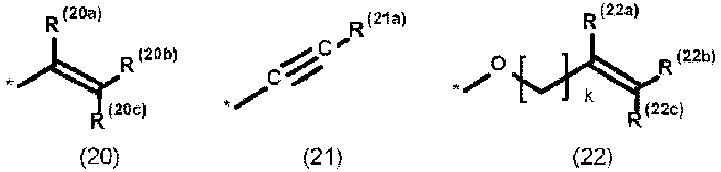 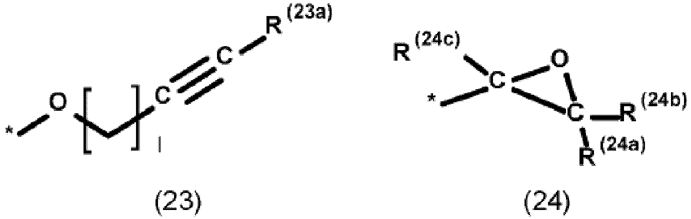
where the R^{(20a)}, R^{(20b)}, R^{(20c)}, R^{(21a)}, R^{(22a)}, R^{(22b)}, R^{(22c)}, R^{(23a)}, R^{(24a)}, R^{(24b)} and R^{(24c)} radicals are each independently hydrogen, methyl, ethyl, n-propyl, isopropyl, n-butyl, sec-butyl, isobutyl or n-pentyl, and where the indices 1 and k are each independently an integer from 0 to 4,
where the R^{(VI)} and R^{(VII)} radicals are each independently hydrogen, alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof,
where the R^{(VIIII)}, R^{(IX)}, R^{(X)} and R^{(XI)} radicals are each independently hydrogen, alkyl which may be straight-chain or branched and has 1 to 5 carbon atoms, *-O-alkyl which may be straight-chain or branched and has 1 to 5 carbon atoms, phenyl or benzyl, where phenyl and benzyl may each independently be unsubstituted or substituted by one or more radicals selected from the group consisting of chlorine, bromine, fluorine, methyl, ethyl, n-propyl, isopropyl, methoxy, ethoxy, and combinations thereof, where the Z radical is in each case independently halogen, hydroxy, alkoxy having 1 to 4 carbon atoms or alkylcarboxy having 1 to 4 carbon atoms, preferably halogen or hydroxy, and where the index p is in each case independently an integer from 0 to 4, and
(b) at least one cured polymeric component,
wherein the at least one phenalen-1-one compound having the general formula (1) is bonded covalently and/or electrostatically, preferably covalently, to the at least one cured polymeric component.

13. Article according to Claim 12, wherein the cured polymer composition comprises
(a) at least one phenalen-1-one compound having the formula (1), at least one phenalen-1-one compound according to either of Claims 4 and 5 or a combination thereof, and
(b) at least one cured polymeric component,
wherein the at least one phenalen-1-one compound having the formula (1), the at least one phenalen-1-one compound according to either of Claims 5 and 6 or a combination thereof is bonded covalently and/or electrostatically, preferably covalently, to the at least one cured polymeric component.

14. Article according to either of Claims 12 and 13, wherein the cured polymer composition is in the form of a coating, a self-supporting film, a fabric or a shaped article.

15. Non-medical use of a photosensitizer composition according to any of Claims 1 to 3 and 6 to 11 and/or of a phenalen-1-one compound according to either of Claims 4 and 5 and/or of an article according to any of Claims 12 to 14 for inactivation, preferably photodynamic inactivation, of microorganisms preferably selected from the group consisting of viruses, archaea, bacteria, bacterial spores, biofilms of bacteria, fungi, fungal spores, protozoa, algae and blood-transmissible parasites, and/or a biofilm thereof.

16. Non-medical use of a photosensitizer composition according to any of Claims 1 to 3 and 6 to 11 and/or of a phenalen-1-one compound according to either of Claims 4 and 5 for photodynamic surface cleaning and/or surface coating of an article or space.

17. Non-medical use according to either of Claims 15 and 16 for surface coating of articles, preferably medical products, food packaging, packaging films, textiles, building materials, toys, electronic devices, furniture or hygiene articles.

18. Non-medical method for inactivation, preferably photodynamic inactivation, of microorganisms which preferably comprise viruses, archaea, bacteria, bacterial spores, biofilms of bacteria, fungi, fungal spores, protozoa, algae and blood-transmissible parasites or combinations thereof, and/or a biofilm thereof, wherein the method comprises the following steps:
(A) contacting the microorganisms and/or a biofilm thereof with at least one coating that has been produced by curing a photosensitizer composition according to any of Claims 1 to 3 and 6 to 11 and/or contains at least one phenalen-1-one compound according to either of Claims 4 and 5, and/or at least one article according to any of Claims 12 to 14, and
(B) irradiating the microorganisms and/or a biofilm thereof and the at least one phenalen-1-one compound present in the coating and/or the article with electromagnetic radiation of suitable wavelength and energy density.

## Revendications

1. Composition photosensibilisante comprenant
(a) au moins un composé de phénalén-1-one de formule générale (1) : 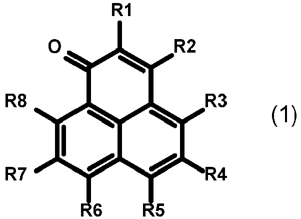
dans laquelle les restes R1 à R8, qui peuvent être chacun de manière indépendante identiques les uns aux autres ou différents les uns des autres, représentent chacun un hydrogène, un halogène, un groupe alkyle contenant 1 à 12 atomes de carbone, alkylaryle contenant 5 à 20 atomes de carbone, aryle contenant 5 à 20 atomes de carbone, *-O-alkyle contenant 1 à 12 atomes de carbone, *-O-alkylaryle contenant 5 à 20 atomes de carbone, *-O-aryle contenant 5 à 20 atomes de carbone, éther contenant 2 à 12 atomes de carbone, un reste de formule *-O-C(=O)-R^{(Ia)}, un reste de formule *-C(=O)-R^{(Ib)}, ou un reste organique W1 contenant au moins un groupe fonctionnel réactif, à la condition que l'un au moins des restes R1 à R7 soit un reste organique W1, chaque reste organique W1 représentant de manière indépendante un reste de formule générale (2) à (6) :
* - [(C(D)(E))_{d} - B]ₐ - (C(D)(E))ₘ - X (2)
* - A - [(C(D)(E))_{d} - B]_{c} - (C(D)(E))ₘ - X (3)
* - (C(D)(E))_{d} - Ar - (C(D)(E))n - X (4)
* - [(C(D)(E))_{d} - B]_{b} - (C(D)(E))g - Ar - (C(D)(E))ₙ - X (5)
* - A - [(C(D)(E))_{d} - B]_{f} - (C(D)(E))_{g} - Ar - (c(D)(E))ₙ - X (6)
dans laquelle chaque A représente indépendamment l'un de l'autre l'oxygène, le soufre ou un reste de formule générale (10a) à (11a) : 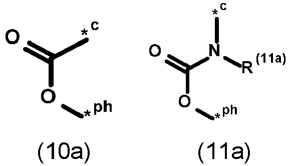
dans laquelle chaque *^{ph} désigne un point de rattachement du reste de formule générale (10a) à (11a) à un atome de carbone du noyau phénalène et chaque *c désigne un point de rattachement du reste de formule générale (10a) à (11a) à un atome de carbone du reste (C(D)(E)), et
dans laquelle chaque reste B représente de manière indépendante l'oxygène, le soufre ou un reste de formule générale (10) à (14) : 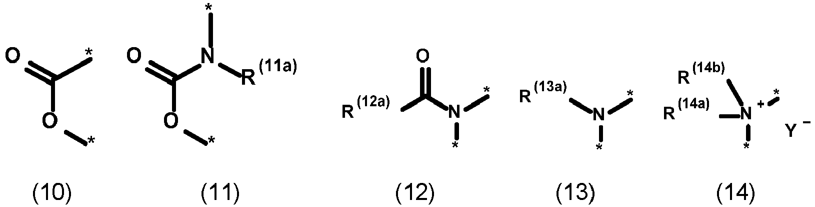
dans laquelle les restes R^{(Ia)}, R^{(Ib)}, R^{(11a)}, R^{(12a)}, R^{(13a)}, R^{(14a)} et R^{(14b)} représentent chacun de manière indépendante l'hydrogène, un groupe méthyle, éthyle, n-propyle, isopropyle, n-butyle, sec-butyle, isobutyle, n-pentyle, phényle ou benzyle, et dans laquelle chaque groupe phényle et benzyle peut être de manière indépendante non substitué ou substitué par un ou plusieurs restes choisis dans le groupe constitué par halogène, amino, hydroxyle, *-O-alkyle pouvant être à chaîne droite ou ramifiée et contenant 1 à 3 atomes de carbone, alkyle pouvant être à chaîne droite ou ramifiée et contenant 1 à 3 atomes de carbone, hydroxyalkyle pouvant être à chaîne droite ou ramifiée et contenant 1 à 3 atomes de carbone et 1 à 3 groupes OH, haloalkyle pouvant être à chaîne droite ou ramifiée et contenant 1 à 3 atomes de carbone et 1 à 3 groupes halogène, et des combinaisons de ceux-ci, de préférence chlore, brome, fluor, méthyle, éthyle, n-propyle, isopropyle, méthoxy, éthoxy et des combinaisons de ceux-ci, dans laquelle Y⁻ est un anion représentant respectivement de manière indépendante un fluorure, chlorure, bromure, iodure, sulfate, hydrogénosulfate, phosphate, hydrogénophosphate, dihydrogénophosphate, au moins un anion carboxylate d'un acide carboxylique contenant 1 à 15 atomes de carbone, au moins un anion sulfonate d'un acide sulfonique contenant 1 à 12 atomes de carbone ou une combinaison de ceux-ci, et
dans laquelle les restes D et E représentent chacun de manière indépendante un hydrogène, halogène, hydroxyle, alkyle pouvant être à chaîne droite ou ramifiée et contenant 1 à 5 atomes de carbone, hydroxyalkyle pouvant être à chaîne droite ou ramifiée et contenant 1 à 5 atomes de carbone et 1 à 5 groupes OH, phényle, benzyle, un reste de formule *-L-R^{(II)}, un reste de formule *-L-C(=L)-R^{(III)}, un reste de formule *-(CH₂)_{q}-X, un reste de formule *-L-(CH₂)_{q}-X, ou un reste de formule
*-(CH₂)ₛ-L-(CH₂)ₜ-X, chaque reste L représentant de manière indépendante l'oxygène ou le soufre, de préférence l'oxygène, les restes R^{(II)} et R^{(III)} représentant chacun de manière indépendante un hydrogène, méthyle, éthyle, n-propyle, isopropyle, n-butyle, sec-butyle, isobutyle, n-pentyle, phényle ou benzyle, et chaque phényle et benzyle pouvant être de manière indépendante non substitué ou substitué par un ou plusieurs restes choisis dans le groupe constitué par le chlore, brome, fluor, méthyle, éthyle, n-propyle, isopropyle, méthoxy, éthoxy, et des combinaisons de ceux-ci, les indices q, s et t représentant chacun de manière indépendante un nombre entier valant de 1 à 5,
dans laquelle les indices a, c, f, g et n représentent chacun de manière indépendante un nombre entier valant de 0 à 5,
et dans laquelle les indices b, d et m représentent chacun de manière indépendante un nombre entier valant de 1 à 5,
dans laquelle chaque reste Ar représente de manière indépendante un reste de formule générale (25a) à (31) : 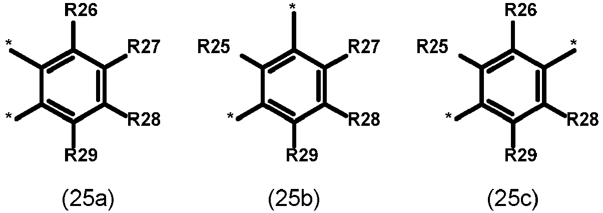 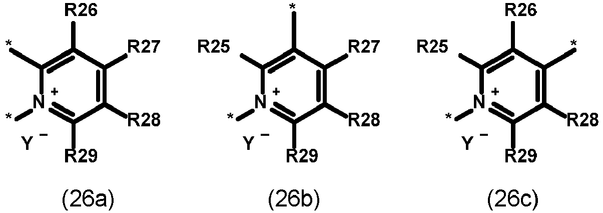 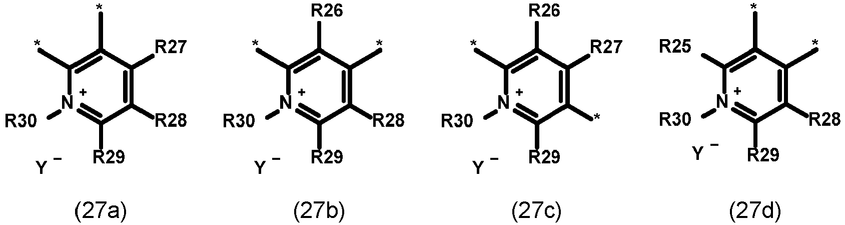 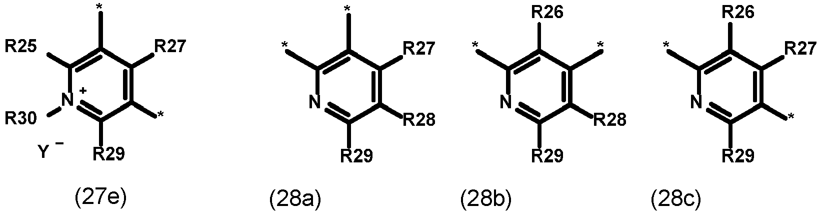 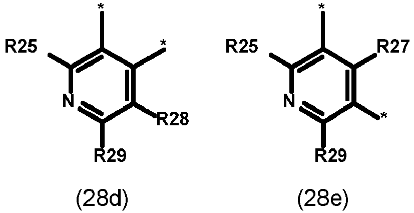 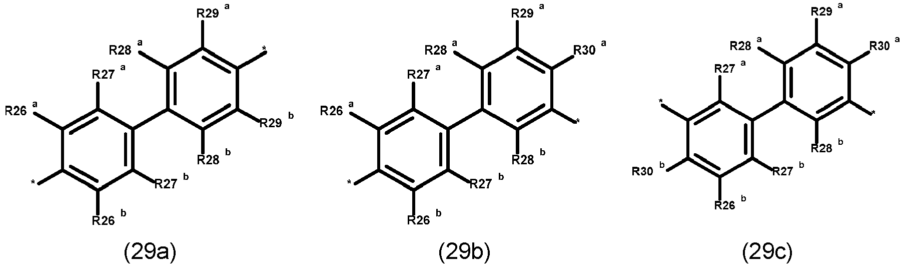 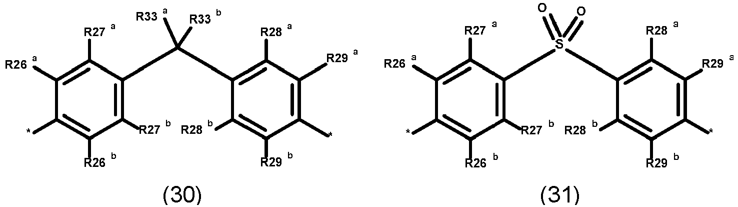
dans laquelle les restes R25, R26, R27, R28, R29, R26^{a}, R26^{b}, R27^{a}, R27^{b}, R28^{a}, R28^{b}, R29^{a}, R29^{b}, R30^{a} et R30^{b} représentent chacun de manière indépendante un hydrogène, hydroxy, amino, alkyle pouvant être à chaîne droite ou ramifiée et contenant 1 à 5 atomes de carbone, *-O-alkyle pouvant être à chaîne droite ou ramifiée et contenant 1 à 5 atomes de carbone, phényle ou benzyle, chaque phényle et benzyle pouvant être de manière indépendante non substitué ou substitué par un ou plusieurs restes choisis dans le groupe constitué par le chlore, brome, fluor, méthyle, éthyle, n-propyle, isopropyle, méthoxy, éthoxy, et des combinaisons de ceux-ci, et chaque reste R30 représentant de manière indépendante un hydrogène ou un alkyle pouvant être à chaîne droite ou ramifiée et contenant 1 à 5 atomes de carbone, et les restes R33^{a} et R33^{b} représentant chacun de manière indépendante un hydrogène, hydroxy, amino, alkyle pouvant être à chaîne droite ou ramifiée et contenant 1 à 5 atomes de carbone, perfluoroalkyle pouvant être à chaîne droite ou ramifiée et contenant 1 à 5 atomes de carbone,
*-O-alkyle pouvant être à chaîne droite ou ramifiée et contenant 1 à 5 atomes de carbone, phényle, benzyle ou, lorsqu'ils sont pris ensemble, un cycloalkyle pouvant être à chaîne droite ou ramifiée et contenant 4 à 9 atomes de carbone, ou un reste 9H-fluorén-9-ylidène, et
dans laquelle Y⁻ est un anion représentant respectivement de manière indépendante un fluorure, chlorure, bromure, iodure, sulfate, hydrogénosulfate, phosphate, hydrogénophosphate, dihydrogénophosphate, au moins un anion carboxylate d'un acide carboxylique contenant 1 à 15 atomes de carbone, au moins un anion sulfonate d'un acide sulfonique contenant 1 à 12 atomes de carbone ou une combinaison de ceux-ci, et
dans laquelle chaque reste X est de manière indépendante un groupe fonctionnel réactif représentant *-N(R^{(VI)})(R^{(VII)}), *-OH, *-SH, *-NCO, *-NCS, *-Si(R^{(VIII)})(R^{(IX)})-[O-Si(R^{(X)})(R^{(XI)})]ₚ-Z ou un reste de formule générale (20) à (24) : 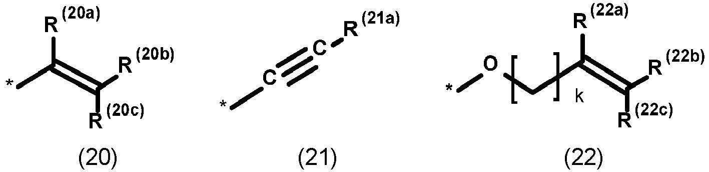 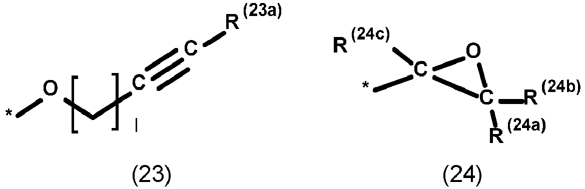
dans laquelle les restes R^{(20a)}, R^{(20b)}, R^{(20c)}, R^{(21a)}, R^{(22a)}, R^{(22b)}, R^{(22c)}, R^{(23a)}, R^{(24a)}, R^{(24b)} et R^{(24c)} représentent chacun de manière indépendante un hydrogène, méthyle, éthyle, n-propyle, isopropyle, n-butyle, sec-butyle, isobutyle ou n-pentyle, et dans laquelle les indices I et k représentent chacun de manière indépendante un nombre entier valant de 0 à 4,
dans laquelle les restes R^{(VI)} et R^{(VII)} représentent chacun de manière indépendante un hydrogène, alkyle pouvant être à chaîne droite ou ramifiée et contenant 1 à 5 atomes de carbone, phényle ou benzyle, le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci,
dans laquelle les restes R^{(VIII)}, R^{(IX)}, R^{(X)} et R^{(XI)} représentent chacun de manière indépendante un hydrogène, alkyle pouvant être à chaîne droite ou ramifiée et contenant 1 à 5 atomes de carbone, *-O-alkyle pouvant être à chaîne droite ou ramifiée et contenant 1 à 5 atomes de carbone, phényle ou benzyle, le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci, dans laquelle chaque reste Z représente de manière indépendante un halogène, hydroxyle, alcoxyle contenant 1 à 4 atomes de carbone ou alkylcarboxyle contenant 1 à 4 atomes de carbone, de préférence halogène ou hydroxyle, et dans laquelle chaque indice p représente de manière indépendante un nombre entier valant de 0 à 4, et
(b) au moins un composant polymère et/ou un précurseur de celui-ci.

2. Composition photosensibilisante selon la revendication 1,
dans laquelle ledit au moins un reste organique W1 représente de manière indépendante un reste de formule générale (40) à (67) ou (72) à (98e) : 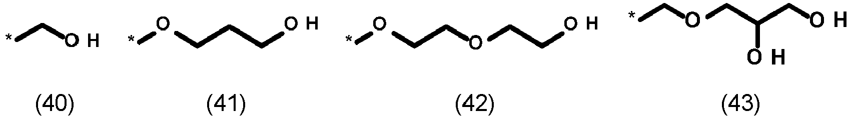 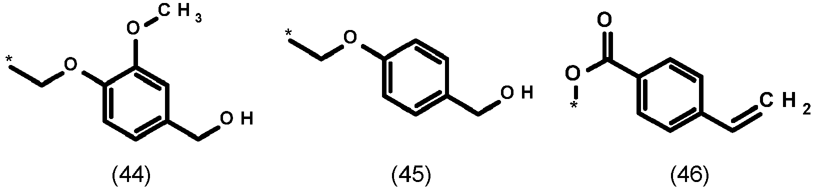 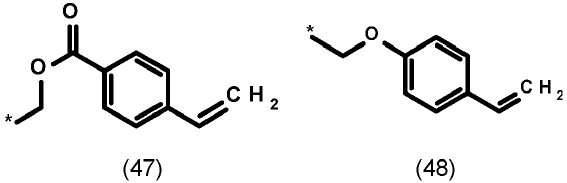 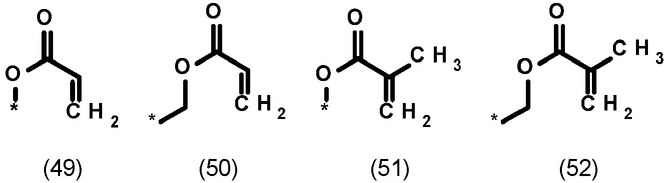 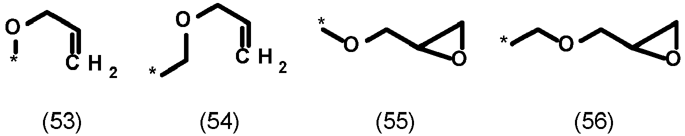 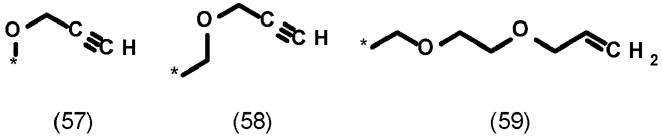 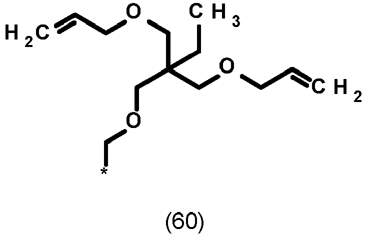 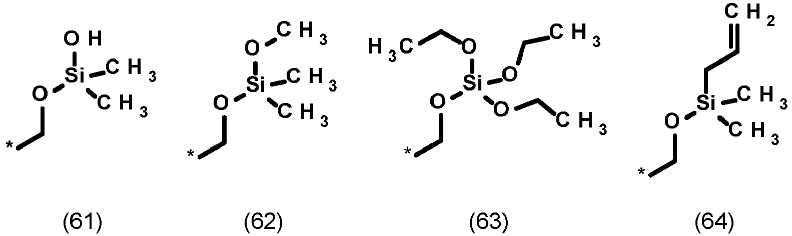 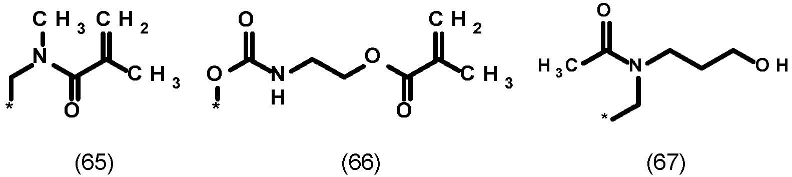 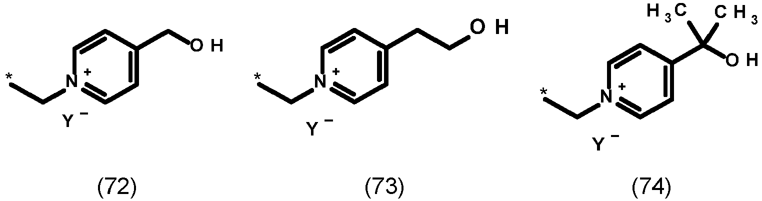 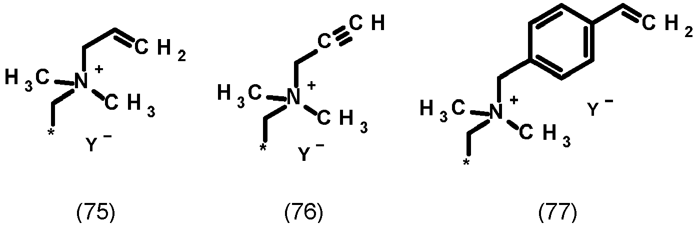 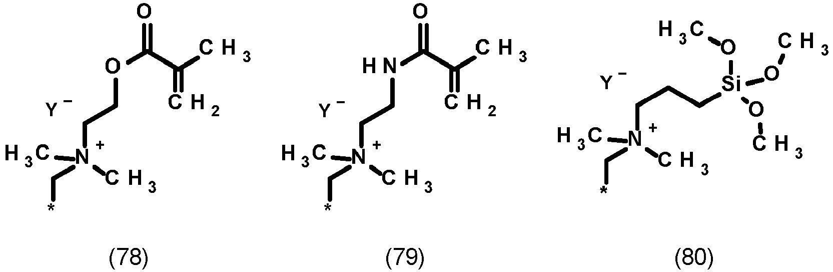 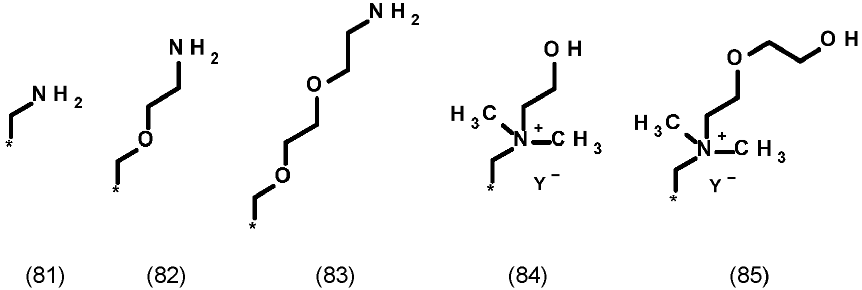 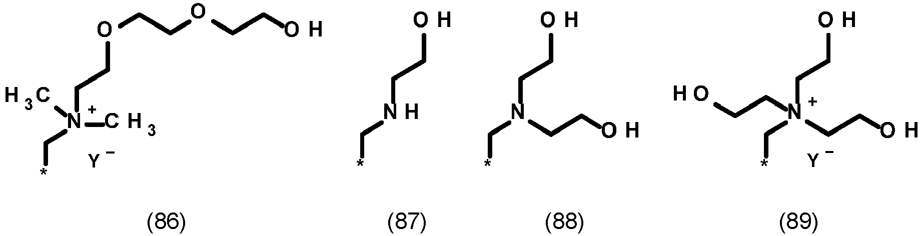 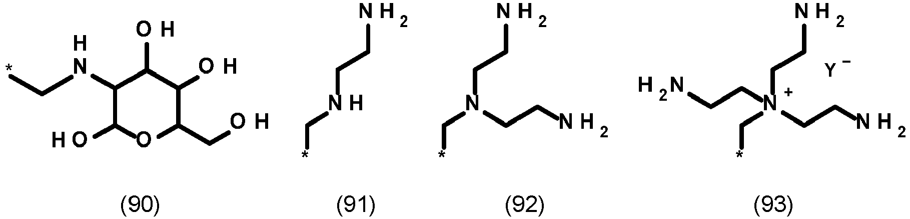 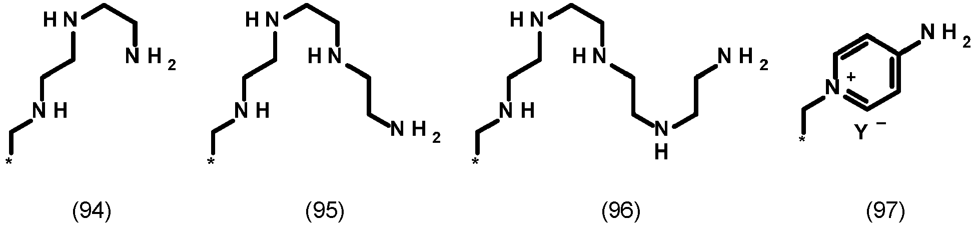 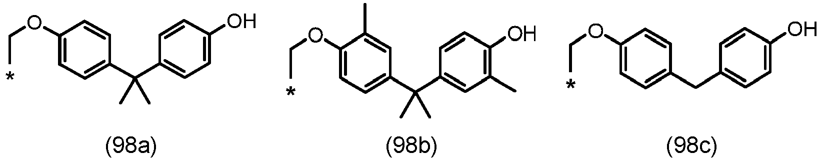 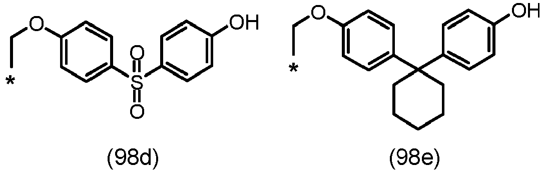 dans laquelle Y⁻ est un anion qui représente respectivement de manière indépendante un fluorure, chlorure, bromure, iodure, sulfate, hydrogénosulfate, phosphate, hydrogénophosphate, dihydrogénophosphate, au moins un anion carboxylate d'un acide carboxylique contenant 1 à 15 atomes de carbone, au moins un anion sulfonate d'un acide sulfonique contenant 1 à 12 atomes de carbone ou une combinaison de ceux-ci.

3. Composition photosensibilisante selon l'une des revendications 1 ou 2, comprenant :
(a) au moins un composé de phénalén-1-one de formule générale (1) choisi dans le groupe constitué par les composés de formule (100) à (127), (132) à (166) : 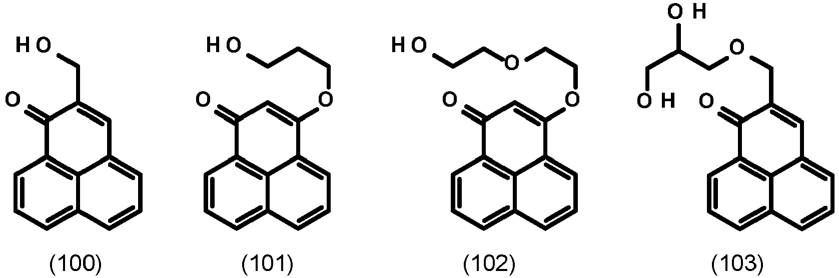 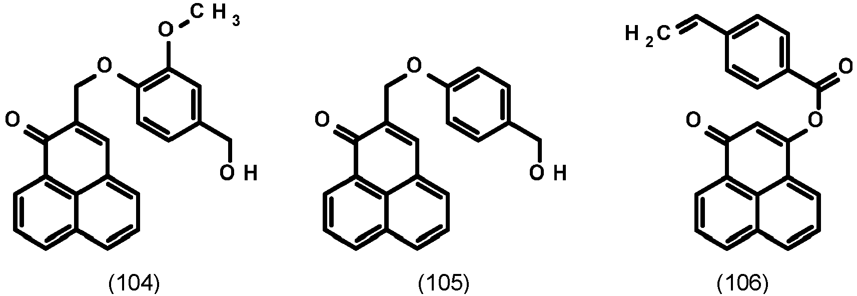 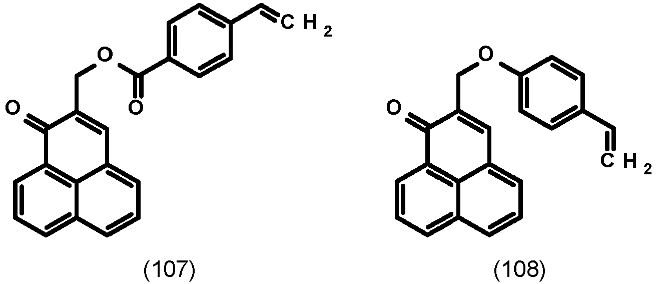 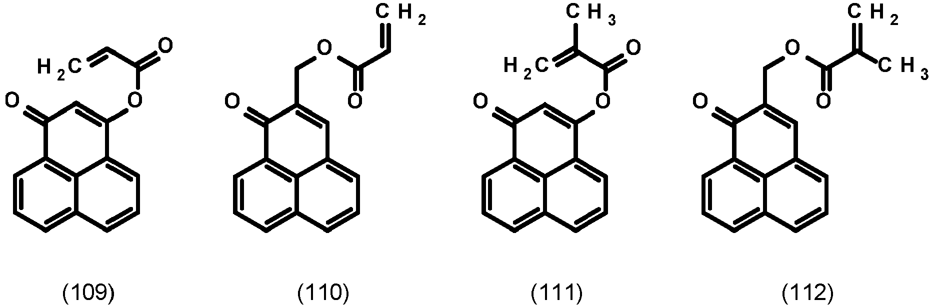 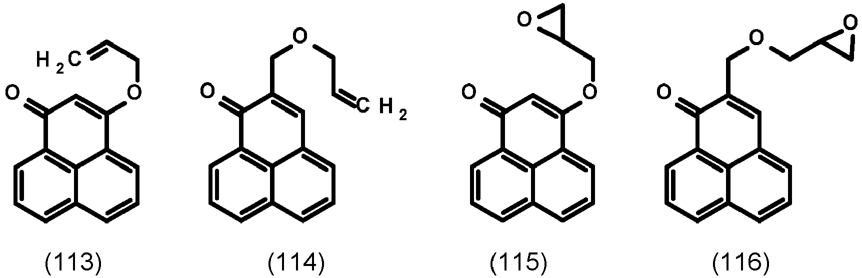 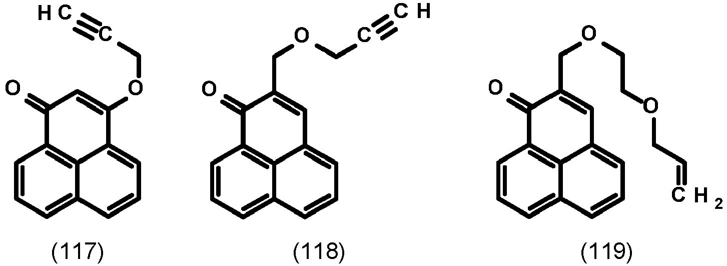 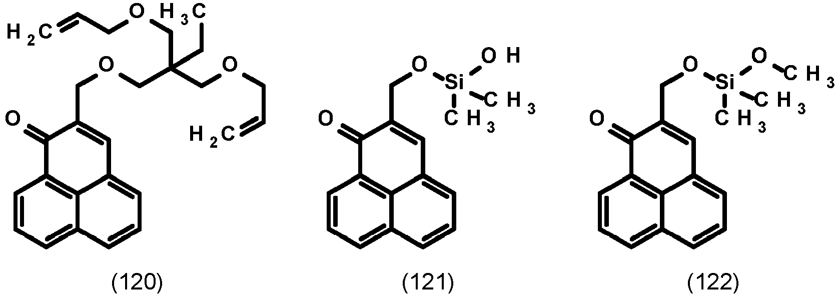 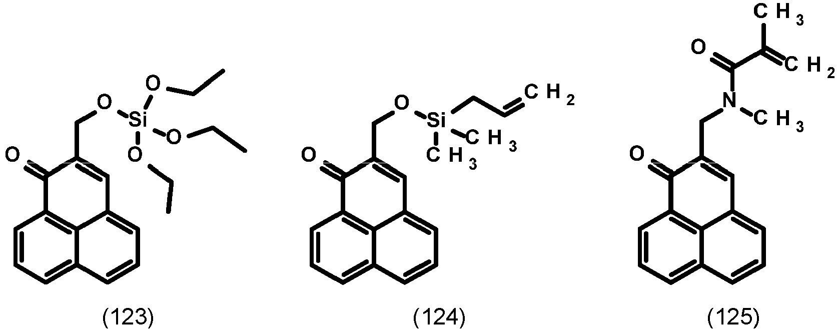 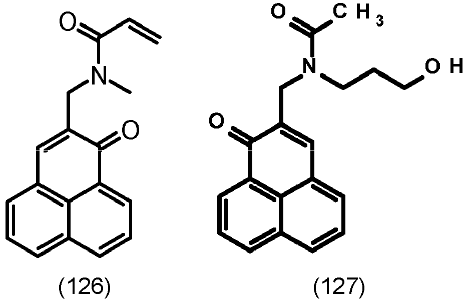 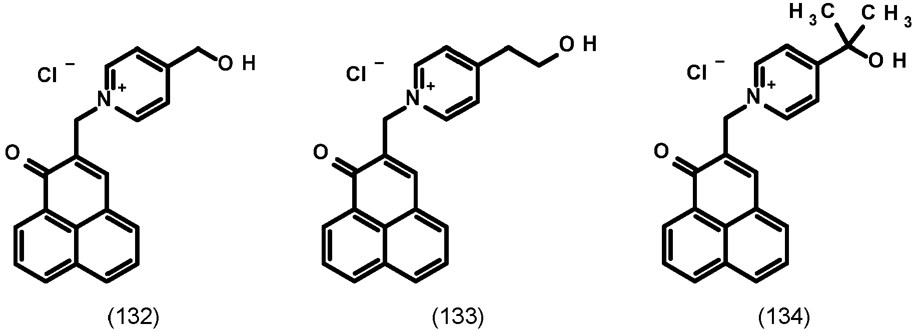 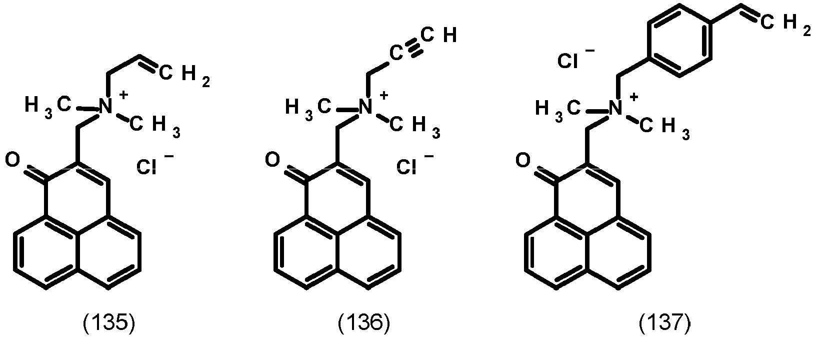 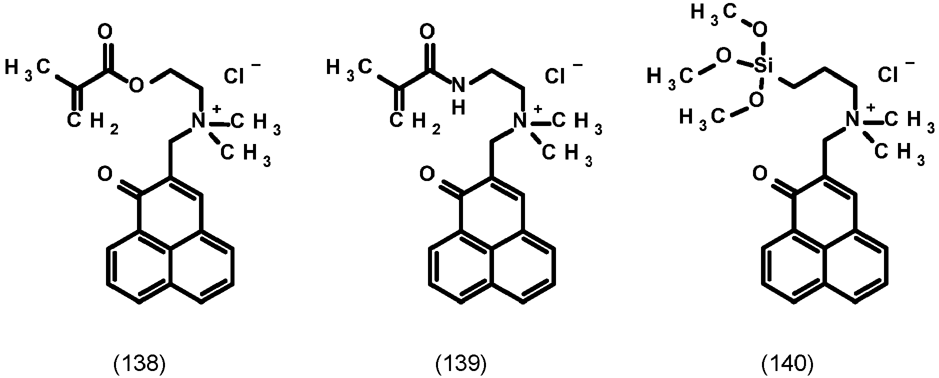 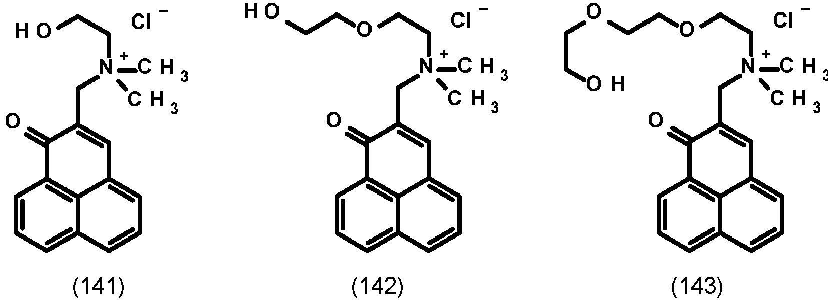 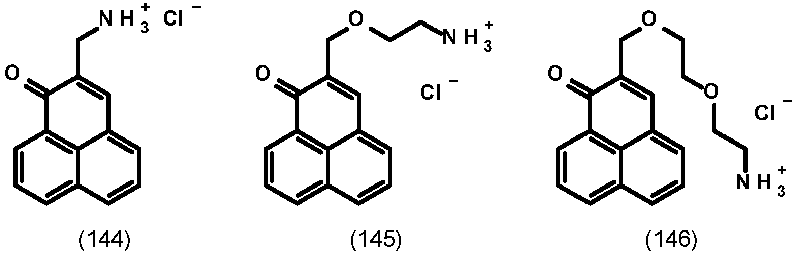 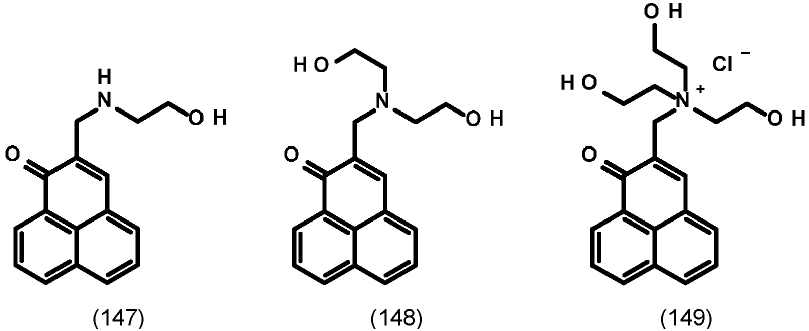 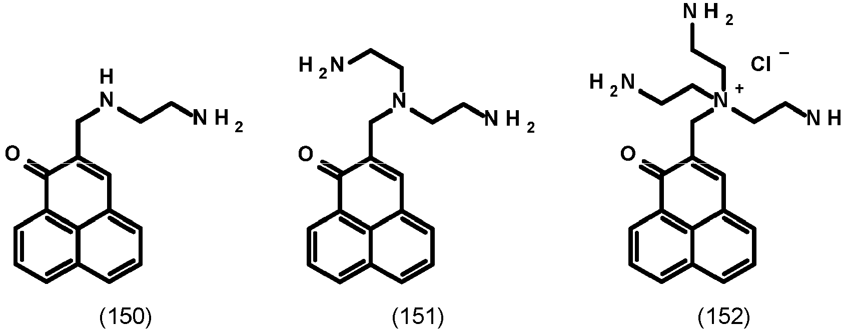 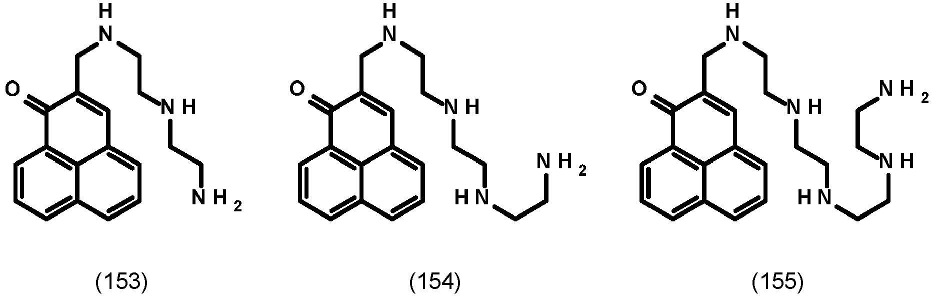 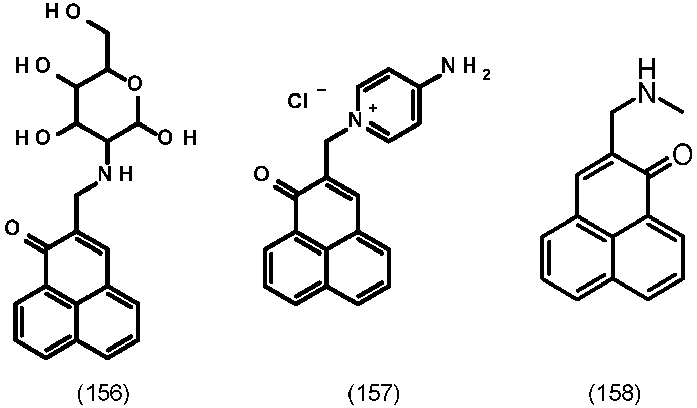 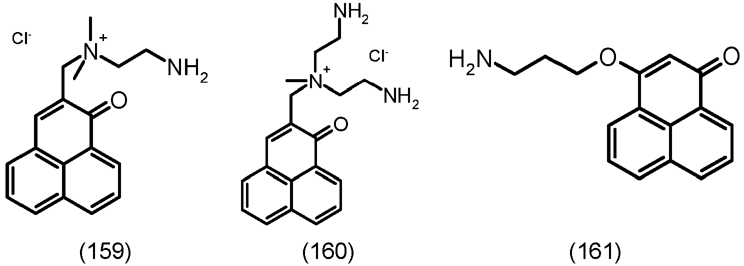 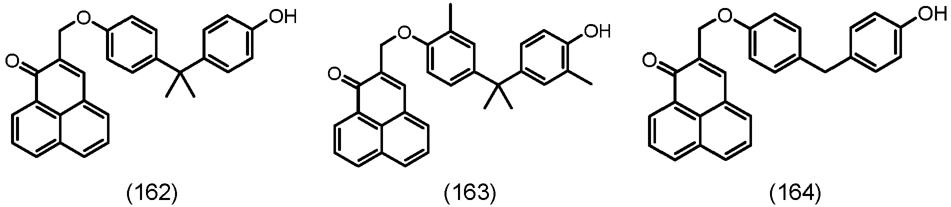 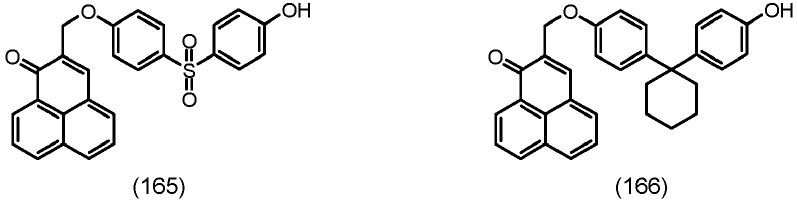 et des combinaisons de ceux-ci, et
(b) au moins un composant polymère et/ou un précurseur de celui-ci.

4. Composé de phénalén-1-one de formule générale (1a) : 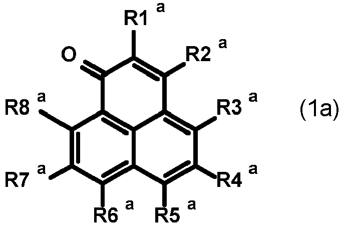 dans laquelle les restes R1 à R8a, qui peuvent être de manière indépendante identiques les uns aux autres ou différents les uns des autres, représentent chacun un hydrogène, halogène, alkyle contenant 1 à 12 atomes de carbone, alkylaryle contenant 5 à 20 atomes de carbone, aryle contenant 5 à 20 atomes de carbone,
*-O-alkyle contenant 1 à 12 atomes de carbone, *-O-alkylaryle contenant 5 à 20 atomes de carbone, *-O-aryle contenant 5 à 20 atomes de carbone, éther contenant 2 à 12 atomes de carbone, un reste de formule *-O-C(=O)-R^{(Ia)}, un reste de formule *-C(=O)-R^{(Ib)}, ou un reste organique W1a contenant au moins un groupe fonctionnel réactif, à la condition que l'un au moins des restes R1^{a} ou R2^{a} soit un reste organique W1a, ledit au moins un reste organique W1a représentant à chaque fois de manière indépendante un reste de formule générale (41) à (67) ou (98a) à (98e) : 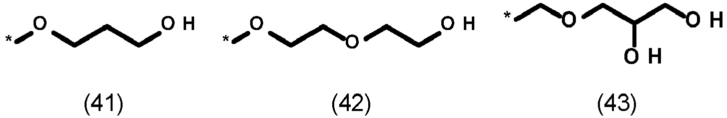 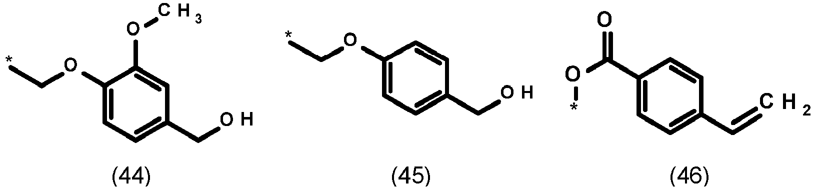 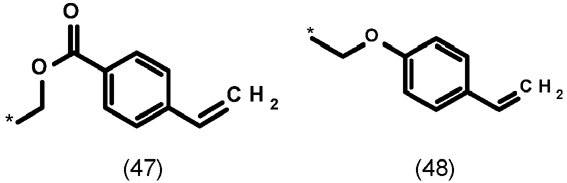 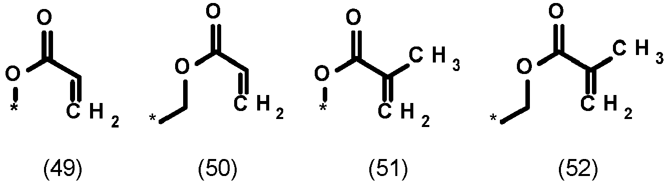 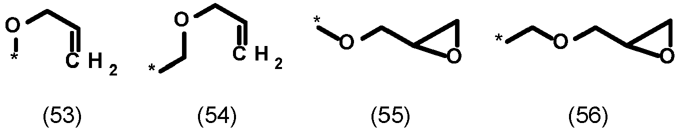 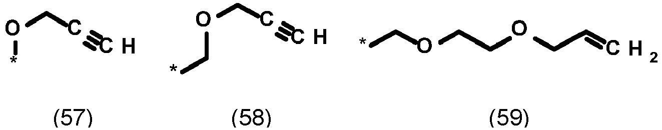 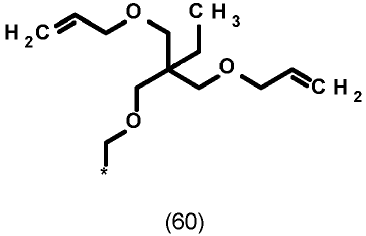 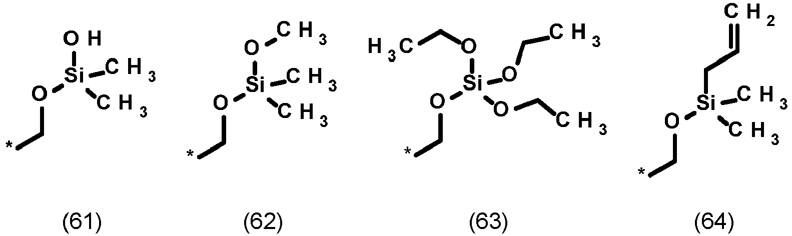 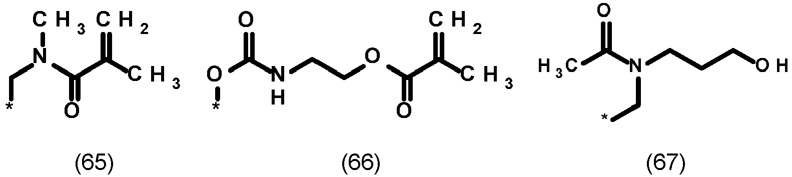 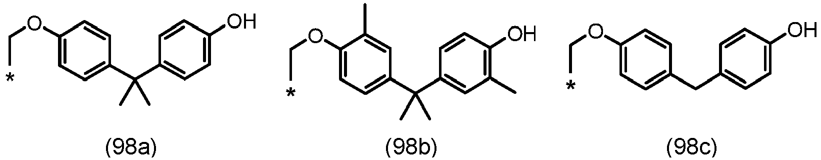 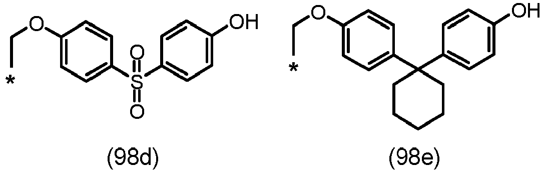 et dans laquelle les restes R^{(Ia)} et R^{(Ib)} représentent chacun de manière indépendante un méthyle, éthyle, n-propyle, isopropyle, n-butyle, sec-butyle, isobutyle, n-pentyle, phényle ou benzyle, et le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci.

5. Composé de phénalén-1-one selon la revendication 4,
dans lequel ledit au moins un composé de phénalén-1-one de formule générale (1a) est choisi dans le groupe constitué par les composés de formule (101) à (127), (162) à (166) et des combinaisons de ceux-ci :

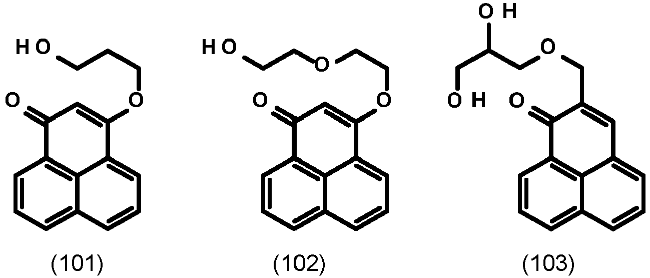

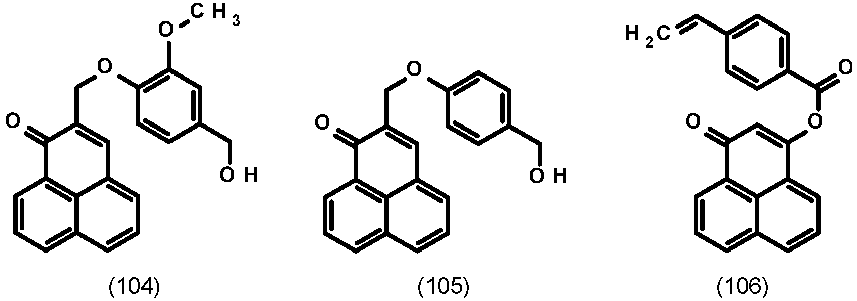

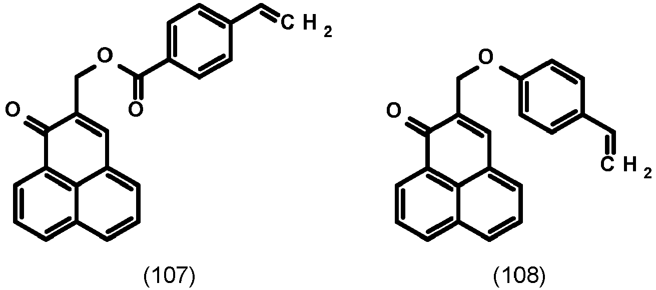

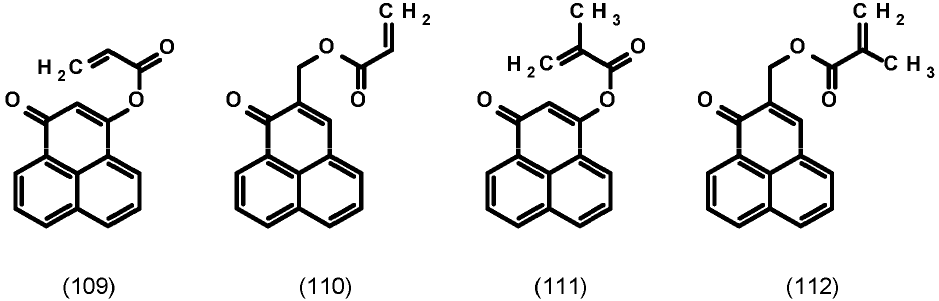

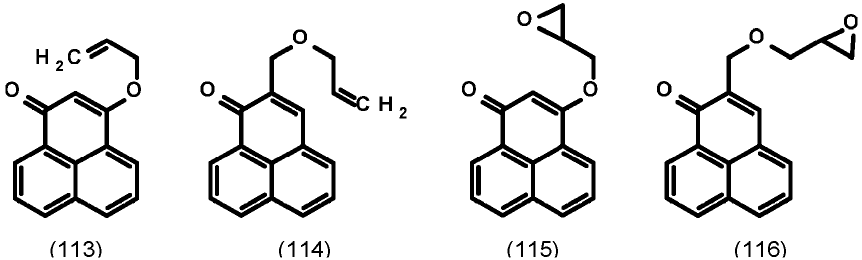

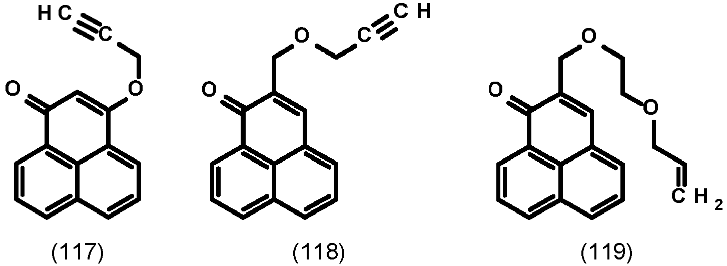

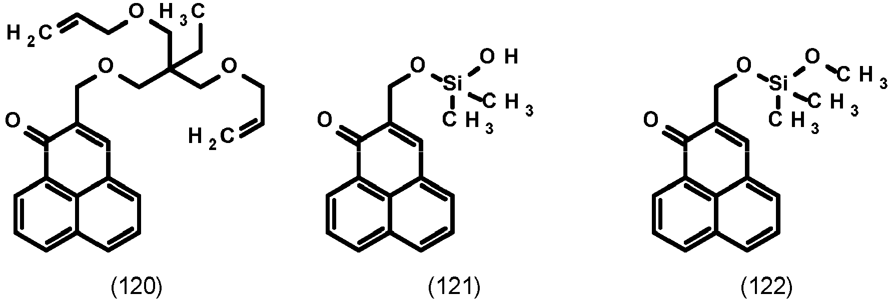

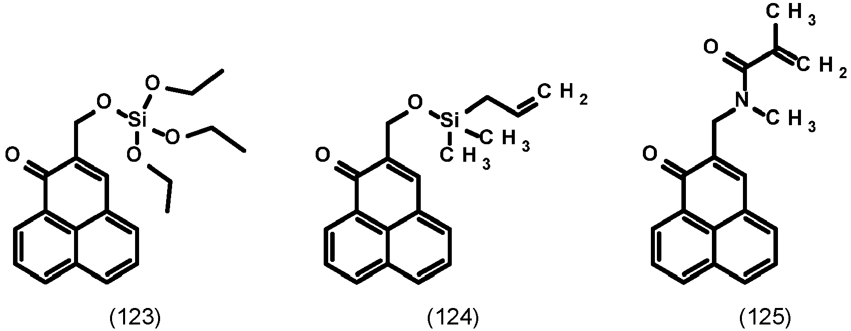

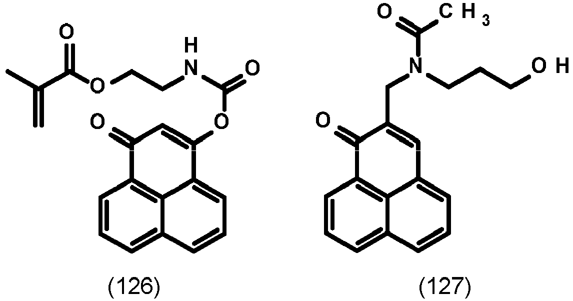

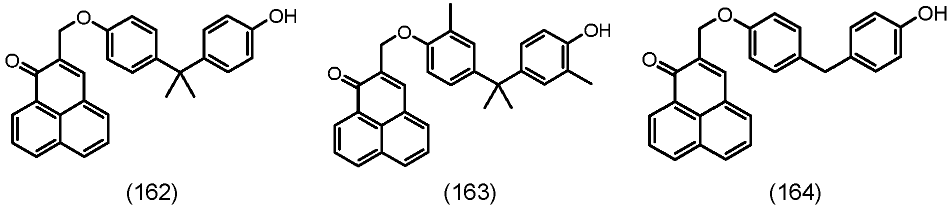

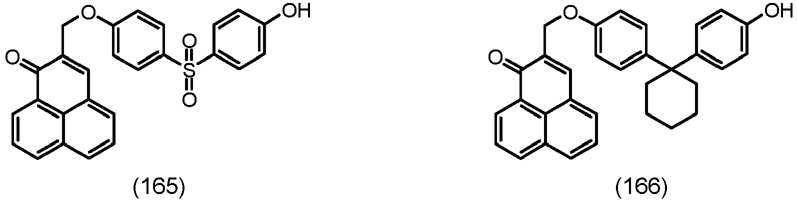

6. Composition photosensibilisante selon l'une des revendications 1 à 3, comprenant :
a) au moins un composé de phénalén-1-one de formule générale (1) qui est un composé de phénalén-1-one selon l'une des revendications 4 à 5, et
b) au moins un composant polymère et/ou un précurseur de celui-ci.

7. Composition photosensibilisante selon l'une des revendications 1 à 3 ou 6, dans laquelle ledit au moins un composant polymère et/ou précurseur de celui-ci est choisi dans le groupe constitué par des polymères et/ou copolymères de l'acide acrylique et ses esters, l'acide méthacrylique et ses esters, l'acide cyanoacrylique et ses esters, l'acrylamide, le méthacrylamide, le styrène, les siloxanes et leurs esters, la mélamine, l'acrylonitrile, le 1,3-butadiène, l'épichlorhydrine, les polyols, les polyisoprènes, les polyéthers, les polyétherimides, les polyacétates de vinyle, les polycarbonates, les polyéthersulfones, la carboxyméthylcellulose, l'alginate et des combinaisons de ceux-ci.

8. Composition photosensibilisante selon l'une des revendications 1 à 3 ou 6 à 7, dans laquelle la composition contient en outre au moins un agent de réticulation contenant un polyisocyanate, un isocyanate bloqué, un diisocyanate d'alkyle ou un diisocyanate de cycloalkyle ou un diisocyanate d'aryle, un composé de formule générale SiZ₄, (R^{XII})SiZ₃, (R^{XII})₂SiZ₂, un composé comprenant au moins deux restes époxyde, un composé comprenant au moins deux restes acrylamide, un composé comprenant au moins deux restes acrylate, un composé comprenant au moins deux restes aldéhyde, un composé comprenant au moins deux groupes alcool, un composé comprenant au moins deux restes amine, une divinylsulfone ou une combinaison de ceux-ci, dans laquelle le reste R^{XII} représente de manière indépendante un alkyle pouvant être à chaîne droite ou ramifiée et contenant 1 à 5 atomes de carbone, un phényle ou un benzyle, de préférence méthyle, éthyle, n-propyle, isopropyle, phényle ou benzyle, et dans laquelle le reste Z représente de manière indépendante un halogène, hydroxyle, alcoxyle contenant 1 à 4 atomes de carbone ou un alkylcarboxyle contenant 1 à 4 atomes de carbone, de préférence un halogène ou un hydroxyle.

9. Composition photosensibilisante selon l'une des revendications 1 à 3 ou 6 à 8, dans laquelle ledit au moins un composé de phénalén-1-one de formule générale (1) est lié de manière covalente et/ou électrostatique, de préférence covalente, audit au moins un composant polymère et/ou précurseur de celui-ci.

10. Composition photosensibilisante selon l'une des revendications 1 à 3 ou 6 à 9, dans laquelle la composition photosensibilisante est une laque, une lasure, une peinture à l'eau, une peinture au latex, une peinture au silicate ou une peinture à la chaux.

11. Composition photosensibilisante selon l'une des revendications 1 à 3 ou 6 à 10, dans laquelle la composition photosensibilisante est un granulé.

12. Objet comprenant au moins une composition polymère durcie, dans lequel la composition polymère durcie comprend
(a) au moins un composé de phénalén-1-one de formule générale (1) : 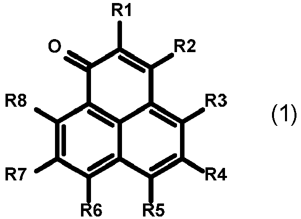
dans laquelle les restes R1 à R8, qui peuvent être chacun de manière indépendante identiques les uns aux autres ou différents les uns des autres, représentent chacun un hydrogène, halogène, alkyle contenant 1 à 12 atomes de carbone, alkylaryle contenant 1 à 18 atomes de carbone, aryle contenant 5 à 20 atomes de carbone, *-O-alkyle contenant 1 à 12 atomes de carbone, *-O-alkylaryle contenant 1 à 18 atomes de carbone, *-O-aryle contenant 5 à 20 atomes de carbone, éther contenant 2 à 12 atomes de carbone, un reste de formule *-O-C(=O)-R(^{Ia}), un reste de formule *-C(=O)-R(^{Ib}), ou un reste organique W1 contenant au moins un groupe fonctionnel réactif, à la condition que l'un au moins des restes R1 à R7 soit un reste organique W1, le reste organique W1 représentant de manière indépendante un reste de formule générale (2) à (6) :
*-[(C(D)(E))_{d}-B]ₐ-(C(D)(E))ₘ-X (2)
*-A-[(C(D)(E))_{d}-B]_{c}-(C(D)(E))ₘ-X (3)
* - (C(D)(E))d - Ar - (C(D)(E))n - X (4)
*-[(C(D)(E))_{d}-B]_{b}-(C(D)(E))_{g}-Ar-(C(D)(E))ₙ-X (5)
*-A-[(C(D)(E))_{d}-B]_{f}-(C(D)(E))_{g}-Ar-(C(D)(E))ₙ-X (6)
dans laquelle chaque reste A représente de manière indépendante l'oxygène, le soufre ou un reste de formule générale (10a) à (11a) : 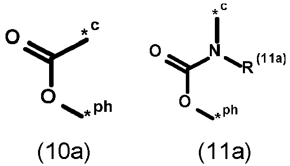
dans laquelle *^{ph} désigne respectivement un point de rattachement du reste de formule générale (10a) à (11a) à un atome de carbone du noyau phénalène et chaque *^{c} désigne un point de rattachement du reste de formule générale (10a) à (11a) à un atome de carbone du reste (C(D)(E)), et
dans laquelle chaque reste B représente de manière indépendante l'oxygène, le soufre ou un reste de formule générale (10) à (14) : 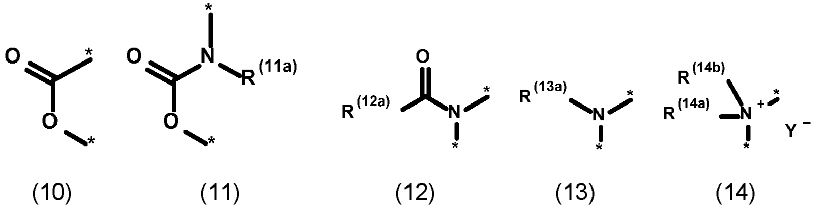
dans laquelle les restes R^{(Ia>}, R^{(Ib)}, R^{(11a)}, R^{(12a)}, R^{(13a)}, R^{(14a)} et R^{(14b)} représentent chacun de manière indépendante un hydrogène, méthyle, éthyle, n-propyle, isopropyle, n-butyle, sec-butyle, isobutyle, n-pentyle, phényle ou benzyle, et dans lequel le phényle et le benzyle peuvent être chacun de manière indépendante non substitués ou substitués par un ou plusieurs restes choisis dans le groupe constitué par halogène, amino, hydroxyle, *-O-alkyle pouvant être à chaîne droite ou ramifiée et contenant 1 à 3 atomes de carbone, alkyle pouvant être à chaîne droite ou ramifiée et contenant 1 à 3 atomes de carbone, hydroxyalkyle pouvant être à chaîne droite ou ramifiée et contenant 1 à 3 atomes de carbone et 1 à 3 groupes OH, haloalkyle pouvant être à chaîne droite ou ramifiée et contenant 1 à 3 atomes de carbone et 1 à 3 groupes halogène, et des combinaisons de ceux-ci, de préférence chlore, brome, fluor, méthyle, éthyle, n-propyle, isopropyle, méthoxy, éthoxy et des combinaisons de ceux-ci, dans laquelle Y⁻ est un anion représentant à chaque fois de manière indépendante un fluorure, chlorure, bromure, iodure, sulfate, hydrogénosulfate, phosphate, hydrogénophosphate, dihydrogénophosphate, au moins un anion carboxylate d'un acide carboxylique contenant 1 à 15 atomes de carbone, au moins un anion sulfonate d'un acide sulfonique contenant 1 à 12 atomes de carbone ou une combinaison de ceux-ci, et
dans laquelle les restes D et E représentent chacun de manière indépendante un hydrogène, halogène, hydroxyle, alkyle pouvant être à chaîne droite ou ramifiée et contenant 1 à 5 atomes de carbone, hydroxyalkyle pouvant être à chaîne droite ou ramifiée et contenant 1 à 5 atomes de carbone et 1 à 5 groupes OH, phényle, benzyle, un reste de formule *-L-R(^{II}), un reste de formule *-L-C(=L)-R^{(III)}, un reste de formule *-(CH₂)_{q}-X, un reste de formule *-L-(CH₂)_{q}-X, ou un reste de formule
*-(CH₂)ₛ-L-(CH₂)ₜ-X, chaque reste L représentant de manière indépendante l'oxygène ou le soufre, de préférence l'oxygène, les restes R^{(II)} et R^{(III)} représentant chacun de manière indépendante un hydrogène, méthyle, éthyle, n-propyle, isopropyle, n-butyle, sec-butyle, isobutyle, n-pentyle, phényle ou benzyle, et le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci, les indices q, s et t représentant chacun de manière indépendante un nombre entier valant de 1 à 5,
dans laquelle les indices a, c, f, g et n représentent chacun de manière indépendante un nombre entier valant de 0 à 5,
et dans laquelle les indices b, d et m représentent chacun de manière indépendante un nombre entier valant de 1 à 5,
dans laquelle chaque reste Ar représente de manière indépendante un reste de formule générale (25a) à (31) : 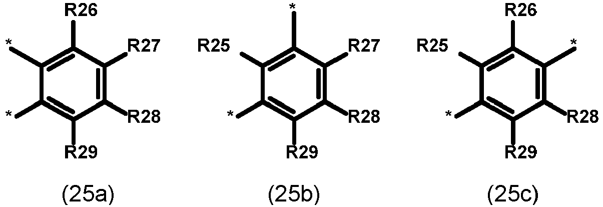 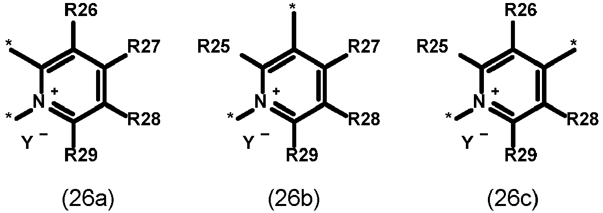 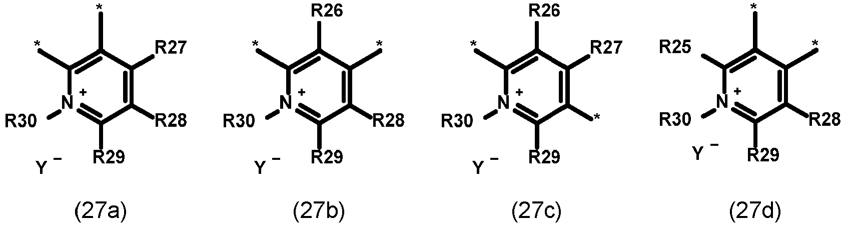 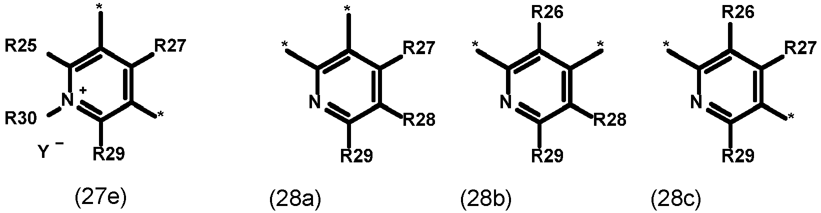 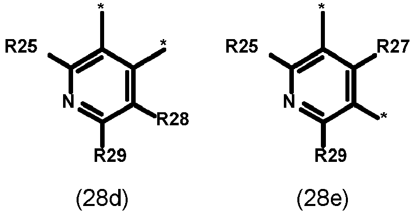 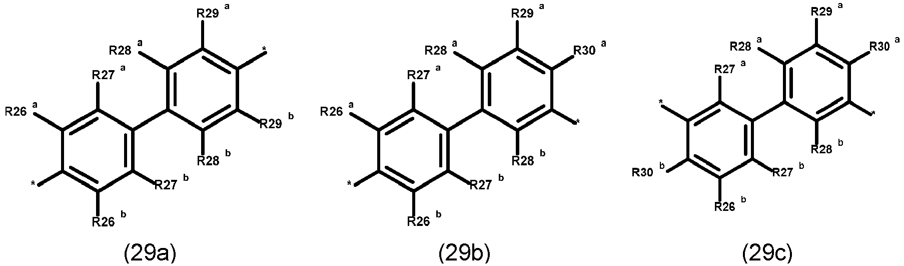 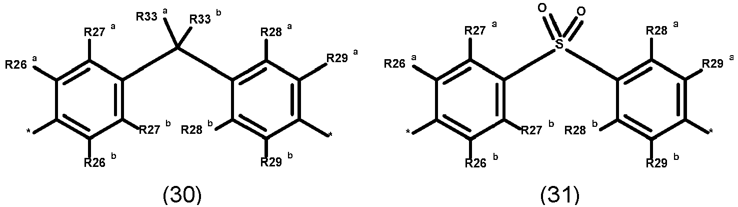
dans laquelle les restes R25, R26, R27, R28, R29, R26^{a}, R26^{b}, R27^{a}, R27^{b}, R28^{a}, R28^{b}, R29^{a}, R29^{b}, R30^{a} et R30^{b} représentent chacun de manière indépendante un hydrogène, hydroxy, amino, alkyle pouvant être à chaîne droite ou ramifiée et contenant 1 à 5 atomes de carbone, *-O-alkyle pouvant être à chaîne droite ou ramifiée et contenant 1 à 5 atomes de carbone, phényle ou benzyle, le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci, et chaque reste R30 représentant de manière indépendante un hydrogène ou un alkyle pouvant être à chaîne droite ou ramifiée et contenant 1 à 5 atomes de carbone, et les restes R33a et R33b représentant chacun de manière indépendante un hydrogène, hydroxy, amino, alkyle pouvant être à chaîne droite ou ramifiée et contenant 1 à 5 atomes de carbone, perfluoroalkyle pouvant être à chaîne droite ou ramifiée et contenant 1 à 5 atomes de carbone,
*-O-alkyle pouvant être à chaîne droite ou ramifiée et contenant 1 à 5 atomes de carbone, phényle, benzyle ou, lorsqu'ils sont pris ensemble, un cycloalkyle pouvant être à chaîne droite ou ramifiée et contenant 4 à 9 atomes de carbone, ou un reste 9H-fluorén-9-ylidène, et
dans laquelle Y⁻ est un anion représentant à chaque fois de manière indépendante un fluorure, chlorure, bromure, iodure, sulfate, hydrogénosulfate, phosphate, hydrogénophosphate, dihydrogénophosphate, au moins un anion carboxylate d'un acide carboxylique contenant 1 à 15 atomes de carbone, au moins un anion sulfonate d'un acide sulfonique contenant 1 à 12 atomes de carbone, ou une combinaison de ceux-ci, et
dans laquelle chaque reste X est de manière indépendante un groupe fonctionnel réactif représentant *-N(R^{(VI)}(R^{(VII)}*-OH, *-SH, *-NCO, *-NCS, *-Si(R^{(VIII)})(R^{(IX)})-[O-Si(R^{(X)})(R^{(XI)})]ₚ-Z ou un reste de formule générale (20) à (24) : 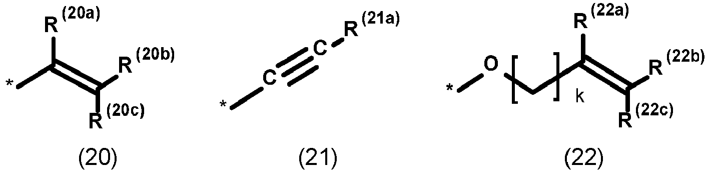 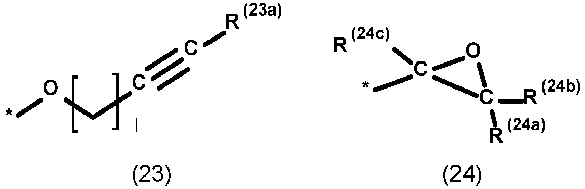
dans laquelle les restes R^{(20a)}, R^{(20b)}, R^{(20c)}, R^{(21a)}, R^{(22a)}, R^{(22b)}, R^{(22c)}, R^{(23a)}, R^{(24a)}, R^{(24b)} et R^{(24c)} représentent chacun de manière indépendante un hydrogène, méthyle, éthyle, n-propyle, isopropyle, n-butyle, sec-butyle, isobutyle ou n-pentyle et dans laquelle les indices I et k représentent chacun de manière indépendante un nombre entier valant de 0 à 4,
dans laquelle les restes R^{(VI)} et R^{(VII)} représentent chacun de manière indépendante un hydrogène, alkyle pouvant être à chaîne droite ou ramifiée et contenant 1 à 5 atomes de carbone, phényle ou benzyle, le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci,
dans laquelle les restes R^{(VIII)}, R^{(IX)}, R^{(X)} et R^{(XI)} représentent chacun de manière indépendante un hydrogène, alkyle pouvant être à chaîne droite ou ramifiée et contenant 1 à 5 atomes de carbone, *-O-alkyle pouvant être à chaîne droite ou ramifiée et contenant 1 à 5 atomes de carbone, phényle ou benzyle, le phényle et le benzyle pouvant être chacun de manière indépendante non substitués ou substitués par un ou plusieurs restes choisis dans le groupe constitué par le chlore, brome, fluor, méthyle, éthyle, n-propyle, isopropyle, méthoxy, éthoxy et des combinaisons de ceux-ci, chaque reste Z représentant de manière indépendante un halogène, hydroxyle, alcoxyle contenant 1 à 4 atomes de carbone ou alkylcarboxyle contenant 1 à 4 atomes de carbone, de préférence un halogène ou un hydroxyle, et chaque indice p représentant de manière indépendante un nombre entier valant de 0 à 4, et
(b) au moins un composant polymère durci,
dans lequel ledit au moins un composé de phénalén-1-one de formule générale (1) est lié de manière covalente et/ou électrostatique, de préférence covalente, audit au moins un composant polymère durci.

13. Objet selon la revendication 12, dans lequel la composition polymère durcie comprend
(a) au moins un composé de phénalén-1-one de formule (1), au moins un composé de phénalén-1-one selon l'une des revendications 4 à 5 ou une combinaison de ceux-ci, et
(b) au moins un composant polymère durci,
dans lequel ledit au moins un composé de phénalén-1-one de formule (1), ledit au moins un composé de phénalén-1-one selon l'une des revendications 5 à 6 ou une combinaison de ceux-ci est lié de manière covalente et/ou électrostatique, de préférence covalente, audit au moins un composant polymère durci.

14. Objet selon l'une des revendications 12 ou 13, dans lequel la composition polymère durcie se présente sous la forme d'un revêtement, d'un film autoporteur, d'une toile ou d'un objet moulé.

15. Utilisation non médicale d'une composition photosensibilisante selon l'une des revendications 1 à 3 ou 6 à 11 et/ou d'un composé de phénalén-1-one selon l'une des revendications 4 à 5 et/ou d'un objet selon l'une des revendications 12 à 14 pour inactiver, de préférence de manière photodynamique, des micro-organismes choisis dans le groupe constitué par des virus, des archées, des bactéries, des spores bactériennes, des biofilms de bactéries, des champignons, des spores fongiques, des protozoaires, des algues et des parasites transmissibles par voie sanguine, et/ou un biofilm de ceux-ci.

16. Utilisation non médicale d'une composition photosensibilisante selon l'une des revendications 1 à 3 ou 6 à 11 et/ou d'un composé de phénalén-1-one selon l'une des revendications 4 à 5 pour nettoyer et/ou revêtir de manière photodynamique la surface d'un objet ou d'un espace.

17. Utilisation non médicale selon l'une des revendications 15 ou 16 pour revêtir la surface d'objets, de préférence des produits médicaux, des emballages alimentaires, des films d'emballage, des textiles, des matériaux de construction, des appareils électroniques, des meubles ou des articles d'hygiène.

18. Procédé non médical pour inactiver, de préférence de manière photodynamique, des micro-organismes choisis dans le groupe constitué par des virus, des archées, des bactéries, des spores bactériennes, des biofilms de bactéries, des champignons, des spores fongiques, des protozoaires, des algues et des parasites transmissibles par voie sanguine, et/ou un biofilm de ceux-ci, ledit procédé comprenant les étapes suivantes :
(A) mise en contact des micro-organismes et/ou d'un biofilm de ceux-ci avec au moins un revêtement obtenu par durcissement d'une composition photosensibilisante selon l'une des revendications 1 à 3 ou 6 à 11 et/ou contenant au moins un composé de phénalén-1-one selon l'une des revendications 4 à 5, et/ou au moins un objet selon l'une des revendications 12 à 14, et
(B) exposition des micro-organismes et/ou d'un biofilm de ceux-ci et dudit au moins un composé de phénalén-1-one contenu dans le revêtement et/ou l'objet à un rayonnement électromagnétique ayant une longueur d'onde et une densité d'énergie appropriées.
